(19) Europäisches Patentamt
European Patent Office
Office européen des brevets

(11) **EP 3 698 832 B1**

(12) **EUROPEAN PATENT SPECIFICATION**

(45) Date of publication and mention
of the grant of the patent:
**13.07.2022 Bulletin 2022/28**

(21) Application number: **20168954.4**

(22) Date of filing: **22.01.2015**

(51) International Patent Classification (IPC):
**A61M 15/06** (2006.01)     **A24F 40/48** (2020.01)
**A61M 11/00** (2006.01)     **A24F 40/46** (2020.01)
**A24F 40/10** (2020.01)

(52) Cooperative Patent Classification (CPC):
**A24F 40/46; A24F 40/48; A24F 40/50;**
**A61M 11/001; A61M 11/042; A61M 15/002;**
**A61M 15/06; H05B 1/0244;** A24F 40/10;
A61M 11/002; A61M 15/0015; A61M 15/0025;
A61M 15/0036; A61M 15/0066; A61M 15/008;

(Cont.)

(54) **METHODS AND DEVICES FOR SMOKING URGE RELIEF**

VERFAHREN UND VORRICHTUNGEN ZUR ENTLASTUNG DES ZWANGS ZU RAUCHEN

PROCÉDÉS ET DISPOSITIFS POUR SOULAGER LE BESOIN DE FUMER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.01.2014 US 201461930391 P**
**07.02.2014 US 201461937313 P**
**07.03.2014 US 201461949771 P**
**10.03.2014 US 201461950775 P**
**27.03.2014 US 201461971456 P**
**09.04.2014 US 201461977591 P**

(43) Date of publication of application:
**26.08.2020 Bulletin 2020/35**

(62) Document number(s) of the earlier application(s) in accordance with Art. 76 EPC:
**15740106.8 / 3 096 636**

(73) Proprietor: **Fontem Holdings 1 B.V.**
**1083 HN Amsterdam (NL)**

(72) Inventors:
• **Wensley, Martin**
**Campbell, CA 95008 (US)**
• **Hufford, Michael**
**Chapel Hill, NC 27516 (US)**
• **Williams, Jeffrey**
**Draper, UT 84020 (US)**
• **Lloyd, Peter**
**Walnut Creek, CA 94597 (US)**

(74) Representative: **Gulde & Partner**
**Patent- und Rechtsanwaltskanzlei mbB**
**Wallstraße 58/59**
**10179 Berlin (DE)**

(56) References cited:
WO-A1-2004/022243     US-A- 5 894 841
US-A1- 2003 108 342     US-A1- 2013 213 418
US-A1- 2013 220 314

(52) Cooperative Patent Classification (CPC): (Cont.)
A61M 15/0083; A61M 2016/0021; A61M 2016/0024;
A61M 2016/0033; A61M 2205/0211;
A61M 2205/0238; A61M 2205/3306;
A61M 2205/3334; A61M 2205/3553;
A61M 2205/3584; A61M 2205/3592;
A61M 2205/3653; A61M 2205/502; A61M 2205/52;
A61M 2205/581; A61M 2205/583; A61M 2205/8206;
A61M 2206/10; A61M 2206/18; A61M 2209/02;
G16H 20/13; Y02A 90/10

**Description**

**BACKGROUND**

There is a need for new methods and devices for administering compounds, such as pharmaceutical agents, to a subject. In particular, there is a need for methods and devices for delivery of compounds to a subject where the compounds are aerosolized to fall within a specified particle size range. In some cases, particles within a specified size range can be efficiently delivered to the deep lung. For example, there is an urgent need for improved methods and devices to deliver nicotine to a subject in specified doses and in a specified particle range size without the carcinogens and other chemicals associated with combustible tobacco products.

In 2011, an estimated 19% of U.S. adults were current smokers (43.8 million people), and an estimated 950 children become addicted to smoking daily. Smokers spend approximately $83 billion to support their habit, and half of smokers will die from their habit. Studies indicate that about 85% of smokers want to quit; however, only about 5% succeed.

Current nicotine replacement therapies (NRTs) are not effective for approximately 85% of users. In some cases, existing NRTs and electronic cigarettes (eCigs) fail to provide sufficient doses of nicotine. Many smokers using NRTs under-dose, resulting in breakthrough cravings, which can lead to smoking lapses and eventual relapse. Smokers also vary widely in terms of their daily nicotine intake, ranging from "social smokers" who may only consume 1 or 2 cigarettes in the presence of friends and/or with alcohol, to heavy smokers who consume 60 or more cigarettes per day. Thus, a need exists to provide effective, customized doses of nicotine to individuals attempting to use recreational nicotine products or to leverage these devices to help quit smoking or nicotine intake all together.

Furthermore, to facilitate nicotine delivery using an electronic nicotine delivery device, a need exists to control nicotine particle size generated from an electronic nicotine delivery device to match the rapid nicotine pharmacokinetics (PK) from smoking, which can result in deep lung absorption of nicotine. Deep lung absorption of nicotine can facilitate rapid delivery of nicotine to the brain, which can result in a subsequent cessation of nicotine cravings. When smoking combustible tobacco products, nicotine laden smoke particles are carried proximally on tar droplets (0.1-1.0 $\mu$m in diameter), are inhaled and travel to the small airways and alveoli in the deep lung. Nicotine off-gasses from particles and defuses to, and deposits on, the alveoli wall where it can be rapidly absorbed into the blood stream. A typical electronic cigarette does not produce an aerosol of nicotine with a particle size for deep lung delivery. Aerosol particles with an aerodynamic diameter larger than 5 $\mu$m can be too large to reach the deep lung because the particles can impact in the mouth and upper airway, resulting in a slow PK. Conversely, aerosol particles with a median aerodynamic diameter of less than 1 $\mu$m can be small enough to reach the deep lung but can be too light to gravitationally settle and can be exhaled, which can result in low dose delivery. Additionally, aerosols with small aerosol particle size can contain a larger percentage of the mass in the gas phase, which rapidly diffuses to the mouth and upper airway. Aerosol particles with an aerodynamic diameter of about 1 $\mu$m to about 5 $\mu$m can be small enough to reach the deep lung but large enough to gravitationally settle in alveoli, which can result in a rapid PK. A need exists for electronic nicotine delivery devices that produce such particles. In addition, a need exists for producing nicotine aerosols that produce such particles using the liquid drug. Moreover, a need exists for methods of using such devices to help users achieve a particular health goal or goals.

There is also a need for a drug delivery platform that is capable of dispensing a variety of drugs to a subject in a specified dose or in a specified particle size range.

There is also a need for a drug delivery platform that is capable of dispensing a variety of drugs to a subject in a specified dose or in a specified particle size range.

US 5894841 A discloses dispenser comprising a reservoir of a physiologically active substance and a droplet ejection device, for example a bubble jet or pizeoelectric device, which is controlled to issue a predetermined number of discrete droplets of the substance from ejection orifices upon actuation. Device may be actuated by a pressure transducer responsive to inhalation and issue the droplets into an airstream which enters at slot and is then inhaled via mouthpiece. In other embodiments, the dispenser is finger actuated and directed by hand for topical application. The number and/or frequency of droplets issued is programmatically controlled by a control circuit whereby average and total dose of the substance are predetermined.

**SUMMARY**

As one example, provided herein is a method for treating an urge of a subject to smoke, the method comprising administering to a subject a condensation aerosol comprising nicotine, wherein the administering comprises: a. producing the condensation aerosol comprising nicotine in an aerosol generating device configured to vaporize a liquid formulation comprising nicotine and condense the vaporized liquid formulation comprising nicotine into the condensation aerosol comprising nicotine, wherein the condensation aerosol comprises a diameter of from about 1 $\mu$m to about 5 $\mu$m; and b. delivering the condensation aerosol comprising nicotine to a subject using the device, wherein the delivering comprises the subject inhaling the condensation aerosol comprising nicotine from the device thereby reducing the urge of the subject to smoke. In some cases, the reduction in the urge to smoke occurs in less than about 1 minute after administeringthe condensation aerosol comprising nicotine. In some cases, the reduction in the urge to smoke is sustained for at least 30 minutes following administering the condensation aerosol comprising nicotine. In some cases, the reduction in the urge to smoke in the subject is at least 50%. In some cases, the reduction in the urge to smoke in the subject is at least 60%. In some cases, the reduction in the urge to smoke in the subject is at least 70%. In some cases, the reduction in the urge to smoke in the subject is at least 80%. In some cases, the reduction in the urge to smoke in the subject is a complete or substantially complete elimination of the urge to smoke in the subject. In some cases, the reduction in the urge to smoke is compared to an urge to smoke in the subject before using the aerosol generating device. In some cases, the reduction in the urge to smoke is compared to an urge to smoke in the subject following administration of a vehicle using the aerosol generating device. In some cases, the reduction in the urge to smoke is assessed using a psychometric response scale. In some cases, the psychometric response scale comprises a smoking urge visual analog scale (SU-VAS). In some cases, the reduction in the urge to smoke is sustained for at least 60 minutes. In some cases, the diameter of the condensation aerosol comprises a mass median aerodynamic diameter (MMAD). In some cases, the diameter of the condensation aerosol comprises a volume median diameter (VMD). In some cases, the condensation aerosol comprises a geometric standard deviation of less than 2. In some cases, the condensation aerosol generating device is configured to deliver the condensation aerosol comprising nicotine to a deep lung of the subject. In some cases, the subject exhales no or substantially no visible vapor following inhalation of the condensation aerosol produced by the device. In some cases, the administering comprises the subject inhaling the condensation aerosol a plurality of times per use of the device, wherein the inhaling a plurality of times administers a pre-determined dose of nicotine to the subject per use of the device. In some cases, the pre-determined dose of nicotine is from about 500 $\mu$g to about 1000 $\mu$g. In some cases, the plurality of times comprises from about 2 to about 10 inhalations from the device. In some cases, the predetermined dose of nicotine produces a nicotine blood concentration that is at least 50% less than the nicotine plasma concentration produced by a cigarette or an electronic cigarette. In some cases, the predetermined dose of nicotine produces a nicotine plasma concentration of from about 0.5 ng/ml to about 1 ng/ml. In some cases, the nicotine plasma concentration is produced in about 30 seconds following the administration of the predetermined dose of nicotine. In some cases, the nicotine plasma concentration is sustained for at least 10 minutes following the administration of the pre-determined dose of nicotine. In some cases, the pre-determined dose of nicotine administered to the subject per use of the device is substantially identical between uses of the device. In some cases, the subject administers the condensation aerosol comprising nicotine according to a prescribed treatment regimen. In some cases, the subject administers the condensation aerosol comprising nicotine on demand. In some cases, the subject administers the condensation aerosol comprising nicotine multiple times per day. In some cases, the aerosol generating device comprises: a. a reservoir comprising the liquid formulation comprising nicotine; b. an air flow channel comprising an inlet and an outlet; and c. a heater element within the airflow channel, wherein the heater element is in fluid communication with the liquid formulation comprising nicotine; and wherein producing the condensation aerosol comprising nicotine with a diameter of from about 1 $\mu$m to about 5 $\mu$m comprises vaporizing the liquid formulation comprising nicotine upon delivery of the liquid formulation comprising nicotine to the heater element and subsequent activation of the heater element. In some cases, the device is hand-held. In some cases, the device is disk-shaped. In some cases, the reservoir comprises a pre-determined number of doses of the liquid formulation comprising nicotine. In some cases, the predetermined number of doses comprises an amount of nicotine sufficient to provide about 1 day of use on demand by a subject. In some cases, the pre-determined number of doses comprises an amount of nicotine sufficient to provide about 1 to about 7 days of use on demand by a subject. In some cases, the pre-determined number of doses comprises an amount of nicotine sufficient to provide about 1 to about 14 days of use on demand by a subject. In some cases, the device further comprises a pump, wherein the pump is configured to deliver the liquid nicotine formulation comprising nicotine from the reservoir to the heater element. In some cases, the pump is located completely within the reservoir. In some cases, the pump is located partially within the reservoir. In some cases, the pump is a diaphragm pump. In some cases, the pump is a piston pump. In some cases, the drive motor for the pump is located outside of the reservoir. In some cases, the heater element comprises a coil comprising electrically resistive material. In some cases, the heater element further comprises a wicking element in fluid communication with the liquid formulation comprising nicotine and wherein the coil comprising electrically resistive material is wrapped around the wicking element. In some cases, the wicking element comprises electrically resistive material. In some cases, the wicking element and the coil are continuous. In some cases, the device further comprises an additional airflow channel connected to the airflow channel. In some cases, the additional airflow channel connects between the outlet and the heater element in the airflow channel. In some cases, the additional airflow channel connects to the airflow channel between the inlet and the heater element. In some cases, the additional airflow channel permits entry of entrainment air, wherein the condensation aerosol is mixed with the entrainment air to produce a total airflow rate out of the mouthpiece of between about 20 LPM and about 80 LPM at a vacuum of about 249 Pa to about 3738 Pa (about 1 inch of water to about 15 inches of water).

As a further example, provided herein is a method for treating an urge to smoke in a subject, the method comprising: administering a condensation aerosol comprising nicotine to the subject, wherein the condensatin erosol comprising nicotine comprises a diameter of from about 1 μm to about 5 μm, wherein the administering comprises the subject inhaling the condensation aerosol comprising nicotine from a device configured to generate the comdensation aerosol comprising nicotine from a liquid formulation comprising nicotine, and wherein the condensation aerosol comprises a pre-determined amount of nicotine, whereby the subject inhales the condensation aerosol a plurality of times in order to administer a predetermined dose of nicotine, thereby reducing the urge to smoke in the subject. In some cases, the diameter comprises a mass median aerodynamic diameter (MMAD). In some cases, the condensation aerosol comprises a geometric standard deviation of less than 2. In some cases, the device is configured to deliver the condensation aerosol comprising nicotine to a deep lung of the subject. In some cases, the reduction in the urge to smoke in the subject is at least 50%. In some cases, the reduction in the urge to smoke in the subject is at least 60%. In some cases, the reduction in the urge to smoke in the subject is at least 70%. In some cases, the reduction in the urge to smoke in the subject is at least 80%. In some cases, the reduction in the urge to smoke in the subject is a complete or substantially complete elimination of the urge to smoke in the subject. In some cases, the reduction in the urge to smoke is compared to an urge to smoke in the subject before using the aerosol generating device. In some cases, the reduction in the urge to smoke is compared to an urge to smoke in the subject following administration of a vehicle using the aerosol generating device. In some cases, the reduction in the urge to smoke is sustained for at least 60 minutes. In some cases, the reduction in the urge to smoke is assessed using a psychometric response scale. In some cases, the psychometric response scale comprises a smoking urge visual analog scale (SU-VAS). In some cases, the reduction in the urge to smoke in the subject occurs within about 1 minute after administering the condensation aerosol comprising nicotine to the subject using the device. In some cases, the subject exhales no or substantially no visible vapor following inhalation of the condensation aerosol produced by the device. In some cases, the pre-determined amount of nicotine is from about 25 to about 100 μg. In some cases, the pre-determined dose of nicotine is from about 500 μg to about 1000 μg. In some cases, the pre-determined dose of nicotine is about 500 μg. In some cases, the pre-determined dose of nicotine is about 1000 μg. In some cases, the plurality of times comprises from about 2 to about 10 inhalations from the device. In some cases, the pre-determined dose of nicotine produces a nicotine plasma concentration that is at least 50% less than the nicotine plasma concentration produced by a cigarette or an electronic cigarette. In some cases, the pre-determined dose of nicotine produces a nicotine plasma concentration of from about 0.5 ng/ml to about 1 ng/ml. In some cases, the nicotine plasma concentration is produced in about 30 seconds following the administration of the pre-determined dose of nicotine. In some cases, the nicotine plasma concentration is sustained for at least 10 minutes following the administration of the pre-determined dose of nicotine. In some cases, the device is hand-held. In some cases, the device is disk-shaped. In some cases, the device further comprises a reservoir and a heater element, wherein the reservoir comprises a pre-determined number of doses of the liquid formulation comprising nicotine. In some cases, the pre-determined number of doses comprises an amount of nicotine sufficient to provide about 1 day of use on demand by a subject. In some cases, the pre-determined number of doses comprises an amount of nicotine sufficient to provide about 1 to about 7 days of use on demand by a subject. In some cases, the pre-determined number of doses comprises an amount of nicotine sufficient to provide about 1 to about 14 days of use on demand by a subject. In some cases, the device further comprises a pump, wherein the pump is adapted to deliver the liquid nicotine formulation comprising nicotine from the reservoir to the heater element. In some cases, the pump is located completely within the reservoir. In some cases, the pump is located partially within the reservoir. In some cases, the pump is a diaphragm pump. In some cases, the pump is a piston pump. In some cases, the drive motor for the pump is located outside of the reservoir. In some cases, the heater element comprises a coil comprising electrically resistive material. In some cases, the heater element further comprises a wicking element in fluid communication with the liquid formulation comprising nicotine and wherein the coil comprising electrically resistive material is wrapped around the wicking element. In some cases, the wicking element comprises electrically resistive material. In some cases, the wicking element and the coil are continuous. In some cases, the device further comprises a first airflow channel and a second airflow channel, wherein the first airflow channel comprises an inlet and an outlet, wherein the heater element is located within the first airflow channel between the inlet and the outlet, and wherein the second airflow channel is connected to the first airflow channel. In some cases, the second airflow channel connects between the outlet and the heater element in the first airflow channel. In some cases, the second airflow channel connects to the first airflow channel between the inlet and the heater element. In some cases, the condensation aerosol is produced in the first airflow channel. In some cases, the second airflow channel permits entry of entrainment air, wherein the condensation aerosol is mixed with the entrainment air to produce a total airflow rate out of the mouthpiece of between about 20 LPM and about 80 LPM at a vacuum of about 249 Pa to about 3738 Pa (about 1 inch of water to about 15 inches of water). In some cases, the pre-determined dose of nicotine administered to the subject per use of the device is substanially identical between uses of the device. In some cases, the subject administers the condensation aerosol comprising nicotine according to a prescribed treatment regimen. In some cases, the subject administers the condensation aerosol comprising nicotine on demand. In some cases, the subject administers the condensation aerosol comprising nicotine multiple times per day.

As yet another example, provided herein is an aerosol generating device for generating a condensation aerosol from a liquid formulation comprising a pharmaceutically active agent, the device comprising: a. a reservoir comprising the liquid formulation comprising a pharmaceutically active agent; b. a pump, wherein the pump is located within the reservoir, and wherein the pump is in fluid communication with the liquid formulation comprising a pharmaceutically active agent; and c. a heater element, wherein the heater element is in fluid communication with the pump, and wherein the pump is configured to deliver the liquid formulation comprising a pharmaceutically active agent to the heater element, wherein the heater element is configured to vaporize the liquid formulation upon activation to generate the condensation aerosol. In some cases, the pump is located completely within the reservoir. In some cases, the pump is located partially within the reservoir. In some cases, the device further comprises an airflow channel comprising an inlet and an outlet, wherein the heater element is located within the airflow channel between the inlet and the outlet. In some cases, the device further comprises an additional airflow channel connected to the airflow channel. In some cases, the additional airflow channel connects between the outlet and the heater element in the airflow channel. In some cases, the additional airflow channel connects to the airflow channel between the inlet and the heater element. In some cases, the additional airflow channel permits entry of entrainment air, wherein the condensation aerosol is mixed with the entrainment air to produce a total airflow rate out of the mouthpiece of between about 20 LPM and about 80 LPM at a vacuum of about 249 Pa to about 3738 Pa (about 1 inch of water to about 15 inches of water). In some cases, the airflow passageway is configured to produce the condensation aerosol in the device. In some cases, the condensation aerosol has a diameter of from about 1 $\mu$m to about 5 $\mu$m. In some cases, the pharmaceutically active agent is nicotine. In some cases, the pump is a diaphragm pump. In some cases, the pump is a piston pump. In some cases, a drive motor of the pump is located outside of the reservoir. In some cases, the drive motor is a magnetic drive motor. In some cases, the heater element comprises a coil comprising electrically resistive material. In some cases, the heater element further comprises a wicking element in fluid communication with the liquid formulation comprising nicotine and wherein the coil comprising electrically resistive material is wrapped around the wicking element. In some cases, the wicking element comprises electrically resistive material. In some cases, the wicking element and the coil are continuous. In some cases, the device further comprises a mouthpiece. In some cases, the mouthpiece comprises a slidable door, wherein the slidable door is configured to slidably cover the mouthpiece. In some cases, the reservoir comprises a pre-determined number of doses of the liquid formulation comprising nicotine. In some cases, the reservoir is disposable. In some cases, the reservoir is refillable. In some cases, the pre-determined number of doses comprises an amount of nicotine sufficient to provide about 1 day of use on demand by a subject. In some cases, the pre-determined number of doses comprises an amount of nicotine sufficient to provide about 1 to about 7 days of use on demand by a subject. In some cases, the pre-determined number of doses comprises an amount of nicotine sufficient to provide about 1 to about 14 days of use on demand by a subject. In some cases, the device is hand-held. In some cases, the device is disk-shaped. In one aspect, provided herein is a method of treating a condition, the method comprising: administering a condensation aerosol comprising nicotine to a subject, wherein the administering comprises the subject inhaling the condensation aerosol comprising nicotine from the device described herein, wherein the inhaling the condensation aerosol comprising nicotine delivers a pre-determined dose of nicotine to the subject, thereby treating the condition. In some cases, the condition is an urge to smoke. In some cases, the administering is self-administering. In some cases, the subject administers the condensation aerosol comprising nicotine on demand. In some cases, the subject administers the condensation aerosol comprising nicotine multiple times per day.

In one aspect, provided herein is an aerosol generating device comprising: a liquid formulation comprising a pharmaceutically active agent, a heater element, and a control program, wherein the control program comprises a first phase and a second phase, wherein the first phase controls delivery of a first amount of the liquid formulation to the heater element to generate a first aerosol comprising a first diameter and the second phase controls delivery of a second amount of the liquid formulation to the heater element to generate a second aerosol comprising a second diameter, wherein the first amount is different from the second amount. In some cases, the pharmaceutically active agent is nicotine. In some cases, the device further comprises an airflow channel comprising an inlet and an outlet, wherein the heater element is located within the airflow channel between the inlet and the outlet. In some cases, the device further comprises an additional airflow channel connected to the airflow channel. In some cases, the additional airflow channel connects between the outlet and the heater element in the airflow channel. In some cases, the additional airflow channel connects to the airflow channel between the inlet and the heater element. In some cases, the additional airflow channel permits entry of entrainment air, wherein each of the first aerosol and the second aerosol is mixed with the entrainment air to produce a total airflow rate out of a mouthpiece on the device. In some cases, the total airflow rate is between about 20 LPM and about 80 LPM at a vacuum of about 249 Pa to about 3738 Pa (about 1 inch of water to about 15 inches of water). In some cases, the airflow channel is configured to produce the first aerosol and the second aerosol in the device. In some cases, the first diameter is a size effective for delivery and absorption in a deep lung of a subject using the device. In some cases, the size effective for delivery and absorption in the deep lung of a subject using the device produces no or substantially no visible vapor upon exhalation by a subject using the device. In some cases, the first diameter is from about 1 $\mu$m to about 5 $\mu$m. In some cases, the second diameter is a size effective for producing a visible vapor upon exhalation by a subject using the device. In some cases, the second diameter is less than about 1

μm. In some cases, the device further comprises a pump, wherein the first phase directs the pump to deliver the first amount to the heater element, and wherein the second phase directs the pump to deliver the second amount to the heater element. In some cases, the first phase directs the pump to operate at a first rate, and wherein the second phase directs the pump to operate at a second rate, wherein the first rate and the second rate are different. In some cases, the heater element comprises a coil comprising electrically resistive material. In some cases, the heater element further comprises a wicking element in fluid communication with the liquid formulation comprising nicotine and wherein the coil comprising electrically resistive material is wrapped around the wicking element. In some cases, the wicking element comprises electrically resistive material. In some cases, the wicking element and the coil are continuous. In some cases, the device is hand-held. In some cases, the device is disk-shaped. In some cases, the first phase and the second phase occur sequentially during a use of the device. In one aspect provided herein is a method of treating a condition, the method comprising: administering a a first aerosol comprising nicotine to a subject, wherein the administering comprises the subject inhaling the first aerosol comprising nicotine from the device as described herein, wherein the inhaling the first aerosol comprising nicotine delivers a pre-determined dose of nicotine to the subject, thereby treating the condition. In some cases, the condition is an urge to smoke. In some cases, the administering is self-administering. In some cases, the subject administers the first aerosol comprising nicotine on demand. In some cases, the subject administers the first aerosol comprising nicotine multiple times per day.

In one aspect, provided herein is a method for generating aerosols from a liquid formulation comprising a pharmaceutically active agent, the method comprising: delivering a first amount of the liquid formulation comprising a pharmaceutically active agent to a heater element in an aerosol generating device, activating the heater element a first time, wherein the first activation of the heater element produces a first aerosol comprising a first diameter, delivering a second amount of the liquid formulation comprising a pharmaceutically active agent to the heater element; and activating the heater element a second time, wherein the second activation of the heater element produces a second aerosol comprising a second diameter, wherein the first amount is different than the second amount. In some cases, the pharmaceutically active agent is nicotine. In some cases, the device comprises an airflow channel comprising an inlet and an outlet, wherein the heater element is located within the airflow channel between the inlet and the outlet. In some cases, the airflow channel is configured to produce the first aerosol and the second aerosol in the device. In some cases, the first diameter is a size effective for delivery and absorption in a deep lung of a subject using the device. In some cases, the size effective for delivery and absorption in the deep lung of the subject using the device produces no or substantially no visible vapor upon exhalation by a subject using the device. In some cases, the first diameter is from about 1 μm to about 5 μm. In some cases, the second diameter is a size effective for producing a visible vapor upon exhalation by a subject using the device. In some cases, the second diameter is less than about 1 μm. In some cases, the device comprises a pump, wherein the pump delivers the first amount to the heater element, and wherein the pump delivers the second amount to the heater element. In some cases, the pump operates at a first rate during the delivering of the first amount, and wherein the pump operates at a second rate during the delivering of the second amount, wherein the first rate and the second rate are different. In some cases, the heater element comprises a coil comprising electrically resistive material. In some cases, the heater element further comprises a wicking element in fluid communication with the liquid formulation comprising nicotine and wherein the coil comprising electrically resistive material is wrapped around the wicking element. In some cases, the wicking element comprises electrically resistive material. In some cases, the wicking element and the coil are continuous. In some cases, the device is hand-held. In some cases, the device is disk-shaped. In some cases, the delivering the second amount occurs after the delivering of the first amount, and wherein the delivering of the first amount and the delivering of the second amount occur during a use of the device by a subject.

As a further example, provided herein is an aerosol generating device for generating a condensation aerosol from a liquid formulation comprising a pharmaceutically active agent, the device comprising: a. a reservoir comprising the liquid formulation comprising a pharmaceutically active agent; b. a pump, wherein the pump is in fluid communication with the reservoir comprising the liquid formulation comprising a pharmaceutically active agent, and wherein the pump is configured to operate at a first rate and a second rate; and c. a heater element, wherein the heater element is in fluid communication with the pump, and wherein the first rate of the pump delivers a first amount of the liquid formulation comprising a pharmaceutically active agent to the heater element, wherein upon activation the heater element vaporizes the first amount that condenses to form a first condensation aerosol comprising a first diameter, and wherein the second rate of the pump delivers a second amount of the liquid formulation comprising a pharmaceutically active agent to the heater element, wherein upon activation the heater element vaporizes the second amount that condenses to form a second condensation aerosol comprising a second diameter, wherein the first amount is different than the second amount. In some cases, the first diameter is a size effective for delivery and absorption in a deep lung of a subject using the device. In some cases, the size effective for delivery and absorption in the deep lung of a subject using the device produces no or substantially no visible vapor upon exhalation by a subject using the device. In some cases, the first diameter is from about 1 μm to about 5 μm. In some cases, the second diameter is a size effective for producing a visible vapor upon exhalation of a subject using the device. In some cases, the second diameter is less than about 1 μm. In some cases, the pharmaceutically active agent is nicotine. In some cases, the pump is located completely within the reservoir. In some cases, the pump is located partially within the reservoir. In some cases, the pump is a diaphragm pump. In some cases, the pump is a piston pump. In some cases, a drive motor of the pump is located outside of the reservoir. In some cases, the drive motor is a magnetic drive motor. In some cases, the heater element comprises a coil comprising electrically resistive material. In some cases, the heater element further comprises a wicking element in fluid communication with the liquid formulation comprising nicotine and wherein the coil comprising electrically resistive material is wrapped around the wicking element. In some cases, the wicking element comprises electrically resistive material. In some cases, the wicking element and the coil are continuous. In some cases, delivery of the second amount occurs after delivery of the first amount, and wherein delivery of the first amount and delivery of the second amount occur during a use of the device by a subject. In some cases, the device comprises an airflow channel comprising an inlet and an outlet, wherein the heater element is located within the airflow channel between the inlet and the outlet. In some cases, the airflow channel is configured to produce the first aerosol and the second aerosol in the device. In one aspect, provided herein is a method of treating a condition, the method comprising: administering a a first aerosol comprising nicotine to a subject, wherein the administering comprises the subject inhaling the first aerosol comprising nicotine from the device as described herein, wherein the inhaling the first aerosol comprising nicotine delivers a pre-determined dose of nicotine to the subject, thereby treating the condition. In some cases, the condition is an urge to smoke. In some cases, the administering is self-administering. In some cases, the subject administers the first aerosol comprising nicotine on demand. In some cases, the subject administers the first aerosol comprising nicotine multiple times per day.

As still another example, provided herein is a method of treating a subject with an urge to smoke comprising administering to the subject a therapeutically effective amount of a condensation aerosol comprising nicotine, wherein the administering comprises the subject inhaling the condensation aerosol comprising nicotine from a device configured to generate the condensation aerosol comprising nicotine from a liquid formulation comprising nicotine, and wherein the administering generates a nicotine plasma concentration in the subject of from about 0.5 ng/ml to 1 ng/ml, thereby reducing the urge to smoke in the subject. In some cases, the therapeutically effective amount is from about 500 $\mu$g to about 1000 $\mu$g. In some cases, the therapeutically effective amount is about 500 $\mu$g. In some cases, the therapeutically effective amount is about 1000 $\mu$g. In some cases, the subject inhales the condensation aerosol comprising nicotine a plurality of times in order to deliver the therapeutically effective amount. In some cases, the plurality of times is from about 2 to about 10 inhalations. In some cases, the subject administers the condensation aerosol on demand. In some cases, the subject administers the condensation aerosol multiple times per day. In some cases, the reduction in the urge to smoke in the subject is at least 50%. In some cases, the reduction in the urge to smoke in the subject is at least 60%. In some cases, the reduction in the urge to smoke in the subject is at least 70%. In some cases, the reduction in the urge to smoke in the subject is at least 80%. In some cases, the reduction in the urge to smoke in the subject is a complete or substantially complete elimination of the urge to smoke in the subject. In some cases, the reduction in the urge to smoke is compared to an urge to smoke in the subject before using the aerosol generating device. In some cases, the reduction in the urge to smoke is compared to an urge to smoke in the subject following administration of a vehicle using the aerosol generating device. In some cases, the reduction in the urge to smoke is sustained for at least 60 minutes. In some cases, the reduction in the urge to smoke is assessed using a psychometric response scale. In some cases, the psychometric response scale comprises a smoking urge visual analog scale (SU-VAS). In some cases, the reduction in the urge to smoke in the subject occurs within about 1 minute after administering the condensation aerosol comprising nicotine to the subject using the device. In some cases, the nicotine plasma concentration is produced in about 30 seconds following the administration of the pre-determined dose of nicotine. In some cases, the nicotine plasma concentration is sustained for at least 10 minutes following the administration of the pre-determined dose of nicotine. In some cases, the condensation aerosol comprising nicotine has a diameter of from about 1 $\mu$m to about 5 $\mu$m. In some cases, the device comprises: a. a reservoir comprising the liquid formulation comprising nicotine; b. an air flow channel comprising an inlet and an outlet; and c. a heater element within the airflow channel, wherein the heater element is in fluid communication with the liquid formulation comprising nicotine; and wherein producing the condensation aerosol comprising nicotine comprises vaporizing the liquid formulation comprising nicotine upon delivery of the liquid formulation comprising nicotine to the heater element and subsequent activation of the heater element. In some cases, the heater element comprises a wire coil continuous with a wicking element, wherein the wire coil and wicking element comprise electrically resistive material. In some cases, the device further comprises a pump, wherein the pump is located within or partially within the reservoir.


**BRIEF DESCRIPTION OF THE DRAWINGS**

Novel features are set forth with particularity in the appended claims. A better understanding of the features and advantages will be obtained by reference to the following detailed description that sets forth illustrative embodiments, in which the principles are utilized, and the accompanying drawings of which:

**FIG. 1** illustrates an embodiment of an electronic nicotine delivery device.

**FIGs. 2A** and **2B** illustrate an embodiment of electronic agent (e.g., nicotine) delivery device.

**FIGs. 3A** and **3B** illustrate embodiments of a heater element.

**FIG. 4** illustrates an embodiment of an agent (e.g., nicotine) reservoir.

**FIG. 5** illustrates another embodiment of an agent (e.g., nicotine) reservoir.

**FIG. 6** illustrates another embodiment of an agent (e.g., nicotine) reservoir.

**FIG. 7** illustrates an embodiment of a heater element.

**FIG. 8** illustrates an embodiment of an electronic agent (e.g., nicotine) delivery device.

**FIG. 9** illustrates another embodiment of a heater element.

**FIGs. 10A** and **10B** illustrate additional embodiments of a heater element.

**FIG 11** illustrates inertial impaction.

**FIG. 12** illustrates an embodiment of a method of removal of an agent (e.g., nicotine) mixture from a reservoir and dispensing the nicotine into desired doses.

**FIG. 13** illustrates another embodiment of a method for measuring an agent (e.g., nicotine) dose.

**FIG. 14** illustrates another embodiment for measuring an agent (e.g., nicotine) dose.

**FIG. 15** illustrates another embodiment for measuring an agent (e.g., nicotine) dose.

**FIGs. 16A** and **16B** illustrate embodiments for applying an agent (e.g., nicotine) to a heater element.

**FIGs. 17A** and **17B** illustrate embodiments of mechanisms for generating an aerosol.

**FIG. 18** illustrates an embodiment of a mechanism for dispensing an agent (e.g., nicotine) mixture.

**FIG. 19** illustrates feedback to a nicotine user regarding nicotine intake and mean craving over time.

**FIG. 20** illustrates customized feedback to a user of an electronic nicotine delivery device.

**FIG. 21** illustrates an embodiment of a method for flow control.

**FIG. 22** illustrates an embodiment of a heater element.

**FIG. 23** illustrates another embodiment for measuring an agent (e.g., nicotine) dose.

**FIG. 24** illustrates another embodiment for measuring an agent (e.g., nicotine) dose.

**FIGs. 25A** and **25B** illustrate another embodiment of a method of removal of an agent (e.g., nicotine) mixture from a reservoir.

**FIG. 26** illustrates a schematic of a test apparatus used for testing the effects of altering system parameters of an aerosol delivery device on particle size distribution.

**FIGs. 27A, 27B, 27C,** and **27D** illustrate a schematic of a test bed used for generating an aerosol in the test apparatus of **FIG. 26.**

**FIG. 28** illustrates a comparison of particle sizes of an aerosol created by an e-cigarette (e-cig) vs. an aerosol created by a device as provided herein.

**FIGs. 29A** and **29B** illustrate a schematic of a test apparatus used for testing flow control. **FIG. 29B** illustrates a close-up of the valve (**2904a**) that is part of the test apparatus in **FIG. 29A.**

**FIGs. 30A** and **30B** illustrates an alternative valve flap for use in the valve (**2904a**) in **FIG. 29A. FIG. 30B** illustrates a slot for use in the bypass (**2908a**) in **FIG. 29A.**

**FIGs. 31A, 31B, 31C, 31D,** and **31E,** illustrate embodiments of airflow configurations and heater element.

**FIGs. 32A, 32B, 32C, 32D,** and **32E** illustrate embodiments of flow-through passageways.

**FIG. 33** illustrates an additional embodiment of a flow-through passageway.

**FIG. 34** illustrates an embodiment of a flow control valve.

**FIG. 35** illustrates an embodiment of a device comprising a primary and secondary airway.

**FIG. 36** illustrates another embodiment of a heater element.

**FIGs. 37A** and **37B** illustrate embodiments of a heater element similar to that shown in **FIG. 36. FIG. 37A** depicts a wire coil spanning a large percentage of the length of one end of the wire. **FIG. 37B** depicts a wire coil spanning a smaller percentage of the length of one end of the wire than shown in **FIG. 37A.**

**FIG. 38** illustrates an enlarged representation of the wire coil from the heater element of **FIG. 36.**

**FIG. 39** illustrates components of eHealth-enabled electronic agent (e.g., nicotine) delivery system, in accordance with an embodiment.

**FIG. 40** illustrates example components of an electronic agent (e.g., nicotine) delivery system, in accordance with an embodiment.

**FIG. 41** illustrates example components of an electronic agent (e.g., nicotine) delivery device for implementing aspects described herein, in accordance with an embodiment.

**FIG. 42** illustrates an escalating dose protocol utilized during part 1 of a two part study for assessing the safety, tolerability, pharmacokinetics, and pharmacodynamics of a condensation aerosol comprising nicotine and propylene glycol produced from an electronic agent (e.g., nicotine) delivery device as provided herein.

FIG. 43 illustrates a trial design for part 2 of a two part study for assessing the safety, tolerability, pharmacokinetics, and pharmacodynamics of a condensation aerosol comprising nicotine and propylene glycol produced from an electronic agent (e.g., nicotine) delivery device as provided herein.

FIGs. 44A, 44B, and 44C illustrate embodiments of a passageway comprising a baffle for removing particles of a non-optimal size. FIGs. 44A and 44B illustrates exterior views of a passageway comprising a baffle. FIG. 44C illustrates an interior view of a passageway comprising a baffle.

FIG. 45 illustrates a schematic of assessments for Part 1 of the two part study described in Examples 13 and 14.

FIG. 46 illustrates the timing of assessments for pre-dosing, dosing, and post-dosing in Part 1 of the two part study described in Examples 13 and 14.

FIG. 47 illustrates the % of doses producing cough for each of the 7 cohorts from Part 1 of the clinical study described in Examples 13 and 14.

FIG. 48 illustrates the % of dose producing cough for each of the 7 cohorts from Part 1 of the clinical study broken down by dose number.

FIG. 49 illustrates the pre-dose and post-dose forced expiration volume in the first second to forced vital capacity (FEV1/FVC) ratio for each of the 7 cohorts from Part 1 of the clinical study, which represents the percentage of subject's vital capacity that they are able to expire in the first second of expiration.

FIG. 50 illustrates the mean change in FEV1 for each of the 7 cohorts.

FIG. 51 illustrates the mean change in FVC for each of the 7 cohorts.

FIG. 52 illustrates the mean change in mean BP (mm Hg) for each of the 7 cohorts.

FIG. 53 illustrates the mean change in pulse (BPM) for each of the 7 cohorts.

FIG. 54 illustrates the median % change from a baseline smoking urge as measured on a visual analog scale (vas) for each of the 7 cohorts 1-min, 15-min, and 30-min post-dose.

FIG. 55 illustrates the mean smoking urge vas for each of the 7 cohorts predose and 1-min, 15-min, and 30-min post-dose.

FIG. 56 illustrates the percent change from a placebo (PBO) baseline for each of the 7 cohorts 1-min, 15-min, and 30-min post-dose.

**FIG. 57** illustrates the % change from a baseline smoking urge as measured on a visual analog scale (vas) for each of the 7 cohorts 1-min, 15-min, and 30-min post-dose.

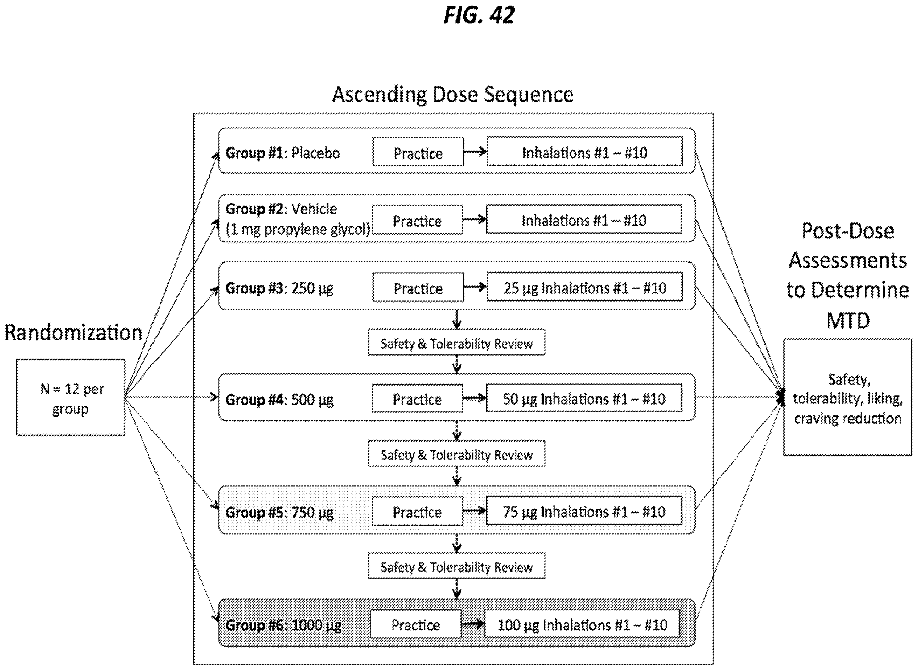

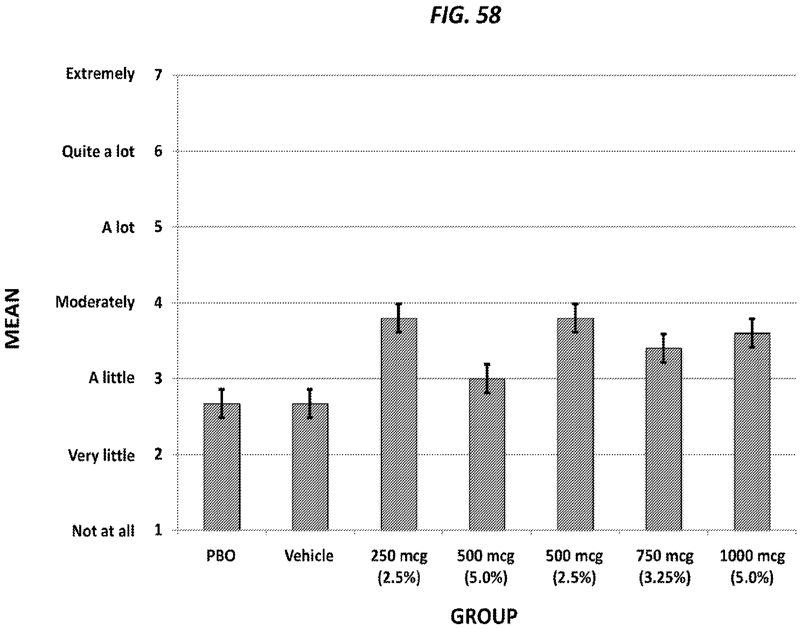

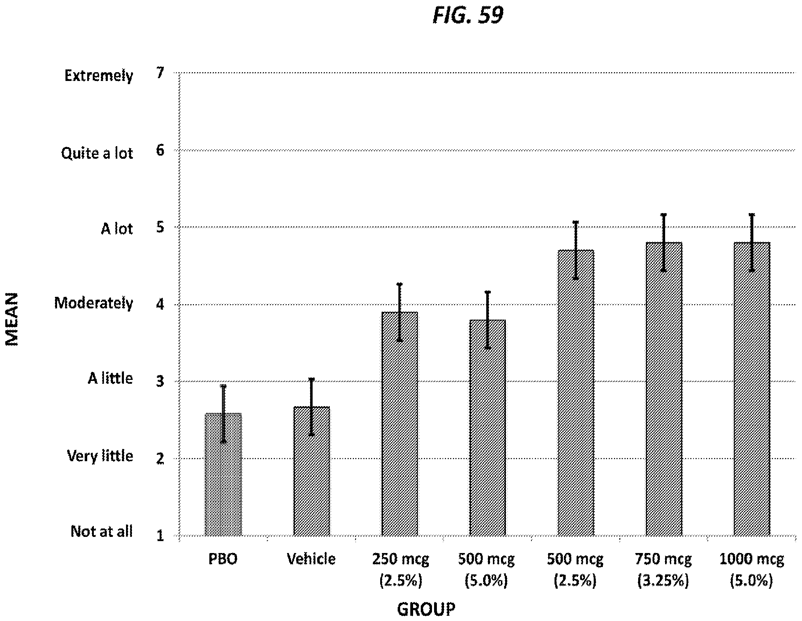

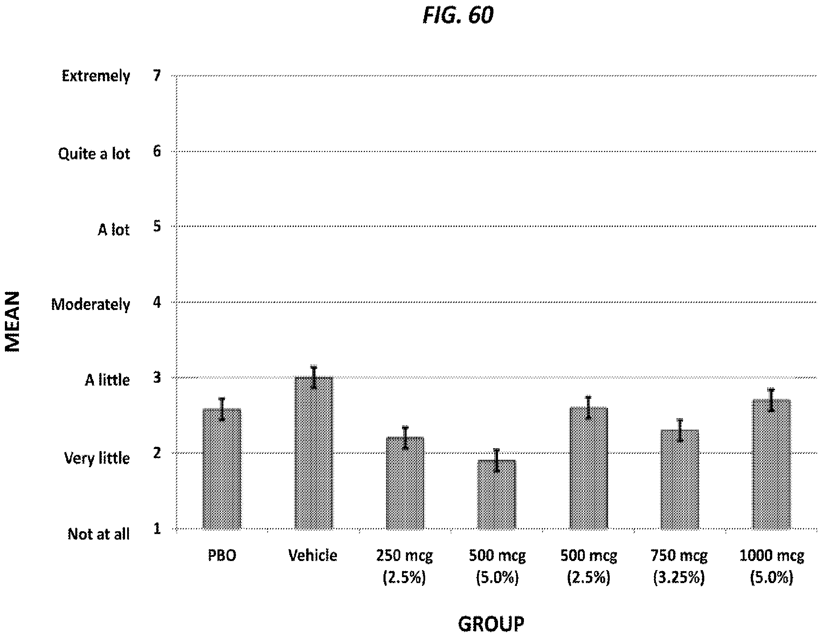

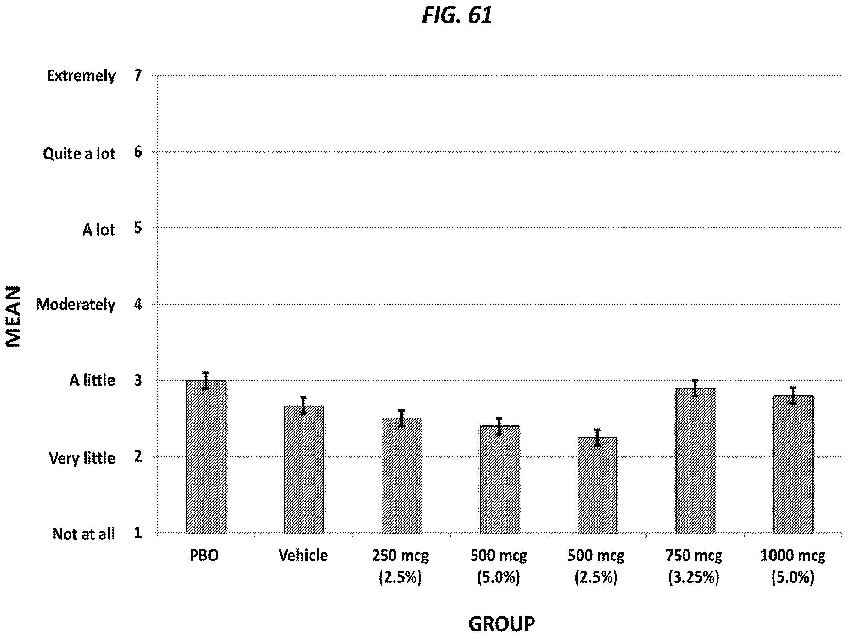

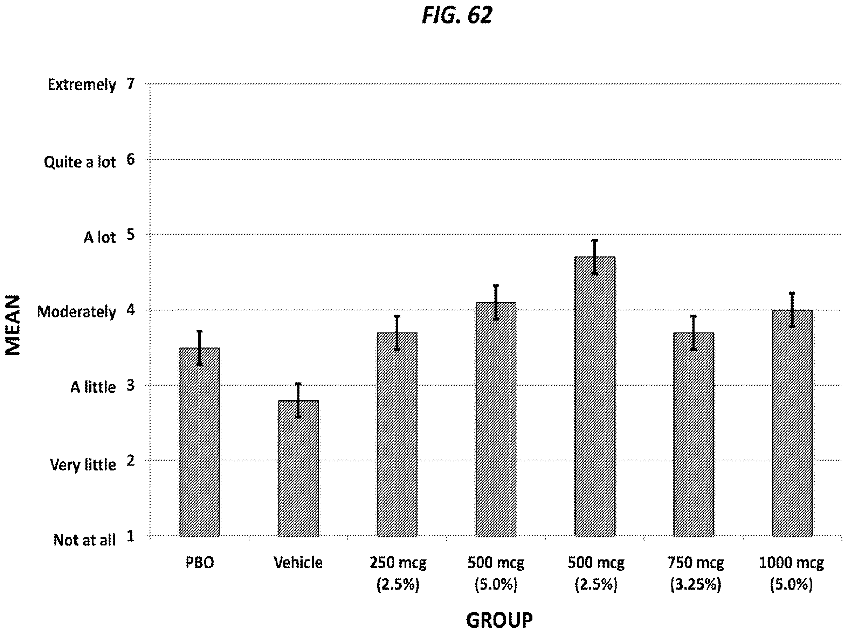

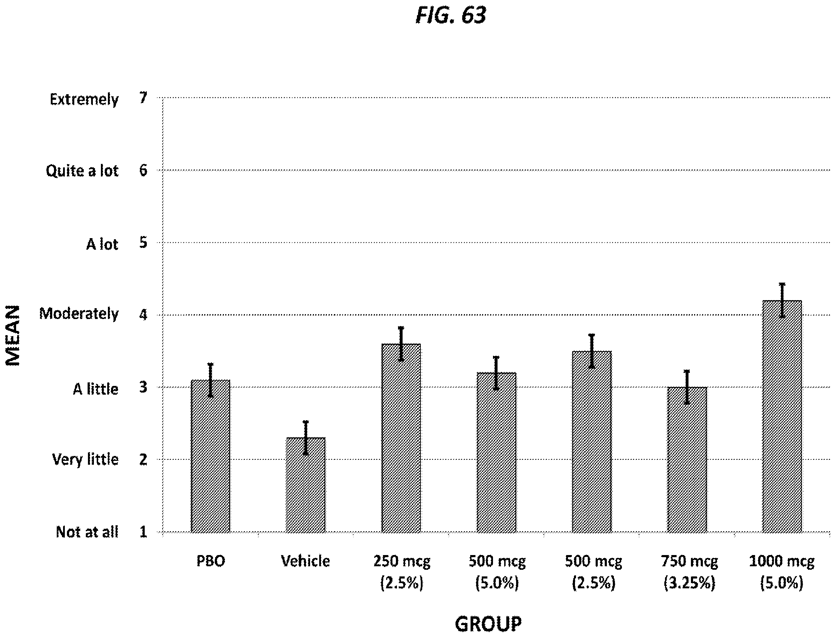

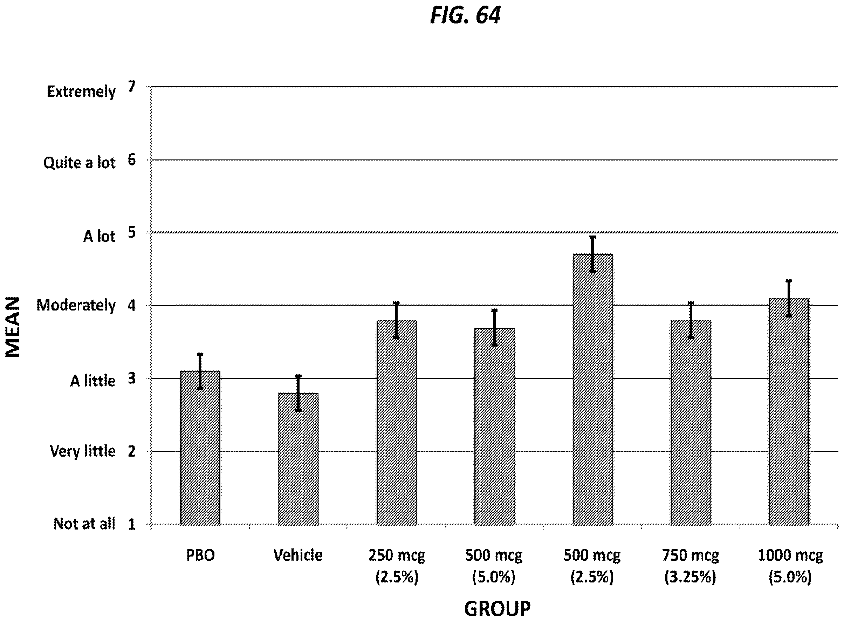

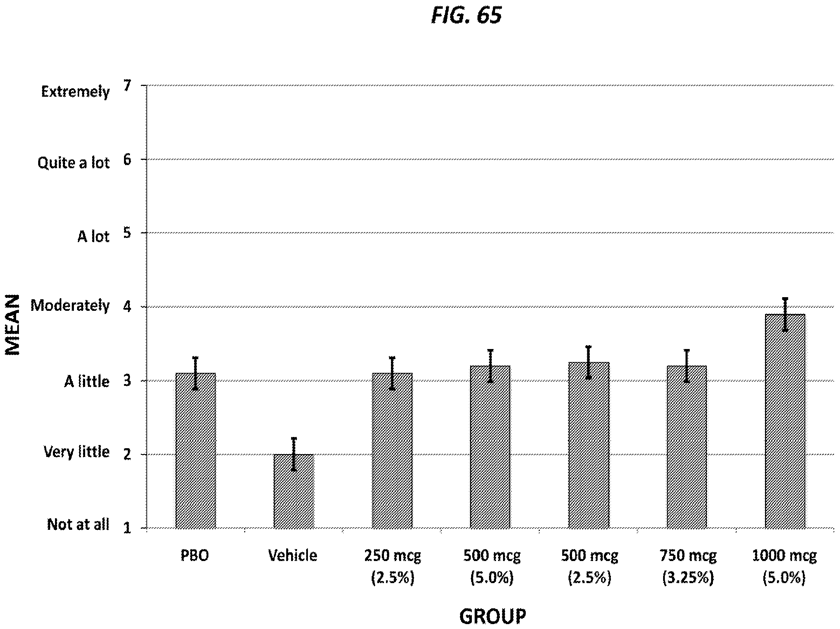

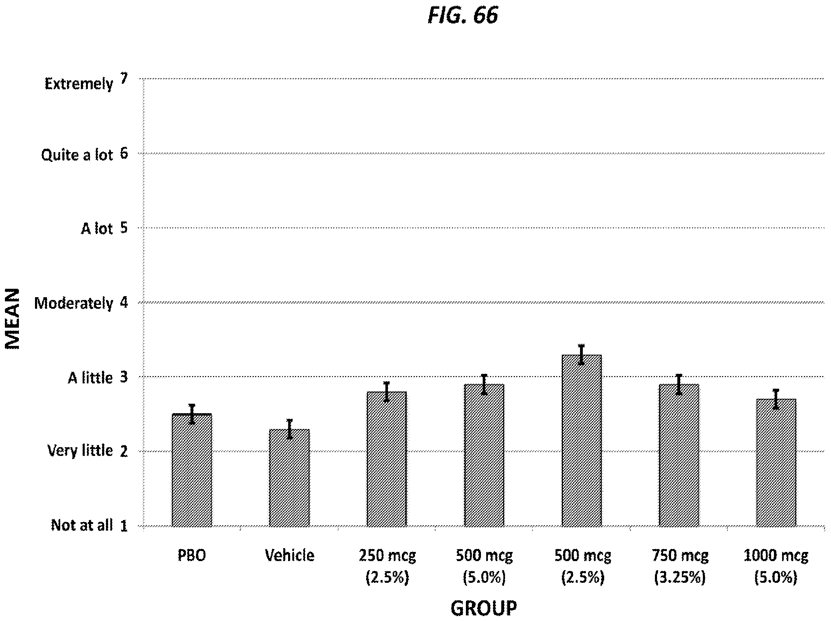

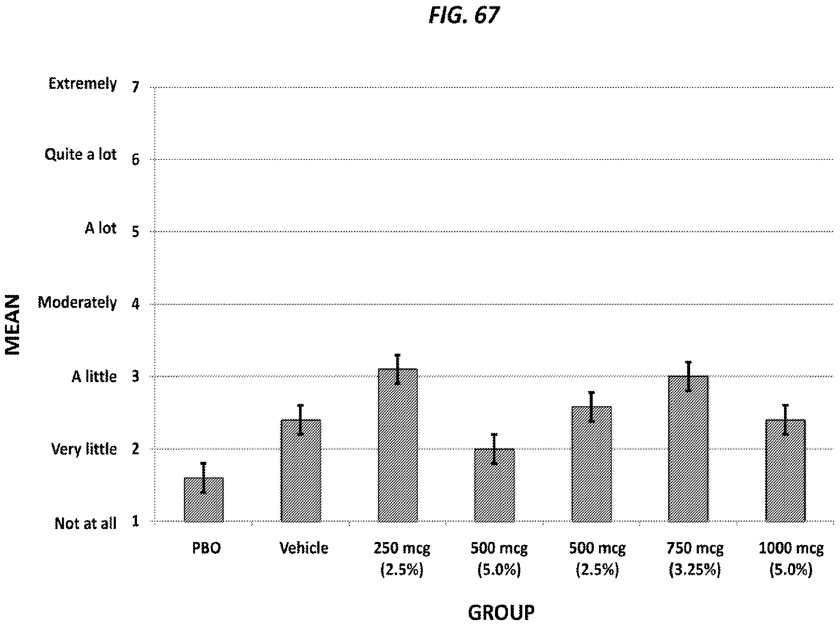

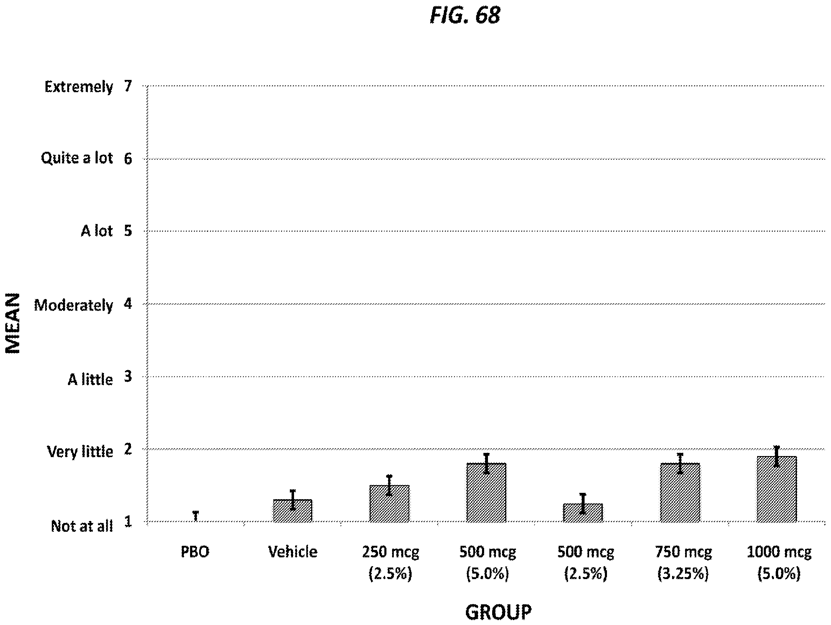

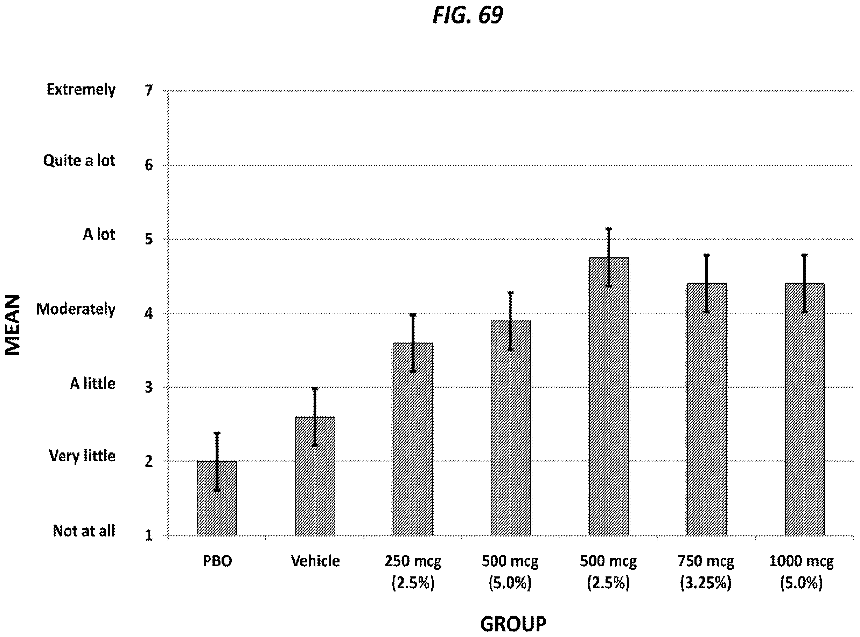

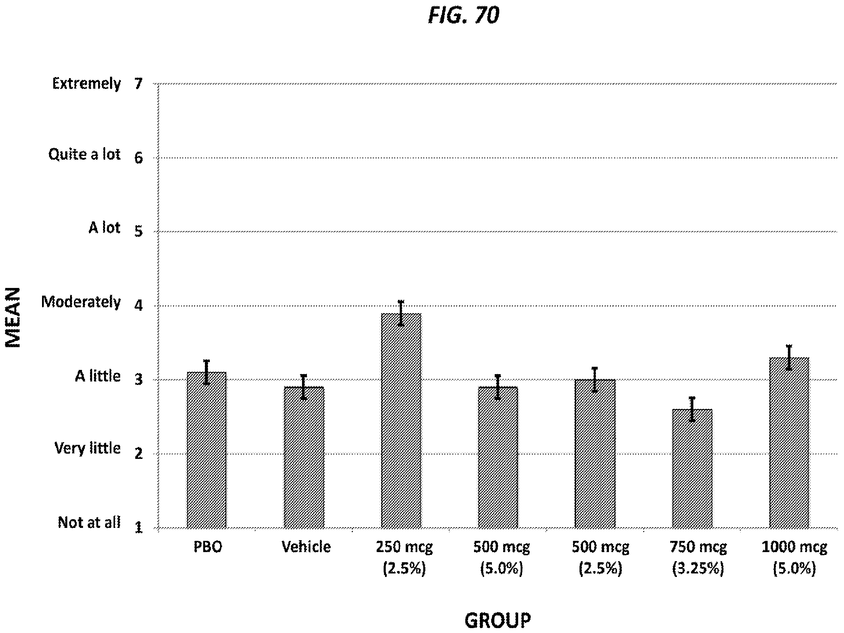

**FIGs. 58-70** illustrate the mean Likert rating response from a 7-point Likert response range for each question of a 13-Item Modified Cigarette Evaluation Scale (mCES) questionnaire completed by subjects in each of the 7 cohorts in Part 1 of the two-part clinical study outlined in **FIGs. 42-43** and described in Examples 13 and 14. **FIG. 58** illustrates the mean rating response for 'was it satisfying?' **FIG. 59** illustrates the mean rating response for 'how high in nicotine?'; **FIG. 60** illustrates the mean rating response for 'did it taste good?'; **FIG. 61** illustrates the mean rating response for 'did you enjoy the sensations in your throat and chest?'; **FIG. 62** illustrates the mean rating response for 'did it calm you?'; **FIG. 63** illustrates the mean rating response for 'did it make you feel more awake?'; **FIG. 64** illustrates the mean rating response for 'did it make you fell less irritable?'; **FIG. 65** illustrates the mean rating response for 'did it help you concentrate?'; **FIG. 66** illustrates the mean rating response for 'did it reduce your hunger for food?'; **FIG. 67** illustrates the mean rating response for 'did it make you dizzy?'; **FIG. 68** illustrates the mean rating response for 'did it make you nauseous?'; **FIG. 69** illustrates the mean rating response for 'did it immediately relieve your craving for a cigarette?'; and **FIG. 70** illustrates the mean rating response for 'did you enjoy it?'.

**FIG. 71** illustrates the mean Likert rating response on the mCES for a select number of questions (i.e., "was it satisfying?"; "how high in nicotine?"; "did it taste good?"; "did you enjoy the sensations in throat and chest?"; "did you enjoy it?").

**FIG. 72** illustrates a bivariate analysis of coughing vs. the mCES question of "did you enjoy it?" which showed that coughing was unrelated to a subject's response to "did you enjoy it?" in the 500 mcg (2.5%) group or 750 mcg (3.25%) cohorts.

**FIG. 73** illustrates a bivariate analysis of coughing vs. the mCES question of "was it satisfying?" which showed that less coughing did predict an increase in satisfaction overall and in the 750 mcg (3.25%) cohort.

**FIG. 74** illustrates the % of "yes" responses of subjects in each of the 7 groups in Part 1 of the two-part study outlined in described in Examples 13 and 14 to the question "if a product was available that was small and easy to use and produced this aerosol, would you consider using it as a replacement for your smoking?"

**FIG. 75** illustrates the median nicotine PK changes from baseline 30 seconds and 5 minutes post-dosing for each of the 7 cohorts between .68 and 2.0 ng/ml within 30 seconds after dosing as compared to baseline.

**FIG. 76** illustrates the raw change in pharmacokinetics (PK) by time (5-min and 10-min post-dosing) for each of the 7 cohorts.

**FIG. 77** illustrates the change in PK by time (5-min and 10-min post-dosing) as compared to baseline for each of the 7 cohorts.

**FIG. 78** illustrates the mean nicotine concentration (ng/ml) in each of the nicotine cohorts as compared to the nicotine concentration (ng/ml) from other products (i.e., nicotine patch, nicotine gum, nicotrol inhaler, NJOY 1st dose, NJOY 2nd dose, and cigarettes).

**FIG. 79** illustrates a box and whisker plot for the nicotine concentration (ng/ml) pre-dosing (PK_0) and 5 min post dosing (PK_5) each of the 7 cohorts.

**FIG. 80** illustrates illustrates a schematic of assessments for Part 2 of the two part study depicted in described in Examples 13 and 14.

**FIG. 81** illustrates the timing of assessments for pre-dosing, dosing, and post-dosing in Part 2 of the two part study described in Example 14.

**FIG. 82** illustrates the eNT-100 clinical device used in the 2 part clinical study outlined in **FIGs. 42** and **43** and described in Examples 13 and 14.

**FIG. 83A** and FIG. **83B** illustrate external structural features of one embodiment of a multi-piece device as provided herein.

**FIG. 84A** and **FIG. 84B** illustrate the internal structural features of the dose cartridge from the device depicted in **FIGs. 83A** and **83B.**

**FIG. 85** illustrates a flow simulation for airflow patterns through the primary and secondary airways in the dose cartridge depicted in **FIG. 84A-B**.

**FIG. 86** illustrates the external structural features of one embodiment of the devices provided herein.

**FIG. 87** illustrates a transverse sectional view of the internal structural features of the device depicted in **FIG. 86.**

**FIG. 88** illustrates a cross-sectional view of the internal structural features of the device depicted in **FIG. 86.**

**FIG. 89** illustrates the external structural features of one embodiment of the devices provided herein.

**FIGs. 90A-D illustrate** internal structural features of a diaphragm pump for use in any of the devices provided herein.

**FIG. 91** illustrates alternate views of the devices depicted in **FIGs. 83A-B, 86,** and **89.**

**FIG. 92 illustrate** the efficient use of nicotine by use of the eNT-100 device (shown in **FIG. 82)** by cohorts as described in Example 14.

**FIG. 93** illustrates the amount of formaldehyde per inhalation of an aerosol produced using a device as provided herein.

**FIGs. 94A-C** illustrate a cyclindrical aerosol generating device that resembles a cigarette. **FIG. 94A** illustrates and exterior view, while **FIG. 94B** and **FIG. 94C** illustrate an interior longitudinal section view of the entire device **(FIG. 94B)** or the mouthpiece end **(FIG. 94C).**

**FIGs. 95A-C** illustrate a removable single unit nicotine reservoir comprising a heater element with a retractable protector. **FIG. 95A** illustrates an exterior view, while **FIGs 95B-C** illustrate interior views of the single unit reservoir.

**FIG. 96** illustrates a nicotine reservoir comprising a pump piston within the reservoir and a magnetic drive motor for use in an aerosol generating device as provided herein.

**FIG. 97** illustrates the percent change from baseline smoking urge visual analog scale (VAS) for the placebo, vehicle, 250 mcg (2.5%), 500 mcg (5.0%), 500 mcg (2.5%) and 1000 mcg (5%) dose groups from Part 1 of the two part study described in Examples 13 and 14 as analyzed by a contract research organization (CRO; Celerion Lincoln NE).

**FIG. 98** illustrates the plasma nicotine concentration for the placebo, vehicle, 250 mcg (2.5%), 500 mcg (5.0%), 500 mcg (2.5%) and 1000 mcg (5%) dose groups from Part 1 of the two part study described in Examples 13 and 14.

**FIG. 99** illustrates the percent change from baseline smoking urge visual analog scale (VAS) for the vehicle, 500 mcg (2.5%), 1000 mcg (5%), NJOY King Bold e-Cig, and usual brand combustible cigarette dose groups from Part 2 of the two part study described in Examples 13 and 14 as analyzed by a contract research organization (CRO; Celerion Lincoln NE).

**FIG. 100** illustrates the smoking urge statistical comparisons excluding device failures for Part 2 of the two part study described in Examples 13 and 14.

**FIG. 101** illustrates the percent change from baseline smoking urge visual analog scale (VAS) for the vehicle, 500 mcg (2.5%), 1000 mcg (5%), NJOY King Bold e-Cig, and usual brand combustible cigarette dose groups from Part 2 of the two part study described in Examples 13 and 14.

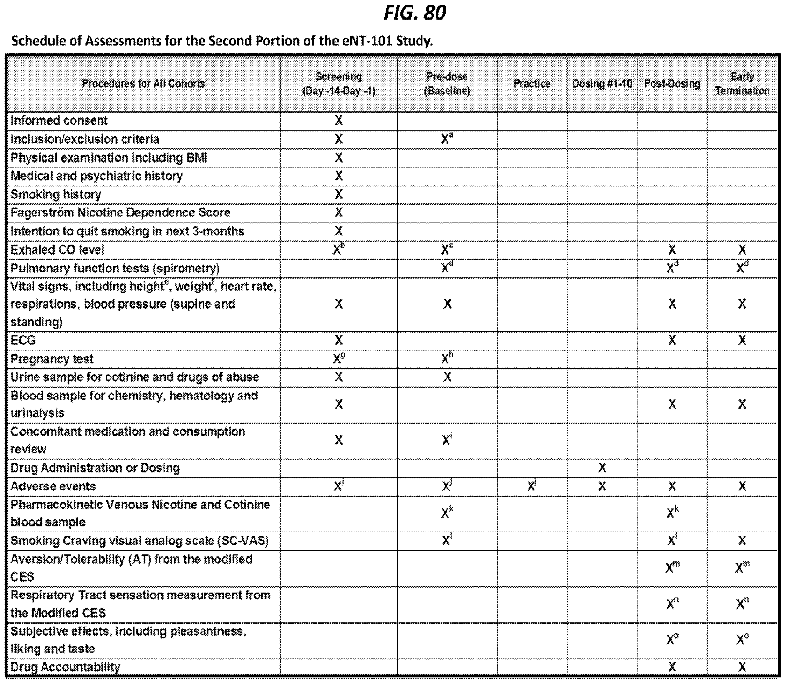

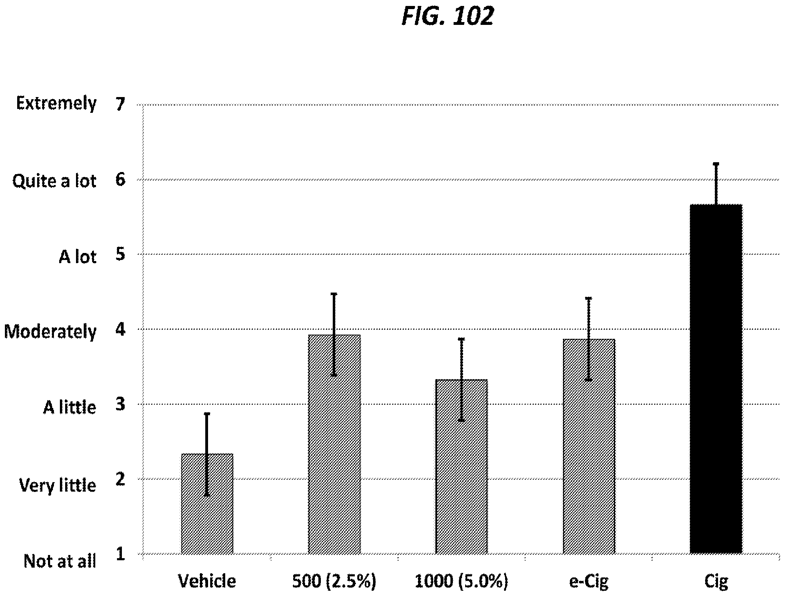

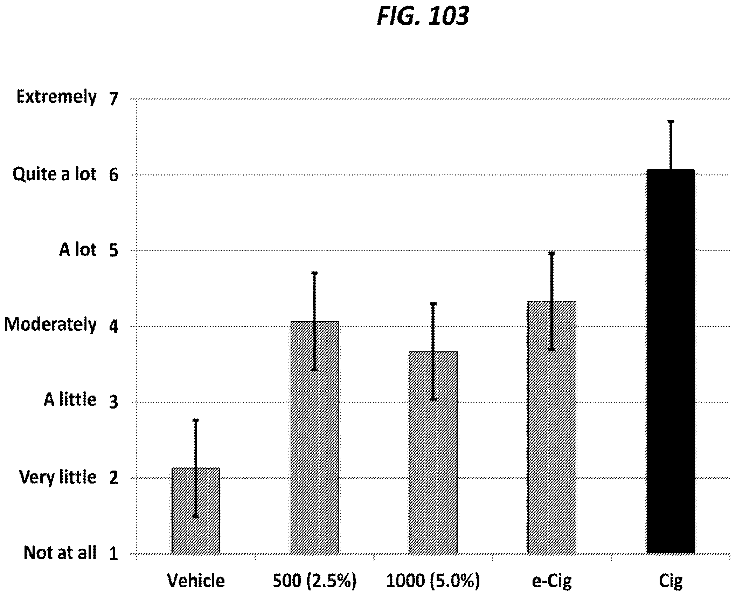

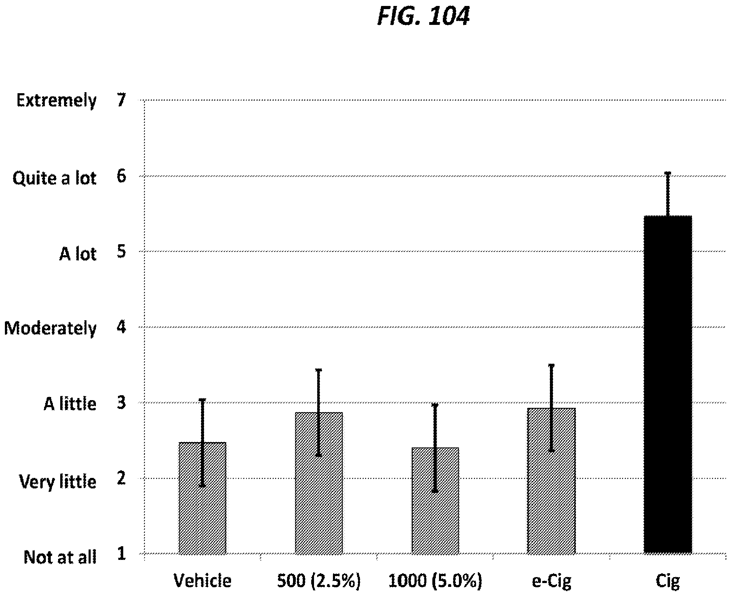

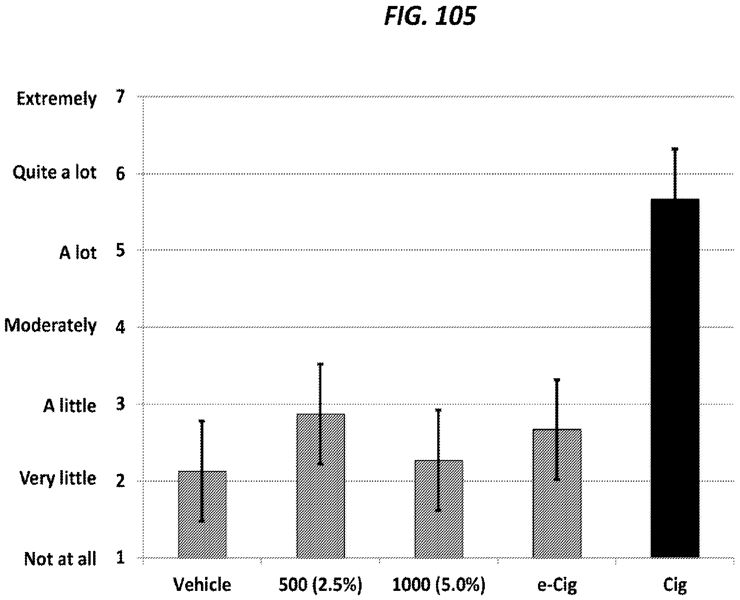

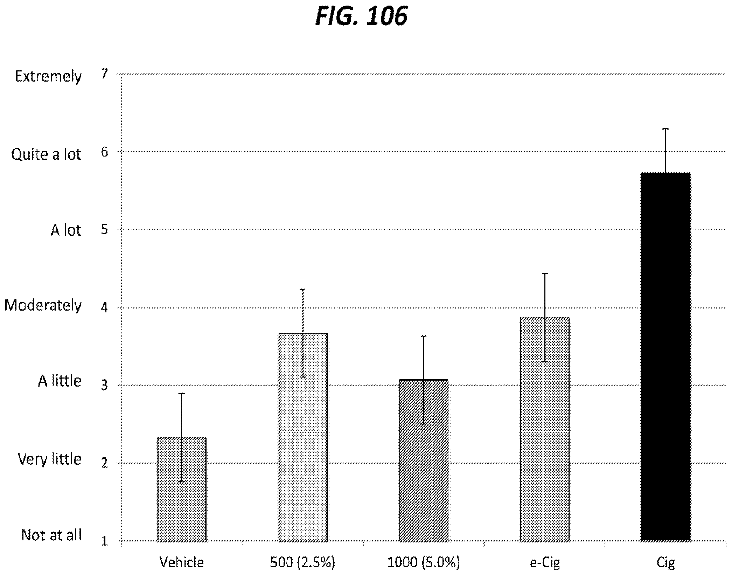

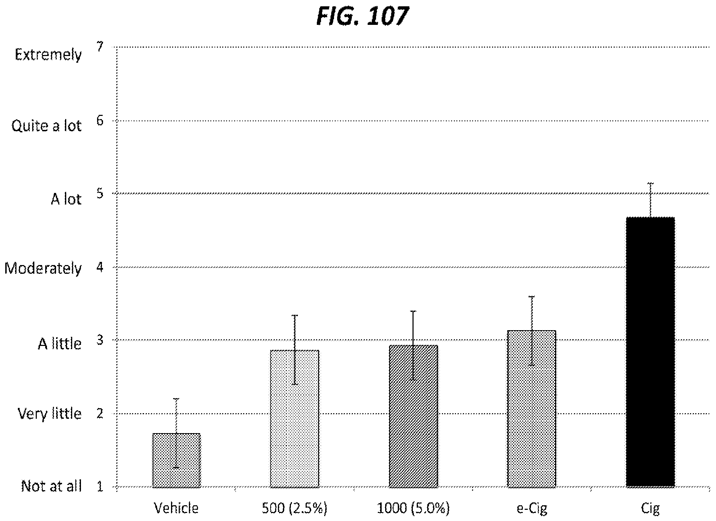

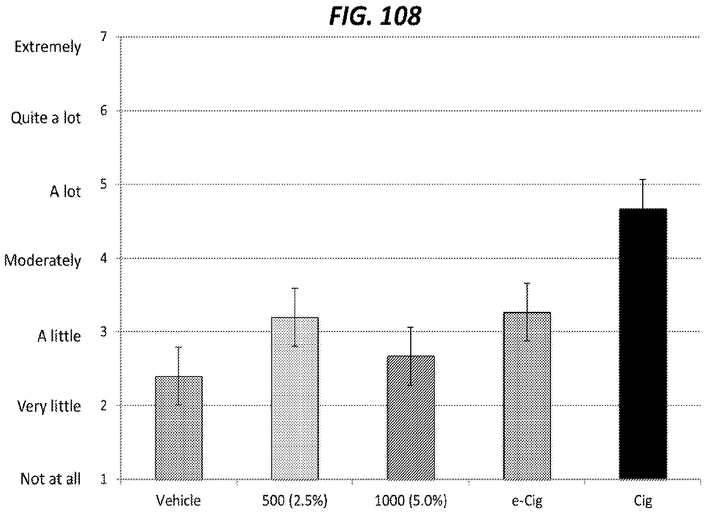

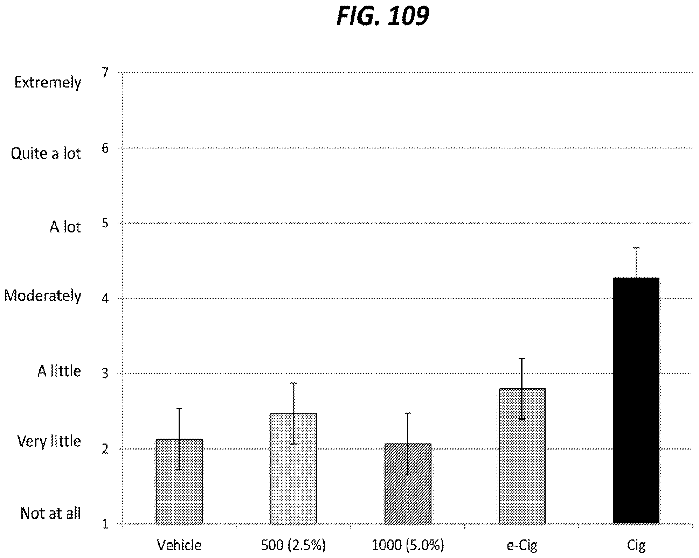

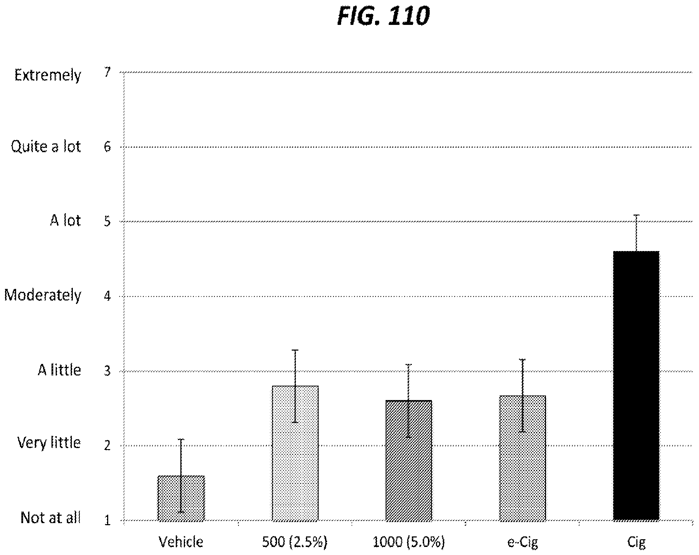

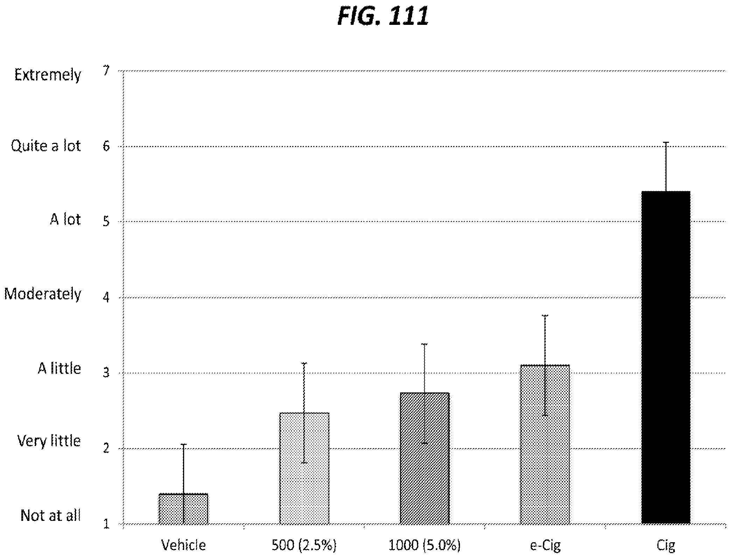

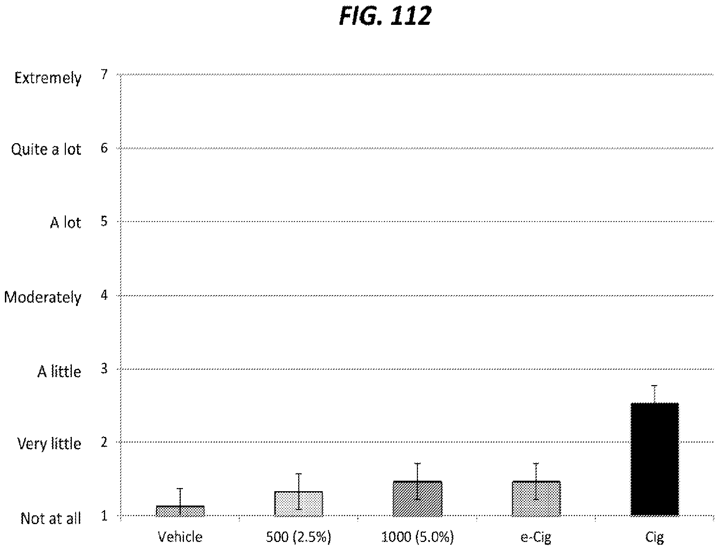

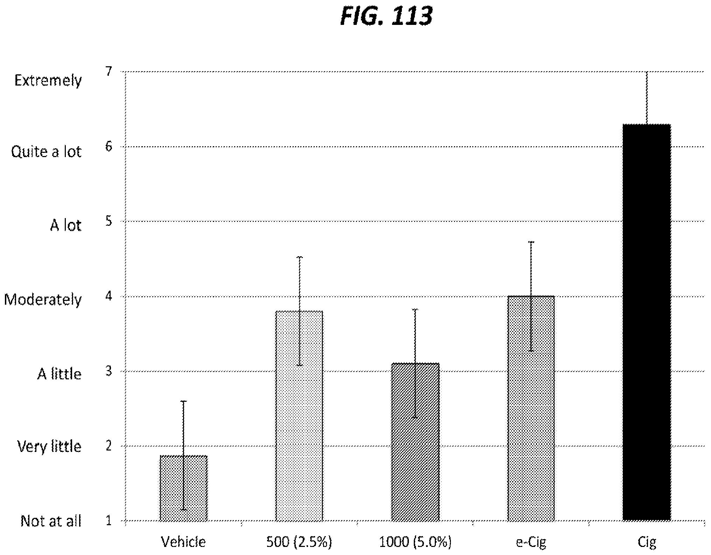

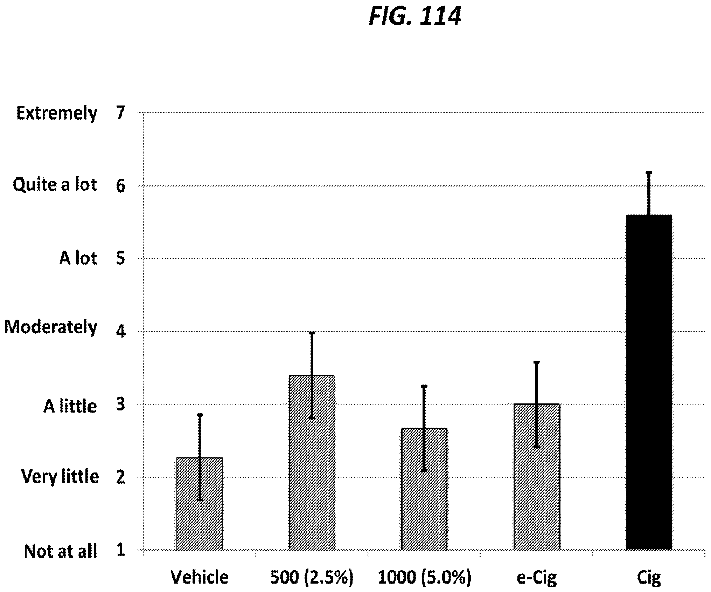

**FIGs. 102-114** illustrate the mean Likert rating response from a 7-point Likert response range for each question of a 13-Item Modified Cigarette Evaluation Scale (mCES) questionnairecompleted by subjects in each of the 5 cohorts in Part 2 of the two-part clinical study depicted in **FIGs. 80-81** and described in Example 14. **FIG. 102** illustrates the mean rating response for 'was it satisfying?' **FIG. 103** illustrates the mean rating response for 'how high in nicotine?'; **FIG. 104** illustrates the mean rating response for 'did it taste good?'; **FIG. 105** illustrates the mean rating response for 'did you enjoy the sensations in your throat and chest?'; **FIG. 106** illustrates the mean rating response for 'did it calm you?'; **FIG. 107** illustrates the mean rating response for 'did it make you feel more awake?'; **FIG. 108** illustrates the mean rating response for 'did it make you fell less irritable?'; **FIG. 109** illustrates the mean rating response for 'did it help you concentrate?'; **FIG. 110** illustrates the mean rating response for 'did it reduce your hunger for food?'; **FIG. 111** illustrates the mean rating response for 'did it make you dizzy?'; **FIG. 112** illustrates the mean rating response for 'did it make you nauseous?'; **FIG. 113** illustrates the mean rating response for 'did it immediately relieve your craving for a cigarette?'; and **FIG. 114** illustrates the mean rating response for 'did you enjoy it?'

FIG. 115 illustrates the baseline adjusted plasma nicotine concentration (ng/ml) as a function of hours from product use for the 5 cohorts from Part 2 of the two-part study described in Examples 13 and 14.

**FIG. 116** illustrates a summary of baseline-adjusted plasma nicotine pharmacokinetic parameters.

**FIG. 117** illustrates a summary of the statistical comparison of pharmacokinetic parameters exlcuding device failures.

**FIG. 118** illustrates a schematic of the dosing protocol for the Consumer Testing study described in Example 15.

**FIG. 119** illustrates the mean pulses after 5 inhalations for subjects in the Consumer Testing study described in Example 15.

**FIG. 120** illustrates the percentage of subjects experiencing coughing after using Product A or Product B for the Consumer Testing study described in Example 15.

**FIG. 121** illustrates the raw smoking urge assessments using the smoking urge VAS for the two groups (Product A first; Product B first) following the first 5 inhalations described in the Consumer Testing study described in Example 15.

**FIG. 122** illustrates the raw smoking urge assessments using the smoking urge VAS for the two groups (Product A first; Product B first) following the second 5 inhalations as described in the Consumer Testing study described in Example 15.

**FIG. 123** illustrates the % change from baseline smoking urge VAS for the raw data in FIG. **121.**

**FIG. 124** illustrates the % change from baseline smoking urge VAS for the raw data in **FIG. 122**.

**FIG. 125** illustrates the distributions of the raw smoking urge VAS data for the subjects in the Consumer Testing study described in Example 15.

**FIG. 126** illustrates the mean Likert rating response from a 7-point Likert response range for each question of a 6-Item Modified Cigarette Evaluation Scale (mCES) questionnairecompleted by subjects in each of the 2 groups of subjects used in the Consumer Testing study described in Example 15.

## DETAILED DESCRIPTION

### I. Overview

Provided herein are devices, systems, kits, compositions, computer readable medium, and methods for electronic delivery of an agent to a subject. For example the devices, systems, computer readable medium, and methods can be used for electronic nicotine delivery, which can facilitate recreational nicotine delivery, or full or partial smoking urge reduction. The devices, systems, computer readable medium, and methods provided herein can be used to allow each user to carefully track their usage and help them to transition completely off of cigarettes, and/or off nicotine entirely if they choose.

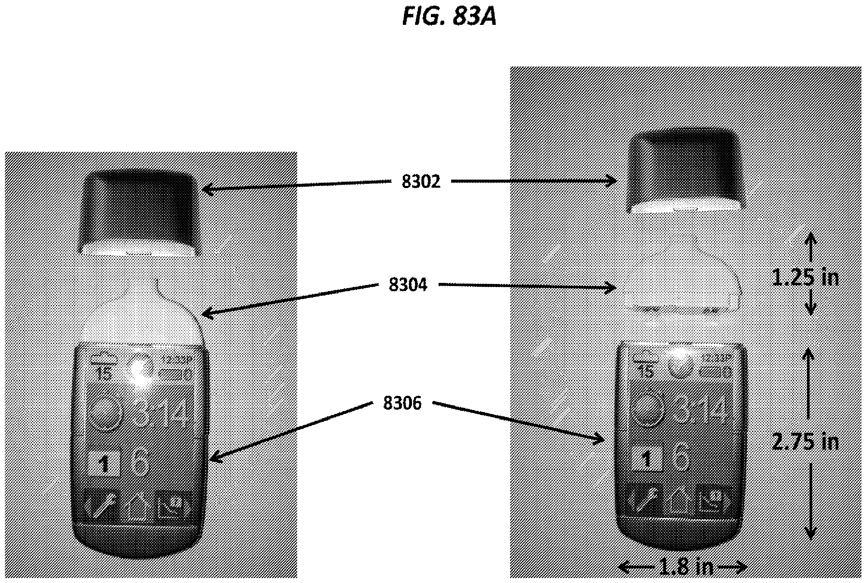

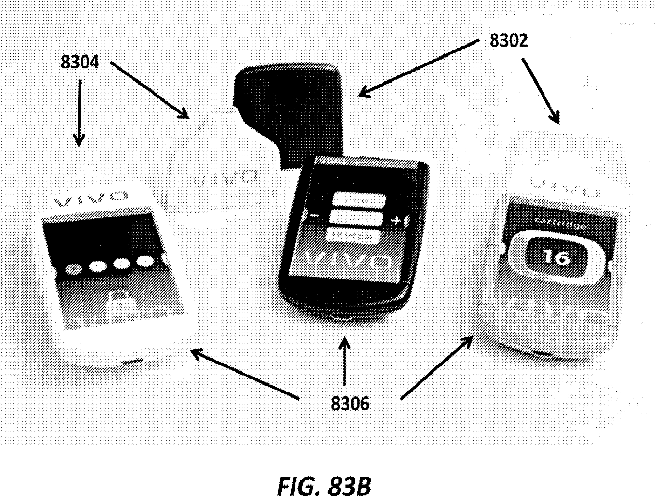

The devices described herein (e.g., FIGs. **83A-B, 86, 89,** or **91)** can be designed to not look like or resemble cigarettes or electronic cigarettes, and to not emit a visible or second hand vapor. The devices described herein can be designed to not glow like a cigarette. The devices provided herein can be designed to not comprise a light emitting diode (LED). The devices described herein can be designed to look like or resemble cigarettes or electronic cigarettes, and to not emit a visible or second hand vapor. The devices described herein can be designed to glow like a cigarette. The devices provided herein can be designed to comprise a light emitting diode (LED). The visible vapor can be an inhaled and/or exhaled vapor. The exhaled visible vapor can be referred to as a second-hand vapor. The subject can be a human. The human subject can be a smoker or an individual who uses tobacco or nicotine containing products. Devices described herein can generate an aerosol comprising an agent (e.g., nicotine), and the agent (e.g., nicotine) aerosol can have a known and consistent amount of agent (e g., nicotine). Also, devices and methods for dose titration are provided. The devices and methods provided herein can help to reduce smoking urges, reduce the amount of nicotine exposure as compared to use of cigarettes, reduce exposure to harmful and potentially harmful constituents, and/ or reduce smoking behavior or similariy to smoking behavior. Also, devices and methods provided herein can track usage and dependence by a user while also guiding said user toward goals using mobile health (mHealth or eHealth) tools.

The devices, systems, kits, compositions, and computer readable medium provided herein can be part of an electronic agent (e.g., nicotine) delivery platform. The electronic platform for delivering an agent (e.g., nicotine) can be used to deliver the agent (e.g., nicotine) to a subject in a particular dose, with a particular mean particle size, pH, and airflow characteristics, which can affect back of the throat impaction and upper airway deposition. In one embodiment, the electronic delivery platform regulates a schedule of delivery of an agent (e.g., nicotine) to a user over time. Furthermore, provided herein are methods of tracking usage of an agent (e.g., nicotine) to suggest a dosing strategy based on the goal or goals of the user of any device as provided herein. In some cases, a user is a human. In some cases, a user is a human who smokes or otherwise uses tobacco or a nicotine containing product.

Provided herein are devices for generating a condensation aerosol comprising particles of a size suitable for delivery to the lungs of a subject. In some cases, a subject is a human. In some cases, a subject is a human who smokes or otherwise uses tobacco or nicotine containing products. The particles can be of a size suitable for delivery to the deep lung (i.e., alveoli) of the subject. The particles can be any of the sizes provided herein. In some cases, the particles can comprise a mass median aerodynamic diameter (MMAD) of from about 1 to about 5 $\mu$m. The particles can have a geometric standard deviation (GSD) of less than 2. The condensation aerosol can be generated from a formulation comprising a pharmaceutically active agent. The formulation can be in a liquid or solid phase prior to vaporization. The agent can be any agent as provided herein; in some cases, the agent is nicotine, and in some cases the nicotine is stabilized using one or more carriers (e.g., vegetable glycerin and/or propylene glycol). The device can comprise a heater element as provided herein and a configuration of flow-through passages or chambers suitable for generating condensation aerosols comprising particles of a size suitable for delivery to the deep lungs of a subject. For example, a device can comprise a primary flow-through chamber in fluid communication with a secondary flow-through chamber. The primary flow-through chamber can comprise an upstream and downstream opening, and the upstream opening can be an inlet for a carrier gas. The device can comprise an aerosol generation chamber, wherein the aerosol generation chamber is located (disposed) between the upstream and downstream openings within the primary flow through chamber. The aerosol generation chamber can comprise a heater element as provided herein and a source of a formulation comprising a pharmaceutically active agent (e.g. nicotine) as provided herein. The aerosol generation chamber can further comprise a configuration whereby the flow rate of the carrier gas entering the aerosol generation chamber is effective to condense a vapor generated from a formulation comprising a pharmaceutically active agent (e.g. nicotine) as provided herein within the aerosol generation chamber.

Provided herein are devices for generating multiple populations of condensation aerosols. In some cases, the devices provided herein generate two populations of condensation aerosols. The first population of condensation aerosols comprise particles of a size suitable for delivery to the deep lungs of a subject. The first population of condensation aerosols suitable for delivery to the lungs of a subject can be non-visible. The second population of condensation aerosols comprise particles of a size suitable to be visible upon exhalation by the subject. Generation of the multiple populations of condensation aerosols from a device as provided herein can occur during a single use of device or between uses of the device. The generation of the multiple populations of condensation aerosols can be directly controlled by a user of the device. The generation of the multiple populations of condensation aerosols can be integrated into electronic circuitry of the device. The electronic circuitry can comprise a control program. The control program can comprise multiple phases such that each phase directs the device to produce a condensation aerosol comprising a specific size (e.g., diameter). The control program can be integrated into a controller. The controller can be programmable. Generation of the multiple populations of condensation aerosols from a device as provided herein can occur by altering an amount or volume of a liquid formulation comprising a pharmaceutically active agent (e.g., nicotine) delivered to or onto a heater element. The amount or volume of liquid formulation delivered can be altered by adjusting the pump rate of a device comprising a pump as provided herein. Alteration of the pump rate can be controlled by a user or by a control program of the device. Generation of the multiple populations of condensation aerosols from a device as provided herein can occur by altering an amount or volume of a carrier gas (e.g., air) flowing through an aerosol generation region of a the device. Alteration of the amount of volume of air can be accomplished by the number and/or size of air inlets configured to provide air inlets to the aerosol generation region of the device.

Provided herein are devices for generating a condensation aerosol comprising a reservoir comprising a liquid formulation comprising apharmaceutically active agent (e.g., nicotine) and a pump. The pump can be a positive displacement pump. In some cases, the pump is a diaphragm pump. In some cases, the pump is a piston pump. The pump can be located completely within the reservoir. The pump can be located patially within the reservoir. In some cases, the pump comprises a pump drive located outside of the reservoir. The pump drive can be located adjacent to the reservoir.The pump drive can be a wire coil. The piston pump can be magnetically coupled to the pump drive such that the piston comprises one or magnets while the pump drive comprises a wire coil. The piston of the piston pump can comprise 3 magnets. The magnet(s) in the piston pump can be magnetically coupled to the wire coil of the pump drive such that the magnetic coupling controls movement of the piston in the piston pump, thereby affecting delivery of the liquid formulation from the reservoir.

Devices and methods for aliquoting an agent (e.g., nicotine) to ensure dose-to-dose uniformity are provided herein. Furthermore, devices and methods are provided herein for sensing an inhalation by a user and triggering a device. Devices and methods are also provided herein for inhalation flow control.

Devices and methods of use of a closed loop design to control heating are provided herein. For example, a device provided herein can incorporate electronics that control for variability in battery condition and ensure consistent heating by direct measurement of resistance through the heater element to control for changes in battery voltage/charge.

eHealth tools provided herein can yield customized doses of an agent (e.g., nicotine) to a subject. In some cases, customized dosing regimens are provided, which can include instructions to dose at specific intervals, driven by reminders on the device. Devices and methods for providing customized feedback and behavioral support to a subject are also provided. In some cases, the customized feedback and/or behavioral support comprise simple instructions. The customized feedback and/or behavioral support can comprise use of social media to leverage social networks to help induce and/or maintain behavior change.

Also provided herein are methods of identifying individual user goals and matching user goals to an agent (e.g., nicotine) dose algorithm. Furthermore, provided herein are devices and methods for giving customized feedback to achieve a nicotine administration goal. Also, provided herein are devices and methods for giving customized feedback to achieve an agent administration goal. In some cases, an individual is a human. In some cases, an individual is a human who smokes or otherwise uses tobacco or a nicotine containing product.

## II. Devices

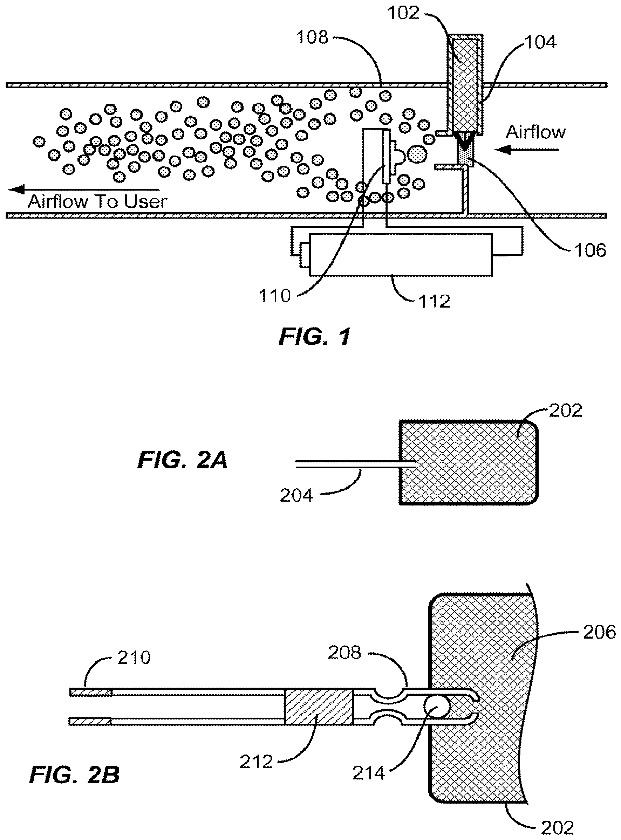

**FIG. 1** illustrates an embodiment of an electronic agent (e.g., nicotine) delivery device for controlling and reducing aerosol particle size for deep lung delivery and rapid pharmacokinetics. An agent, e.g., nicotine (102) is held in an agent (e.g., nicotine) reservoir (104), and can be wicked into a dosing mechanism (106). Upon inhalation, agent (e.g., nicotine) droplets are pulled out of the dosing mechanism. Small droplets are entrapped in airflow in the airway (108). A heater (110) can be in electrical communication with a battery (112). Larger droplets inertially impact with a heater (110), deposit, and are vaporized and reduced in size. Vapor condenses to form an optimum size aerosol by controlling airflow and vaporization rate. Any of the devices as provided herein can be rechargeable. Any of the devices as provided herein can be disposable. Any of the devices as provided herein can be rechargeable and comprise disposable components.

**Shape**

An electronic agent (e.g., nicotine) delivery device as provided herein can be disk-shaped, oval shaped, ovoid shaped, rectangular shaped, cyclindrically shaped, or triangular shaped. An electronic agent (e.g., nicotine) delivery device as provided herein can be in the shape of any smoking article known in the art. An electronic agent (e.g., nicotine) delivery device as provided herein can be in the shape of a cigarette, cigar, or smoking pipe.

**Agent doses**

An electronic agent (e.g., nicotine) delivery device provided herein can provide doses of agent (e.g., nicotine) in a consistent and known amount. A dose of an agent (e.g., nicotine) can about, more than, less than, or at least 1, 2, 3, 4, 5, 6, 7, 8, 9, 10, 11, 12, 13, 14, 15, 16, 17, 18, 19, 20, 21, 22, 23, 24, 25, 26 27, 28, 29, 30, 31, 32, 33, 34, 35, 36, 37, 38, 39, 40, 41, 42, 43, 44, 45, 46, 47, 48, 94, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000 $\mu$g of agent (e.g., nicotine). In some cases, a device can deliver a dose of an agent of about, more than, less than, or at least 1, 2, 3, 4, 5, 6, 7, 8, 9, 10, 11, 12, 13, 14, 15, 16, 17, 18, 19, 20, 21, 22, 23, 24, 25, 26 27, 28, 29, 30, 31, 32, 33, 34, 35, 36, 37, 38, 39, 40, 41, 42, 43, 44, 45, 46, 47, 48, 94, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 mg.

In one embodiment, a dose of an agent (e.g., nicotine) is about 1 $\mu$g to about 1000 $\mu$g, about 1 $\mu$g to about 500 $\mu$g, about 1 $\mu$g to about 1000 $\mu$g, about 10 $\mu$g to about 500 $\mu$g, about 20 $\mu$g to about 500 $\mu$g, about 25 $\mu$g to about 500 $\mu$g, about 30 $\mu$g to about 500 $\mu$g, about 40 $\mu$g to about 500 $\mu$g, about 50 $\mu$g to about 500 $\mu$g, about 10 $\mu$g to about 250 $\mu$g, about 20 $\mu$g to about 250 $\mu$g, about 30 $\mu$g to about 250 $\mu$g, about 40 $\mu$g to about 250 $\mu$g, about 50 $\mu$g to about 250 $\mu$g, about 1 $\mu$g to about 200 $\mu$g, about 10 $\mu$g to about 200 $\mu$g, about 20 $\mu$g to about 200 $\mu$g, about 30 $\mu$g to about 200 $\mu$g, about 40 $\mu$g to about 200 $\mu$g, about 50 $\mu$g to about 200 $\mu$g, about 25 $\mu$g to about 50 $\mu$g, about 25 $\mu$g to about 100 $\mu$g, about 25 $\mu$g to about 150 $\mu$g, about 25 $\mu$g to about 200 $\mu$g, about 25 $\mu$g to about 250 $\mu$g, about 25 $\mu$g to about 300 $\mu$g, about 25 $\mu$g to about 350 $\mu$g, about 25 $\mu$g to about 400 $\mu$g, about 25 $\mu$g to about 450 $\mu$g, about 25 $\mu$g to about 500 $\mu$g, about 50 $\mu$g to about 750 $\mu$g, or about 25 $\mu$g to about 1000 $\mu$g of agent (e.g., nicotine). In some cases, a dose of an agent is about 1 mg to about 100 mg, about 1 mg to about 50 mg, about 10 mg to about 50 mg, about 20 mg to about 50 mg, about 25 mg to about 50 mg, about 30 mg to about 50 mg, about 40 mg to about 50 mg, about 50 mg to about 100 mg, about 1 mg to about 25 mg, about 2 mg to about 25 mg, about 3 mg to about 25 mg, about 4 mg to about 25 mg, about 5 mg to about 25 mg, about 1 mg to about 20 mg, about 1 mg to about 20 mg, about 2 mg to about 20 mg, about 3 mg to about 20 mg, about 4 mg to about 20 mg, or about 5 mg to about 20 mg of agent.

An emitted dose of an agent (e.g., nicotine) can be about, more than, less than, or at least 1, 2, 3, 4, 5, 6, 7, 8, 9, 10, 11, 12, 13, 14, 15, 16, 17, 18, 19, 20, 21, 22, 23, 24, 25, 26 27, 28, 29, 30, 31, 32, 33, 34, 35, 36, 37, 38, 39, 40, 41, 42, 43, 44, 45, 46, 47, 48, 94, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 $\mu$g of agent (e.g., nicotine). In some cases, an emitted dose of an agent is about, more than, less than, or at least 1, 2, 3, 4, 5, 6, 7, 8, 9, 10, 11, 12, 13, 14, 15, 16, 17, 18, 19, 20, 21, 22, 23, 24, 25, 26 27, 28, 29, 30, 31, 32, 33, 34, 35, 36, 37, 38, 39, 40, 41, 42, 43, 44, 45, 46, 47, 48, 94, 50, 51, 52, 53, 54, 55, 56,

57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 mg. In one embodiment, an emitted dose of an agent (e.g., nicotine) is about 1 $\mu$g to about 1000 $\mu$g, about 1 $\mu$g to about 500 $\mu$g, about 1 $\mu$g to about 1000 $\mu$g, about 10 $\mu$g to about 500 $\mu$g, about 20 $\mu$g to about 500 $\mu$g, about 25 $\mu$g to about 500 $\mu$g, about 30 $\mu$g to about 500 $\mu$g, about 40 $\mu$g to about 500 $\mu$g, about 50 $\mu$g to about 500 $\mu$g, about 10 $\mu$g to about 250 $\mu$g, about 20 $\mu$g to about 250 $\mu$g, about 30 $\mu$g to about 250 $\mu$g, about 40 $\mu$g to about 250 $\mu$g, about 50 $\mu$g to about 250 $\mu$g, about 1 $\mu$g to about 200 $\mu$g, about 10 $\mu$g to about 200 $\mu$g, about 20 $\mu$g to about 200 $\mu$g, about 30 $\mu$g to about 200 $\mu$g, about 40 $\mu$g to about 200 $\mu$g, about 50 $\mu$g to about 200 $\mu$g, about 25 $\mu$g to about 50 $\mu$g, about 25 $\mu$g to about 100 $\mu$g, about 25 $\mu$g to about 150 $\mu$g, about 25 $\mu$g to about 200 $\mu$g, about 25 $\mu$g to about 250 $\mu$g, about 25 $\mu$g to about 300 $\mu$g, about 25 $\mu$g to about 350 $\mu$g, about 25 $\mu$g to about 400 $\mu$g, about 25 $\mu$g to about 450 $\mu$g, about 25 $\mu$g to about 500 $\mu$g, about 50 $\mu$g to about 750 $\mu$g, or about 25 $\mu$g to about 1000 $\mu$g (e.g., nicotine). In some cases, an emitted dose of an agent is about 1 mg to about 100 mg, about 1 mg to about 50 mg, about 10 mg to about 50 mg, about 20 mg to about 50 mg, about 25 mg to about 50 mg, about 30 mg to about 50 mg, about 40 mg to about 50 mg, about 50 mg to about 100 mg, about 1 mg to about 25 mg, about 2 mg to about 25 mg, about 3 mg to about 25 mg, about 4 mg to about 25 mg, about 5 mg to about 25 mg, about 1 mg to about 20 mg, about 1 mg to about 20 mg, about 2 mg to about 20 mg, about 3 mg to about 20 mg, about 4 mg to about 20 mg, or about 5 mg to about 20 mg of agent. In another embodiment, a device according to any of the embodiments described herein delivers only a single emitted dose of an agent (e.g., nicotine).

In some cases, the emitted dose can be about, more than, less than, or at least 1, 2, 3, 4, 5, 6, 7, 8, 9, 10, 11, 12, 13, 14, 15, 16, 17, 18, 19, 20, 21, 22, 23, 24, 25, 26 27, 28, 29, 30, 31, 32, 33, 34, 35, 36, 37, 38, 39, 40, 41, 42, 43, 44, 45, 46, 47, 48, 94, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100% of the dose (or loaded dose). In some cases, the emitted dose can be between 1%-10%, 10%-20%, 20%-30%, 30%-40%, 40%-50%, 50%-60%, 60%-70%, 70%-80%, 80%-90%, or 90%-100% of the dose (or loaded dose). In some cases, the emitted dose is more than 20% of the dose (or loaded dose). In some cases, the emitted dose is less than 20% of the dose (or loaded dose). The dose (or loaded dose) can be the amount of nicotine solution delivered onto the heater element prior to the creation of the aerosol and can be about 2% of the target dose (the label claimed dose or goal dose). The emitted dose can be 92% to 97% of the dose. For example, the amount actually delivered to the lung if the label claim dose is 100 $\mu$g can be between 90% and 99%.

## Dosing

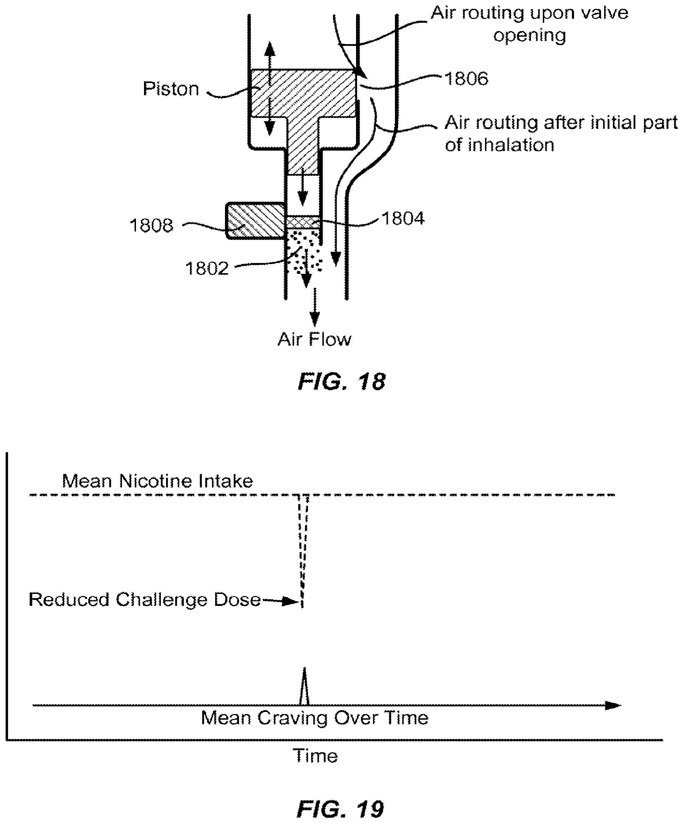

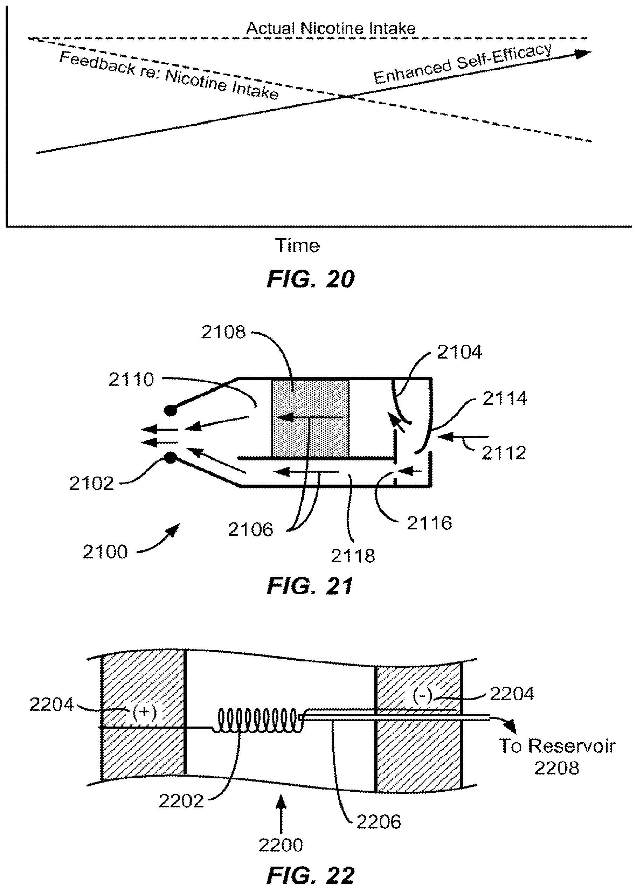

Provided herein are methods for administering an agent (e.g., nicotine) challenge doses to a subject. The administration of the challenge doses comprising nicotine can serve to reduce craving for nicotine in a subject using the device (see **FIG. 19).** In some cases, an electronic nicotine delivery device or web backend system as provided herein used in methods to administer an agent (e.g., nicotine) can give the user feedback regarding his/her mean nicotine dose, so as to enhance self-efficacy (see **FIG. 20).** In some cases, a subject is a human. In some cases, a subject is a human who smokes or otherwise uses tobacco or nicotine containing products. Methods are provided herein for generating condensation aerosols comprising particles comprising a mass median aerodynamic diameter (MMAD) effective for delivery to the deep lung of a subject. The condensation aerosols produced by devices as provided herein can provide a consistent nicotine delivery to a user of the device. The methods can comprise supplying or delivering a liquid formulation comprising a pharmaceutically active agent (e.g. nicotine) to a passageway; vaporizing the liquid formulation using a heater element in the passageway to produce a vaporized liquid formulation; and flowing a carrier gas through the passageway at a flow rate effective to allow condensation of the vaporized liquid formulation into particles comprising a size effective for delivery to the deep lung. The size of the particles following condensation can be an MMAD of from about 1 to about 5 $\mu$m. The flow rate can be about 1 to about 10 liters per minute (LPM) (a range from about 1.667 x $10^{-5}$ m$^3$/s to about 1.667 x $10^{-4}$ m$^3$/s), e.g., at a vacuum of about 1 to about 15 inches of water (a range from about 249 Pa to about 3738 Pa). The flow resistance of the device can be about 0.05 to about 0.15 (cm of $H_2O$)$^{1/2}$/LPM. The flow resistance of the device as provided herein for use in a method as provided herein can be about the same flow resistance as that of a combustible cigarette. The flow resistance through a device as provided herein for use in a method as provided herein can be around 2.5 (cm of $H_2O$)$^{1/2}$/LPM. In some cases, a device as provided herein for use in a method as provided herein comprises a flow rate of 1 LPM at a vacuum of 7.6 cm of $H_2O$. In some cases, a device as provided herein for use in a method as provided herein comprises a flow rate of 1.5 LPM at a vacuum of 16 cm of $H_2O$. In some cases, a device as provided herein for use in a method as provided herein comprises a flow rate of 2 LPM at a vacuum of 26 cm of $H_2O$. The liquid formulation can be supplied or delivered from a reservoir. The reservoir can comprise a tube, e.g., a capillary tube. The reservoir can be in fluid communication with the heater element.

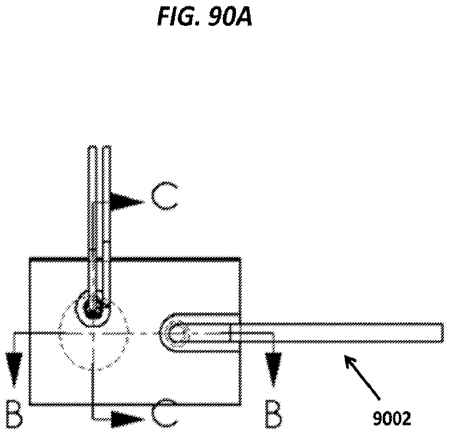

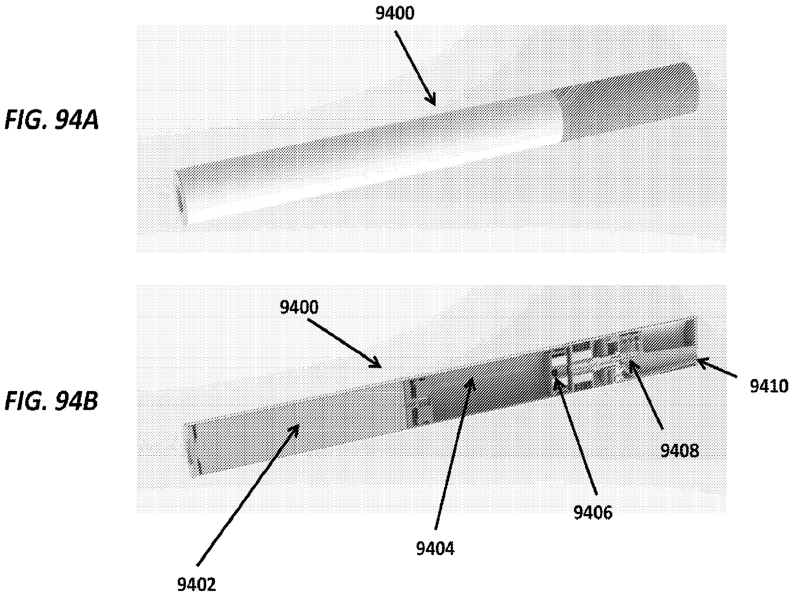

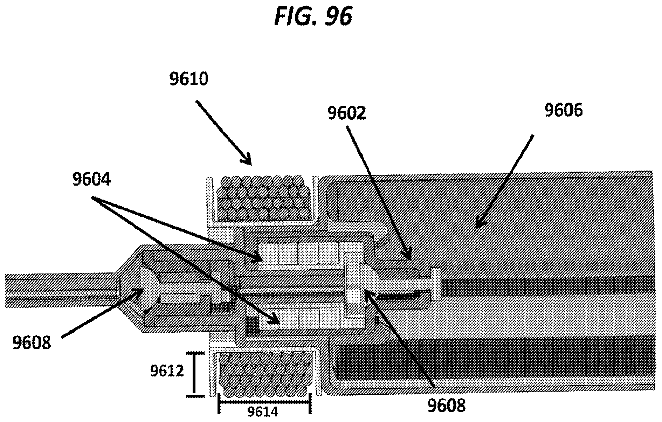

In some cases, the liquid formulation comprising a pharmaceutically active agent (e.g., nicotine) is delivered to the heater element through the use of a positive displacement pump. The positive displacement pump can be a reciprocating, metering, rotary-type, hydraulic, peristaltic, gear, screw, flexible impeller, diaphragm, piston, or progressive cavity pump, or any other pump utilizing positive displacement as known in the art. The positive displacement pump can be in fluid communication with the heater element. The positive displacement pump can be in fluid communication or fluidically coupled to a reservoir comprising a pharmaceutically active agent (e.g., nicotine). The positive displacement pump can be in fluid communication with the heater element and a reservoir comprising a pharmaceutically active agent (e.g., nicotine). The pharmaceutically active agent (e.g., nicotine) can be a liquid formulation. The pump (e.g., positive displacement pump) can be within the passageway or external to the passageway. The pump (e.g., positive displacement pump) can be fully or partially located within a reservoir comprising a liquid formulation comprising a pharmaceutically active agent (e.g., nicotine) in any device as provided herein. A drive motor for a pump (e.g., positive displacement pump) can be located external to a reservoir in a device as provided herein. In some cases, an aerosol generating device as provided herein comprises a pump housed or located within a reservoir comprising a liquid formulation comprising a pharmaceutically active agent (e.g., nicotine) and a drive motor located outside of the reservoir such that the drive motor is in mechanical communication with the pump. The drive motor can be a magnetic drive motor as shown in **FIG. 96.** The pump can be any pump as provided herein. In some cases, the pump is a piston pump as provided in **FIG. 94A-C** or **FIG. 96.** The pump can be a diaphragm pump as depicted in **FIG. 90A-D.**

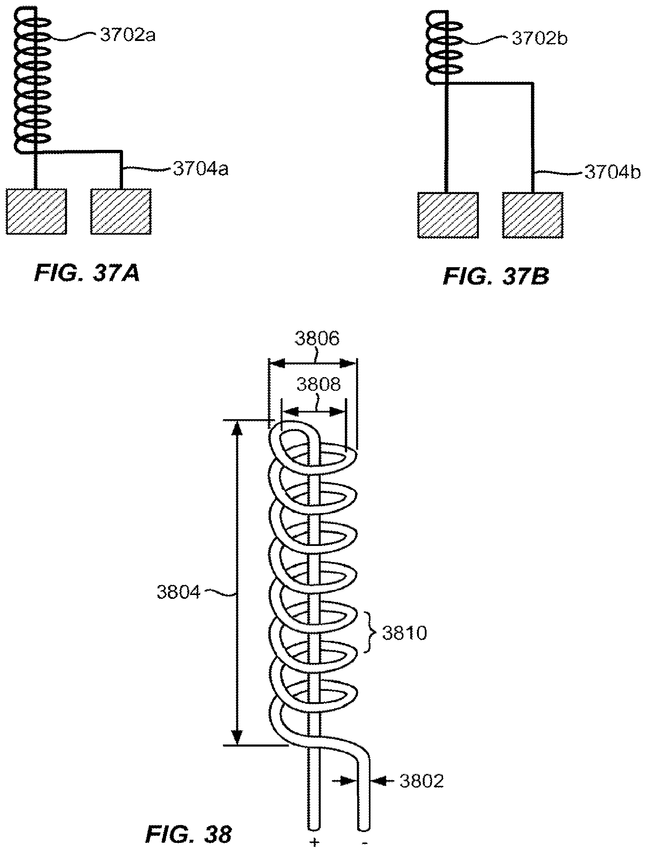

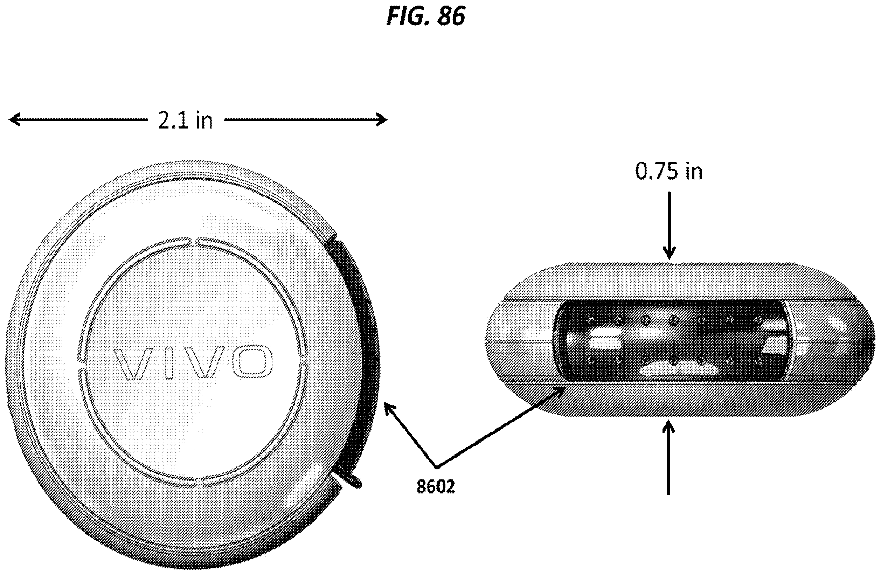

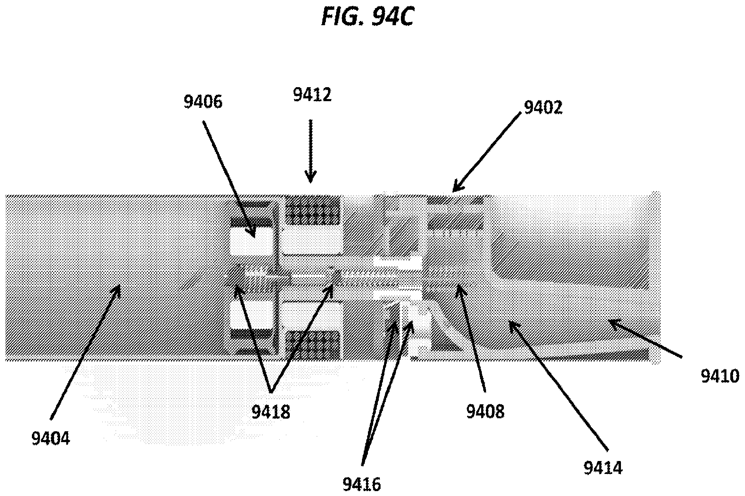

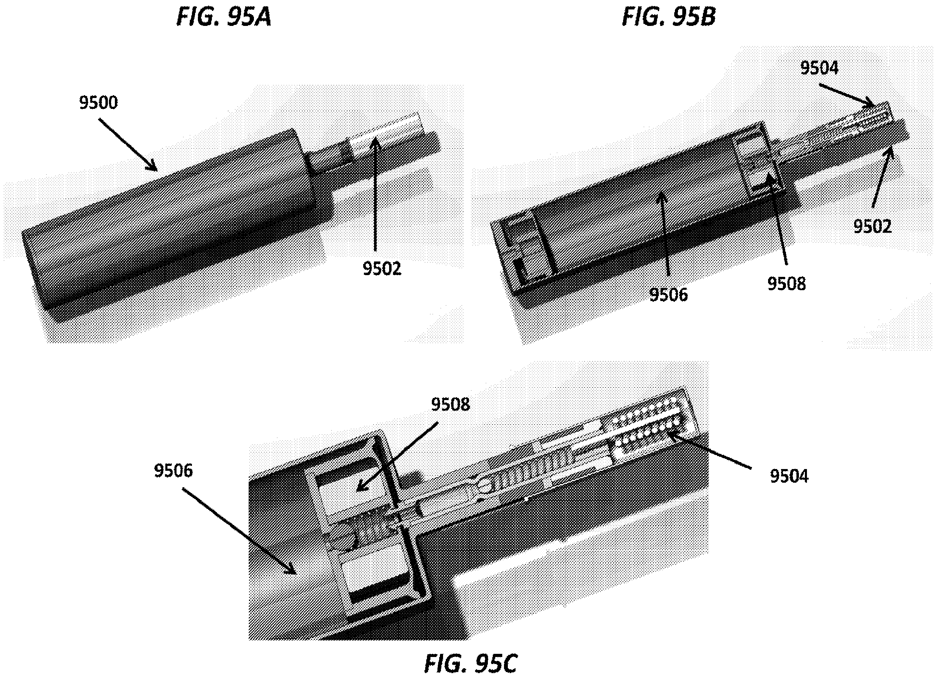

**FIG. 94A** illustrates an example of anaerosol generating device **(9400)** that is cylindrical in shape. As shown in **FIG. 94B,** the device of **FIG. 94A** comprises a battery **(9402)**, a nicotine reservoir **(9404)** comprising a liquid nicotine formulation as provided herein, a piston pump **(9406)** located within the nicotine reservoir **(9404),** a heater element **(9408)** and a mouthpiece **(9410).** A pump (e.g., piston or diaphragm) for use in an aerosol generating device as provided herein can be used to dispense a liquid formulation comprising a pharmaceutically active agent (e.g., nicotine) from a reservoir comprising the liquid formulation comprising a pharmaceutically active agent (e.g., nicotine) to a heater element. **FIG. 94C** illustrates a close up view of the mouthpiece end of the device in **FIGs. 94A** and **94B** and shows that the piston pump **(9406)** is flanked by check valves **(9418)** and is coupled to a pump drive **(9412)** located adjacent to but outside of the nicotine reservoir **(9404).** The pump (e.g., piston or diaphragm pump) can be mechanically or magnetically coupled to a pump drive. As can be seen, one of the check valves **(9418)** is located within the piston within the nicotine reservoir **(9404)** and can serve as an inlet of for entry of a volume of liquid from the reservoir **(9404)** to the pump **(9406)** for subsequent delivery to or onto the heater element **(9408).** Furthermore, the heater element **(9408)** comprises a coil and resides within an airway **(9414)** comprising an air inlet **(9402)** and an outlet (i.e., mouthpiece; **9410).** The nicotine reservoir **(9404)** can be any reservoir as provided herein. In some cases, the nicotine reservoir can hold the equivalent of 500 puffs (inhalations) (at the 4mg/puff). The nicotine reservoir can be part of a reservoir or cartridge as depicted in **FIG. 95A-C.** The heater element **(9408)** can be any heater element as described herein. In some cases, the heater element is a coil comprising electrically resistive material. An example of a suitable heater element comprising a coil that can be used is represented by the heater element depicted in **FIG. 38.** The piston pump **(9406)** can comprise a pump drive **(9412)** located outside of the nicotine reservoir **(9404)** such that it is coupled to and can control movement of the piston pump **(9406).** The piston pump can be mechanically coupled to the pump drive. The piston pump can be magnetically coupled to the pump drive such as shown in **FIG. 96.** The pump drive **(9412)** can be adjacent to the nicotine reservoir **(9404).** In operation, the pump drive **(9412)** can control the pump piston **(9406)** to deliver a volume of a liquid formulation comprising nicotine from the nicotine reservoir **(9404)** onto the heater element **(9408).** The heater element **(9408)** can vaporize the volume of liquid formulation delivered to it such that air flowing through the air inlet **(9402)** can serve to condense the vaporized liquid formulation into a condesation aerosol comprising a desired diameter within the airway **(9414)** prior to the condensation aerosol flowing through the mouthpiece **(9410).** The desired diameter can be any diameter provided herein. The desired diameter can be from about 1 μm to about 5 μm. The pump drive **(9412)** can comprise a magnetic drive motor. The magnetic drive motor can be a magnetic drive motor seated in an aerosol generating device as shown in **FIG. 96.** Alternatively, the aerosol generating device can be a disk-shaped device (e.g., the devices in **FIGs.86-89).** The pump can be designed to oscillate back and forth at a slow frequency (e.g., between 1 and 10 hz). The volume pumped per stroke can be determined by the preset stroke and diameter.

**FIG. 96** depicts an embodiment of a reservoir comprising a pharmaceucially active agent (e.g., nicotine **(9606))** for use in an aerosol generating device as provided herein. The reservoir in **FIG. 96** can be a single unit or component (see **FIG. 95)** that can be used in a multi-component aerosol generating device as described herein. As shown in **FIG. 96,** the pump drive **(9610)** can be located adjacent to the niotine reservoir **(9606).** The pump piston **(9602)** comprises magnets **(9604)** and check valves **(9608)** such that the magnets **(9604)** can be located between the check valves **(9608)** and can be used to control movement of the pump piston **(9602)** located partially within the nicotine reservoir **(9606).** The pump drive can comprise a wire coil.

A piston pump comprising magnets as illustrated in FIG. 96 can comprise 1, 2, 3, 4, 5, 6, 7, 8, 9 or 10 magnets. In some cases, a piston pump comprises 3 magnets. Each of the magnets in a piston pump comprising magnets can have an inner diameter (ID), an outer diameter (OD), and a length. The inner diameter can be 1, 2, 3, 4, or 5 mm. The inner diameter can be from 1mm to 2mm, 2 mm to 3 mm, 3mm to 4 mm, 4 mm to 5 mm, or 2 mm to 4 mm. The outer diameter can be 1, 2, 3, 4, 5, or 6 mm. The outer diameter can be from 1mm to 2mm, 2 mm to 3 mm, 3mm to 4 mm, 4 mm to 5 mm, 5 mm to 6 mm, or 3 mm to 6 mm. The length can be 1, 2, 3, 4, or 5 mm. The length can be from 1 mm to 2mm, 2 mm to 3 mm, 3mm to 4 mm, 4 mm to 5 mm, 2 mm to 4 mm, or from 1 mm to 5 mm. In some cases, the ID of a magnet in a piston pump comprising one or magnets is 3mm, while the OD is 4mm, and the length is 1 mm.

The pump rate of a piston pump (e.g., **FIG. 94** or **FIG. 96)** for use in anaerosol generating device as provided herein can be controlled by varying the voltage applied to the pump motor, the number of coils in a pump drive comprising wire coils, the gauge of the wire coil in a pump drive comprising wire coils, the size of the magnets (see **FIG. 96),** the travel distance of the piston, the diameter of the piston, and the frequency of the drive current applied to the pump. The pump rate of a pump in an aerosol generating device as provided herein (e.g., **FIG. 94** or **FIG. 96)** can be controlled. As provided herein, controlling the pump rate can be used to controll aerosol (e.g. condensation aerosol) size (e.g., diameter). The pump rate can less than, more than, at least, at most or about 0.1, 0.5, 1, 1.5, 2, 2.5, 3, 3.5, 4, 4.5, 5, 5.5, 6, 6.5, 7, 7.5, 8. 8.5, 9, 9.5, or 10 mg/second (mg/sec). The pump rate can be from about 0.1 to about 1, about 1 to about 2, about 2 to about 3, about 3 to about 4, about 4 to about 5, about 5 to about 6, about 6 to about 7, about 8 to about 9, about 9 to about 10, or about 0.1 to about 10 mg/sec. In some cases, the pump rate is 2 mg/sec.

The wire coil of a pump drive comprising a wire coil (e.g.,9610 in FIG. 96) can comprise from 25 to 50, 50 to 75, 75 to 100, 100 to 125, 125 to 150, 150 to 175, 175 to 200, 200 to 225, 225 to 250, 250 to 275, 275 to 300, 300 to 325, 325 to 350, 350 to 375, 375 to 400, 400 to 425, 425 to 450, 450 to 475, 475 to 500, 500 to 550, 550 to 600, 600 to 650, 650 to 700, 700 to 750, 750 to 800, 800 to 900, or 900 to 1000 coil turns. In some cases, the wire coil of a pump drive comprising a wire coil (e.g., 9610 in FIG. 96) comprises 350. In some cases, the wire coil of a pump drive comprising a wire coil (e.g., 9610 in FIG. 96) comprises from about 50 to about 500.

The gauge of the wire coil of a pump drive comprising a wire coil (e.g., 9610 in FIG. 96) can be from about 32 to about 38. In some cases, the gauge of the wire coil of a pump drive comprising a wire coil (e.g., 9610 in FIG. 96) is 36.

The wire coil of a pump drive comprising a wire coil (e.g.,9610 in FIG. 96) can comprise a depth (see 9612 in FIG. 96) of less than, more than, at least, at most or about 0.005, 0.01, 0.015, 0.02,0.025, 0.03, 0.035, 0.04, 0.045, 0.05, 0.055, 0.06, 0.065, 0.07, 0.075, 0.08, 0.085, 0.09, 0.095, 0.1, 0.2, 0.3, 0.4, 0.5, 0.6, 0.7, 0.8, 0.9 or 1 inches. The wire coil of a pump drive comprising a wire coil (e.g., 9610 in FIG. 96) can comprise a depth of from about 0.01 to about 0.05, about 0.01 to about 0.1, about 0.01 to about 0.5, about 0.01 to about 1, about 0.05 to about 0.1, about 0.05 to about 0.5, about 0.05 to about 1, about 0.1 to about 0.5, or about 0.1 to about 1 inches. In some cases, the wire coil of a pump drive comprising a wire coil (e.g., 9610 in FIG. 96) has a depth of 0.055 inches.

The wire coil of a pump drive comprising a wire coil (e.g., 9610 in FIG. 96) can comprise a width (see 9614 in FIG. 96) of less than, more than, at least, at most or about0.05, 0.055, 0.06, 0.065, 0.07, 0.075, 0.08, 0.085, 0.09, 0.095, 0.1, 0.125, 0.150, 0.175, 0.2, 0.225, 0.250, 0.275, 0.3, 0.325, 0.350, 0.375, 0.4, 0.425, 0.450, 0.475, 0.5, 0.525, 0.550, 0.575, 0.6, 0.625, 0.650, 0.675, 0.7, 0.725, 0.750, 0.775, 0.8, 08125, 0.850, 0.875, 0.9, 0.925, 0.950, 0.975, or 1 inches. The wire coil of a pump drive comprising a wire coil (e.g., 9610 in FIG. 96) can comprise a width of from about 0.05 to about 0.1, about 0.05 to about 0.2, about 0.05 to about 0.3, about 0.1 to about 0.2, about 0.1 to about 0.3, or about 0.2 to about 0.3 inches. In some cases, the wire coil of a pump drive comprising a wire coil (e.g., 9610 in FIG. 96) has a width of 0.175 inches.

The wire coil of a pump drive comprising a wire coil (e.g.,9610 in FIG. 96) can be driven at a frequency of less than, more than, at least, at most, or about 1, 2, 3, 4, 5, 6, 7, 8, 9, 10, 11, 12, 13, 14, 15, 16, 17, 18, 19, 20, 21, 22, 23, 24, 25, 26 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 Hertz (Hz). In some cases, the wire coil is driven at a frequency of 2 Hz. The drive of the pump can be an electrical. The electrical drive can comprise a waveform. The waveform can be a square wave, sign wave or saw wave. In some cases, the electrical drive waveform is a square waveform.

The diameter of a piston in a piston pump (e.g., see **FIG 94A-C** and **FIG.** 96) in an aerosol generating device as provided herein can be less than, more than, at least, at most or about 0.5, 0.55, 0.6, 0.65, 0.7, 0.75, 0.8, 0.85, 0.9, 0.95, 0.1, 0.150, 0.2, 0.250, 0.3, 0.350, 0.4, 0.450, 0.5, 0.550, 0.6, 0.650, 0.7, 0.750, 0.8, 0.850, 0.9, 0.950, 1, 1.5, 2, 2.5, 3, 3.5, 4, 4.5, or 5 mm. The diameter of a piston in a piston pump (e.g., see **FIG 94A-C** and **FIG. 96)** in an aerosol generating device as provided herein can be from about 0.5 to about 1, about 0.5 to about 2, about 0.5 to about 3, about 1 to about 2, about 1 to about 3, or about 2 to about 3mm. In some cases, the wire coil of a pump drive comprising a wire coil (e.g.,9610 in FIG. 96) is from about 0.75 mm to 2 mm. In some cases, the diameter of the piston is 1 mm. The distance a piston in a piston pump (e.g., see **FIG 94A-C** and **FIG. 96)** travels in an aerosol generating device as provided herein can be less than, more than, at least, at most or about 0.1, 0.2, 0.3, 0.4, 0.5, 0.6, 0.7, 0.8, 0.9, 1, 1.5, 2, 2.5, 3, 3.5, 4, 4.5, or 5 mm. The distance a piston in a piston pump (e.g., see **FIG 94A-C** and **FIG. 96)** travels in an aerosol generating device as provided herein can be from about 0.1 to about 1, about 0.1 to about 2, about 0.1 to about 3, about 1 to about 2, about 1 to about 3, or about 2 to about 3mm. In some cases, the distance a piston in a piston pump (e.g., see **FIG 94A-C** and **FIG. 96)** travels in an aerosol generating device as provided herein is 2mm.

The voltage applied to a pump for an aerosol generating device as applied herein (e.g., 9610 in FIG. 96) can be less than, more than, at least, at most or about 1, 2, 3, 4, 5, 6, 7, 8, 9, or 10 volts. The voltage applied to a pump as provided herein can be from about 1 to about 2, about 1 to about 3, about 1, to about 4, about 1 to about 5, about 1 to about 6, about 1 to about 7, about 1 to about 8, about 1 to about 9, about 1 to about 10, about 2 to about 4, about 4 to about 6, about 6 to about 8, or about 8 to about 10. In some cases, the voltage applied to a pump in an aerosol generating device as provided herein is 3 volts. The pump can be a piston pump (e.g., FIG. 94A-C or FIG. 96). The pump can be a diaphragm pump (e.g., FIG. 90A-D).

In some cases, an aerosol generating device as provided herein (e.g., 9400 in FIGs. 94A-C) comprises a piston pump comprising a magnetic drive coil (e.g., FIG. 96). In some cases, the magnetic drive coil comprises three magnets such that the each magent has a size of 3 mm ID, 4 mm OD and a length of 1 mm (total length of the 3 magnets is 3mm). The coil can be designed to maximize the turns of the coil to the voltage available and the current desired. In some cases, the device comprises a single cell Lithium battery and provides 3.0 volts, while the current provided is about 0.2 amps. In some cases, the wire coil is 36 gage. In some cases, the wire coil has a width of 0.175 in. and a depth of about 0.055 inches such that the wire coil has a resistance of about 10 Ohms. Application of 3 volts to the wire coil comprising a resistance of about 10 Ohms can produce a current of .33 Amps. In some cases, a current of 0.33 Amps is sufficient to drive the piston in a piston pump as provided herein. In some cases, the wire coil is driven with a square wave at a frequency of about 2 Hz.

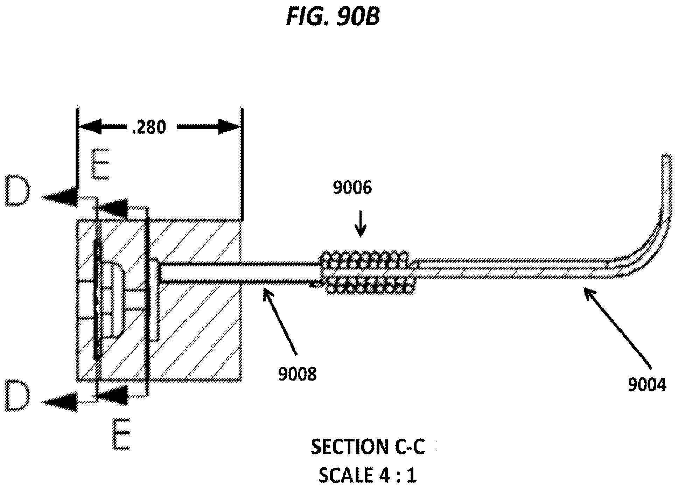

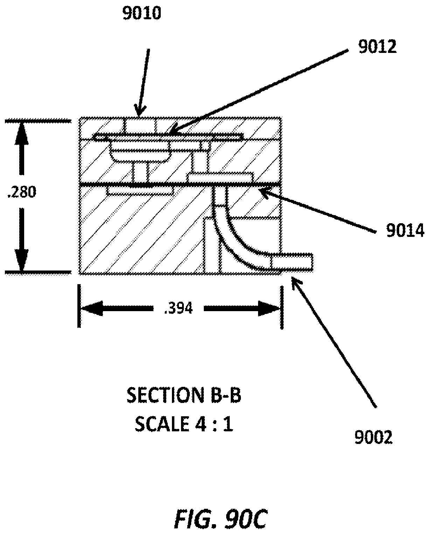

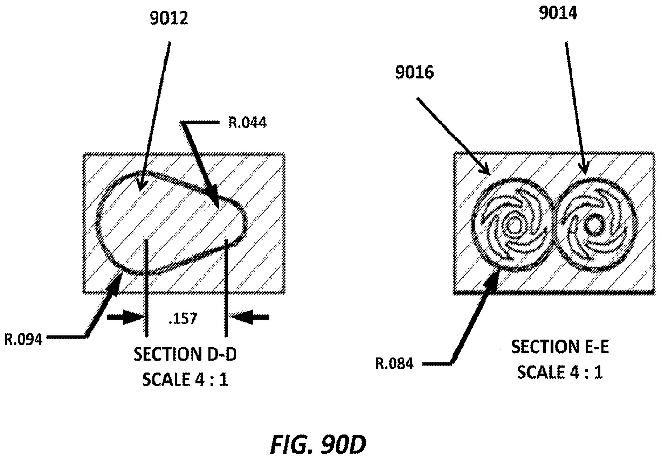

The pump in an aerosol generating device as provided herein that comprises a pump housed or located within a reservoir comprising a liquid formulation comprising a pharmaceutically active agent (e.g., nicotine) can be a diaphragm pump as depicted in **FIG. 90A-D.** The aerosol generating device can be a disk-shaped device (e.g., the devices in **FIGs.86-89).** The aerosol generating device can be a cylindrical device (e.g., the devices in **FIG. 94A-C).** The cylindrical device can resemble a cigarette. **FIG. 90A** illustrates a diaphragm pump comprising a sub-assembly, while **FIGs. 90B** and **90C** depict interior views of **FIG. 90A** sectioned along the C-C and B-B axes, respectively. **FIG. 90D** depicts internal views of **FIG. 90B** sectioned the D-D and E-E lines. In some cases, the diaphragm pump sub assembly in **FIGs. 90A-D** comprises two check valves (inlet valve **9014;** outlet valve **9016),** a pump diaphragm **(9012),** and an activation hole for a linear push **(9010)** for a motor (e.g., linear motor) as well as a heater element **(9006)** and electrical leads **(9004)** for providing power to the heater element **(9006).** One **(9014)** of the check valves is on an inlet tube **(9002)** of the diaphragm pump, while the other **(9016)** of the check valves is on an outlet tube **(9008)** of the diaphragm pump. The check valves **(9014; 9016)** can ensure that the pump stays primed and that the correct volume of material can be displaced. The 2 check valves can be made from an elastomeric material or, alternatively, a thin stiff material. The material can have slots cut in it so that the material can deflect out of the way of the opening allowing liquid material (e.g., liquid formulation comprising nicotine) to pass. The valve material can then close (seat) on the opening. The pump can be powered through the activation hole **(9010)** by a linear motion from either an electrical solenoid or a crank or cam powered by a rotational motion from a motor. Additionally the motion can be from the release of a cocked mechanical mechanism that can be cocked by the user. Inhalation can serve to cause the stored mechanical energy to be released. For example a spring can be compressed and upon inhalation the spring can be released. The chamber for the diaphragm pump can be designed so that the volume of the chamber can be the volume that needs to be pumped. As shown in **FIG. 90A-D,** a pump sub-assembly can be rectangular in shape with an inlet and outlet port. One side of the pump sub assembly can be about 0.280 inches in length, and another side of the pump sub assembly can be about 0.394 inches in length. In some cases, an outlet of the pump sub assembly is connected to an outlet tube **(9008),** which can be a capillary,that is configured to deliver liquid material (e.g., liquid nicotine formulation) to a heater element **(9006)** powered by electrical leads **(9004).** The heater element **(9006)** can be a coil (e.g., wire coil). The heater element can be any heater element as provided herein. The carrier gas can be air. The pump can be designed to oscillate back and forth at a slow frequency (e.g., between 1 and 50 hz). The volume pumped per stroke can be determined by the preset stroke and diameter.

Methods for aliquoting an agent (e.g., nicotine) to ensure dose-to-dose uniformity are provided herein. For example, an element comprising porous materials can wick out fluid comprising agent (e.g., nicotine) at a particular rate in order to measure out a dose to provide dose-to-dose uniformity. A tube, e.g., a capillary tube can be used to measure out a dose. In one embodiment, heat is used as a means of ejecting a dose. A material or geometry of a device can be used to measure out a dose. In one embodiment, providing dose consistency controls for variability in environment and device. In another embodiment, inhalation flow control ensures that variability in inhalations by a user are controlled and corrected for, which can result in dose-to-dose consistency and predictable and desirable aerosol particle sizes.

In some cases, an agent (e.g., nicotine) is metered out into a prevaporization area in a device (dosing mechanism) through capillary action. The metering can occur between inhalations of a user of a device. Upon inhalation by a subject, an agent (e.g., nicotine) can be drawn into a vaporization chamber or onto a heater element. The agent can be a pharmaceutically active agent. The agent can be in a formulation that is liquid. The liquid formulation comprising a pharmaceutically active agent (e.g., nicotine) can be drawn or metered out into a vaporization chamber or onto a heater element upon inhalation by a subject. The subject can be a human. The human subject can be a smoker or user of tobacco or nicotine containing substances. The agent (e.g., nicotine) in the vaporization chamber or heater element can be vaporized and subsequently condense to form an aerosol. The aerosol can comprise agent (e.g., nicotine) particles of an optimum size to achieve certain biological effects (e.g., deep lung delivery producing rapid pharmacokinetics).

Devices described herein can comprise a mechanism for separating out and reducing large aerosol particles to a size that can navigate to the deep lung of a subject. In the deep lung, the particles can settle and be rapidly absorbed. Also provided herein are methods for controlling aerosol particle size, pH, and other inhalation characteristics, which can ensure deep lung delivery and rapid pharmacokinetics. For example, the aerosol size control can result in rapid, cigarette-like nicotine absorption, which can help to satisfy nicotine cravings. In some cases, aerosol particles comprising nicotine produced by a heater element or device as provided herein can achieve peak plasma concentrations similar to peak plasma concentrations achieved by smoking a cigarette. In some cases, aerosol particles comprising nicotine produced by a heater element or device as provided herein can achieve peak plasma concentrations in a time frame similar to the time frame required to achieve peak plasma concentrations achieved by smoking a cigarette. The condensation aerosol comprising nicotine produced by any of the devices provided herein can result in rapid, cigarette-like nicotine absorption resulting in nicotine blood, serum or plasma concentrations similar or substantially similar to the nicotine blood, serum or plasma concentration achieved from smoking a cigarette. In some cases, the plasma concentration can be an arterial plasma concentration. In some cases, the plasma concentration can be a venous plasma concentration. Smoking a single cigarette can produce peak increments of plasma nicotine concentration of 5-30 ng/ml. In some cases, the blood concentration can be an arterial blood concentration. In some cases, the blood concentration can be a venous blood concentration.

In some cases, a device as provided hereinfor generating a condensation aerosol comprising nicotine produces a blood, serum or plasma nicotine concentration in the user of the device of about 0.5 ng/mL to about 200 ng/mL, about 0.5 ng/mL to about 150 ng/mL, about 0.5 ng/mL to about 100 ng/mL, about 0.5 ng/mL to about 75 ng/mL, about 0.5 ng/mL to about 50 ng/mL, about 0.5 ng/mL to about 40 ng/mL, about 0.5 ng/mL to about 30 ng/mL, about 0.5 ng/mL to about 20 ng/mL, about 0.5 ng/mL to about 10 ng/mL, about 0.5 ng/mL to about 5 ng/mL, about 0.5 ng/mL to about 1 ng/mL, about 10 ng/mL to about 200 ng/mL, about 10 ng/mL to about 150 ng/mL, about 10 ng/mL to about 100 ng/mL, about 10 ng/mL to about 75 ng/mL, about 10 ng/mL to about 50 ng/mL, about 10 ng/mL to about 40 ng/mL, about 10 ng/mL to about 30 ng/mL, about 10 ng/mL to about 20 ng/mL, about 10 ng/mL to about 15 ng/mL, about 20 ng/mL to about 200 ng/mL, about 20 ng/mL to about 150 ng/mL, about 20 ng/mL to about 100 ng/mL, about 20 ng/mL to about 75 ng/mL, about 20 ng/mL to about 50 ng/mL, about 20 ng/mL to about 40 ng/mL, about 20 ng/mL to about 30 ng/mL, about 20 ng/mL to about 24 ng/mL, about 30 ng/mL to about 200 ng/mL, about 30 ng/mL to about 150 ng/mL, about 30 ng/mL to about 100 ng/mL, about 30 ng/mL to about 75 ng/mL, about 30 ng/mL to about 50 ng/mL, about 30 ng/mL to about 40 ng/mL, or about 30 ng/mL to about 35 ng/mL, 0.1 ng/mL to about 20 ng/mL, 0.1 ng/mL to about 15 ng/mL, 0.1 ng/mL to about 10 ng/mL, about 0.1 ng/mL to about 5 ng/mL, about 0.1 ng/mL to about 2 ng/mL, about 0.1 ng/mL to about 1 ng/mL, about 0.1 ng/mL to about 0.5 ng/mL. In some cases, a device as provided herein for generating a condensation aerosol comprising nicotine produces a blood, serum or plasma nicotine concentration in a user of the device of about, more than, less than, or at least 0.1, 0.2, 0.3, 0.4, 0.5, 0.6, 0.7, 0.8, 0.9, 1, 1.25, 1.5, 1.75, 2, 2.25, 2.5, 2.75, 3, 3.25, 3.5, 3.75, 4, 4.25, 4.5, 4.75, 5, 5.5, 6, 6.5, 7, 7.5, 8, 8.5, 9, 9.5, 10, 11, 12, 13, 14, 15, 16, 17, 18, 19, 20, 21, 22, 23, 24, 25, 26 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 ng/mL. The blood, serum or plasma nicotine concentration can be produced after inhaling from the device a plurality of times. The plurality of times can be 2, 3, 4, 5, 6, 7, 8, 9, or 10 times from the condensation aerosol generating device. The blood, serum or plasma nicotine concentration can be arterial or venous. In some cases, a blood, serum or plasma nicotine concentration of from about 0.5 ng/ml to about 2 ng/ml is produced following a plurality of doses or inhalations from a device provided herein for generating a condensation aerosol comprising nicotine. In some cases, a blood, serum or plasma nicotine concentration of from about 1 ng/ml to about 2 ng/ml is produced following a plurality of doses or inhalations from a device provided herein for generating a condensation aerosol comprising nicotine. In some cases, a blood, serum or plasma nicotine concentration of from about 1 ng/ml to about 5 ng/ml is produced following a plurality of doses or inhalations from a device provided herein for generating a condensation aerosol comprising nicotine. In some cases, a blood, serum or plasma nicotine concentration of from about 0.5 ng/ml to about 1 ng/ml is produced following a plurality of doses or inhalations from a device provided herein for generating a condensation aerosol comprising nicotine. In some cases, a blood, serum or plasma nicotine concentration of from about 0.5 ng/ml to about 1.5 ng/ml is produced following a plurality of doses or inhalations from a device provided herein for generating a condensation aerosol comprising nicotine. The plurality of doses or inhalations can be 2 to 10 doses or inhalations. The plurality of doses or inhalations can be 10 doses or inhalations. The amount of nicotine per dose or inhalation can be 25, 50, 75, or 100 $\mu$g. In some cases, a blood, serum or plasma nicotine concentration of from about 0.5 ng/ml to about 5 ng/ml is produced following 10 inhalations from a device provided herein for generating a condensation aerosol comprising nicotine, wherein the condensation aerosol comprising nicotine comprises 25 $\mu$g of nicotine. In some cases, a blood, serum or plasma nicotine concentration of from about 0.5 ng/ml to about 2 ng/ml is produced following 10 inhalations from a device provided herein for generating a condensation aerosol comprising nicotine, wherein the condensation aerosol comprising nicotine comprises 50 $\mu$g of nicotine. In some cases, a blood, serum or plasma nicotine concentration of from about 0.5 ng/ml to about 1.5 ng/ml is produced following 10 inhalations from a device provided herein for generating a condensation aerosol comprising nicotine, wherein the condensation aerosol comprising nicotine comprises 50 µg of nicotine. In some cases, a blood, serum or plasma nicotine concentration of from about 0.5 ng/ml to about 1 ng/ml is produced following 10 inhalations from a device provided herein for generating a condensation aerosol comprising nicotine, wherein the condensation aerosol comprising nicotine comprises 50 µg of nicotine. In some cases, a blood, serum or plasma nicotine concentration of from about 0.5 ng/ml to about 5 ng/ml is produced following 10 inhalations from a device provided herein for generating a condensation aerosol comprising nicotine, wherein the condensation aerosol comprising nicotine comprises 75 µg of nicotine. In some cases, a blood, serum or plasma nicotine concentration of from about 0.5 ng/ml to about 2 ng/ml is produced following 10 inhalations from a device provided herein for generating a condensation aerosol comprising nicotine, wherein the condensation aerosol comprising nicotine comprises 100 µg of nicotine.

In some cases, a device as provided herein can produce a plasma, serum or blood nicotine concentration in a user of the device within about 1 second to about 30 minutes, about 1 second to 20 minutes, about 1 second to 10 minutes, about 1 second to 5 minutes, about 1 second to 2 minutes, about 1 second to 1 minute, about 1 second to about 30 seconds, about 30 seconds to 30 minutes, about 30 seconds to 20 minutes, about 30 seconds to 10 minutes, about 30 seconds to 5 minutes, about 30 seconds to 2 minutes, about 30 seconds to about 1 minute, about 1 minute to about 30 minutes, about 1 minute to about 25 minutes, about 1 minute to about 20 minutes, about 1 minute to about 15 minutes, about 1 minute to about 10 minutes, about 5 minutes to about 30 minutes, about 5 minutes to about 25 minutes, about 5 minutes to about 20 minutes, about 5 minutes to about 15 minutes, about 5 minutes to about 10 minutes, about 10 minutes to about 30 minutes, about 10 minutes to about 25 minutes, about 10 minutes to about 20 minutes, or about 10 minutes to about 15 minutes of use of the device. The plasma, serum or blood nicotine concentration can be a peak concentration or an average concentration. A use of the device can be an inhalation of a dose delivered by the device. In some cases, a device as provided herein can produce a plasma, serum or blood nicotine concentration in a user of the device in less than, more than, or about 30 minutes, 20 minutes, 15 minutes, 10 minutes, 5 minutes, 2 minutes, 1 minute, 30 seconds, 15 seconds, 10 seconds, or 5 seconds. The plasma, serum or blood nicotine concentration in a user of a device as provided herein can be sustained for about, or more than 5 minutes, 10 minutes, 15 minutes, 20 minutes, 25 minutes, 30 minutes, 45 minutes, 60 minutes, 75 minutes, 90 minutes, 120 minutes, 150 minutes, 180 minutes, or 360 minutes. The blood, serum or plasma nicotine concentration can be arterial or venous. The plasma, serum or blood nicotine concentration can be a peak concentration or an average concentration. In some cases, a blood, serum or plasma nicotine concentration of from about 1 ng/ml to about 3 ng/ml is produced in less than 10 minutes following a plurality of doses or inhalations from a device provided herein for generating a condensation aerosol comprising nicotine. In some cases, a blood, serum or plasma nicotine concentration of from about 0.5 ng/ml to about 4 ng/ml is produced in less than 1 minutes following a plurality of doses or inhalations from a device provided herein for generating a condensation aerosol comprising nicotine. In some cases, a blood, serum or plasma nicotine concentration of from about 1 ng/ml to about 4 ng/ml is produced in less than 1 minute following a plurality of doses or inhalations from a device provided herein for generating a condensation aerosol comprising nicotine. The blood, serum or plasma nicotine concentration can be arterial or venous. In some cases, a blood, serum or plasma nicotine concentration of from about 0.5 ng/ml to about 4 ng/ml is produced in about 30 seconds following a plurality of doses or inhalations from a device provided herein for generating a condensation aerosol comprising nicotine. In some cases, a blood, serum or plasma nicotine concentration of from about 1 ng/ml to about 4 ng/ml is produced in about 30 seconds following a plurality of doses or inhalations from a device provided herein for generating a condensation aerosol comprising nicotine. The plurality of doses or inhalations can be 2 to 10 doses or inhalations. The plurality of doses or inhalations can be 10 doses or inhalations. The amount of nicotine per dose or inhalation can be 25, 50, 75, or 100 µg. In some cases, a blood, serum or plasma nicotine concentration of from about 0.5 ng/ml to about 4 ng/ml is produced in less than 1 minute following 10 inhalations from a device provided herein for generating a condensation aerosol comprising nicotine, wherein the condensation aerosol comprising nicotine comprises 25, 50, 75, or 100 µg of nicotine. In some cases, a blood, serum or plasma nicotine concentration of from about 0.5 ng/ml to about 4ng/ml is produced in about 30 seconds following 10 inhalations from a device provided herein for generating a condensation aerosol comprising nicotine, wherein the condensation aerosol comprising nicotine comprises 25, 50, 75, or 100 µg of nicotine.

The plasma, serum or blood nicotine concentration produced in a user of device as provided herein for generating a condensation aerosol comprising nicotine can be substantially similar to the plasma, serum or blood nicotine concentration from a subject smoking a cigarette. The plasma, serum or blood nicotine concentration can be a peak plasma, serum or blood nicotine concentration, wherein the peak plasma, serum or blood nicotine concentration from smoking a cigarette can be achieved within 10 minutes. The plasma, serum or blood nicotine concentration can be an average concentration. The nicotine blood, serum or plasma concentration can be about, more than, less than, or at least 1%, 2%, 3%, 4%, 5%, 6%, 7%, 8%, 9%, 10%, 11%, 12%, 13%, 14%, 15%, 16%, 17%, 18%, 19%, 20%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%,

93%, 94%, 95%, 96%, 97%, 98%, 99%, or 100% of the nicotine blood, serum or plasma concentration achieved by smoking a cigarette. The nicotine blood, serum or plasma concentration can be between 1%-10%, 10%-20%, 20%-30%, 30%-40%, 40%-50%, 50%-60%, 60%-70%, 70%-80%, 80%-90%, or 90%-100% of the nicotine blood, serum or plasma concentration achieved by smoking a cigarette. The nicotine blood, serum or plasma concentration can be about 1% to about 10%, about 10% to about 20%, about 20% to about 30%, about 30% to about 40%, about 40% to about 50%, about 50% to about 60%, about 60% to about 70%, about 70% to about 80%, about 80% to about 90%, or about 90% to about 100% of the nicotine blood, serum or plasma concentration achieved by smoking a cigarette. Smoking or using a single cigarette can produce blood, serum or plasma nicotine concentrations of 5-30 ng/ml. The blood, serum or plasma concentrations of 5-30 ng/ml can be peak increments. The blood, serum or plasma nicotine concentration can be arterial or venous. In some cases, use of a device for generating a condensation aerosol comprising nicotine as provided herein by a subject produces a plasma, serum or blood nicotine concentration in the subject that is substantially less than the blood, serum or plasma nicotine concentration in the subject following use of or smoking a cigeratte.

The plasma, serum or blood nicotine concentration produced in a user of device as provided herein for generating a condensation aerosol comprising nicotine can be substantially similar to the plasma, serum or blood nicotine concentration from a subject smoking, using or vaping from an electronic cigarette. The plasma or blood nicotine concentration can be a peak plasma, serum or blood nicotine concentration, wherein the peak plasma, serum or blood nicotine concentration from smoking, using or vaping from an electronic cigarette can be achieved within 1 or more puffs. The plasma, serum or blood nicotine concentration can be an average concentration. The nicotine blood, serum or plasma concentration can be about, more than, less than, or at least 1%, 2%, 3%,4%,5%, 6%, 7%, 8%, 9%, 10%, 11%, 12%, 13%, 14%, 15%, 16%, 17%, 18%, 19%, 20%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% of the nicotine blood, serum or plasma concentration achieved by smoking, using or vaping from an electronic cigarette. The nicotine blood, serum or plasma concentration can be between 1%-10%, 10%-20%, 20%-30%, 30%-40%, 40%-50%, 50%-60%, 60%-70%, 70%-80%, 80%-90%, or 90%-100% of the nicotine blood, serum or plasma concentration achieved by smoking, using or vaping from an electronic cigarette. The nicotine blood, serum or plasma concentration can be about 1% to about 10%, about 10% to about 20%, about 20% to about 30%, about 30% to about 40%, about 40% to about 50%, about 50% to about 60%, about 60% to about 70%, about 70% to about 80%, about 80% to about 90%, or about 90% to about 100% of the nicotine blood, serum or plasma concentration achieved by smoking, using or vaping from an electronic cigarette. The blood, serum or plasma nicotine concentration can be arterial or venous. In some cases, use of a device for generating a condensation aerosol comprising nicotine as provided herein by a subject produces a plasma, serum or blood nicotine concentration in the subject that is substantially less than the blood, serum or plasma nicotine concentration in the subject following smoking, using or vaping from an electronic cigarette. The electronic cigarette can be any electronic cigarette that is commercially available. In some cases, the electronic cigarette comprises a 4.5% nicotine solution. In some cases, a deivce as provided herein is adapted to or configured to comprise an amount of a pharmaceutically active agent (e.g., nicotine) sufficient to provide a number of days of use by a subject. The use can be an on demand use. The subject can be a smoker. A smoker can be a new smoker, a trough maintainer smoker, an intermittent smoker, a light smoker, a weight-loss smoker, a heavy smoker, or a very heavy smoker. An intermittent smoker can be an individual who does not smoke every day. A light smoker can be an individual who smokes 1 to 9 cigarettes per day. A moderate smoker can be an individual who smokes 10 to 19 cigarettes a day. A heavy smoker can be an individual who smokes 20 to 29 cigarettes per day. A very heavy smoker can be an individual who smokes 30 or more cigarettes per day. The number of days of use a device as provided herein can provide to a subject can be about, more than, less than, at least, or at most 0.1, 0.25, 0. 5..75, 1, 2, 3, 4, 5, 6, 7, 8, 9, 10, 11, 12, 14, 15, 16, 17, 18, 19, 20, 21, 22, 23, 24, 25, 26, 27, 28, 29,30, 35, 40, 45, 50,, 55, 60, 65, 70, 75, 80, 85, 90, 95, 100, 125, 150, 175, 200, 225, 250, 275, 300, 325, 350, or 365 days. The number of days of use can be from about 0.1 to about 1, about 1 to about 2, about 1, to about 7, about 1 to about 14, about 1 to about 21, or about 1 to about 30 days. The number of days of use can be between 0.1 to 1, 1 to 2, 1 to 7, 1 to 14, 1 to 21, or 1 to 30 days. The number of days can be from about 1 day to about 1 month, about 1 day to about 2 months, about 1 day to about 3 months, about 1 day to about 6 months, about 1 day to about 1 year or about 1 to about 5 years. In some cases, a reservoir in a device as provided herein comprises a predetermined number of doses of a pharmaceutically active agent (e.g., nicotine). The pre-deteremined number of doses can be amount sufficient to provide about, more than, less than, at least, or at most 0.1, 0.25, 0. 5..75, 1, 2, 3, 4, 5, 6, 7, 8, 9, 10, 11, 12, 14, 15, 16, 17, 18, 19, 20, 21, 22, 23, 24, 25, 26, 27, 28, 29,30, 35, 40, 45, 50,, 55, 60, 65, 70, 75, 80, 85, 90, 95, 100, 125, 150, 175, 200, 225, 250, 275, 300, 325, 350, or 365 days of use by a subject. The amount can be sufficient to provide from about 0.1 to about 1, about 1 to about 2, about 1, to about 7, about 1 to about 14, about 1 to about 21, or about 1 to about 30 days of use by a subject. The amount can be sufficient to provide between 0.1 to 1, 1 to 2, 1 to 7, 1 to 14, 1 to 21, or 1 to 30 days of use by a subject. The amount can be sufficient to provide from about 1 day to about 1 month, about 1 day to about 2 months, about 1 day to about 3 months, about 1 day to about 6 months, about 1 day to about 1 year or about 1 to about 5 years of use by a subject.

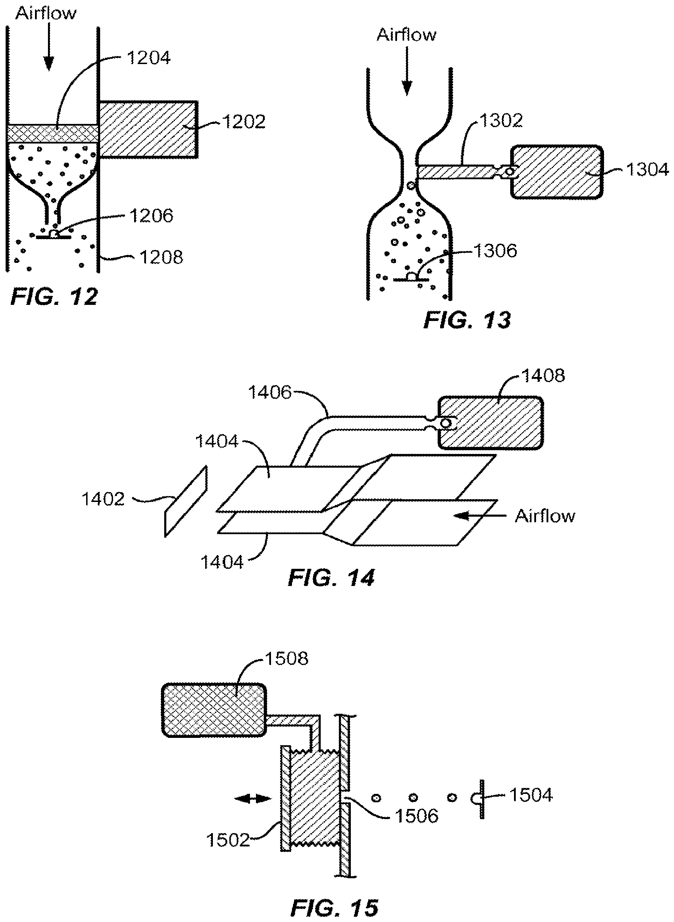

FIG. 12 illustrates an embodiment of a method of removal of an agent (e.g., nicotine) mixture from a reservoir and dispensing the agent (e.g., nicotine) into desired doses. FIG. 12 shows an agent (e.g., nicotine) reservoir (1202) next to a frit (1204) or porous material, such as a metal (stainless steel) or a ceramic, and allowing the agent (e.g., nicotine) to wick into it. Then, upon inhalation, the air can draw the agent (e.g., nicotine) into the airway (1208) and onto the heater element (1206). In some cases, the mixture is a liquid formulation comprising an agent (e.g., nicotine).

FIG. 13 illustrates another embodiment of a method for measuring a dose. Another method of dosing out the mixture is to draw the material out using a venturi. The device can comprise a tube, e.g., a capillary tube (1302), an agent (e.g., nicotine) reservoir (1304), and a heater element (1306). In some cases, the mixture is a liquid formulation comprising an agent (e.g., nicotine).

FIG. 14 illustrates another embodiment of a method for measuring a dose. In this embodiment, an agent (e.g., nicotine) mixture can be wicked into a space between two parallel plates. The device can comprise a heater element (1402), plates (1404), tube, e.g., capillary tube (1406), and an agent (e.g., nicotine) reservoir (1408). In some cases, the mixture is a liquid formulation comprising an agent (e.g., nicotine).

FIG. 15 illustrates another embodiment for measuring a dose. An agent (e.g., nicotine) mixture can be ejected using a piezoelectric device (1502) and an attached chamber with an opening or orifice (1506). When the piezo is activated, either as a single pulse or as a series of pulses (vibrated) the mixture can be driven from the opening. By controlling the amplitude of the pulse or the number of pulses, the amount of material dosed can be controlled. The device can comprise an agent (e.g., nicotine) reservoir (1508) and a heater element (1504). In one embodiment, a piezo electric device is mounted on an end or a side of the reservoir and receipt of an electrical pulse causes the piezo to deflect and push a small amount of the agent (e.g., nicotine) formulation out of a tube, e.g., capillary tube mounted on another end of the reservoir onto a heater element. In some cases, the agent formulation is liquid.

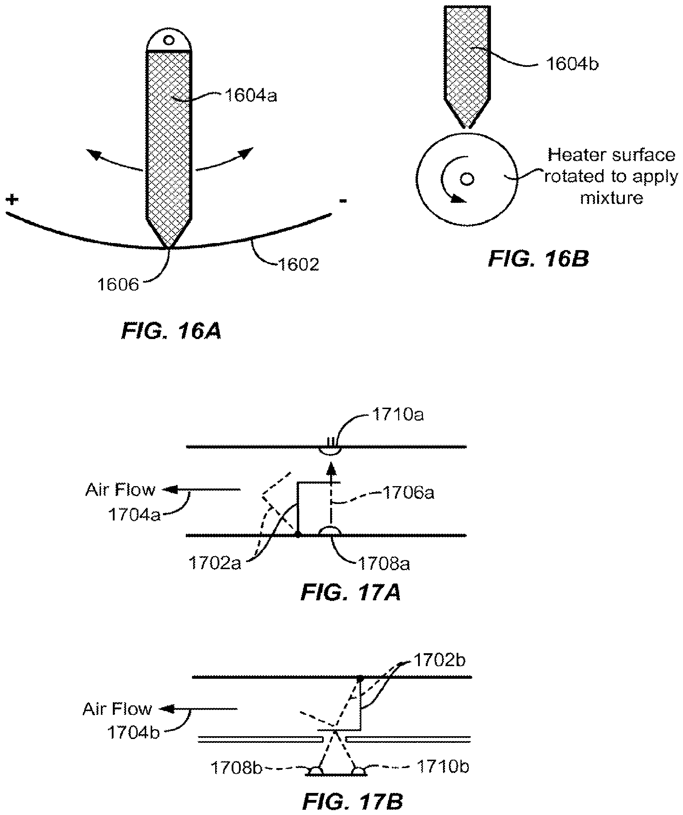

All of the forgoing mechanisms to power the dispensing of a mixture (heat, piezo) can be powered by a user performing a maneuver such as pushing a button or lever. Mechanical energy from the user can also allow for alternative methods of applying agent (e.g., nicotine) to a heater surface. An agent (e.g., nicotine) can be applied to the heater element (1602), where the reservoir is moved over the heater surface in a sweeping (see FIG. 16A) or rolling motion (see FIG. 16B). The heater surface can be etched or pitted to accept the mixture.

To have the device generate an agent (e.g., nicotine) aerosol upon inhalation by a user, a movable member (e.g., vane (1702a or 1702b)) can be used that moves upon air flow (1704a or 1704b) caused by inhalation (see e.g., FIG. 17A or 17B). This member can break an optical path (1706a) (e.g., when no inhalation is occurring), move out of an optical path (1706a) when inhalation occurs (see e.g., FIG. 17A), or can complete an optical path when inhalation occurs (by, e.g., reflection; see e.g., FIG. 17B). An LED (1708a or 1708b) can be used to generate the light. To ensure that a sensor or detector (1710a or 1710b) does not get confused by stray light, the LED (1708a or 1708b) can be strobed in a particular pattern and only when that pattern is detected is an inhalation present. In some cases, optical light pipes can be used to route the light to the valve and to route the light back to the detector.

To dispense the agent (e.g., nicotine) mixture (1802) out of some of the frits (1804) or capillaries using the pressure from the inhalation a valve can be designed to create increased pressure in the initial part of the inhalation and decrease the resistance for the duration of the inhalation (see e.g., FIG. 18).

In one embodiment, an electronic agent (e.g., nicotine) delivery device is provided that provides a dose of from 25 to 200 $\mu$g of freebase agent (e.g., nicotine). The agent (e.g., nicotine) can be in a mixture of propylene glycol at a ratio of agent (e.g., nicotine) to propylene glycol of from about 1:1 to about 1:20, or about 1:5 to about 1:10. In some cases, a mixture comprises propylene glycol and about 1.25% to about 20% nicotine. In some cases, the mixture is liquid formulation comprising an agent (e.g., nicotine). In some cases, the mixture is liquid formulation comprising an agent (e.g., nicotine) during use of the device. An aerosol can have an MMAD of about 1 to about 5 microns with a geometric standard deviation (GSD) of less than 2.0. An aerosol can have an VMD of about 1 to about 5 microns with a geometric standard deviation (GSD) of less than 2.0. Dose to dose consistency over the lifetime of the product can be no greater than $\pm$ 30%. The device can have a dose to dose consistency over the lifetime of the product that can be about, more than, less than, at least, or at most + 1%, 2%, 3%,4%,5%, 6%, 7%, 8%, 9%, 10%, 11%, 12%, 13%, 14%, 15%, 16%, 17%, 18%, 19%, 20%, 30%, 31%, 32%, 33%, 34%, 35%, 36%, 37%, 38%, 39%, 40%, 41%, 42%, 43%, 44%, 45%, 46%, 47%, 48%, 49%, or 50%. The device can be activated by an inhalation. The device can have an interior air resistance (to inhalation) no greater than that of a cigarette. The device can have an interior air resistance (to inhalation) no greater than 0.08 (cm $H_2O$)$^{1/2}$/LPM. The flow resistance of a device as provided herein can be about the same flow resistance as through that of a combustible cigarette. The device can have an interior air resistance (to inhalation) about, more than, less than, at least, or at most 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, 0.20, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, or 3.0 (cm $H_2O$)$^{1/2}$/LPM. The flow resistance through a device as provided herein can be around 2.5 (cm of $H_2O$)$^{1/2}$/LPM. In some cases, a device as provided herein comprises a flow rate of 1 LPM at a vacuum of 7.6 cm of $H_2O$. In some cases, a device as provided herein comprises a flow rate of 1.5 LPM at a vacuum of 16 cm

of $H_2O$. In some cases, a device as provided herein comprises a flow rate of 2 LPM at a vacuum of 26 cm of $H_2O$.

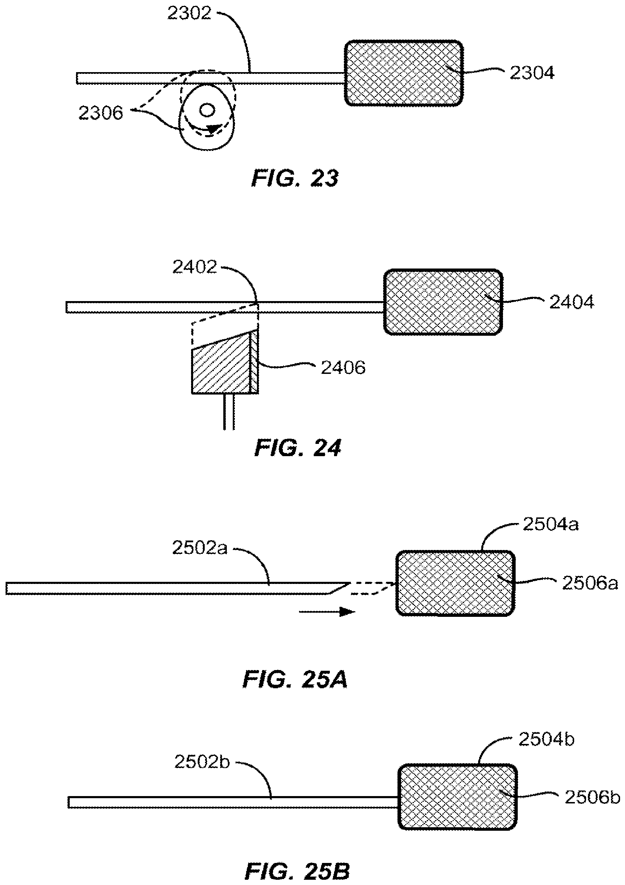

**FIG. 23** illustrates another embodiment of a method for measuring a dose. Another method of dosing out the mixture is to draw the material out using a peristaltic pump comprising a rotatable cam. The device can comprise a tube, e.g., capillary tube **(2302),** agent (e.g., nicotine) reservoir **(2304),** and a rotatable cam **(2306)** to pull or draw an agent (e.g., nicotine) mixture from the nicotine reservoir. In one embodiment, an agent (e.g., nicotine) delivery device comprises a disposable component that comprises the tube, e.g., capillary tube, and agent (e.g, nicotine) reservoir and a reusable component comprising the rotatable cam, wherein the tube, e.g., capillary tube and agent (e.g., nicotine) reservoir are mechanically connected to the rotatable cam by mating the disposable component to the reusable component. In some cases, the mixture is a liquid formulation comprising an agent (e.g., nicotine). In some cases, a device as provided herein is disposable.

**FIG. 24** illustrates another embodiment of a method for measuring a dose. The device can comprise a tube, e.g, capillary tube **(2402),** agent (e.g., nicotine) reservoir **(2404),** and a cam made of variable durometer material **(2406).** The cam can comprise an area of high durometer material surrounded by low durometer material, wherein the tube, e.g., capillary tube can be sealed within the high durometer material. In one embodiment, an agent (e.g., nicotine) mixture can be pushed out of the tube, e.g., capillary tube by compression, wherein pressure is exerted on the low durometer material of the cam to cause compression of the tube, e.g., capillary tube, within the high durometer material. In one embodiment, an agent (e.g., nicotine) delivery device comprises a disposable component that comprises the tube, e.g., capillary tube and the agent (e.g., nicotine) reservoir and a reusable component comprising the cam made of variable durometer material, wherein the tube, e.g., capillary tube and agent (e.g., nicotine) reservoir are mechanically connected to the cam made of variable durometer material by mating the disposable component to the reusable component. In some cases, the mixture is a liquid formulation comprising an agent (e.g., nicotine).

**FIGs. 25A** and **25B** illustrate an embodiment of a method of removal of an agent (e.g., nicotine) mixture from a reservoir. **FIG. 25A** shows a tube, e.g., capillary tube **(2502a)** adjacent to, but separate from, an agent (e.g., nicotine) reservoir **(2504a)** comprising an agent (e.g., nicotine) mixture **(2506a). FIG. 25B** shows that the tube, e.g., capillary tube **(2502b)** can pierce the agent (e.g., nicotine) reservoir **(2504b)** such that the agent (e.g., nicotine) mixture **(2506b)** within the agent (e.g., nicotine) reservoir can move into the tube, e.g., capillary tube and subsequently onto a heater element as provided herein. In one embodiment, the agent (e.g., nicotine) reservoir comprises a septum or seal, wherein the tube, e.g., capillary tube pierces the septum or seal. In one embodiment, the agent (e.g., nicotine) reservoir is a collapsible bag or container. In one embodiment, the collapsible bag or container is made of plastic, foil, or any other collapsible material known in the art. In a further embodiment, the tube, e.g., capillary tube can directly pierce an agent (e.g., nicotine) reservoir that is made of a collapsible material. In one embodiment, the tube, e.g., capillary tube is not inserted into the agent (e.g., nicotine) reservoir prior to a first use of the device, wherein upon first use, the tube, e.g., capillary tube, is inserted into the agent (e.g., nicotine) reservoir such that an agent (e.g., nicotine) mixture can move from the agent (e.g., nicotine) reservoir into the tube, e.g., capillary tube and subsequently onto a heater element as provided herein. In some cases, the mixture is a liquid formulation comprising an agent (e.g., nicotine).

### Carriers/excipients

In some cases, an agent (e.g., nicotine) is mixed with one or more other substances. When mixed with an agent (e.g., nicotine) as provided herein, the mixture can be liquid at room temperature. When mixed with an agent (e.g., nicotine) as provided herein, the mixture can be liquid during use of the device such that the liquid mixture is delivered to the heater element during use of the device. The one or more other substances can be pharmaceutically acceptable excipients or carriers. The suitable pharmaceutically acceptable excipients or carriers can be volatile or nonvolatile. The volatile excipients, when heated, can be volatilized, aerosolized and inhaled with the agent (e.g. nicotine). Classes of such excipients are known in the art and include, without limitation, gaseous, supercritical fluid, liquid and solid solvents. The excipient/carriers or substances can be water; terpenes, such as menthol; alcohols, such as ethanol, propylene glycol, glycerol and other similar alcohols; dimethylformamide; dimethylacetamide; wax; supercritical carbon dioxide; dry ice; lipids, triglycerides, acids, surfactants and mixtures or combinations thereof. The candidate acids can be those acids that can be in the lung with minimal, low, no, or substantially no detrimental toxicological effects. The candidate surfactants can be those surfactants that can be in the lung with minimal, low, no, or substantially no detrimental toxicological effects. The acids can be citric acid, tartaric acid, and/or lactic acid. The surfactants can be Ceteareth-25, Cocamide MEA, Cocamidapropyl betaine, Coceth-4, Coceth-7 Coconut Alcohol ethoxylate, Hydroxyethelcellulose, Lauryl polyglucose, Pareth-7, Polyglucose, Polyglucoside, PPG -10-Laureth; PPG-8 -Laureth - 8, PPG-6C12-15-Pareth-12, and/or Sodium lauraminopropionate.

The one or more other substances can be, e.g., propylene glycol (1,2-dihydroxypropane, 1,2-propanediol, methyl glycol, or trimethyl glycol ). The ratio of agent (e.g., nicotine) to propylene glycol can be about, more than, less than, or at least 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 2:1, 1:1:, 1:2, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, or 1:100. The ratio of agent (e.g., nicotine) to propylene glycol can be from about 100:1 to about 1:100, about 75:1 to about 1:100, about 50:1 to about 1:100, about 25:1 to about 1:100, about 25:1 to about 1:50, about 10:1 to about 1:100, about 10:1 to about 1:50, about 10:1 to about 1:20, about 5:1 to about 1:20, or about 1:1 to about 1:20. In one example, a 100 $\mu$g dose of agent (e.g., nicotine) and 1:10 ratio yields a volume of 1 mm$^3$ (1 mg). A mixture of agent (e.g., nicotine) and another substance, e.g., propylene glycol, can be held in an agent (e.g., nicotine) reservoir (e.g., as a liquid). In some cases, a liquid formulation comprising nicotine for use in a device as provided herein comprises 25, 50, 75, or 100 ug of nicotine mixed with 1 mg of propylene glycol. In some cases, a liquid formulation comprising nicotine for use in a device as provided herein comprises 25, 50, 75, or 100 ug of nicotine mixed with 2 mg of propylene glycol. In some cases, a liquid formulation comprising nicotine for use in a device as provided herein comprises 25, 50, 75, or 100 ug of nicotine mixed with 0.5 mg of propylene glycol.

In one embodiment, the one or more other substances is vegetable glycerin. The ratio of an agent (e.g., nicotine) to vegetable glycerin can be about, more than, less than, or at least 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 2:1, 1:1:, 1:2, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, or 1:100. The ratio of an agent (e .g., nicotine) to vegetable glycerin can be from about 100:1 to about 1:100, about 75:1 to about 1:100, about 50:1 to about 1:100, about 25:1 to about 1:100, about 25:1 to about 1:50, about 10:1 to about 1:100, about 10:1 to about 1:50, about 10:1 to about 1:20, about 5:1 to about 1:20, or about 1:1 to about 1:20. In one example, a 100 $\mu$g dose of agent (e.g., nicotine) and 1:10 ratio yields a volume of 1 mm$^3$ (1 mg). A mixture of agent (e.g., nicotine) and vegetable glycerin can be held in an agent (e.g., nicotine) reservoir (e.g., as a liquid). In some cases, a liquid formulation comprising nicotine for use in a device as provided herein comprises 25, 50, 75, or 100 ug of nicotine mixed with 1 mg of vegetable glycerin. In some cases, a liquid formulation comprising nicotine for use in a device as provided herein comprises 25, 50, 75, or 100 ug of nicotine mixed with 2 mg of vegetable glycerin. In some cases, a liquid formulation comprising nicotine for use in a device as provided herein comprises 25, 50, 75, or 100 ug of nicotine mixed with 0.5 mg of vegetable glycerin.

In another embodiment, the one or more other substances comprise vegetable glycerin and propylene glycol. The ratio of vegetable glycerin to propylene glycol can be about, more than, less than, or at least 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 2:1, 1:1:, 1:2, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, or 1:100. The ratio of agent (e.g., nicotine) to vegetable glycerin can be from about 100:1 to about 1:100, about 75:1 to about 1:100, about 50:1 to about 1:100, about 25:1 to about 1:100, about 25:1 to about 1:50, about 10:1 to about 1:100, about 10:1 to about 1:50, about 10:1 to about 1:20, about 5:1 to about 1:20, or about 1:1 to about 1:20.

The ratio of agent (e.g., nicotine) to mixture of vegetable glycerin and propylene glycol can be about, more than, less than, or at least 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 2:1, 1:1:, 1:2, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, or 1:100. The ratio of agent (e.g., nicotine) to vegetable glycerin and glycerin can be from about 100:1 to about 1:100, about 75:1 to about 1:100, about 50:1 to about 1:100, about 25:1 to about 1:100, about 25:1 to about 1:50, about 10:1 to about 1:100, about 10:1 to about 1:50, about 10:1 to about 1:20, about 5:1 to about 1:20, or about 1:1 to about 1:20.

In another embodiment, the one or more other substances can be polyethylene glycol (PEG). The PEG can be PEG200, PEG300, PEG400, PEG600, PEG1000, PEG2000, PEG4000, or PEG6000.

In one embodiment, the one or more other substances is glycerol.

In one embodiment, the one or more other substances is propylene glycol and additional alcohols. The additional alcohols can be ethanol, glycerol and other similar alcohols. In one embodiment, the one or more other substances is an alcohol in place of propylene glycol.

In one embodiment, the one or more other substances is propylene glycol and an acid. The acid can be citric acid, tartaric acid, lactic acid and/or any acid with minimal, no, low or substantially no toxicity in the lungs of a user of a device as provided herein. In one embodiment, the one or more other substances is an acid as provided herein in place of propylene glycol.

In one embodiment, the one or more other substances is propylene glycol and a surfactant. The surfactant can be Ceteareth-25, Cocamide MEA, Cocamidapropyl betaine, Coceth-4, Coceth-7 Coconut Alcohol ethoxylate, Hydroxyethelcellulose, Lauryl polyglucose, Pareth-7, Polyglucose, Polyglucoside, PPG -10-Laureth; PPG-8 -Laureth - 8, PPG-6C12-15-Pareth-12, and/or Sodium lauraminopropionate. and/or any surfactant with minimal, no, low or substantially no toxicity in the lungs of a user of a device as provided herein. In one embodiment, the one or more other substances is a surfactant as provided herein in place of propylene glycol.

In one embodiment, the one or more other substances is a mixture comprising propylene glycol and one or more additional components that aid in the transport of a pharmaceutically active agent (e.g., nicotine) in the mixture to the lungs and/or circulatory system of a user of any of the devices as provided herein. The one or more additional components can be an alcohol, acid, and/or surfactant. The alcohol can be ethanol, glycerol and other similar alcohols. The acid can be citric acid, tartaric acid, lactic acid and/or any acid with minimal, no, low or substantially no toxicity in the lungs of a user of a device as provided herein. The surfactant can be Ceteareth-25, Cocamide MEA, Cocamidapropyl betaine, Coceth-4, Coceth-7 Coconut Alcohol ethoxylate, Hydroxyethelcellulose, Lauryl polyglucose, Pareth-7, Polyglucose, Polyglucoside, PPG -10-Laureth; PPG-8 -Laureth - 8, PPG-6C12-15-Pareth-12, and/or Sodium lauraminopropionate. and/or any surfactant with minimal, no, low or substantially no toxicity in the lungs of a user of a device as provided herein.

In another embodiment, an electronic agent (e.g., nicotine) delivery device comprises a mixture of agent (e.g., nicotine) and polyethylene glycol. A mixture can comprise an agent (e.g., nicotine), polyethylene glycol, and vegetable glycerin. A mixture can comprise an agent (e.g., nicotine), polyethylene glycol, vegetable glycerin, and propylene glycol. In another embodiment, a mixture comprises an agent (e.g., nicotine), polyethylene glycol, and propylene glycol. A mixture can comprise an agent (e.g., nicotine), propylene glycol, and vegetable glycerin.

In one embodiment, the percentage of an agent (e.g., nicotine) in a formulation (e.g., solution) comprising an agent (e.g., nicotine) can be about, more than, less than, or at least 0.25, 0.5, 0.75, 1, 1.25, 1.5, 1.75, 2, 2.25, 2.5, 2.75, 3, 3.25, 3.5, 3.75, 4, 4.25, 4.5, 4.75, 5, 5.25, 5.5, 5.75, 6, 6.25, 6.5, 6.75, 7, 7.25, 7.5, 7.75, 8, 8.25, 8.5, 8.75, 9, 9.25, 9.5, 9.75, 10, 10.25, 10.5, 10.75, 11, 11.25, 11.5, 11.75, 12, 12.25, 12.5, 12.75, 13, 13.25, 13.5, 13.75, 14, 14.25, 14.5, 14.75, 15, 15.25, 15.5, 15.75, 16, 16.25, 16.5, 16.75, 17, 17.25, 17.5, 17.75, 18, 18.25, 18.5, 18.75, 19, 19.25, 19.5, 19.75, 20, 20.5, 21, 21.5, 22, 22.5, 23, 23.5, 24, 24.5, or 25% by volume. The percentage of an agent (e.g., nicotine) in a formulation (e.g., solution) comprising an agent (e.g., nicotine) can be from about 0.25 to about 1.25, about 1.25 to about 2.5, about 2.5 to about 5, about 5 to about 7.5, about 7.5 to about 10, about 10 to about 12.5, about 12.5 to about 15, about 15 to about 17.5, about 17.5 to about 20, or about 20 to about 25 % by volume. The formulation (e.g., solution) can further comprise one or more substances. The one or more substances can be propylene glycol and/or vegetable glycerin. The formulation can be liquid at room temperature or at temperatures at which the device is generally used by a subject.

In one embodiment, the percentage of an agent (e.g., nicotine) in a formulation (e.g., solution) comprising an agent (e.g., nicotine) can be about, more than, less than, or at least 0.25, 0.5, 0.75, 1, 1.25, 1.5, 1.75, 2, 2.25, 2.5, 2.75, 3, 3.25, 3.5, 3.75, 4, 4.25, 4.5, 4.75, 5, 5.25, 5.5, 5.75, 6, 6.25, 6.5, 6.75, 7, 7.25, 7.5, 7.75, 8, 8.25, 8.5, 8.75, 9, 9.25, 9.5, 9.75, 10, 10.25, 10.5, 10.75, 11, 11.25, 11.5, 11.75, 12, 12.25, 12.5, 12.75, 13, 13.25, 13.5, 13.75, 14, 14.25, 14.5, 14.75, 15, 15.25, 15.5, 15.75, 16, 16.25, 16.5, 16.75, 17, 17.25, 17.5, 17.75, 18, 18.25, 18.5, 18.75, 19, 19.25, 19.5, 19.75, 20, 20.5, 21, 21.5, 22, 22.5, 23, 23.5, 24, 24.5, or 25% by weight. The percentage of an agent (e.g., nicotine) in a formulation (e.g., solution) comprising an agent (e.g., nicotine) can be from about 0.25 to about 1.25, about 1.25 to about 2.5, about 2.5 to about 5, about 5 to about 7.5, about 7.5 to about 10, about 10 to about 12.5, about 12.5 to about 15, about 15 to about 17.5, about 17.5 to about 20, or about 20 to about 25 % by weight. The formulation (e.g., solution) can further comprise one or more substances. The one or more substances can be propylene glycol and/or vegetable glycerin. The formulation can be liquid at room temperature or at temperatures at which the device is generally used by a subject

The source of nicotine for use in the devices and methods as provided herein can be a tobacco or tobacco material. Here, a tobacco or tobacco material can be defined as any combination of natural and synthetic material that can be vaporized for pleasure or medicinal use. The formulation comprising nicotine can comprise flue-cured tobacco, glycerin, and flavorings. The formulation comprising nicotine can comprise flue-cured tobacco, propylene glycol, and flavorings. A liquid formulation comprising nicotine can be produced by chopping tobacco into fine pieces (less than 3 mm diameter, less than 2 mm), adding the other ingredients (e.g., propylene glycol, vegetable glycerin, water, and/or flavorings), and mixing until even consistency is achieved.

### pH

An electronic agent (e.g., nicotine) delivery device described herein can control a pH of an agent (e.g., nicotine) mixture or aerosol. The pH of the agent (e.g., nicotine) mixture or aerosol can be about, more than, less than, or at least 1, 1.5, 2, 2.5, 3, 3.5, 4, 4.5, 5, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9 9, 9.5, 10, 10.5, 11, 11.5, 12, 12.5, 13, 13.5, or 14. In some cases, the pH of an agent (e.g., nicotine) mixture or aerosol can be about 1 to about 14, about 2 to about 13, about 3 to about 12, about 4 to about 11, about 5 to about 10, about 6 to about 9, about 6 to about 8, about 6 to about 7, about 4 to about 6, about 4 to about 8, about 5 to about 8, about 5 to about 7, about 7 to about 9, about 5.5 to about 8.5, about 6.5 to about 8.5, about 6.5 to about 7.5, about 7.5 to about 9, or about 7 to about 8.5. One or more substances can be added to a mixture to adjust the pH of the mixture. The one or more substances that can be added to the mixture can be one or more buffers.

The one or more buffers can be any buffers known in the art. The one or more buffers can be acidic buffers or alkaline buffers. The one or more buffers can be a phosphate, bicarbonate or protein buffer system. Examples of buffers can include, but are not limited to, TAPS (3-{[tris(hydroxymethyl)methyl]amino}propanesulfonic acid), Bicine (N,N-bis(2-hydroxyethyl)glycine), Tris (tris(hydroxymethyl)methylamine), Tricine (N-tris(hydroxymethyl)methylglycine), TAPSO (3-[N-Tris(hydroxymethyl)methylamino]-2-hydroxypropanesulfonic Acid), HEPES (4-2-hydroxyethyl-1-piperazineethanesulfonic acid), TES (2-{[tris(hydroxymethyl)methyl]amino}ethanesulfonic acid), MOPS (3-(N-morpholino)propanesulfonic acid), PIPES (piperazine-N,N'-bis(2-ethanesulfonic acid)), cacodylate (dimethylarsinic acid), SSC (saline sodium citrate), MES (2-(N-morpholino)ethanesulfonic acid), succinic acid (2(R)-2-(methylamino)succinic acid), sodium acetate/acetic acid, sodium citrate/citric acid, CHES, sodium borate/boric acid, diethyl barbituric acid, potassium dihydrogen phosphate, Carmody buffer, Britton-Robinson buffer, sodium lactate/latic acid, sodium tarttraic scid or mixtures thereof.

Flavorings

In one embodiment, a mixture comprises one or more flavorings. The one or more flavorings can be a flavor offered by, e.g., Flavourart (Italy), Flavor Apprentice, or LorAnn. A flavor can be, e.g., almond, almond amaretto, apple, Bavarian cream, black cherry, black sesame seed, blueberry, brown sugar, bubblegum, butterscotch, cappuccino, caramel, caramel cappuccino, cheesecake (graham crust), cinnamon redhots, cotton candy, circus cotton candy, clove, coconut, coffee, clear coffee, double chocolate, energy cow, graham cracker, grape juice, green apple, Hawaiian punch, honey, Jamaican rum, Kentucky bourbon, kiwi, koolada, lemon, lemon lime, tobacco, maple syrup, maraschino cherry, marshmellow, menthol, milk chocolate, mocha, Mountain Dew, peanut butter, pecan, peppermint, raspberry, banana, ripe banana, root beer, RY4, spearmint, strawberry, sweet cream, sweet tarts, sweetner, toasted almond, tobacco, tobacco blend, vanilla bean ice cream, vanilla cupcake, vanilla swirl, vanillin, waffle, Belgian waffle, watermelon, whipped cream, white chocolate, wintergreen, amaretto, banana cream, black walnut, blackberry, butter, butter rum, cherry, chocolate hazelnut, cinnamon roll, cola, creme de menthe, eggnog, English toffee, guava, lemonade, licorice, maple, mint chocolate chip, orange cream, peach, pina colada, pineapple, plum, pomegranate, pralines and cream, red licorice, salt water taffy, strawberry banana, strawberry kiwi, tropical punch, tutti frutti, or vanilla. The number of flavors in a mixture can be about, more than, less than, or at least 1, 2, 3, 4, 5, 6, 7, 8, 9, 10.

A flavoring can be used to pair nicotine administration with certain gustatory and/or olfactory sensations. Subsequent administration of agent (e.g., nicotine) doses can be reduced while retaining the flavoring to help the user reduce their agent (e.g., nicotine) dependency and enable cravings to be fully or partially sated using the flavoring as a conditioned stimulus.

## Particle size

A device provided herein can generate an aerosol. The aerosol can comprise particles of an optimum size for delivery to the deep lung. The aerosol can be a condensation aerosol. The aerosol can comprise a pharmaceutically active agent as provided herein (e.g., nicotine). The particle size can be about, more than, less than, or at least 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 0.71, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, or 20 microns. The particle size can be from about 1 to about 10 microns, about 1 to about 9 microns, about 1 to about 7 microns, about 1 to 6 microns, about 1 to about 5 microns, about 1 to about 4 microns, about 1 to about 3 microns, or about 1 to about 2 microns. The particle size can be from about 0.5 to about 10 microns, about 0.5 to about 9.5 microns, about 0.5 to about 9 microns, about 0.5 to about 8.5 microns, about 0.5 to about 8 microns, about 0.5 to about 7.5 microns, about 0.5 to about 7microns, about 0.5 to about 6.5 microns, about 0.5 to about 6 microns, about 0.5 to about 5.5 microns, about 0.5 to about 5 microns, about 0.5 to about 4.5 microns, about 0.5 to about 4.0 microns, about 0.5 to about 3.5 microns, about 0.5 to about 3 microns, about 0.5 to about 2.5 microns, about 0.5 to about 2 microns, about 0.5 to about 1.5 microns, or about 0.5 to about 1 microns. The particle size can be less than 1 micron. The particle size can be greater than 5 microns. The particle size can be less than 5 microns. The particle size can be greater than 1 micron. In one embodiment, the particle size is from about 1 to about 5 microns. In one embodiment, the particle size is from about 1 to about 3 microns. The particle size can be a mean or average. In some cases, a condensation aerosol produced by any device as provided herein comprises a mean or average particle size. The mean can be an arithmetic or geometric mean. The particle size can be a diameter, radius, or circumference. The particle size can represent a single particle or a population of particles. The population of particles can be an aerosol or condensation aerosol produced by a device as provided herein. In some cases, the population of particles is a condensation aerosol. In some cases, the particle size is a diameter. The diameter can be a physical diameter (e.g., Feret's diameter, Martin's diameter, or equivalent projected area diameter), a fiber diameter, a Stokes' diameter, a thermodynamic diameter, a volumetric diameter, or an aerodynamic diameter. In one embodiment, the particle size is a volume median diameter (VMD). In one embodiment, the particle size is a mass median aerodynamic diameter (MMAD). In one embodiment, the particle size is a physical diameter (e.g., Feret's diameter, Martin's diameter, or equivalent projected area diameter). The particle size can be created at any of the flow rates for any of the devices provided herein. In some cases, a device as provided herein comprises a flow rate of 1 LPM at a vacuum of 7.6 cm of $H_2O$. In some cases, a device as provided herein comprises a flow rate of 1.5 LPM at a vacuum of 16 cm of $H_2O$. In some cases, a device as provided herein comprises a flow rate of 2 LPM at a vacuum of 26 cm of $H_2O$. In some cases, a device for generating a condensation aerosol as provided herein generates a condensation aerosol comprising a pharmaceutically active agent (e.g., nicotine) comprising a particle size of 2.5 microns at a flow rate of 20 liters/minute (LPM). In some cases, a device for generating a condensation aerosol as provided herein generates a condensation aerosol comprising a pharmaceutically active agent (e.g., nicotine) comprising a particle size of 1.4 microns at a flow rate of 50 liters/minute (LPM).

A device provided herein can generate an aerosol comprising particles. The aerosol can comprise particles of an optimum size for delivery to the deep lung. The aerosol can be a condensation aerosol. In some cases, a condensation aerosol produced by any device as provided herein comprises a standard deviation. In some cases, the standard deviation is for a particle size distribution of a condensation aerosol produced by a device as provided herein. The standard deviation can be an arithmetic or geometric standard deviation (GSD). In some cases, a condensation aerosol generated by a device as provided herein comprises a particle size distribution comprising an arithmetic standard deviation (ASD). The ASD can be about, more than, less than, or at least 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, or 3 microns. The ASD can be from about 1 to about 3, about 1 to about 2, about 0.1 to about 1, or about 0.1 to about 0.5 microns. The ASD can be between about 0.1 to about 0.5, about 0.5 to about 1, about 1 to about 1.5, about 1.5 to about 2, or about 2 to about 3 microns. The ASD can be between 0.1 and 0.5, 0.5 and 1, 1 and 1.5, 1 and 2, 1 and 3, 1.5 and 2, 1.5 and 3, or 2 and 3 microns. In one embodiment, the ASD is less than 2 microns. In some cases, a condensation aerosol generated by a device as provided herein comprises a particle size distribution comprising a GSD. The GSD can be about, more than, less than, or at least 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, or 3. The GSD can be from about 1 to about 3, about 1 to about 2, about 0.1 to about 1, or about 0.1 to about 0.5. The GSD can be between about 0.1 to about 0.5, about 0.5 to about 1, about 1 to about 1.5, about 1.5 to about 2, or about 2 to about 3. The GSD can be between 0.1 and 0.5, 0.5 and 1, 1 and 1.5, 1 and 2, 1 and 3, 1.5 and 2, 1.5 and 3, or 2 and 3. In one embodiment, the GSD is less than 2. The particle size can be a diameter, radius, or circumference. The diameter can be a physical diameter (e.g., Feret's diameter, Martin's diameter, or equivalent projected area diameter), a fiber diameter, a Stokes' diameter, a thermodynamic diameter, a volumetric diameter, or an aerodynamic diameter. In some cases, the diameter of the particles of a condensation aerosol generated by a device as provided herein comprises an ASD. In some cases, the diameter of the particles of a condensation aerosol generated by a device as provided herein comprises a GSD. In some cases, a device provided herein generates a condensation aerosol comprising an MMAD of from about 1 to about 5 $\mu$m with a GSD of less than 2. In some cases, a device provided herein generates a condensation aerosol comprising an MMAD of from about 1 to about 3 $\mu$m with a GSD of less than 2. In some cases, a device provided herein generates a condensation aerosol comprising an MMAD of from about 1 to about 5 $\mu$m with a GSD of from about 1 to about 2. In some cases, a device provided herein generates a condensation aerosol comprising an MMAD of from about 1 to about 3 $\mu$m with a GSD of less than 1. In some cases, a device provided herein generates a condensation aerosol comprising a VMD of from about 1 to about 5 $\mu$m with a GSD of less than 2. In some cases, a device provided herein generates a condensation aerosol comprising a VMD of from about 1 to about 3 $\mu$m with a GSD of less than 2. In some cases, a device provided herein generates a condensation aerosol comprising a VMD of from about 1 to about 5 $\mu$m with a GSD of less than 1. In some cases, a device provided herein generates a condensation aerosol comprising a VMD of from about 1 to about 3 $\mu$m with a GSD of less than 1. The GSD can be for any of the particle sizes that can be created at any of the flow rates for any of the devices provided herein. The GSD can be around the diameter, MMAD, or VMD. In some cases, a device for generating a condensation aerosol as provided herein generates a condensation aerosol comprising a pharmaceutically active agent (e.g., nicotine) comprising a particle size of 2.5 microns with a GSD of 1.6 at a flow rate of 20 liters/minute (LPM). In some cases, a device for generating a condensation aerosol as provided herein generates a condensation aerosol comprising a pharmaceutically active agent (e.g., nicotine) comprising a particle size of 1.4 microns with a GSD of 1.2 at a flow rate of 50 liters/minute (LPM).

A device provided herein can generate an aerosol. The aerosol can comprise particles of an optimum size for delivery to the deep lung. The aerosol can be a condensation aerosol. The aerosol can comprise a pharmaceutically active agent as provided herein (e.g., nicotine). A device provided herein can produce a condensation aerosol wherein greater than 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% of the condensation aerosol has a diameter of from about 1 to about 5 $\mu$m. A device provided herein can produce a condensation aerosol wherein greater than 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%,

81%, 82%, 83%, 84%, 85%, 86%, 87%, 88%, 89%, 90%, 91%, 92%, 93%, 94%, 95%, 96%, 97%, 98%, or 99% of the condensation aerosol has a diameter of from about 1 to about 3 $\mu$m. In some cases, between 60-70%, 70-80%, 80-90%, or 90-100% of the condensation aerosol produced by a device herein comprises a diameter of from about 1 to about 5 $\mu$m. In some cases, between 60-70%, 70-80%, 80-90%, or 90-100% of the condensation aerosol produced by a device herein comprises a diameter of from about 1 to about 5 $\mu$m. In some cases, about 60 to about 70%, about 70 to about 80%, or about 80 to about 90%, or about 90 to about 100% of the condensation aerosol produced by a device herein comprises a diameter of from about 1 to about 5 $\mu$m. In some cases, about 60 to about 70%, about 70 to about 80%, about 80 to about 90%, or about 90 to about 100% of the condensation aerosol produced by a device herein comprises a diameter of from about 1 to about 3 $\mu$m. In some cases, a device as provided herein produces a condensation aerosol comprising a pharmaceutically active agent (e.g., nicotine), wherein greater than 90% of the condensation aerosol comprises a particle diameter of from about 1 to about 5 $\mu$m. In some cases, a device as provided herein produces a condensation aerosol comprising a pharmaceutically active agent (e.g., nicotine), wherein greater than 90% of the condensation aerosol comprises a particle diameter of from about 1 to about 3 $\mu$m. In some cases, a device as provided herein produces a condensation aerosol comprising a pharmaceutically active agent (e.g., nicotine), wherein greater than 95% of the condensation aerosol comprises a particle diameter of from about 1 to about 5 $\mu$m. In some cases, a device as provided herein produces a condensation aerosol comprising a pharmaceutically active agent (e.g., nicotine), wherein greater than 95% of the condensation aerosol comprises a particle diameter of from about 1 to about 3 $\mu$m. The particle sizes can be generated at any of the flow rates as described herein for any of the devices for generating a condensation as provided herein. In some cases, the flow rate is 20 LPM. In some cases, the flow rate is 50 LPM. In some cases, a device as provided herein comprises a flow rate of 1 LPM at a vacuum of 7.6 cm of $H_2O$. In some cases, a device as provided herein comprises a flow rate of 1.5 LPM at a vacuum of 16 cm of $H_2O$. In some cases, a device as provided herein comprises a flow rate of 2 LPM at a vacuum of 26 cm of $H_2O$. A device provided herein can produce a condensation aerosol comprising a pharmaceutically active agent (e.g., nicotine), wherein the average mass and/or size of a particle from the condensation aerosol is substantially greater than a particle from an anerosol produced by an e-cigarette. A device provided herein can produce a condensation aerosol comprising a pharmaceutically active agent (e.g., nicotine), wherein the average mass and/or size distribution of the condensation aerosol is substantially greater than the average size and/or mass distribution of an anerosol produced by an e-cigarette. The e-cigarette can be any commerically available e-cigarette. The e-cigarette can be an NJOY or Finiti e-cig. In one embodiment, the particle size is a diameter. In one embodiment, the particle size is a volume median diameter (VMD). In one embodiment, the particle size is a mass median aerodynamic diameter (MMAD).

In some cases, an aerosol generating device as provided herein is configured to produce a plurality of aerosols such that each of the plurality of aerosols comprises a size that is different than the sizeof a separate aerosol produced by the aerosol generating device. Each of the plurality of aerosols can comprise a population of aerosols possessing a range of sizes that is different or substantially different than a separate aerosol of the plurality of aerosols. The plurality of aerosols can be 1, 2, 3, 4, or 5 aerosols. In some cases, an aerosol generating device as provided herein produces a first aerosol and a second aerosol such that the size of the first aerosol is different or substantially different than the size of the second aerosol. The size of the aerosol can be a diameter. The diameter can be an MMAD or VMD. The device can be configured to produce the plurality of aerosols during a single use by a subject using the device. In some cases, an aerosol generating device as provided herein produces a first aerosol and a second aerosol during a single use of the device by a subject. In some cases, an aerosol generating device as provided herein produces a first aerosol and a second aerosol during a single use of the device by a subject such that the diameterof the first aerosol is different or substantially different than the diameter of the second aerosol. In some cases, an aerosol generating device as provided herein produces a first aerosol and a second aerosol during separate uses of the device by a subject. In some cases, an aerosol generating device as provided herein produces a first aerosol and a second aerosol during separate uses of the device by a subject such that the diameterof the first aerosol is different or substantially different than the diameter of the second aerosol. The first aerosol can comprise a size (e.g., diameter) suitable for delivery and absorption into the deep lungs of a user of the device. In some cases, the diameter (e.g., MMAD or VMD) of the first aerosol is from about 1$\mu$m to about 5 $\mu$m. The second aerosol can comprise a size (e.g., diameter) suitable for exhalation from a user of the device such that the exhaled aerosol is visible. In some cases, the diameter (e.g., MMAD or VMD) of the second aerosol is less than about 1 $\mu$m.

Provided herein are devices and methods for generating multiple aerosols as provided herien from asingle aerosol generating device comprising an airflow channel or passageway as provided herein by altering the volume of air through an aerosol generation region of the airflow channel or passageway. In some cases, each of the multiple aerosols produced by the single device is a different size (e.g., diameter). The aerosol generation region of the device can comprise a heater element as provided herein. The heater element can comprise a wire coil as provided herein. The heater element can comprise a wire coil and wicking element as provided herein (e.g., **FIG. 38**). The volume or amount of air in the aerosol generation region of the airflow channel or passageway can serve to condense the vaporized liquid formulation into a condensation aerosol as described herein which can subsequently exit an outlet in the airflow channel and be inhaled by a subject using the device. The amount or volume of air in the aerosol generation region of the airflow channel or passageway can be altered or adjusted by changing the number and/or size of inlets to the airflow channel.

In some cases, the volume or amount of air flowing through the aerosol generation region of the device can be altered by changing the number of air inlets serving the aerosol generation region by moving adjustable rings or sliders located on the outside of the airflow channel such as described in EP0845220B1 or WO2013083635A1. The alteration in the number of inlets supplying air to the aerosol generation chamber can be achieved manually or automatically under the control of the electrical circuitry within the device. The electric circuitry can be controlled by a controller. The controller can be a component of the device and can be programmable as provided herein. Manual control of the number of air inlets can be achieved by a user of the device moving the adjustable slider or shutter to block or open an air inlet or inlets. Alteration in the number of air inlets providing air to the ariflow channel can effectively alter the air flow rate through the aerosol generation region. In some cases, the number of air inlets generates a flow rate of air through an aerosol generation region of an aerosol generating device as provided herein such that the flow rate generates a condensation aerosol of a desired size. The desired size can be a diameter. The diameter can be effective for deep lung delivery of the condensation aerosol and absorption into the blood stream of a user. The diameter effective for deep lung delivery can be from about 1 $\mu$m to about 5 $\mu$m. The diameter can be an MMAD or a VMD. The flow rate effective for generating condensation aerosol particles comprising a size (e.g., diameter) effective for deep lung delivery can be from about 1 LPM to about 10 LPM. In some cases, a device as provided herein comprises a flow rate of 1 LPM at a vacuum of 7.6 cm of $H_2O$. In some cases, a device as provided herein comprises a flow rate of 1.5 LPM at a vacuum of 16 cm of $H_2O$. In some cases, a device as provided herein comprises a flow rate of 2 LPM at a vacuum of 26 cm of $H_2O$. The number of air inlets can be altered during a single use or between uses of an aerosol generating device in order to alter the size (e.g., diameter) of a condensation aerosol generated by the device. The cross-section of the airway in a device configured to generate a condensation aerosol of a size (e.g., diameter) suitable for deep lung delivery as well as the vaporization rate of a liquid formulation delivered to or onto the heater element can remain constant in the device such that an increase in the air flow rate can result in a condensation aerosol comprising a smaller size (e.g., diameter) sutiable for exhalation of a visible vapor. Thus, the size (e.g., diameter) of the condensation aerosol can be altered from a size effective for deep lung delivery as provided herein to a size (e.g., diameter) effective for exhalation of a visible vapor. The diameter effective for exhalition of a visible vapor can be less than about 1 $\mu$m. The flow rate effective for generating condensation aerosol particles comprising a size (e.g., diameter) effective for exhalation of a visible vapor can be greater than 10 LPM. The flow rate can be from about 20 LPM to about 40 LPM. The alteration in the size of the condensation aerosol by altering the number of the air inlets can be performed automatically during use of the device as described herein. The alteration in the size of the condensation aerosol by altering the number of the air inlets can be performed manually during use of the device as described herein.

In some cases, the volume or amount of air flowing through the aerosol generation region of the device can be altered by changing the size of the air inlets serving the aerosol generation region such as described in WO2013083635A1. In this embodiment, a second air inlet located between the heater in the aerosol generation region and an outlet of the aerosol generation region can be larger than an air inlet located before the aerosol generation region. The larger second inlet can serve to provide a greater flow of air through the second air inlet for a given inhalation by a user of the device such that a greater flow of air air can be drawn through the second air inlet than the first air inlet. The second air inlets can be larger than the fir st air inlets. The second air inlets can be larger and more numerous than the first air inlets. In some cases, the size of air inlets generates a flow rate of air through an aerosol generation region of an aerosol generating device as provided herein such that the flow rate generates a condensation aerosol of a desired size. The desired size can be a diameter. The diameter can be effective for deep lung delivery of the condensation aerosol and absorption into the blood stream of a user. The diameter effective for deep lung delivery can be from about 1 $\mu$m to about 5 $\mu$m. The diameter can be an MMAD or a VMD. The flow rate effective for generating condensation aerosol particles comprising a size (e.g., diameter) effective for deep lung delivery can be from about 1 LPM to about 10 LPM. The sizeof air inlets can be altered during a single use or between uses of an aerosol generating device in order to alter the size (e.g., diameter) of a condensation aerosol generated by the device. The cross-section of the airway in a device configured to generate a condensation aerosol of a size (e.g., diameter) suitable for deep lung delivery as well as the vaporization rate of a liquid formulation delivered to or onto the heater element can remain constant in the device such that an increase in the air flow rate can result in a condensation aerosol comprising a smaller size (e.g., diameter) sutiable for exhalation of a visible vapor. Thus, the size (e.g., diameter) of the condensation aerosol can be altered from a size effective for deep lung delivery as provided herein to a size (e.g., diameter) effective for exhalation of a visible vapor. The diameter effective for exhalation of a visible vapor can be less than about 1 $\mu$m. The flow rate effective for generating condensation aerosol particles comprising a size (e.g., diameter) effective for exhalation of a visible vapor can be from greater than 10 LPM. The flow rate can be from about 20 LPM to about 40 LPM. The alteration in the size of the condensation aerosol by altering the size of the air inlets can be performed automatically during use of the device as described herein. The alteration in the size of the condensation aerosol by altering the size of the air inlets can be performed manually during use of the device as described herein. Alteration in the size of the air inlets can be achieved through the use of adjustable shutters located adjacent to or over air inlets to the air flow channel. The adjustable shutters can be moved to partially occlude or block one or more air inlets thereby effectively changing the respective air inlet's size.

Provided herein are devices and methods for generating multiple aerosols as provided herein from a single aerosol generating device by altering an amount or volume of a liquid formulation comprising a phramceuctically active agent (e.g., nicotine) that is delivered to or onto a heater element and vaporized by the heater element. In some cases, each of the multiple aerosols produced by the single device is a different size (e.g., diameter). The heater element can be any heater element as provided herein.The heater element can comprise a wire coil as provided herein. The heater element can comprise a wire coil and wicking element as provided herein (e.g., **FIG. 38**). In some cases, the aerosol generating device comprises an airflow channel or passageway such that air flowing through the channel serves to condense the vaporized liquid formulation into a condensation aerosol which subsequently exits an outlet in the airflow channel and is inhaled by a subject using the device. The amount of the liquid formulation delivered to or onto the heater element can be controlled by a pump located within the device. The pump can be any pump provided herein. The pump can be a positive displacement pump. The pump can be a piston pump (e.g., **FIGs. 94A-C**) or a diaphragm pump (e.g., **FIGs 90A-C**). In some cases, the device comprises a reservoir housing the liquid formulation and the pump is located within the reservoir as provided herein. In some cases, the amount of the liquid formulation delivered by the pump is controlled by setting a pump rate such that a specific pump rate corresponds to a specific volume that can be delivered by the pump. Adjusting the pump rate from a first pump rate to a second pump rate can result in the pump delivering a different amount or volume of liquid formulation. In some cases, a pump in an aerosol generating device as provided herein is set at a first controlled rate such that a first amount of a liquid formulation comprising a pharmaceutically active agent (e.g., nicotine) is delivered to or onto a heater element within the device which generates a first aerosol comprising a first size (e.g., diameter) and the pump is altered to operate at a second controlled rate such that a second amount of the liquid formulation is delivered to or onto the heater element which generates a second aerosol comprising a second size (e.g., diameter). The first and second aerosols can have different sizes (e.g., diameters). The first aerosol can comprise a size (e.g., diameter) suitable for delivery and absorption into the deep lungs of a user of the device. In some cases, the diameter (e.g., MMAD or VMD) of the first aerosol is from about 1 $\mu$m to about 5 $\mu$m. The second aerosol can comprise a size (e.g., diameter) suitable for exhalation from a user of the device such that the exhaled aerosol is visible. In some cases, the diameter (e.g., MMAD or VMD) of the second aerosol is less than about 1 $\mu$m. Alteration of the rates of the pump in an aerosol generating device as provided herein can occur during a single use of the device by a user. Alteration of the pump rate during a single use can occur automatically or manually. Alteration of the rates of the pump in an aerosol generating device as provided herein can occur during separate uses of the device by a user.

Automatic alteration of the pump rate can be accomplished by electrically coupling the pump to a circuit configured to switch the pump rate during operation of the device. The ciruit can be controlled by a control program. The control program can be stored in a controller as provided herein. The controller can be programmable and/ or can be a component of the aerosol generating device. A user of the device can select a desired aerosol size or sets of aerosol sizes by selecting a specific program on the controller of the device prior to use of the device. In some cases, a specific program is associated with a specific pump rate for delivering a specific volume of a liquid formulation in order to produce an aerosol comprising a desired size. If the user desires an aerosol with a different size (e.g., diameter) for a subsequent use, then the user can select a different program associated with a different pump rate for delivering a different volume of the liquid formulation in order to produce an aerosol with the newly desired size (e.g., diameter). In some cases, a specific program is associated with specific pump rates for delivering specific volumes of a liquid formulation in order to produce multiple aerosols comprising desired sizes. Each of the specific pump rates in a specific program comprising a set of specific pump rates can deliver in succession a specific amount or volume of the liquid formulation in order to produce a succession of aerosols of differing sizes (e.g., diameters) during a single use of the device. The aerosol or aerosols can be condensation aerosols. The conensation aerosols can be produced within an airway within the device as provided herein.

Manual alteration of thepump rate can be accomplished by the user of the device pressing a button or switch on the device during use of the device. Manual alteration can occur during a single use of the device or between separate uses of the device. The button or switch can be electrically coupled to the pump and/or a controller. The controller can be a component of the device and can be programmable. The controller can comprise program(s) designed to control the operation of the pump such that the pressing of a button or switch can cause the controller to alter the operation (e.g., pump rate) of the pump in order to affect delivery of a differing volume of the liquid formulation. The user of the device can press the button or flip the switch while using the device. The user of the device can press the button or flip the switch between uses of the device.

In some cases, an aerosol generating device as provided herein is configured to produce a condensation aerosol comprising a diameter of from about 1 $\mu$m to about 1,2 $\mu$m. Upon inhaling from an outlet of the device, a user can perform a breathing maneuver in order to facilitate delivery of the condensation aerosol comprising a diameter of

from about 1 μm to about 1,2 μm into the user's deep lungs for subsequent absorption into the user's bloodstream. The breathing maneuver can comprise the user holding his/her's breath following inhalation of the condensation aerosol and subsequently exhaling. The breath-hold can be for 1, 2, 3, 4, 5, 6, 7, 8, 9, or 10 seconds. The breath-hold can be from about 2 to about 5 seconds. Alternatively, the user can inhale and directly exhale the condensation aerosol comprising a diameter of from about 1 μm to about 1.2 μm. Inhalation followed by direct exhalation can cause the generation of a visible vapor since a large percentage of the condensation aerosol can be exhaled. The diameter can be an MMAD or VMD as provided herein.

## Agent (e.g., nicotine) reservoir

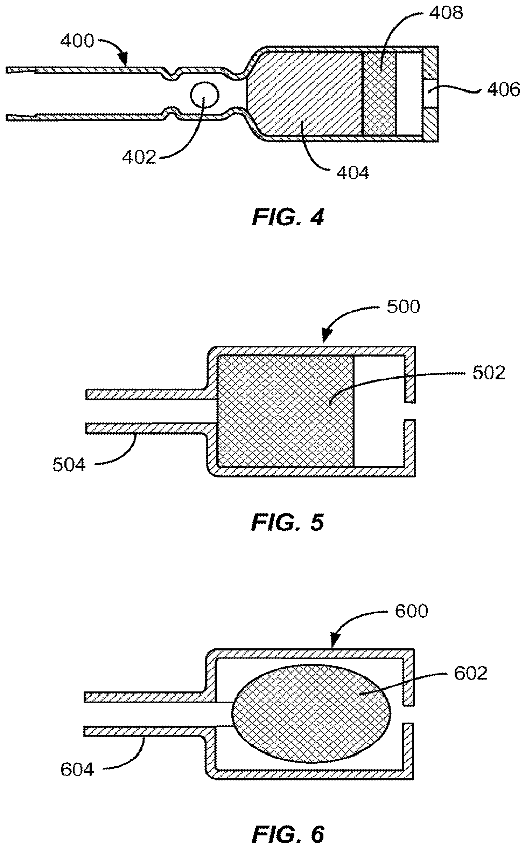

FIG. 4 illustrates an embodiment of an agent (e.g., nicotine) reservoir (404) that can be used in an electronic agent (e.g., nicotine) delivery device provided herein. A tube, e.g., capillary tube (400) with a valve (402) does not need to be inserted into a separate reservoir, but can be the reservoir (404) itself by extending away from the ejection end. The diameter of the tube, e.g., capillary tube, can be increased to store more mixture. To allow for the mixture to be pulled from the reservoir without creating a low pressure, which could resist the mixture leaving, the back end can have a vent (406). To stop an agent (e.g., nicotine) from vaporizing or evaporating from the back end a section of the reservoir could be filled with a soft material such as a wax or grease plug. This plug (408) can be drawn along the reservoir as the mixture is used. In one embodiment, the agent (e.g., nicotine) reservoir is cylindrical. In one embodiment, the agent (e.g., nicotine) reservoir holds a formulation comprising 200 mg of agent (e.g., nicotine) mixed with 1000 mg of propylene glycol. In one embodiment, the agent (e.g., nicotine) reservoir holds a formulation comprising 200 ug of agent (e.g., nicotine) mixed with 1000 ug of propylene glycol. In some cases, the agent (e.g., nicotine) formulation is a liquid formulation.

FIG. 5 illustrates another embodiment of a reservoir. An agent (e.g., nicotine) reservoir (500) can be a porous, open cell foam (502) within a cartridge; a tube, e.g., capillary tube (504) can extend from the reservoir.

FIG. 6 illustrates another embodiment of an agent (e.g., nicotine) reservoir. The mixture can be held in a collapsible bag (602) which can be held within a secondary container (600). A tube, e.g., capillary tube (604) can extend from the reservoir.

In one embodiment, doses of a liquid agent (e.g., liquid nicotine) are held in a safe dose cartridge container until needed. A container for an agent (e.g., nicotine) can comprise a sealing mechanism that can keep the agent (e.g., nicotine) in the container even if the container is crushed. In one embodiment, the sealing mechanism comprises septum sealing. Methods are provided herein for safely puncturing and reclosing access to a drug (e.g., nicotine) cartridge. In one embodiment, a septum and a puncturing needle is used to extract an agent (e.g., nicotine) from a cartridge. A semi-porous material can be used to ensure that the rate of agent (e.g., nicotine) transfer is safe. For example, materials can include a frit or other material (e.g., ceramic, foam, or metal) that has a convoluted or open structure.

In one embodiment, a device comprises a dose cartridge. In one embodiment, the dose cartridge is a disposable dose cartridge. In another embodiment, the dose cartridge houses an agent (e.g., nicotine) formulation and an aerosol creation mechanism as described herein. In another embodiment, the agent (e.g., nicotine) formulation is housed in a reservoir. In one embodiment, the dose cartridge comprises a reservoir comprising an agent (e.g., nicotine) formulation. In one embodiment, the dose cartridge comprises a reservoir comprising an agent (e.g., nicotine) formulation and dispensing tube, e.g., capillary tube, for dispensing the agent (e.g., nicotine) formulation. In one embodiment, the dose cartridge comprises a mouthpiece. In another embodiment, the mouthpiece comprises a cap. The cap can help prevent contamination. The cap can provide a tamper resistance feature. The cap can provide a child resistance feature. In one embodiment, the cap covers both the mouthpiece and any air inlets. In another embodiment, the cap is reusable. In one embodiment, the dose cartridge comprises a mouthpiece at one end and a mating mechanism whereby the dose cartridge can connect to a controller at another end. In one embodiment, the dose cartridge comprises a mechanism for breath detection. In one embodiment, the dose cartridge comprises a flow control valve. In one embodiment, the dose cartridge comprises a flow control valve that can regulate inhalation. The mechanism for breath detection or inhalation sensing can comprise breath sensory components. The breath sensory components can comprise an optical chase whereby light can be routed to and from a flow sensor.

In one embodiment, the dose cartridge comprises a heater element. In one embodiment, the heater element comprises a metal foil. The metal foil can be made of stainless steel or any other electrically resistive material. In one embodiment, the metal foil is made of stainless steel. In one embodiment, the heater element comprises a steel or metal foil that can be about 0.013 mm thick in order to ensure rapid vaporization. In one embodiment, the heater element comprises a coil of wire or wire coil. The coil of wire or wire coil can be from about 0.12 to about 0.5 mm in diameter. In another embodiment, the dose cartridge comprises more than one heater element. In one embodiment, the dose cartridge comprises two heater elements. In some cases, the heater element can be rapidly heated. In one embodiment, a heating element can comprise a heating rate of about $1600^0$ C ($1873.15°K$) per second for a duration of 250 msec, which can cause a $400^0$ C ($673.15°K$) rise in the temperature of the heater element. In some cases, a heater element is activated for a duration of about 10 msec to about 2000 msec, about 10 msec to about 1000 msec, about 10 msec to about 500 msec, about 10 msec to about 250 msec, about 10 msec to about 100 msec, about 50 msec to about 1000 msec, about 50 msec to about 500 msec, about 50 msec to about 250 msec, about 100 msec to about 1000 msec, about 100 msec to about 500 msec, about 100 msec to about 400 msec, or about 100 msec to about 300 msec. In some cases, a heater element is activated for about 10, 50, 100, 150, 200, 250, 300, 350, 400, 450, 500, 550, 600, 650, 700, 750, 800, 850, 900, 950, or 1000 msec. In some cases, a heater element is activated for at least 10, 50, 100, 150, 200, 250, 300, 350, 400, 450, 500, 550, 600, 650, 700, 750, 800, 850, 900, 950, or 1000 msec. In some cases, the maximum temperature of the heater element is about 100, 150, 200, 250, 300, 350, 400, 450, 500, 550, 600°C (a range from about 373.15°K to about 873.15°K). In some cases, the maximum temperature of the heater element is at least 100, 150, 200, 250, 300, 350, 400, 450, 500, 550, 600°C (a range from about 373.15°K to about 873.15°K).

In one embodiment, a device provided herein is made up of multiple components. In one embodiment, the device provided herein is comprised of two components wherein one component comprises a controller and the other component comprises a dose cartridge. In a further embodiment, the controller is reusable and the dose cartridge is replaceable. In yet another embodiment, the dose cartridge is mated to the controller. Mating of the dose cartridge to the controller can be accomplished by inserting the dose cartridge into an interlocking channel in the controller and engaging a locking mechanism. The locking mechanism can comprise a tab or button on the controller which can be depressed. In one embodiment, the dose cartridge is detachable from the controller. In one embodiment, detachment of the dose cartridge is accomplished by releasing the locking mechanism. In one embodiment, releasing the locking mechanism entails depressing the tab or button on the controller. Electrical connection between the dose cartridge and the controller can be accomplished through a set of mating electrical contacts. In one embodiment, attachment or mating of the dose cartridge to the controller establishes a breath detection mechanism. The breath detection mechanism can comprise breath sensory components. In one embodiment, the breath detection mechanism comprises detecting an alteration in an optical signal, wherein attachment or mating of the dose cartridge to the controller establishes an optical path through which the optical signal can be sent and received. In one embodiment, a source and detector of an optical signal is present in the controller, while the dose cartridge comprises an optical path. The optical path can comprise reflectors for reflecting an optical signal. The optical path can comprise a vane, wherein an inhalation can move the vane in such a way as to cause an alteration in an optical signal. In one embodiment, the dose cartridge comprises a vane, wherein an inhalation can move the vane in such a manner as to cause an alteration in an optical signal. The optical signal can be light of any wavelength.

In some cases, a reservoir comprising a liquid formulation comprising a pharmaceutically active agent (e.g., nicotine) is a single unit comprising a pump within the reservoir, a heater element, and a tube in fluid communication with the pump and the heater element. The reservoir can further comprise a protective element that can serve to cover and protect the heater element when the reserovir is not part of an aerosol generating device. The protective element can be retractable. FIG. 95A-C depicts a single unit reservoir. FIG. 95A shows an exterior view of the single unit reservoir (9500), while FIGs. 95B-C show that internally the reserovir comprises a nicotine reservoir (9506) comprising a pump (9508b) connected to an elongated housing comprising a heater element (9504) at the tip. The elongated housing comprising the heater element (9504) can be surrounded by a retractable heater element protector (9508). The single unit reservoir depicted in FIG 95A-C can be one component in a multi-component aerosol generating device as provided herein. The single unit reservoir can be disposable. The single unit reservoir can be refillable. The single-unit reservoir can be non-refillable. In some cases, the single unit reservoir comprises a retractable heater element protector that is retracted when the reservoir is inserted or connected to a separate component to form an aerosol generating device.

## Tube, e.g., Capillary Tube

FIGs. 2A and 2B illustrate embodiments of components of an electronic nicotine delivery device. FIG. 2A illustrates an agent (e.g., nicotine) reservoir (202) and a tube, e.g., capillary tube (204). FIG. 2B illustrates an expanded view of the device. The agent (e.g., nicotine) reservoir can comprise an agent (e.g., nicotine)/propylene glycol (PG) mixture (206). The tube, e.g., capillary tube can comprise a region on the interior which has been coated with an agent (e.g., nicotine)/PG philic material (208) to promote wicking out of a reservoir. A region on the interior which has been coated with an agent (e.g., nicotine)/PG phobic material (210) (such as polytetrafluoroethylene (PTFE)) can lie at the open end. This coating can cause the agent (e.g., nicotine)/PG to stop wicking short of the open end, thereby reducing the surface area of the mixture exposed to air, and air devoid of agent (e.g., nicotine) vapor. The tube, e.g., capillary tube can comprise a heated section (212) of the tube, e.g., capillary tube which, upon heating, can cause the mixture in the tube to vaporize and expand, pushing the mixture from the open end. A ball valve (214) can be trapped between two indentations in the tube, e.g., capillary tube, the end indentation being such that the ball, if pushed by fluid, will form a seal. This configuration can allow the liquid to be ejected from the end upon heating rather than back into the reservoir. All four of these elements can form a pump which can eject a known dose of the mixture from the end of the tube, e.g., capillary tube.

To eject a dose of an agent (e.g., nicotine)/PG mix with a 1:10 ratio, 1 mm$^3$ of material can be in the tube, e.g., capillary tube. For a tube, e.g., capillary tube with an interior diameter of 0.5 mm, the length can be ~5 mm.

### Valve

A valve can be a check valve, and the check valve can be a ball which can be made of a metal, such as stainless steel or can be made of a plastic, such as nylon, delrin, or a homopolymer acetal. The ball can have a diameter less than the interior diameter of the tube, e.g., capillary tube sufficient to allow an agent (e.g., nicotine)/PG mix to wick by it.

### Heater element

A heater element as provided herein can comprise an electrically resistive material. In some cases, an electronic agent (e.g., nicotine) delivery device provided herein comprises a heater element comprising a coil, wherein the coil comprises electrically resistive/conductive material as provided herein. Electrically conductive/ resistive materials that can be useful as resistive heater elements can be those having low mass, low density, and moderate resistivity and that are thermally stable at the temperatures experienced during use of the aerosol generating device. In some cases, a heater element heats and cools rapidly, and can efficiently use energy. Rapid heating of the heater element can provide almost immediate volatilization of an aerosol forming substrate (e.g., liquid formulation comprising nicotine) in proximity thereto. Rapid cooling to a temperature below the volatilization temperature of the substrate can prevent substantial volatilization (and hence waste) of the substrate during periods when aerosol formation is not desired. Such heater elements also permit relatively precise control of the temperature range experienced by the substrate, e.g., when time based current control is employed. In some cases, electrically conductive/resistive materials are chemically non-reactive with the materials being heated (e.g., aerosol precursor materials and other inhalable substance materials) so as not to adversely affect the flavor or content of the aerosol or vapor that is produced. Exemplary, non-limiting, materials that can be used as the electrically conductive/resistive material include carbon, nickel, iron, chromium, graphite, tantalum, stainless steel, gold, platinum, tungsten molybdenum alloy, metal ceramic matrices, carbon/graphite composites, metals, metallic and non-metallic carbides, nitrides, silicides, inter-metallic compounds, cermets, metal alloys (e.g., aluminum alloys, iron alloys, etc.), and metal foils. In some cases, a refractory material is used. Various, different materials can be mixed to achieve the desired properties of resistivity, mass, and thermal conductivity. In some cases, metals that can be utilized include, for example, nickel, chromium, alloys of nickel and chromium (e.g., nichrome), and steel. Suitable metal-ceramic matrices can include silicon carbide aluminum and silicon carbide titanium. Oxidation resistant intermetallic compounds, such as aluminides of nickel and aluminides of iron are also suitable. Of the listed materials, stainless steel and the aluminum, iron or chromium alloys can be encapsulated in a suitable ceramic material because of their reactivity. Suitable ceramic materials for encapsulation include silica, alumina, and sol gels. The heater element can be made of a thin stainless steel foil or wires of the materials described herein. Materials that can be useful for providing resistive heating are described in U.S. Pat. No. 5,060,671; U.S. Pat. No. 5,093,894; U.S. Pat. No. 5,224,498 ; U.S. Pat. No. 5,228,460; U.S. Pat. No. 5,322,075; U.S. Pat. No. 5,353,813; U.S. Pat. No. 5,468,936; U.S. Pat. No. 5,498,850 ; U.S. Pat. No. 5,659,656; U.S. Pat. No. 5,498,855; U.S. Pat. No. 5,530,225; U.S. Pat. No. 5,665,262; U.S. Pat. No. 5,573,692; and U.S. Pat. No. 5,591,368.

A heater element (e.g., resistive heater element) in an aerosol generating device as provided herein can be provided in a form that enables the heater element to be positioned in intimate contact with or in close proximity to the substrate (i.e. to provide heat to the substrate through, for example, conduction, radiation, or convection). In some cases, the substrate is a liquid substrate or formulation comprising a pharmaceutically active agent (e.g., nicotine). In some cases, the heater element can be provided in a form such that the substrate (e.g., liquid substrate) can be delivered to the heater element for vaporization. The delivery of the liquid substrate can take on a variety of embodiments, such as wicking of the liquid substrate to the heater element using a wick (e.g., fibrous wick) in fluid communication with the liquid substrate or flowing the liquid substrate to the heater element, such as through a capillary, which can include valve flow regulation. As such, the liquid substrate can be in one or more reservoirs positioned sufficiently away from the heater element to prevent premature vaporization, but positioned sufficiently close to the heater element to facilitate transport of the liquid substrate, in the desired amount, to the heater element for vaporization. In some cases, the one or more reservoirs comprising a liquid substrate can be located in an annular space surrounding a tubular or cylindrical air flow channel or passageway. In some cases, the heater element is in fluid communication with the liquid substrate stored in one or more reservoirs located in an annular space surrounding an air flow channel or passageway, wherein the heater element is located within the air flow channel or passageway. In some cases, the liquid substrate comprising a pharmaceutically active agent (e.g., nicotine) is delivered to the heater element through the use of a positive displacement pump. The positive displacement pump can be a reciprocating, metering, rotary-type, hydraulic, peristaltic, gear, screw, flexible impeller, diaphragm, piston, or progressive cavity pump, or any other pump utilizing positive displacement as known in the art. The positive displacement pump can be in fluid communication with the heater element. The positive displacement pump can be in fluid communication or fluidically coupled to a reservoir comprising a pharmaceutically active agent (e.g., nicotine). The positive displacement pump can be in fluid communication with the heater element and a reservoir comprising a pharmaceutically active agent (e.g., nicotine). The positive displacement pump can be within an air-flow channel or passageway in an aerosol generating device as provided herein or external to the air flow channel or passageway. The pump can be located within a source of the liquid substrate as provided herein.

The heater element (e.g., electrically resistive material) can be provided in a variety forms, such as in the form of straight line, a foil, a foam, discs, spirals (e.g., single spiral, double spiral, cluster or spiral cluster), fibers, wires, films, yarns, strips, ribbons, or cylinders, as well as irregular shapes of varying dimensions. In some cases, a heater element can be a resistive heater element comprising a conductive substrate, such as described in US20130255702A1 to Griffith et al. In some cases, a heater element can be a resistive heater element that can be present as part of a micro-heater component, such as described in US20140060554A1. In some cases, a heater element is a droplet ejection type heater element such as described in US5894841. In some cases, a heater element comprises an ejector in combination with a heater element (e.g., electrically resistive coil or thin film or foil), such as described in US20050016550A1. In some cases, a heater element comprises a wire coil comprising electrically resistive material wrapped around a wick, wherein the wick has one end within a reservoir comprising the liquid substrate, such as described in US20110094523A1. In some cases, a heater element in an aerosol generating device as provided herein comprises a "cartomizer," wherein the heater element and the reservoir comprising the liquid substrate are configured as a single disposable cartridge or unit. The cartomizer can be a first part of a two part aerosol generating device, wherein the second part can comprise the battery, LED, and a control apparatus (e.g., air-flow switch and any associated processor). In some cases, a heater element in an aerosol generating device as provided herein comprises an improved cartomizer that comprises: (a) a tube shape having an inlet and outlet; (b) a foam substrate for receiving a liquid formulation, the foam substrate defining an an aerosol generation region; (c) a fiberglass member disposed within the aerosol generation region and in contact with the foam substrate to draw the liquid formulation into the region; and (d) a heater element disposed within the aerosol generation region and about the fiberglass member to vaporize the liquid formulation in the aerosol generation region, such as described in US20120199146A1. In some cases, a heater element in an aerosol generating device as provided herein comprises an electrically resistive heater element (e.g., wire coil) with a liquid formulation permeating component (e.g., wicking element) directly sleeved thereon with the liquid permeating component in direct contact with a liquid containing reservoir that surrounds the heater element such as described in US20120111347A1 and US20120279512A1. In some cases, a heater element in an aerosol generating device as provided herein comprises a porous wicking component surrounding a heating rod with an electrically resistive wire coil wrapped thereon, such as described in US20110209717A1, US20130125906A1, US7832410, US8156944, US8393331, or a wire coil wrapped around a fibrous wicking component such as described in US8375957. In some cases, a heater element in an aerosol generating device as provided herein comprises an electrically resistive heater element within an atomization and spray device, such as described in US20110005535A1. In some cases, a heater element comprises an atomizer, wherein the atomizer comprises an atomizer cover, a rubber sleeve, an atomizer sleeve, fibrous storage component infused with a liquid formulation (e.g., nicotine solution), two wires, a heating wire, a rubber pad, a threaded sleeve, a propping pin, a first fiber pipe, wicking element and a second fiber pipe, such as described in US20120145169A1. In some cases, an aerosol generating device as provided herein comprises a vaporization nozzle. The vaporization nozzle can be located within an air flow channel in the aerosol generating device. The vaporization nozzle can be composed of any of the high-temperature resistant with low thermal conductivity materials provided herein. For example, the vaporization nozzle can be made of conventional ceramics or be made of aluminum silicate ceramics, titanium oxide, zirconium oxide, yttrium oxide ceramics, molten silicon, silicon dioxide, molten aluminum oxide. The vaporization nozzle can be made in the shape of a straight line or spiral, and can also be made from polytetrafluoethylene, carbon fiber, glass fiber, or other materials with similar properties. The vaporization nozzle can be a tubule comprising a heater element within the nozzle or on the outside of the nozzle, or can comprise no heater element and the tubule can be directly applied with heating current, such as described in US8511318, US20060196518A1, and US20120090630A1. The heater element arranged within the vaporization nozzle can be made of wires of nickel chromium alloy, iron chromium aluminum alloy, stainless steel, gold, platinum, tungsten molybdenum alloy, etc., and can be in the shape of straight line, single spiral, double spiral, cluster or spiral cluster. The heating function of the heater element in the vaporization nozzle can be achieved by applying a heating coating on the inner wall of the tube, and the coating can be made from electro-thermal ceramic materials, semiconductor materials, or corrosion-resistant metal films, such as gold, nickel, chromium, platinum and molybdenum.

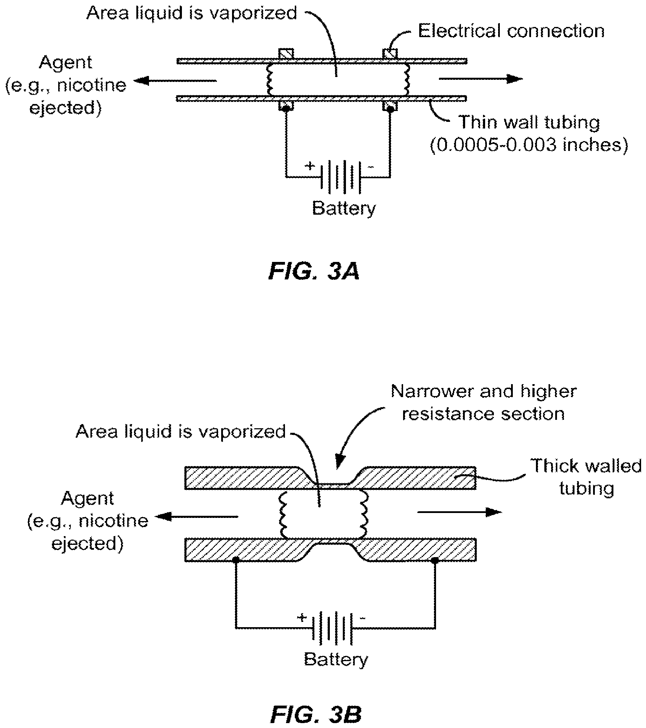

**FIGs. 3A** and **3B** illustrate configurations of a heater element. The tube, e.g., capillary tube can be made of stainless steel, or a similar matter, which has an electrical resistance substantially greater than other metals (aluminum, brass, iron). The tube, e.g., capillary tube can be made of a thin wall material (**FIG. 3A**), or a section of the wall can be narrowed (**FIG. 3B**) to result in that section having an electrical resistance such that when an electrical current is passed across the section heating happens. Alternately the tube, e.g., capillary tube can be wrapped with a heater wire. This configuration can allow for the tube, e.g., capillary tube to be made of a non-electrically conductive material such as Kapton (polyimide), which can withstand heat. Electrical heating can be powered directly from a battery or can be powered from a charged capacitor.

A heater element can be used to vaporize an agent (e.g., nicotine)/PG mixture to form an aerosol with a particle size (MMAD= Mass Median Aerodynamic Diameter) of about 1 to about 5 μm. Aerosols with this particle size can deposit in the deep lung and result in rapid PK.

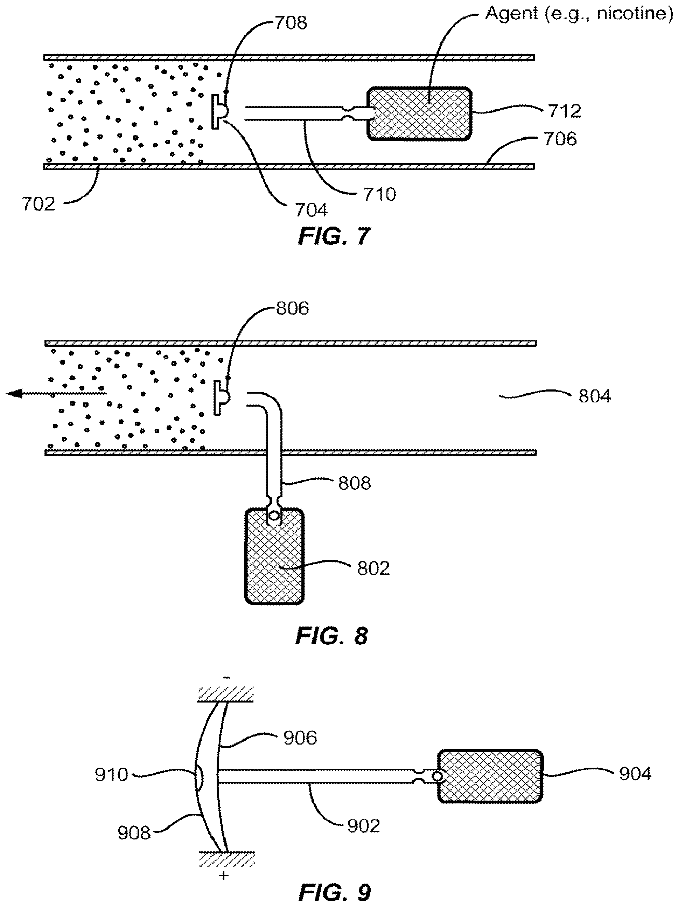

FIG. 7 illustrates a configuration of a heater element (704) in an airway (706). The heater element can be made of a thin stainless steel foil. The foil can be of a thickness of about 0.0005 to about 0.005 inches (a range from about 0.01 mm to about 0.13 mm) thick, or from about 0.0005 to about 0.001 inches (a range from about 0.01 mm to about 0.025 mm) so that less electrical current is needed to vaporize the mixture. The foil can be of a thickness of about, less than, more than, at least or at most 0.0005, 0.0006, 0.0007, 0.0008, 0.0009, 0.001, 0.002, 0.003, 0.003, 0.004, or 0.005 inches (a range from about 0.01 mm to about 0.13 mm). The heater element (704) can be positioned at the exit of the tube, e.g., capillary tube (710) so that the mixture can deposit (708) on the heater element (704). The heater element (704) can be positioned in an airway (706) so that a user upon inhalation can cause the aerosol to pass through the mouthpiece (702) and be drawn into the lungs. The agent (e.g., nicotine) reservoir (712) can be in the airway. FIG. 8 illustrates that in some cases, an agent (e.g., nicotine) reservoir (802) can be placed outside of an airway (804), while the heater element (806) can be in the airway (804). A tube, e.g., capillary tube (808) can enter the airway (804).

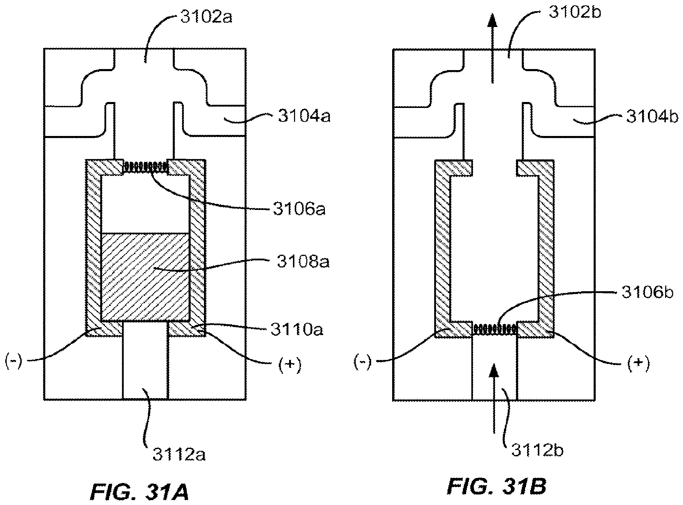

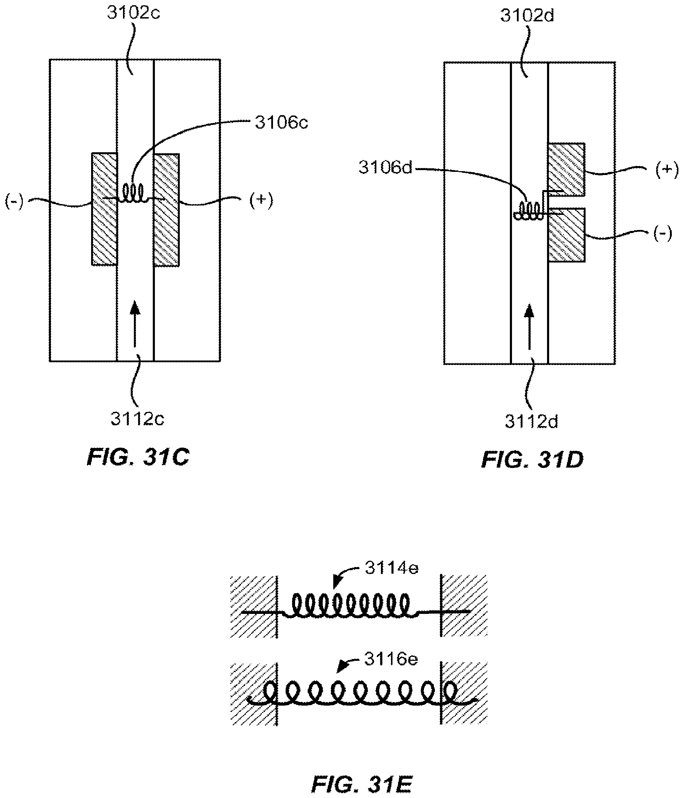

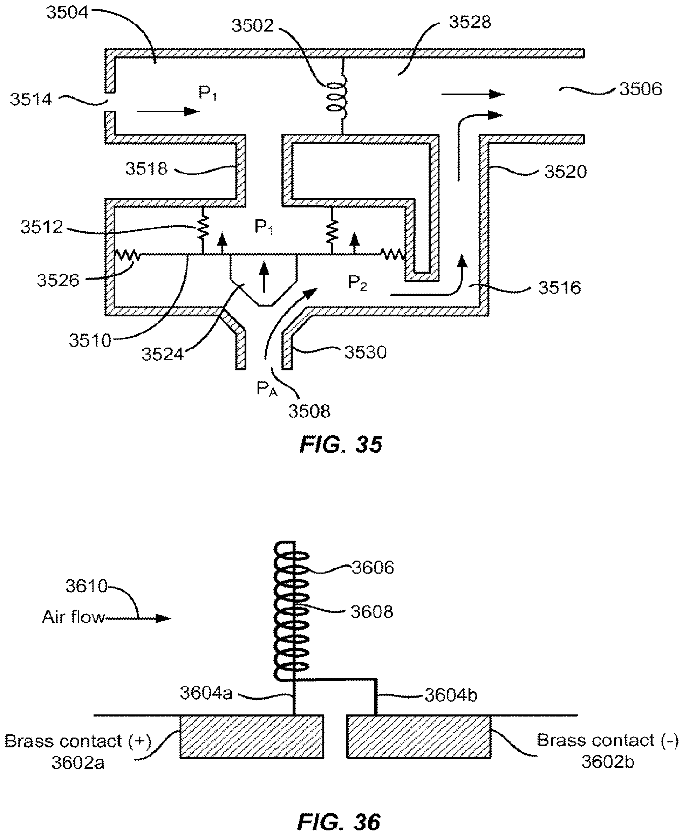

FIGs. 31A-D illustrates another configuration of a heater element (3106a-d) in an airway (3112a-d). FIG. 31A depicts a device (ENT-100-A), comprising a primary carrier gas inlet (3112a), positive and negative brass contacts (3110a), a heater element (3106a) comprising a coil located distally from the inlet to the primary airway (3112a) and two bypass inlets (3104a) located (disposed) downstream of the heater element but prior to the outlet (3102a). FIG. 31B depicts a device designated ENT-100-B, which is the same as ENT-100-A except that the heater element has been moved to be proximal to the inlet of the primary airway (3112b). FIG. 31C depicts a device designated ENT-100-C, which is similar to the ENT-100-A device except that the wire coil heater element has been moved to an intermediate position relative to the location of the coil in ENT-100-A and ENT-100-B. Any of the devices depicted in FIG. 31A-C can comprise the wire coil heater element designated "A Coil" (3114e) or "B Coil" (3116e) as illustrated in FIG. 31E. The coil in both types of heater elements comprise inner diameter of 0.26 inches (about 6.6 mm). The "A Coil" comprises a stretch of coil followed by a straight lead on either end of the coil which connects to the brass contacts. The "B Coil" comprises a stretch of coil, wherein the coil itself connects to the brass contacts. FIG. 31D depicts a device designated ENT-100-D with a primary passageway (3112d) for air to flow through, brass contacts (+/-) embedded within the wall of the primary passageway, and a heater element (3106d) comprising a wire wherein one end of the wire wraps around another segment of the wire, wherein a wire coil is formed with an end of the wire passes through the center of the wire coil. An example of this type of heater element is shown in FIGs. 36-38. In some cases, a liquid formulation comprising a pharmaceutically active agent (e.g., nicotine) is delivered to the heater element of FIGs. 31A-D from a reservoir comprising the liquid formulation comprising a pharmaceutically active agent (e.g., nicotine) through the use of a tube, e.g., capillary tube as provided herein, wherein the tube, e.g., capillary tube is coupled or capable of being coupled to the reservoir. In some cases, a liquid formulation comprising a pharmaceutically active agent (e.g., nicotine) is delivered to the heater element of FIGs. 31A-D from a reservoir comprising the liquid formulation comprising a pharmaceutically active agent (e.g., nicotine) through the use of a positive displacement pump as provided herein, wherein the positive displacement pump is fluidically coupled to the reservoir.

FIG. 9 illustrates another embodiment for a heater element. To aid in reducing an agent (e.g., nicotine) from evaporating from the end of a tube, e.g., capillary tube (902) (attached to an agent (e.g., nicotine) reservoir (904)), the heater element (906) can be positioned to cover the end of the tube, e.g., capillary tube when cold. Upon heating the heater would move away from the end (908) due to thermal expansion, opening up the end and allowing the mixture to leave. The position of deposited material (910) is shown.

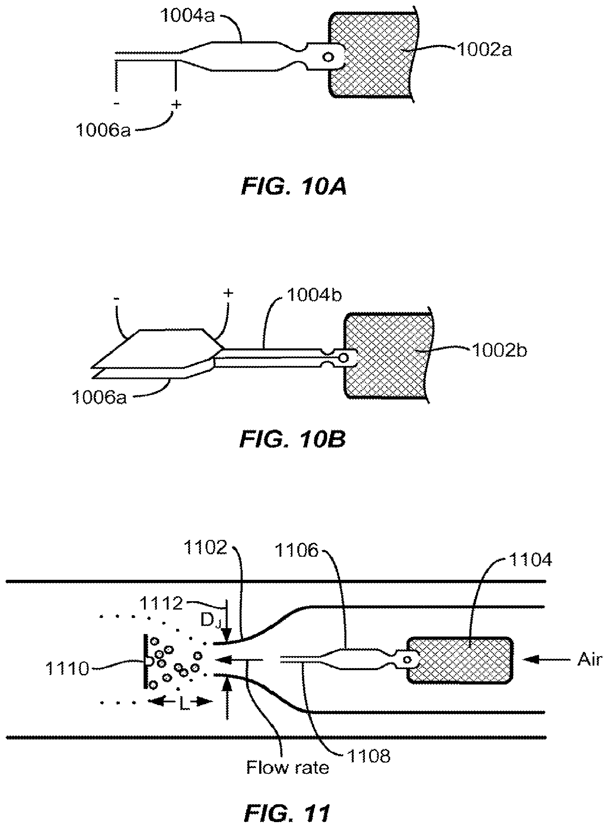

FIGs. 10A and 10B illustrate additional configurations of a heater element. FIG. 10A illustrates that a heater element (1006a) can be positioned at the end of the tube, e.g., capillary tube (1004a), where the tube, e.g., capillary tube can be attached to an agent (e.g., nicotine) reservoir (1002a). FIG. 10B illustrates an agent (e.g., nicotine) reservoir (1002b) and a tube, e.g., capillary tube (1004b), where the geometry of the tube, e.g., capillary tube is modified at the end (1006b) by narrowing or flattening to aid in vaporization.

FIG. 22 illustrates another embodiment of a heater element. The heater element (2200) can be a rod comprising a coil (2202) that can be made of stainless steel, or a similar matter, which has an electrical resistance substantially greater than other metals (aluminum, brass, iron). In some cases, the rod is a wire, wherein the coil is a wire coil. The rod can comprise an electrically resistive material. The electrically resistive material can have an electrical resistance such that when an electrical current is passed across the rod heating happens. The rod is connected to brass contacts (2204) through segments of the rod that do not form the coil. In some cases, the segments of the rod that connect to the brass contacts comprise leads. The brass contacts can serve to pass electrical current across the rod, including the coil. The electrical current can serve to heat the coil and vaporize material (i.e. an agent (e.g., nicotine) mixture) that contacts or is delivered to the coil. The coil can be an open coil that can allow for air to flow between the coils and carry away the vaporized material. In **FIG. 22**, the brass contacts (**2204**) are located (disposed) on either side of an airflow channel and the rod, including the coil, span the channel. In some cases, the coil can be oriented parallel to the flow of a carrier gas (e.g, air). In some cases, the coil can be oriented perpendicular to the flow of a carrier gas (e.g., air). In **FIG. 22,** a tube, e.g., capillary tube (**2206**) attached to a reservoir (**2208**) comprising an agent (e.g., nicotine) mixture is located at one end of the coil and an agent (e.g., nicotine) mixture is dispensed from the end of the tube, e.g., capillary tube onto the coil. The agent (e.g., nicotine) mixture, once dispensed, can wick along the coil to cover the entire or part of the coil. The coil can be heated which can vaporize the agent (e.g., nicotine) mixture.

**FIGs. 36-38** illustrate yet another embodiment of a heater element. In this embodiment, a first (**3602a; +**) and a second (**3602b; -**) brass contact or terminal are located adjacent to each other. The brass contacts can be embedded within or placed proximal to a wall of a housing or channel of a device for generating an aerosol as provided herein. The heater element can be a rod comprising electrically resistive material, wherein a first end or lead (**3604a**) is connected to one brass contact (**3602a; +**), while a second end or lead (**3604b**) is connected to another, separate brass contact (**3602b; -**). As illustrated in **FIG. 36**, a portion or segment of the rod between the leads is configured into a coil (**3606**). In addition, a separate portion or segment (**3608**) of the rod passes through the interior of the coil (**3606**). Supplying current to the rod through the brass contacts (**3602a,b**) can serve to heat both the coil (**3606**) as well as the segment (**3608**) of the rod that passes through the interior of the coil (**3606**). In some cases, the segment of the rod that runs through the center of the coil is capable of holding a liquid formulation comprising an agent (i.e. nicotine) as provided herein. The liquid formulation can wick or be delivered by any of dosing mechanisms provided herein onto the segment of the rod that runs through the center of the coil from a source of the liquid formulation (e.g., a reservoir). In some cases, supplying current to the rod through the brass contacts (**3602a,b**) serves to heat both the coil (**3606**) as well as the segment (**3608**) of the rod that passes through the interior of the coil (**3606**)**,** wherein a liquid formulations that wicks or is delivered by any of dosing mechanisms provided herein onto the segment of the rod running through the coil is vaporized. In **FIG. 36**, the coil is oriented perpendicular to the the flow of a carrier gas (e.g. air flow) (**3610**). In some cases, the coil is oriented parallel to the flow of a carrier gas (e.g. air flow) in a device for generating a condensation aerosol as described herein. **FIGs. 37A** and **37B** depict alternate embodiments to the heater element illustrated in **FIG. 36**, wherein the number of coils shown in the heater element of **FIG. 37A** is reduced in the heater element of **FIG. 37B.** As shown in **FIGs. 37A-B,** alternating the number of coils (**3702b, 3702b**) in the coil serves to increase the length of the non-coil segments (**3704a, 3704b**) of the rod and decrease the length of the rod covered by the coil. **FIG. 38** illustrates components of the rod and coil in the heater element illustrated in **FIG. 36**, including the diameter of the rod (**3802**)**,** total length of the coil (**3804**) (e.g., 0.1 to 0.15 inches (a range from about 2.54 mm to about 3.81mm)), inner diameter of the coil (**3808**) (e.g., 0.027-0.040 inches (about 0.6 mm to about 1.02 mm)), outer diameter of the coil (**3806**) (e.g., 0.047-0.06 inches (a range from about 1.19 mm to about 1.53 mm)), and pitch of the coil (**3810**).

In some cases, the heater element can comprise a rod comprising electrically resistive material. The rod can be a wire. The wire can be made of any of the electrically resistive/conductive materials described herein. The rod can be a pliable rod. A heater element comprising a rod as provided herein can comprise a coil and a wick element around which the coil can be wrapped. The wick element can be capable of being heated. The wick element can be connected to the rod. The wick element can be continuous with the rod. The wick element can be independent of the rod. In some cases, the wick element is capable of being heated, and wherein the wick element is connected to the rod. The rod can be a wire. The coil can be a wire coil. The rod can comprise a coil along the entire length of the wick element. The wick element can be capable of wicking or holding a liquid formulation comprising an agent as provided herein. The wick element can be a capillary (a self wicking tube). The liquid formulation comprising an agent as provided herein can be in fluid communication with a source of the liquid formulation. The source of the liquid formulation can be any source as provided herein, including but not limited to, a reservoir. The liquid formulation comprising an agent as provided herein can be delivered to the wick element by any means known in the art. The delivery can be through capillary action or through the use of a pump. In some cases, the rod comprises a capillary wherein the capillary is in fluid communication with a reservoir, wherein the reservoir comprises a liquid formulation comprising a pharmaceutically active agent (e.g. nicotine), and wherein the capillary is capable of holding the liquid formulation comprising a pharmaceutically active agent (e.g. nicotine). The wick element can be made of any material known in the art capable of wicking or holding a liquid formulation comprising an agent as provided herein. In some cases, the coil connects to a source of electricity. The coil can connect to the source of electricity through one or more leads protruding from both ends of the coil. The source of electricity can be a battery or a charged capacitor. The battery can be rechargeable.

In some cases, the coil can wrap around or span exactly, about, more than, less than, at least, or at most 1%, 2%, 3%,4%,5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100% of the length of the wick element. In some cases, the coil can wrap around or span between 1-10%, 10-20%, 20-30%, 30-40%, 40-50%,

50-60%, 60-70%, 70-80%, 80-90%, or 90-100%, 10-20% of the length of the wick element. In some cases, the coil can wrap around or span of about 1 to about 10%, about 10 to about 20%, about 20 to about 30%, about 30 to about 40%, about 40 to about 50%, about 50 to about 60%, about 60 to about 70%, about 70 to about 80%, about 80 to about 90%, or about 90 to about 100% of the length of the wick element.

The total length of the coil can be exactly, about, more than, less than, at least, or at most 0.01, 0.0125, 0.015, 0.0175, 0.02, 0.0225, 0.025, 0.0275, 0.03, 0.0325, 0.035, 0.0375, 0.04, 0.0425, 0.045, 0.0475, 0.05, 0.0525, 0.055, 0.0575, 0.06, 0.0625, 0.065, 0.0675, 0.07, 0.0725, 0.075, 0.0775, 0.08, 0.0825, 0.085, 0.0875, 0.09, 0.0925, 0.095, 0.0975, 0.1, 0.11, 0.115, 0.12, 0.125, 0.13, 0.135, 0.14, 0.145, 0.15, 0.155, 0.16, 0.165, 0.17, 0.175, 0.18, 0.185, 0.19, 0.195 or 0.2 inches (a range from about 0.25 mm to about 5.08 mm). The total length of the coil can be between 0.01-0.015, 0.015-0.02, 0.02-0.025, 0.025-0.030, 0.03-0.035, 0.035-0.04, 0.04-0.045, 0.045-0.05, 0.05-0.055, 0.055-0.06, 0.06-0.065, 0.065-0.07, 0.07-0.075, 0.075-0.08, 0.08-0.085, 0.085-0.09, 0.09-0.095, 0.095-0.1, 0.1-0.12, 0.12-0.13, 0.13-0.14, 0.14-0.15, 0.15-0.16, 0.16-0.17, 0.17-0.18, 0.18-0.19, or 0.19-0.2 inches (a range from about 0.25 mm to about 5.08 mm). The total length of the coil can be about 0.01 to about 0.015, about 0.015 to about 0.02, about 0.02 to about 0.025, about 0.025 to about 0.03, about 0.03 to about 0.035, about 0.035 to about 0.04, about 0.04 to about 0.045, about 0.045 to about 0.05, about 0.05 to about 0.055, about 0.055 to about 0.06, about 0.06 to about 0.065, about 0.065 to about 0.07, about 0.07 to about 0.075, about 0.075 to about 0.08, about 0.08 to about 0.085, about 0.085 to about 0.09, about 0.09 to about 0.095, about 0.095 to about 0.1, about 0.1 to about 0.12, about 0.12 to about 0.13, about 0.13 to about 0.14, about 0.14 to about 0.15, about 0.15 to about 0.16, about 0.16 to about 0.17, about 0.17 to about 0.18, about 0.18 to about 0.19, or about 0.19 to about 0.2 inches (a range from about 0.25 mm to about 5.08 mm).

A heater element comprising a rod as provided herein can comprise one or more segments comprising a coil and one or more segments not comprising a coil (i.e. non-coil segment). The rod can be a wire. The coil can be a wire coil. One or more non-coil segments of the rod can be capable of wicking or holding a liquid formulation comprising an agent as provided herein. The non-coil segment can act as a capillary or wick. In some cases, one or more non-coil segments of the rod comprise a wick element. One or more wick elements can be capable of being heated, thereby forming one or more heated wick elements. The liquid formulation comprising an agent as provided herein can be in fluid communication with a source of the liuid formulation. The source of the liquid formulation can be any source as provided herein, including, but not limited to, a reservoir. The liquid formulation comprising an agent as provided herein can be delivered to a non-coil segment of the rod by any means known in the art. The delivery can be through capillary action or through the use of a pump. In some cases, the non-coil segment is in fluid communication with a reservoir, wherein the reservoir comprises a liquid formulation comprising a pharmaceutically active agent (e.g. nicotine), and wherein the non-coil segment is capable of holding the liquid formulation comprising a pharmaceutically active agent (e.g., nicotine).

The non-coil segments can serve as electrical leads for connecting the rod to a source of electricity. The rod can comprise a coil along the entire length of the rod. In some cases, the coil connects to the source of electricity. The source of electricity can be a battery or a charged capacitor. The battery can be rechargeable.

In some cases, a distance between the first and second leads of the rod when the first lead is connected to either the first or second terminal of the power source while the second lead is connected to the other of the first or second terminal of the power source is about, more than, less than, or at least 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.11, 0.12, 0.13, 0.14, 0.15, 0.16, 0.17, 0.18, 0.19, or 0.2 inches (a range from about 0.254 mm to about 5.08 mm). A distance between the first and second leads of the rod when the first lead is connected to either the first or second terminal of the power source while the second lead is connected to the other of the first or second terminal of the power source is from about 0.01 to about 0.1 inches, about 0.02 to about 0.09 inches, or about 0.025 to about 0.8 inches (a range from about 0.254 mm to about 20.32 mm).

In some cases, the coil can wrap around a non-coil segment of the rod, wherein the non-coil segment passes through the coil. In these cases, the coil can wrap around or span exactly, about, more than, less than, at least, or at most 1%, 2%, 3%,4%,5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100% of the length a non-coil segment of the rod. In these cases, the coil can wrap around or span between 1-10%, 10-20%, 20-30%, 30-40%, 40-50%, 50-60%, 60-70%, 70-80%, 80-90%, or 90-100%, 10-20% of the length a non-coil segment of the rod. In these cases, the coil can wrap around or span of about 1 to about 10%, about 10 to about 20%, about 20 to about 30%, about 30 to about 40%, about 40 to about 50%, about 50 to about 60%, about 60 to about 70%, about 70 to about 80%, about 80 to about 90%, or about 90 to about 100% of the length of a non-coil segment of the rod.

The total length of the coil can be exactly, about, more than, less than, at least, or at most 0.01, 0.0125, 0.015, 0.0175, 0.02, 0.0225, 0.025, 0.0275, 0.03, 0.0325, 0.035, 0.0375, 0.04, 0.0425, 0.045, 0.0475, 0.05, 0.0525, 0.055, 0.0575, 0.06, 0.0625, 0.065, 0.0675, 0.07, 0.0725, 0.075, 0.0775, 0.08, 0.0825, 0.085, 0.0875, 0.09, 0.0925, 0.095, 0.0975, 0.1, 0.11, 0.115, 0.12, 0.125, 0.13, 0.135, 0.14, 0.145, 0.15, 0.155, 0.16, 0.165, 0.17, 0.175, 0.18, 0.185, 0.19, 0.195 or 0.2 inches (a range from about 0.254 mm to about 5.08 mm). The total length of the coil can be between 0.01-0.015, 0.015-0.02, 0.02-0.025, 0.025-0.030, 0.03-0.035, 0.035-0.04, 0.04-0.045, 0.045-0.05, 0.05-0.055, 0.055-0.06, 0.06-0.065, 0.065-0.07, 0.07-0.075, 0.075-0.08, 0.08-0.085, 0.085-0.09, 0.09-0.095, 0.095-0.1, 0.1-0.12, 0.12-0.13, 0.13-0.14, 0.14-0.15, 0.15-0.16, 0.16-0.17, 0.17-0.18, 0.18-0.19, or 0.19-0.2 inches (a range from about 0.254 mm to about 5.08 mm). The total length of the coil can be about 0.01 to about 0.015, about 0.015 to about 0.02, about 0.02 to about 0.025, about 0.025 to about 0.03, about 0.03 to about 0.035, about 0.035 to about 0.04, about 0.04 to about 0.045, about 0.045 to about 0.05, about 0.05 to about 0.055, about 0.055 to about 0.06, about 0.06 to about 0.065, about 0.065 to about 0.07, about 0.07 to about 0.075, about 0.075 to about 0.08, about 0.08 to about 0.085, about 0.085 to about 0.09, about 0.09 to about 0.095, about 0.095 to about 0.1, about 0.1 to about 0.12, about 0.12 to about 0.13, about 0.13 to about 0.14, about 0.14 to about 0.15, about 0.15 to about 0.16, about 0.16 to about 0.17, about 0.17 to about 0.18, about 0.18 to about 0.19, or about 0.19 to about 0.2 inches (a range from about 0.254 mm to about 5.08 mm).

A heater element comprising a rod as provided herein can comprise one or more coils. The rod can be a wire. The coil can be a wire coil. The coil can have exactly, about, more than, less than, at least or at most 1, 1.5, 2, 2.5, 3, 3.5, 4, 4.5, 5, 5.5, 6, 6.5, 7, 7.5, 8, 8.5, 9, 9.5 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35, 35.5, 36, 36.5, 37, 37.5, 38, 38.5, 39, 39.5, 40, 41, 42, 43, 44, 45, 46, 47, 4, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 105, 110, 115, 120, 125, 130, 135, 140, 145, 150, 155, 160, 165, 170, 175, 180, 185, 190, 195, or 200 coils. The coil can comprise 1-2, 2-4, 4-6, 6-8, 8-10, 10-12, 12-14, 14-16, 16-18, or 18-20 coils. The coil can comprise 2-20, 4-20, 6-20, 8-20, 10-20, 12-20, 14-20, or 16-20 coils. The coil can comprise between 1-5, 5-10, 10-15, or 15-20 coils. The coil can comprise between 1-10, 1-20, 1-30, 1-40, 1-60, 1-70, 1-80, 1-90, 1-100 coils. The coil can comprise from about 1 to about 5, about 5 to about 10, about 10 to about 15, or about 15 to about 20 coils. The coil can comprise from about 1 to about 10, about 1 to about 20, about 1 to about 30, about 1 to about 40, about 1 to about 60, about 1 to about 70, about 1 to about 80, about 1 to about 90, or about 1 to about 100 coils. In one embodiment, the coil comprises from about 5 to about 10 coils. In one embodiment, the coil comprises from about 1 to about 10 coils. The distance between successive coils or the pitch of the coils can be exactly, about, more than, less than, at least or at most 0.01, 0.0125, 0.015, 1.17, 0.0175, 0.02, 0.0225, 0.025, 0.0275, 0.03, 0.0325, 0.035, 0.0375, 0.04, 0.0425, 0.045, 0.0475, 0.05, 0.0525, 0.055, 0.0575, 0.06, 0.0625, 0.065, 0.0675, 0.07, 0.0725, 0.075, 0.0775, 0.08, 0.0825, 0.085, 0.0875, 0.09, 0.0925, 0.095, 0.0975, 0.1, 0.11, 0.115, 0.12, 0.125, 0.13, 0.135, 0.14, 0.145, 0.15, 0.155, 0.16, 0.165, 0.17, 0.175, 0.18, 0.185, 0.19, 0.195 or 0.2 inches (a range from about 0.254 mm to about 5.08 mm). The distance between successive coils or the pitch of the coils can be between 0.01-0.015, 0.015-0.3, 0.01-0.02, 0.015-0.02, 0.020-0.025, 0.025-0.03, 0.03-0.035, 0.035-0.04, 0.04-0.045, 0.045-0.05, 0.05-0.055, 0.055-0.06, 0.06-0.065, 0.065-0.07, 0.07-0.075, 0.075-0.08, 0.08-0.085, 0.085-0.09, 0.09-0.095, 0.095-0.1, 0.1-0.12, 0.12-0.13, 0.13-0.14, 0.14-0.15, 0.15-0.16, 0.16-0.17, 0.17-0.18, 0.18-0.19, or 0.19-0.2 inches (a range from about 0.254 mm to about 5.08 mm). The distance between successive coils or the pitch of the coils can be about 0.01 to about 0.015, about 0.01 to about 0.02, about 0.015 to about 0.3, about 0.015 to about 0.02, about 0.02 to about 0.025, about 0.025 to about 0.03, about 0.03 to about 0.035, about 0.035 to about 0.04, about 0.04 to about 0.045, about 0.045 to about 0.05, about 0.05 to about 0.055, about 0.055 to about 0.06, about 0.06 to about 0.065, about 0.065 to about 0.07, about 0.07 to about 0.075, about 0.075 to about 0.08, about 0.08 to about 0.085, about 0.085 to about 0.09, about 0.09 to about 0.095, about 0.095 to about 0.1, about 0.1 to about 0.12, about 0.12 to about 0.13, about 0.13 to about 0.14, about 0.14 to about 0.15, about 0.15 to about 0.16, about 0.16 to about 0.17, about 0.17 to about 0.18, about 0.18 to about 0.19, or about 0.19 to about 0.2 inches (a range from about 0.254 mm to about 5.08 mm).

A rod in a heater element comprising a rod as provided herein can have a diameter of exactly, about, more than, less than, at least or at most 0.005, 0.006, 0.007, 0.008, 0.009, 0.01, 0.011, 0.012, 0.013, 0.014, 0.015, 0.016, 0.017, 0.018, 0.019, or 0.02 inches (a range from about 0.127 mm to about 0.51 mm). The rod can have a diameter between 0.005 and 0.01, 0.01 and 0.015, or 0.015 and 0.02 inches (a range from about 0.127 mm to about 0.51 mm). In one embodiment, the rod has a diameter between 0.005 and 0.02 inches (a range from about 0.127 mm to about 0.51 mm). In one embodiment, the rod has a diameter between 0.008 and 0.0012 inches (a range from about 0.2032 mm to about 0.03 mm).. The rod can have a diameter of about 0.005 to about 0.01, about 0.01 to about 0.015, or about 0.015 to about 0.02 inches (a range from about 0.127 mm to about 0.508 mm). In one embodiment, the rod has a diameter of about 0.005 to about 0.02 inches (a range from about 0.127 mm to about 0.508 mm). In one embodiment, the rod has a diameter of about 0.008 to about 0.0012 inches (a range from about 0.2031 mm to about 0.03 mm). The rod can be a wire.

A heater element comprising a coil as provided herein can have a coil with an inner or internal diameter of exactly, about, more than, less than, at least or at most 0.01, 0.012, 0.0125, 0.015, 0.0175, 0.02, 0.022, 0.0225, 0.025, 0.0275, 0.03, 0.032, 0.0325, 0.035, 0.0375, 0.04, 0.042 0.0425, 0.045, 0.0475, 0.05, 0.0520.0525, 0.055, 0.0575, 0.06, 0.062, 0.0625, 0.065, 0.0675, 0.07, 0.072, 0.0725, 0.075, 0.0775, 0.08, 0.0825, 0.085, 0.0875, 0.09, 0.0925, 0.095, 0.0975, 0.1, 0.11, 0.115, 0.12, 0.125, 0.13, 0.135, 0.14, 0.145, 0.15, 0.155, 0.16, 0.165, 0.17, 0.175, 0.18, 0.185, 0.19, 0.195, 0.2, 0.21, 0.22, 0.23, 0.24, 0.25, 0.26, 0.27, 0.28, 0.29, 0.3, 0.35, 0.4, 0.45, or 0.5 inches (a range from about 0.254 mm to about 12.7 mm). The inner or internal diameter of the coil can be between 0.01-0.015, 0.015-0.02, 0.02-0.025, 0.0250-0.03, 0.03-0.035, 0.035-0.04, 0.04-0.045, 0.045-0.05, 0.05-0.055, 0.055-0.06, 0.06-0.065, 0.065-0.07, 0.07-0.075, 0.075-0.08, 0.08-0.085, 0.085-0.09, 0.09-0.095, 0.095-0.1, 0.1-0.12, 0.12-0.13, 0.13-0.14, 0.14-0.15, 0.15-0.16, 0.16-0.17, 0.17-0.18, 0.18-0.19, 0.19-0.2, 0.2-0.21, 0.21-0.22, 0.22-0.23, 0.23-0.24, 0.24-0.25, 0.25-0.26, 0.26-0.27, 0.27-0.28, 0.28-0.29, 0.29-0.3, 0.3-0.35, 0.35-0.4, 0.4-0.45, or 0.45-0.5 inches (a range from about 0.254 mm to about 12.7 mm). The inner or internal diameter of the coil can be about 0.01 to about 0.015, about 0.015 to about 0.02, about 0.02 to about 0.025, about 0.025 to about 0.03, about 0.03 to about 0.035, about 0.035 to about 0.04, about 0.04 to about 0.045, about 0.045 to about 0.05, about 0.05 to about 0.055, about 0.055 to about 0.06, about 0.06 to about 0.065, about 0.065 to about 0.07, about 0.07 to about 0.075, about 0.075 to about 0.08, about 0.08 to about 0.085, about 0.085 to about 0.09, about 0.09 to about 0.095, about 0.095 to about 0.1, about 0.1 to about 0.15, about 0.15 to about 0.2, about 0.2 to about 0.25, about 0.25 to about 0.3, about 0.3 to about 0.35, about 0.35 to about 0.4, about 0.4 to about 0.45, or about 0.45 to about 0.5 inches (a range from about 0.254 mm to about 12.7 mm). The inner or internal diameter of the coil can be between 0.02 and 0.04, 0.04 and 0.06, or 0.02 and 0.06 inches (a range from about 0.508 mm to about 1.524 mm). In one embodiment, the inner or internal diameter of the coil is between 0.03 and 0.04 inches (a range from about 0.3 mm to about 1.02 mm). The inner or internal diameter of the coil can be about 0.02 to about 0.04, about 0.04 to about 0.06, or about 0.02 to about 0.06 inches (a range from about 0.508 mm to about 1.524 mm). In one embodiment, the inner or internal diameter of the coil is about 0.03 to about 0.04 inches (a range from about 0.3 mm to about 1.02 mm). In one embodiment, the inner or internal diameter of the coil is about 0.02 to about 0.04 inches (a range from about 0.508 mm to about 1.02 mm). In one embodiment, the inner or internal diameter of the coil is between 0.02 to about 0.04 inches (a range from about 0.508 mm to about 1.02 mm). The coil can be a wire coil.

A heater element comprising a coil as provided herein can have a coil with an outer or external diameter of exactly, about, more than, less than, at least or at most 0.01, 0.0125, 0.015, 0.0175, 0.02, 0.0225, 0.025, 0.0275, 0.03, 0.0325, 0.035, 0.0375, 0.04, 0.0425, 0.045, 0.0475, 0.05, 0.0525, 0.055, 0.0575, 0.06, 0.0625, 0.065, 0.0675, 0.07, 0.0725, 0.075, 0.0775, 0.08, 0.0825, 0.085, 0.0875, 0.09, 0.0925, 0.095, 0.0975, 0.1, 0.11, 0.115, 0.12, 0.125, 0.13, 0.135, 0.14, 0.145, 0.15, 0.155, 0.16, 0.165, 0.17, 0.175, 0.18, 0.185, 0.19, 0.195, 0.2, 0.21, 0.22, 0.23, 0.24, 0.25, 0.26, 0.27, 0.28, 0.29, 0.3, 0.35, 0.4, 0.45, or 0.5 inches (a range from about 0.254 mm to about 12.7 mm). The outer or external diameter of the coil can be between 0.01-0.015, 0.015-0.02, 0.02-0.025, 0.025-0.03, 0.03-0.035, 0.035-0.04, 0.04-0.045, 0.045-0.05, 0.05-0.055, 0.055-0.06, 0.06-0.065, 0.065-0.07, 0.07-0.075, 0.075-0.08, 0.08-0.085, 0.085-0.09, 0.09-0.095, 0.095-0.1, 0.1-0.12, 0.12-0.13, 0.13-0.14, 0.14-0.15, 0.15-0.16, 0.16-0.17, 0.17-0.18, 0.18-0.19, 0.19-0.2, 0.2-0.21, 0.21-0.22, 0.22-0.23, 0.23-0.24, 0.24-0.25, 0.25-0.26, 0.26-0.27, 0.27-0.28, 0.28-0.29, 0.29-0.3, 0.3-0.35, 0.35-0.4, 0.4-0.45, or 0.45-0.5 inches (a range from about 0.254 mm to about 12.7 mm). The outer or external diameter of the coil can be about 0.01 to about 0.015, about 0.015 to about 0.02, about 0.02 to about 0.025, about 0.025 to about 0.03, about 0.03 to about 0.035, about 0.035 to about 0.04, about 0.04 to about 0.045, about 0.045 to about 0.05, about 0.05 to about 0.055, about 0.055 to about 0.06, about 0.06 to about 0.065, about 0.065 to about 0.07, about 0.07 to about 0.075, about 0.075 to about 0.08, about 0.08 to about 0.085, about 0.085 to about 0.09, about 0.09 to about 0.095, about 0.095 to about 0.1, about 0.1 to about 0.15, about 0.15 to about 0.2, about 0.2 to about 0.25, about 0.25 to about 0.3, about 0.3 to about 0.35, about 0.35 to about 0.4, about 0.4 to about 0.45, or about 0.45 to about 0.5 inches (a range from about 0.254 mm to about 12.7 mm). The outer or external diameter of the coil can be between 0.02 and 0.04, 0.04 and 0.06, or 0.02 and 0.06 inches (a range from about .02 mm and 1.02 mm to about 0.02 mm and 1.524 mm). In one embodiment, the outer or external diameter of the coil is between 0.03 and 0.04 inches (a range from about 0.0.762 mm to about 1.02 mm). The outer or external diameter of the coil can be about 0.02 to about 0.04, about 0.04 to about 0.06, about 0.02 to about 0.06 inches, about 0.02 to about 0.08 inches, about 0.02 to about 0.1 inches (a range from about 0.508 mm to about 2.54 mm). In one embodiment, the outer or external diameter of the coil is about 0.03 to about 0.04 inches (a range from about 0.762 mm to about 1.02 mm). In one embodiment, the outer or external diameter of the coil is about 0.02 to about 0.04 inches (a range from about 0.508 mm to about 1.02 mm). In one embodiment, the outer or external diameter of the coil is between 0.02 to about 0.04 inches (a range from about 0.508 mm to about 1.02 mm). The coil can be a wire coil.

A heater element comprising a coil as provided herein can have a coil with a length to width aspect ratio exactly, about, more than, less than, at least or at most 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, 10, 10.5, 11, 11.5, 12, 12.5, 13, 13.5, 14, 14.5, or 15. The length to width aspect ratio of the coil can be between 0.1-0.15, 0.15-0.2, 0.2-0.25, 0.25-0.3, 0.3-0.35, 0.35-0.4, 0.4-0.45, 0.45-0.5, 0.5-0.55, 0.55-0.6, 0.6-0.65, 0.65-0.7, 0.7-0.75, 0.75-0.8, 0.8-0.85, 0.85-0.9, 0.9-0.95, 0.95-1, 1-1.5, 1.5-2, 2-2.5, 2.5-3, 3-3.5, 3.5-4, 4-4.5, 4.5-5, 5-5.5, 5.5-6, 6-6.5, 6.5-7, 7-7.5, 7.5-8, 8-8.5, 8.5-9, 9-9.5, 9.5-10, 10-10.5, 10.5-11, 11-11.5, 11.5-12, 12.5-13, 13-13.5, 13.5-14, 14-14.5, or 14.5-15. The length to width aspect ratio of the coil can be about 0.1 to about 0.15, about 0.15 to about 0.2, about 0.2 to about 0.25, about 0.25 to about 0.3, about 0.3 to about 0.35, about 0.35 to about 0.4, about 0.4 to about 0.45, about 0.45 to about 0.5, about 0.5 to about 0.55, about 0.55 to about 0.6, about 0.6 to about 0.65, about 0.65 to about 0.7, about 0.7 to about 0.75, about 0.75 to about 0.8, about 0.8 to about 0.85, about 0.85 to about 0.9, about 0.9 to about 0.95, about 0.95 to about 1, about 1 to about 1.5, about 1.5 to about 2, about 2 to about 2.5, about 2.5 to about 3, about 3 to about 3.5, about 3.5 to about 4, about 4 to about 4.5, about 4.5 to about 5, about 5 to about 5.5, about 5.5 to about 6, about 6 to about 6.5, about 6.5 to about 7, about 7 to about 7.5, about 7.5 to about 8, about 8 to about 8.5, about 8.5 to about 9, about 9 to about 9.5, about 9.5 to about 10, about 10 to about 10.5, about 10.5 to about 11, about 11 to about 11.5, about 11.5 to about 12, about 12.5 to about 13, about 13 to about 13.5, about 13.5 to about 14, 14 to about 14.5, or about 14.5 to about 15. The width of the coil in the length to width aspect ratio can be the inner or internal diameter, or the outer or external diameter. The coil can be a wire coil.

A heater element comprising a rod as provided herein, wherein the rod can have a coil wherein a ratio of the diameter of the rod to the diameter of the coil can be exactly, about, more than, less than, at least or at most 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, 10, 10.5, 11, 11.5, 12, 12.5, 13, 13.5, 14, 14.5, or 15. The ratio of the diameter of the rod to the diameter of the coil can be between 0.1-0.15, 0.15-0.2, 0.2-0.25, 0.25-0.3, 0.3-0.35, 0.35-0.4, 0.4-0.45, 0.45-0.5, 0.5-0.55, 0.55-0.6, 0.6-0.65, 0.65-0.7, 0.7-0.75, 0.75-0.8, 0.8-0.85, 0.85-0.9, 0.9-0.95, 0.95-1, 1-1.5, 1.5-2, 2-2.5, 2.5-3, 3-3.5, 3.5-4, 4-4.5, 4.5-5, 5-5.5, 5.5-6, 6-6.5, 6.5-7, 7-7.5, 7.5-8, 8-8.5, 8.5-9, 9-9.5, 9.5-10, 10-10.5, 10.5-11, 11-11.5, 11.5-12, 12.5-13, 13-13.5, 13.5-14, 14-14.5, or 14.5-15. The ratio of the diameter of the rod to the diameter of the coil can be about 0.1 to about 0.15, about 0.15 to about 0.2, about 0.2 to about 0.25, about 0.2 to about 0.4, about 0.25 to about 0.3, about 0.3 to about 0.35, about 0.35 to about 0.4, about 0.4 to about 0.45, about 0.45 to about 0.5, about 0.5 to about 0.55, about 0.55 to about 0.6, about 0.6 to about 0.65, about 0.65 to about 0.7, about 0.7 to about 0.75, about 0.75 to about 0.8, about 0.8 to about 0.85, about 0.85 to about 0.9, about 0.9 to about 0.95, about 0.95 to about 1, about 1 to about 1.5, about 1.5 to about 2, about 2 to about 2.5, about 2.5 to about 3, about 3 to about 3.5, about 3.5 to about 4, about 4 to about 4.5, about 4.5 to about 5, about 5 to about 5.5, about 5.5 to about 6, about 6 to about 6.5, about 6.5 to about 7, about 7 to about 7.5, about 7.5 to about 8, about 8 to about 8.5, about 8.5 to about 9, about 9 to about 9.5, about 9.5 to about 10, about 10 to about 10.5, about 10.5 to about 11, about 11 to about 11.5, about 11.5 to about 12, about 12.5 to about 13, about 13 to about 13.5, about 13.5 to about 14, 14 to about 14.5, or about 14.5 to about 15. The width of the coil in the ratio of the diameter of the rod to a diameter of the coil can be the inner or internal diameter, or the outer or external diameter. The rod can be a wire. The coil can be a wire coil.

A heater element comprising a rod is provided herein, wherein the rod can have a coil wherein the volume of the rod can be less than the volume of the coil. The volume of the rod can be exactly, about, more than, less than, at least or at most 1%, 2%, 3%,4%,5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100% of the volume of the coil. The volume of the rod can be between 1-10%, 10-20%, 20-30%, 30-40%, 40-50%, 50-60%, 60-70%, 70-80%, 80-90%, or 90-100% of the volume of the coil. The volume of the rod can be about 1 to about 10%, about 10 to about 20%, about 20 to about 30%, about 30 to about 40%, about 40 to about 50%, about 50 to about 60%, about 60 to about 70%, about 70 to about 80%, about 80 to about 90%, or about 90 to about 100% of the volume of the coil. The rod can be a wire. The coil can be a wire coil. In some cases, the volume of the coil be about, more than, less than, at least, or no greater than 1.1, 1.2, 1.3, 1.4, 1.5, 1.6, 1.7, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.5, 5, 5.5, 6, 6.5, 7, 7.5, 8, 8.5, 9, 9.5, or 10 times the volume of the rod.

A heater element comprising a rod is provided herein, wherein the rod can have a coil, wherein the surface area of the rod can be less than, greater than or equal to the surface area of the outer or external surface of the coil. The surface area of the rod can be exactly, about, more than, less than, at least or at most 1%, 2%, 3%,4%,5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100% greater than or less than the outer surface area of the coil. The surface area of the rod can be between 1-10%, 10-20%, 20-30%, 30-40%, 40-50%, 50-60%, 60-70%, 70-80%, 80-90%, or 90-100% greater than or less than the outer surface area of the coil. The surface area of the rod can be about 1 to about 10%, about 10 to about 20%, about 20 to about 30%, about 30 to about 40%, about 40 to about 50%, about 50 to about 60%, about 60 to about 70%, about 70 to about 80%, about 80 to about 90%, or about 90 to about 100% greater than or less than the outer surface area of the coil. The rod can be a wire. The coil can be a wire coil. In some cases, a surface area of a rod can be 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, or 10 times greater than a surface area of a coil.

A heater element as provided herein can comprise an electrical resistance that can be exactly, about, more than, less than, at least or at most 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, or 10 Ohms. The electrical resistance can be between 0.1-0.15, 0.15-0.2, 0.2-0.25, 0.25-0.3, 0.3-0.35, 0.35-0.4, 0.4-0.45, 0.45-0.5, 0.5-0.55, 0.55-0.6, 0.6-0.65, 0.65-0.7, 0.7-0.75, 0.75-0.8, 0.8-0.85, 0.85-0.9, 0.9-0.95, 0.95-1, 1-1.5, 1.5-2, 2-2.5, 2.5-3, 3-3.5, 3.5-4, 4-4.5, 4.5-5, 5-5.5, 5.5-6, 6-6.5, 6.5-7, 7-7.5, 7.5-8, 8-8.5, 8.5-9, 9-9.5, or 9.5-10 Ohms. The electrical resistance can be about 0.1 to about 0.15, about 0.15 to about 0.2, about 0.2 to about 0.25, about 0.25 to about 0.3, about 0.3 to about 0.35, about 0.35 to about 0.4, about 0.4 to about 0.45, about 0.45 to about 0.50, about 0.5 to about 0.55, about 0.55 to about 0.6, about 0.6 to about 0.65, about 0.65 to about 0.7, about 0.7 to about 0.75, about 0.75 to about 0.8, about 0.8 to about 0.85, about 0.85 to about 0.9, about 0.9 to about 0.95, about 0.95 to about 1, about 1 to about 1.5, about 1.5 to about 2, about 2 to about 2.5, about 2.5 to about 3, about 3 to about 3.5, about 3.5 to about 4, about 4 to about 4.5, about 4.5 to about 5, about 5 to about 5.5, about 5.5 to about 6, about 6 to about 6.5, about 6.5 to about 7, about 7 to about 7.5, about 7.5 to about 8, about 8 to about 8.5, about 8.5 to about 9, about 9 to about 9.5, or about 9.5 to about 10 Ohms. The electrical resistance can be the electrical resistance at room temperature.

A heater element as provided herein can vaporize a liquid formulation comprising a pharmaceutically active agent (e.g., nicotine) as provided herein, wherein substantially all of the liquid formulation in contact with or delivered to the heater element is vaporized. The vaporization of the liquid formulation that contacts or is delivered to a heater element as provided herein can be exactly, about, more than, less than, at most, or at least 1%, 2%, 3%,4%,5%, 6%, 7%, 8%, 9%, 10%, 11%, 12%, 13%, 14%, 15%, 16%, 17%, 18%, 19%, 20%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%. The vaporization of the liquid formulation that contacts or is delivered to a heater element as provided herein can be between 1%-10%, 10%-20%, 20%-30%, 30%-40%, 40%-50%, 50%-60%, 60%-70%, 70%-80%, 80%-90%, or 90%-100%. The vaporization of the liquid formulation that contacts or is delivered to a heater element as provided herein can be about 1% to about 10%, about 10% to about 20%, about 20% to about 30%, about 30% to about 40%, about 40% to about 50%, about 50% to about 60%, about 60% to about 70%, about 70% to about 80%, about 80% to about 90%, or about 90% to about 100%. The vaporization of the liquid formulation that contacts or is delivered to a heater element as provided herein can be greater than 95%, 99%, or 99.5%.

The amount of residue or build-up of non-vaporized liquid formulation comprising a pharmaceutically active agent (e.g., nicotine) that contacts or is delivered to a heater element as provided herein can be reduced by exactly, about, more than, less than, at most, or at least 1%, 2%, 3%,4%,5%, 6%, 7%, 8%, 9%, 10%, 11%, 12%, 13%, 14%, 15%, 16%, 17%, 18%, 19%, 20%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%. The amount of residue or build-up of non-vaporized liquid formulation comprising a pharmaceutically active agent that contacts or is delivered to a heater element as provided herein can be between 1%-10%, 10%-20%, 20%-30%, 30%-40%, 40%-50%, 50%-60%, 60%-70%, 70%-80%, 80%-90%, or 90%-100%. The amount of residue or build-up of non-vaporized liquid formulation comprising a pharmaceutically active agent that contacts or is delivered to a heater element as provided herein can be reduced by about 1% to about 10%, about 10% to about 20%, about 20% to about 30%, about 30% to about 40%, about 40% to about 50%, about 50% to about 60%, about 60% to about 70%, about 70% to about 80%, about 80% to about 90%, or about 90% to about 100%. The amount of residue or build-up of non-vaporized liquid formulation comprising a pharmaceutically active agent that contacts or is delivered to a heater element as provided herein can be reduced by greater than 95%, 99%, or 99.5%.

Methods of renewal of a heater element are provided herein. Heating elements can be renewed with changes in an agent (e.g., nicotine) dose cartridge to ensure dose consistency by removal of any build up of combusted material on the heater element.

In some cases, the heater element comprises a coil and a wick element, wherein the coil wraps around the wick element, and wherein the liquid formulation wicks onto the heated wick element, wherein the liquid formulation is vaporized through heating of the coil and wick element.

The heater element can be in fluid communication with a source of liquid formulation comprising an agent (e.g., nicotine) as provided herein. In some cases, the heater element further comprises a source of a liquid formulation comprising an agent (e.g., nicotine), wherein the source is in fluid communication with the wick element capable of being heated, wherein the liquid formulation comprising an agent (e.g., nicotine) wicks onto the wick element capable of being heated, whereby the liquid formulation is aerosolized by heating of the coil and wick element capable of being heated upon activation of a power source, wherein the power source is electrically coupled to the heater element. In some cases, the heater element further comprises a source of a liquid formulation comprising an agent, wherein the source is in fluid communication with the heatable wick element, wherein the liquid formulation comprising an agent wicks onto the heatable wick element, wherein the heatable wick element is heated after the formulation has wicked onto the heatable wick element, whereby the liquid formulation is aerosolized by heating of the coil and heatable wick element upon activation of the power source.

The heater element comprising a coil with a center exit wick element capable of being heated as described herein can vaporize substantially all of the liquid formulation comprising the pharmaceutically active agent (e.g., nicotine) that wicks onto the center wick element. The heater element comprising a coil with a center exit wick element capable of being heated can have a reduced or substantially no splatter. In some cases, the heater element comprises a coil with a center exit wick element capable of being heated, wherein a liquid formulation comprising a pharmaceutically active agent (e.g., nicotine) is held or wicks onto the center exit wick element capable of being heated, and wherein both the wick element capable of being heated and coil are heated, thereby vaporizing the liquid formulation, wherein substantially all of the liquid formulation is vaporized. The heater element comprising a coil with a center exit wick element capable of being heated can vaporize greater than 95 % of the liquid formulation wicked onto the wick element. The amount of residue or build-up of non-vaporized liquid formulation comprising a pharmaceutically active agent (e.g., nicotine) can be substantially reduced. Following vaporization of a liquid formulation as provided herein by a heater element comprising a coil and a center exit wick element capable of being heated less than 5% residue of non-vaporized liquid formulation can remain on the heater element.

In some cases, a heater element is connected to a timing device.

**Control Apparatus**

In some cases, an aerosol generating device (e.g., electronic cigarette) as provided herein comprises a control apparatus for regulating activation of a heater element. In some cases, the control apparatus is in electrical communication with the heater element. The electrical communication can be direct or indirect. In some cases, the control apparatus is a valve or flap as provided herein, wherein the valve or flap comprises an electrical component that serves to control activation of the heater element. The valve or flap can be a gas-control valve or flap. The heater element can be any heater element as provided herein. The control apparatus can activate the heater element at a trip point or activation trip point as described herein.

In some cases, the control apparatus can comprise a switch. The switch can be any switch known in the art. The switch can comprise a diaphragm. The switch can be an air-flow switch. The diaphragm can be a component of a pressure sensor in the air-flow switch. The switch can be configured for detecting air flow or inhalation from the device by a user.

In some cases, the control apparatus comprises a processor or microprocessor. In some cases, the control apparatus comprises a switch and a processor, wherein the switch detects an air flow rate (or pressure change) due to inhalation by a user and the processor serves to activate the heater element based on data from the sensor.

In some cases, a control apparatus comprising a switch is constructed to activate the heater element prior to the air-flow rate in an aerosol generation region of an aerosol generating device as provided herein reaching a desired or predetermined rate. Timing of activation is such that the heater element begins vaporization of a substrate (e.g., liquid nicotine solution) at about the time or after the air-flow through the aerosol generation region reaches the desired air-flow rate. In some cases, the heater element is activated when the air-flow rate through the aerosol generation region reaches the desired air-flow rate. In some cases, the heater element is activated at a selected time after the desired flow rate has been reached in the aerosol generation region. The desired rate can be detected in the aerosol generation region. The desired rate can be any rate as provided herein. The desired rate can be any trip point or activation trip point as provided herein. The desired rate can be less than 3 LPM. The desired rate can be less than 1 LPM. The desired rate can be up to 0.5 LPM. The desired rate can be about 0.15 LPM. The switch in the device can be configured for activating the heater element in relation to airflow through the aerosol generation region, such that the heater element produces an aerosol when the air flow rate through the aerosol generation region is sufficient for producing desired-size aerosol particles. The desired-size aerosol particles can comprise a desired diameter. The desired diameter can be from about 1 $\mu$m to about 5 $\mu$m. The desired diameter can be from about 1 $\mu$m to about 3 $\mu$m. The desired diameter can be an MMAD or a VMD. The desired-size aerosol particles can be condensation aerosol particles. In some cases, the switch is controlled by airflow through the aerosol generation region, such that the heater element is activated when (or just prior to, or after) the rate of airflow in the device reaches its desired rate. Alternatively, the switch can be user activated, allowing the user to initiate aerosol formation as air is being drawn into the device. In this manner, the device can provide a signal, such as an audible tone, to the user, when the desired rate of airflow through the aerosol generation region is reached.

A trip point can be a flow rate (or vacuum applied to the mouthpiece that can result in a flow rate) which causes an electrical current to be applied to a heater element, which activates (heats) the heater element and results in generation of an aerosol from a substrate in contact with the heater element. The flow rate (or vacuum applied to the mouthpiece that can result in a flow rate) can be detected by the control apparatus, wherein the control apparatus can subsequently activate the heater element. In some cases, a flow rate that is detected by the control apparatus and causes the control apparatus to activate a heater element of an aerosol generating device is the flow rate at which an aerosol comprising a desired diameter is generated following vaporization of a substrate in contact with the activated heater element. The desired diameter can be from about 1 $\mu$m to about 5$\mu$m. The diameter can be an MMAD. The diameter can be a VMD.

## Removal of Particles

In some cases, an issue with vaporization within the capillary can arise. First, liquid droplets can be ejected by vapor pushing the material out. Second, because the high vapor concentration can be high within the capillary end, rapid condensation and aggregation leading to larger than optimum particle size can result. To reduce the particle size of the aerosol the large particles can be removed and revaporized. Removal can be accomplished thru inertial impaction (**FIG. 11**). **FIG. 11** shows an agent (e.g., nicotine) reservoir (**1104**), tube, e.g., capillary tube (1106), heater element 1 (**1108**), and a heater element 2 (**1110**). One consideration is whether a restriction in a nozzle (1102) can cause an unacceptable increase in the air flow resistance. The following formula can be used to calculate the diameter of an orifice ($D_J$) (**1112**).

$$d_{50}\sqrt{C_c} = \left[\frac{9\pi N D_j^3 (Stk_{50})}{4P_p Q}\right]^{1/2}$$

Where $d_{50}$ = is the average aerosol practice size.
Where:

N = viscosity (of air) = $1.81 \times 10^{-5}$ P$_a$ sec
$D_J$ = The nozzle diameter in meters
$Stk_{50}$ =Stokes number for a round nozzle = .24 (dimensionless)
$P_p$ = Density of particle, for liquids assumed to be 1000kg/meter$^3$
Q = Flow rate in liters/mixture (assume 15L/min (about 2.5 x $10^{-4}$ m$^3$/s))

Additionally to correct for slip factor the following equation can be used:

$$d_{50} = d_{50}\sqrt{C_c} - 0.078 \; in \; microns$$

Using the above, a table of nozzle sizes vs. particle sizes that will impact can be generated as shown in Table 1:

Table 1.

| Nozzle Size (mm) | Particle Size ($\mu$m) |
|---|---|
| 7 | 6.41 |
| 6 | 5.07 |
| 5 | 3.84 |
| 4 | 2.72 |

If a particle size of approximately 5 $\mu$m is desired, a nozzle with a diameter of about 6 mm can be used, which can be acceptable for a pressure drop at 15L/min (about 2.5 $\times$ $10^{-4}$ m$^3$/s) flow rate of inhalation.
In some cases, a device for generating a condensation aerosol from a liquid formulation comprising a pharmaceutically active agent (e.g., nicotine) as provided herein comprises a means for removing aerosol particles of a size not optimal for deep lung delivery and subsequent rapid PK. The non-optimal particles can have a particle size of about, greater than, at least, or at most 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, or 20 microns. The particle size can be about, more than, less than, or at least 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 0.71, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, or 20 microns. The particle size can be from about 1 to about 10 microns, about 1 to about 9 microns, about 1 to about 7 microns, about 1 to 6 microns, about 1 to about 5 microns, about 1 to about 4 microns, about 1 to about 3 microns, or about 1 to about 2 microns. In some cases, the non-optimal particle sizes are greater than 1, 1,5, 2, 2.5, 3, 3.5, 4, 4.5, or 5 microns. The means for removing the non-optimal particles can be a solid structure within a passageway in which a condensation aerosol generated as provided herein flows. The structure can be an impactor, a baffle or baffle plate. In some cases, the structure (e.g., impactor, baffle, or baffle plate) is within a passageway in a device as provided herein. In some cases, the structure is located between a heater element and an outlet in a passageway of a device for generating a condensation aerosol comprising a pharmaceutically active agent (e.g., nicotine) as provided herein. In some cases, the structure is located downstream of an aerosol generation area and upstream of an outlet in a passageway of a device for generating a condensation aerosol comprising a pharmaceutically active agent (e.g., nicotine) as provided herein. In some cases, the structure (e.g., impactor, baffle, or baffle plate) comprises a surface attached to the passageway such that the surface has a diameter or width that occupies a portion of the diameter or width of the passageway such that only particles of an optimal size flow or are diverted around the surface while non-optimally sized particles impact or are substantially retained by the surface (e.g., impactor, baffle, or baffle plate) and are thereby incapable of flowing or being diverted around the surface. The surface can be a planar surface. The particles that flow or are diverted passed, around, by, beyond or are not substantially retained by the structure (e.g., impactor, baffle, or baffle plate) and thereby exit an outlet in a device for producing a condensation aerosol as provided herein can have a particle size of less than, at least or about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 0.71, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, 1, 1.5, 2, 2.5, 3, 3.5, 4, 4.5, or 5 microns. The particle size can be from about 1 to about 5 microns, about 1 to about 4 microns, about 1 to about 3 microns, or about 1 to about 2 microns. In some cases, the optimal particle sizes are less than 1, 1,5, 2, 2.5, 3, 3.5, 4, 4.5, or 5 microns. The particle size can be a diameter, radius, or circumference. In some cases, the particle size is a diameter. The diameter can be an average or mean. The mean can be arithmetic or geometric. The particle size can be an average or mean diameter. The particle size can be a mass median aerodynamic diameter (MMAD). The particle size can be a volumetric median diameter (VMD). In some cases, the optimally sized particles have an MMAD of less than or equal to 5 $\mu$m. In some cases, the optimally sized particles have an MMAD of about 1 to about 5 $\mu$m. In some cases, the non-optimally sized particles have an MMAD of greater than 5 $\mu$m. In some cases, the optimally sized particles have an MMAD of less than or equal to 3 $\mu$m. In some cases, the optimally sized particles have an MMAD of about 1 to about 3 $\mu$m. In some cases, the optimally sized particles have an MMAD of less than or equal to 2 $\mu$m. In some cases, the optimally sized particles have an MMAD of about 1 to about 2 $\mu$m. In some cases, the non-optimally sized particles have an MMAD of greater than 3 $\mu$m. In some cases, the non-optimally sized particles have an MMAD of greater than 2 $\mu$m. In some cases, a baffle or impactor in a passageway of a device as provided herein for generating a condensation aerosol comprising a pharmaceutically active agent (e.g., nicotine) substantially retains large particles of the condensation aerosol. In some cases, a baffle or impactor in a passageway of a device as provided herein for generating a condensation aerosol comprising a pharmaceutically active agent (e.g., nicotine) removes large particles from the condensation aerosol that exits an outlet of the device. The baffle or impactor can retain about, at least, at most, or more than 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% of the large particles of a condensation aerosol produced by a device as provided herein, thereby preventing or inhibiting exit of the large particles from an outlet of the device. The baffle or impactor can retain from about 50% to about 60%, about 60% to about 70%, about 70% to about 80%, about 80% to about 90%, or about 90% to about 100% of the large particles of a condensation aerosol produced by a device as provided herein, thereby preventing or inhibiting exit of the large particles from an outlet of the device. The large particles can have a size of about, more than, less than, or at least 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 0.71, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, or 20 microns. The large particles can have a size of from about 1 to about 10 microns, about 1 to about 9 microns, about 1 to about 7 microns, about 1 to 6 microns, about 1 to about 5 microns, about 1 to about 4 microns, about 1 to about 3 microns, or about 1 to about 2 microns. In some cases, a baffle or impactor in a passageway of a device as provided herein for generating a condensation aerosol comprising a pharmaceutically active agent (e.g., nicotine) does not substantially retains small particles of the condensation aerosol. The baffle or impactor can retain about, at most, or less than 1%, 2%, 3%,4%,5%, 6%, 7%, 8%, 9%, 10%, 11%, 12%, 13%, 14%, 15%, 16%, 17%, 18%, 19%, 20%, 30%, 31%, 32%, 33%, 34%, 35%, 36%, 37%, 38%, 39%, 40%, 41%, 42%, 43%, 44%, 45%, 46%, 47%, 48%, 49%, or 50%, of the small particles of a condensation aerosol produced by a device as provided herein. The baffle or impactor retains from about 10% to about 20%, about 20% to about 30%, about 30% to about 40%, or about 40% to about 50% of the small particles of a condensation aerosol produced by a device as provided herein. The small particles can have a size of less than, at least or about 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 0.71, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, 1, 1.5, 2, 2.5, 3, 3.5, 4, 4.5, or 5 microns. The small particles can have a size of from about 1 to about 5 microns, about 1 to about 4 microns, about 1 to about 3 microns, or about 1 to about 2 microns. The size of the small and/or large particles can be a diameter, radius, or circumference. In some cases, the size of the small particles is a diameter. In some cases, the size of the large particles is a diameter. The diameter can be a physical diameter (e.g., Feret's diameter, Martin's diameter, or equivalent projected area diameter), a fiber diameter, a Stokes diameter, a thermodynamic diameter, a volumetric diameter, or an aerodynamic diameter. The size of the small and/or large particles can be an MMAD or a VMD. In some cases, a baffle or impactor in a passageway of a device as provided herein for generating a condensation aerosol comprising a pharmaceutically active agent (e.g., nicotine) removes large particles from the condensation aerosol that exits an outlet of the device, wherein the condensation aerosol that exits the outlet comprises a particles size with a GSD of less than 2. In some cases, the GSD of the particle size is less than 1. The particle size with a GSD can be a diameter, radius, or circumference. In some cases, a baffle or impactor in a passageway of a device as provided herein for generating a condensation aerosol comprising a pharmaceutically active agent (e.g., nicotine) removes large particles from the condensation aerosol that exits an outlet of the device, wherein the condensation aerosol that exits the outlet comprises a diameter with a GSD of less than 2. In some cases, a baffle or impactor in a passageway of a device as provided herein for generating a condensation aerosol comprising a pharmaceutically active agent (e.g., nicotine) removes large particles from the condensation aerosol that exits an outlet of the device, wherein the condensation aerosol that exits the outlet comprises an average particles size of from about 1 to about 5 μm. In some cases, a baffle or impactor in a passageway of a device as provided herein for generating a condensation aerosol comprising a pharmaceutically active agent (e.g., nicotine) removes large particles from the condensation aerosol that exits an outlet of the device, wherein the condensation aerosol that exits the outlet comprises an average particles size of from about 1 to about 3 μm. In some cases, a baffle or impactor in a passageway of a device as provided herein for generating a condensation aerosol comprising a pharmaceutically active agent (e.g., nicotine) removes large particles from the condensation aerosol that exits an outlet of the device, wherein the condensation aerosol that exits the outlet comprises an average or mean particles size of from about 1 to about 2 μm. The average or mean particle size can be a diameter, radius, or circumference. In some cases, the average or mean particles size is a diameter. The diameter can be a physical diameter (e.g., Feret's diameter, Martin's diameter, or equivalent projected area diameter), a fiber diameter, a Stokes diameter, a thermodynamic diameter, a volumetric diameter, or an aerodynamic diameter. In some cases, a baffle or impactor in a passageway of a device as provided herein for generating a condensation aerosol comprising a pharmaceutically active agent (e.g., nicotine) reduces the average or mean particle size of the condensation aerosol that exits an outlet of the device. The average or mean particle size can be reduced by about, at least, at most, more than or less than 1%, 2%, 3%,4%,5%, 6%, 7%, 8%, 9%, 10%, 11%, 12%, 13%, 14%, 15%, 16%, 17%, 18%, 19%, 20%, 30%, 31%, 32%, 33%, 34%, 35%, 36%, 37%, 38%, 39%, 40%, 41%, 42%, 43%, 44%, 45%, 46%, 47%, 48%, 49%, or 50% of the average or mean particle size prior to encountering the baffle or impactor within a device as provided herein. The average or mean particle size can be reduced from about 10% to about 20%, about 20% to about 30%, about 30% to about 40%, or about 40% to about 50% of the average or mean particle size prior to encountering the baffle or impactor within a device as provided herein, The average or mean can be geometric or arithmetic. The average or mean particle size can be an average or mean diameter, radius, or circumference. In some cases, a baffle or impactor in a passageway of a device as provided herein for generating a condensation aerosol comprising a pharmaceutically active agent (e.g., nicotine) reduces the average or mean diameter of the particles of the condensation aerosol that exits an outlet of the device.

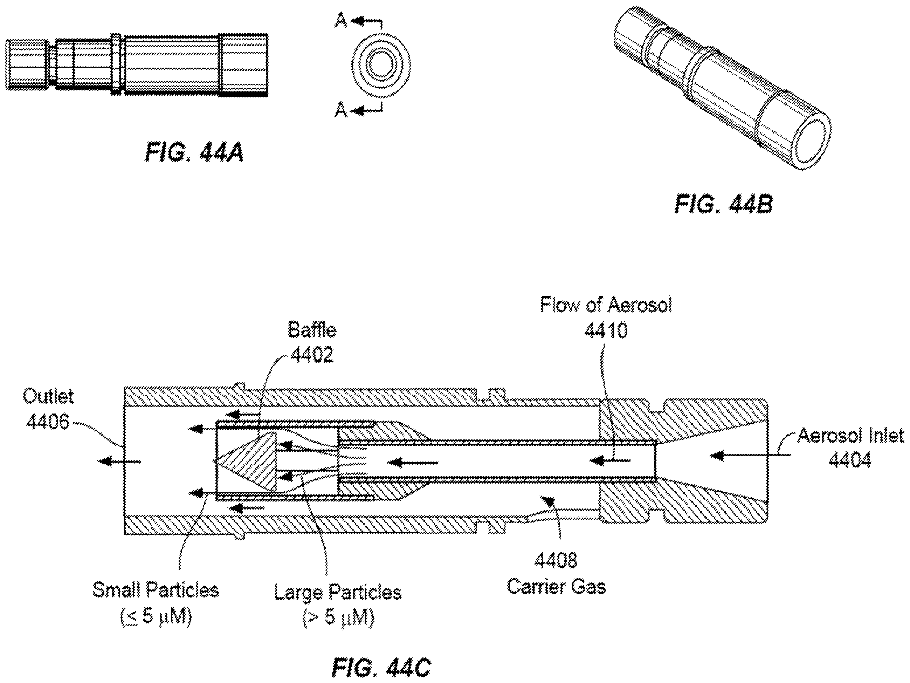

**FIGs. 44 A-C** illustrate an embodiment of a passageway comprising a baffle or impactor for removing condensation aerosol particles whose size is not optimal for deep lung delivery and subsequent rapid PK. **FIGs. 44A** and **B** illustrate exterior views of the passageway comprising the baffle, while **FIG. 44C** provides an interior view of a cone shaped baffle (**4402**) and its orientation within a passageway through which a condensation aerosol flows (**4410**). In **FIG. 44C**, a condensation aerosol comprising a pharmaceutically active agent (e.g., nicotine) generated by any means as provided herein enters a portion of a passageway comprising the baffle (**4402**) through an aerosol inlet (**4404**). The aerosol inlet can be a portion of a passageway downstream of a heater element that narrows following the area of the passageway that comprises the heater element. The aerosol inlet (**4404**) serves to funnel the aerosol through a narrowed passageway prior to the aerosol encountering the planar surface of the cone-shaped baffle (**4402**). Prior to the baffle (**4402**), the passageway widens, wherein the diameter of the planar surface of the baffle occupies a substantial portion of the diameter of the widened passageway. Upon entry into the widened passageway, the aerosol flows toward the baffle (**4402**), wherein large particles flow into the planar surface of the baffle, while small particles,flow around the edges of the baffle (**4402**). As the small particles flow around the baffle (**4402**), they flow into a wider passageway towards the outlet (**4406**) of the passageway. The widened passageway downstream of the baffle (**4402**) entrains the small particles into additional carrier gas (**4408**) that enters through secondary carrier gas (**4408**) inlets. In some cases, a flow of carrier gas through the passageway is about 1 to about 10 LPM (a range from about $1.667 \times 10^{-5}$ m$^3$/s to about $1.667 \times 10^{-4}$ m$^3$/s) (e.g., at a vacuum of about 1 to about 15 inches of water (a range from about 249 Pa to about 3738 Pa)), while the carrier gas (**4408**) entering through the secondary carrier gas (**4408**) inlets entrains the small particles in an air flow of about 20 to about 80 LPM (a range from about $3 \times 10^{-4}$ m$^3$/s to about $1.3 \times 10^{-3}$ m$^3$/s). The large particles can be about, greater than, at least or at most 1, 1.5, 2, 2.5, 3, 3.5, 4, 4.5, or 5 microns in diameter. The small particles can be about, less than, at least or at most 1, 1.5, 2, 2.5, 3, 3.5, 4, 4.5, or 5 microns in diameter. The diameter can be an MMAD or VMD. The diameter of the condensation aerosol particles that exit the device depicted in **FIGs. 44A-C** can have a GSD of less than 2. The diameter of the condensation aerosol particles that exit the device depicted in **FIGs. 44A-C** can have a GSD of less than 1. In some cases, the passageway depicted in **FIG. 44C** is connected to and downstream of the passageways depicted in any one of **FIGs. 31A-D,** wherein the passageway depicted in **FIG. 44C** is connected at the aerosol inlet (**4404**). In some cases, the aerosol inlet of the passageway depicted in **FIG. 44C** is a downstream extension of the passageways depicted in any one of **FIGs. 31A-D.**

The inner diameter of the passageway at the aerosol inlet of **FIG. 44C** and downstream of the narrow channel can be can be exactly, about, more than, less than, at least or at most 0.2, 0.21, 0.22, 0.23, 0.24, 0.25, 0.26, 0.27, 0.28, 0.29, 0.3, 0.35, 0.4, 0.45, or 0.5 inches (a range from about 0.508 mm to about 12.7 mm). The inner diameter of the passageway at the aerosol inlet of **FIG. 44C** and downstream of the narrow channel can be between 0.2-0.21, 0.21-0.22, 0.22-0.23, 0.23-0.24, 0.24-0.25, 0.25-0.26, 0.26-0.27, 0.27-0.28, 0.28-0.29, 0.29-0.3, 0.3-0.35, 0.35-0.4, 0.4-0.45, or 0.45-0.5 inches (a range from about 0.508 mm to about 12.7 mm). The inner diameter of the passageway at the aerosol inlet of **FIG. 44C** and downstream of the narrow channel can be about 0.2 to about 0.25, about 0.25 to about 0.3, about 0.3 to about 0.35, about 0.35 to about 0.4, about 0.4 to about 0.45, or about 0.45 to about 0.5 inches (a range from about 0.508 mm to about 12.7 mm). The inner diameter of the outlet (**4406**) can be exactly, about, more than, less than, at least or at most 0.2, 0.21, 0.22, 0.23, 0.24, 0.25, 0.26, 0.27, 0.28, 0.29, 0.3, 0.35, 0.4, 0.45, or 0.5 inches (a range from about 0.508 mm to about 12.7 mm). The inner diameter of the outlet (**4406**) can be between 0.2-0.21, 0.21-0.22, 0.22-0.23, 0.23-0.24, 0.24-0.25, 0.25-0.26, 0.26-0.27, 0.27-0.28, 0.28-0.29, 0.29-0.3, 0.3-0.35, 0.35-0.4, 0.4-0.45, or 0.45-0.5 inches (a range from about 0.508 mm to about 12.7 mm). The inner diameter of the outlet (**4406**) can be about 0.2 to about 0.25, about 0.25 to about 0.3, about 0.3 to about 0.35, about 0.35 to about 0.4, about 0.4 to about 0.45, or about 0.45 to about 0.5 inches (a range from about 0.508 mm to about 12.7 mm). The inner diameter of the narrow channel can be exactly, about, more than, less than, at least or at most 0.01, 0.0125, 0.015, 0.0175, 0.02, 0.0225, 0.025, 0.0275, 0.03, 0.0325, 0.035, 0.0375, 0.04, 0.0425, 0.045, 0.0475, 0.05, 0.0525, 0.055, 0.0575, 0.06, 0.0625, 0.065, 0.0675, 0.07, 0.0725, 0.075, 0.0775, 0.08, 0.0825, 0.085, 0.0875, 0.09, 0.0925, 0.095, 0.0975, 0.1, 0.11, 0.115, 0.12, 0.125, 0.13, 0.135, 0.14, 0.145, or 0.15 inches (a range from about 0.254 mm to about 3.81 mm). The inner diameter of the narrow channel can be between 0.01-0.015, 0.015-0.02, 0.02-0.025, 0.025-0.03, 0.03-0.035, 0.035-0.04, 0.04-0.045, 0.045-0.05, 0.05-0.055, 0.055-0.06, 0.06-0.065, 0.065-0.07, 0.07-0.075, 0.075-0.08, 0.08-0.085, 0.085-0.09, 0.09-0.095, 0.095-0.1, 0.1-0.12, 0.12-0.13, 0.13-0.14, or 0.14-0.15 inches (a range from about 0.254 mm to about 3.81 mm). The inner diameter of the narrow channel can be about 0.01 to about 0.015, about 0.015 to about 0.02, about 0.02 to about 0.025, about 0.025 to about 03, about 0.03 to about 0.035, about 0.035 to about 0.04, about 0.04 to about 0.045, about 0.045 to about 0.05, about 0.05 to about 0.055, about 0.055 to about 0.06, about 0.06 to about 0.065, about 0.065 to about 0.07, about 0.07 to about 0.075, about 0.075 to about 0.08, about 0.08 to about 0.085, about 0.085 to about 0.09, about 0.09 to about 0.095, about 0.095 to about 0.1, or about 0.1 to about 0.15 inches (a range from about 0.254 mm to about 3.81 mm).

**Flow regulation**

A device provided herein can be configured to limit a flow of a carrier gas through the passageway or aerosol generation area/chamber to permit condensation of the vaporized liquid formulation. The carrier gas can be air. The flow of a carrier gas through the aerosol generation chamber or passageway comprising or in fluid communication with the heater element can be limited to about 1 to about 10 liters per minute (LPM) (a range from about $1.667 \times 10^{-5}$ m$^3$/s to about $1.667 \times 10^{-4}$ m$^3$/s). The device can be configured to comprise a flow resistance (to inhalation) of about 0.05 to about 0.15 sqrt (cm-H$_2$O)/LPM. The device can be configured to comprise an inhalation resistance comprising a vacuum pressure of about 1 to about 10 inches of H20 (a range from about 249 Pa to about 2488 Pa). The flow resistance of the device as provided herein for use in a method as provided herein can be about the same flow resistance as through that of a combustible cigarette. The flow resistance through a device as provided herein for use in a method as provided herein can be around 2.5 (cm of H$_2$O)$^{1/2}$/LPM. In some cases, a device as provided herein for use in a method as provided herein comprises a flow rate of 1 LPM at a vacuum of 7.6 cm of H$_2$O. In some cases, a device as provided herein for use in a method as provided herein comprises a flow rate of 1.5 LPM at a vacuum of 16 cm of H$_2$O. In some cases, a device as provided herein for use in a method as provided herein comprises a flow rate of 2 LPM at a vacuum of 26 cm of H$_2$O.

Methods are provided herein for sensing an inhalation by a user and triggering a device. For example, an optical sensor that uses a deformable member (e.g., a vane) that moves during inhalation can be used to either open or close an optical path. In some embodiments, a Hall effect sensor is used to measure inhalation. In one embodiment, inhalation sensing is accomplished using an optical signal wherein a unique pattern of light pulses is sent along an optical path or light pipe and resent back along the optical path to a light detector. In one embodiment, the optical signal is sent from a controller into a dose cartridge whereby it is resent back into the controller to a light detector. In one embodiment, a vane is positioned in the path of an airway such that when an inhalation occurs, the vane is deflected out of the way and interrupts the optical signal. In this case, the device notes the absence of the optical signal and triggers the creation of an aerosol.

Methods are provided herein for inhalation flow control. In some cases, a valve system to allow for a user to experience an initial high pressure and low flow rates, followed by low pressure is used. An initial high-pressure drop through the device to facilitate the ejection of an agent (e.g., nicotine) from a dosing mechanism can be used. The following high flow rate can facilitate deep lung delivery. In one embodiment, a slide valve with an attached piston mechanism is used to eject an agent (e.g., nicotine) from a dosing reservoir. In one embodiment, air flow over a vaporizing agent (e.g., nicotine) formulation is regulated and controlled to an optimum level using a valve system, resulting in optimum particle sizing and dosing effectiveness. In a one embodiment, a valve system is used to create an internal air or inhalation resistance that is low (e.g., 0.08 to 0.12 (cm H$_2$O)$^{1/2}$/LPM). In a one embodiment, a valve system is used to create an internal air or inhalation resistance that is similar to that of a combustible cigarette (e.g., about 2.5 (cm H$_2$O)$^{1/2}$/LPM).

In some cases, a device for generating a condensation aeorosol as provided herein can comprise a heater element. In some cases, a device provided herein can comprise a passageway, wherein the passageway comprises a heater element and a reservoir. In some cases, the device comprises a passageway, a reservoir, and a housing which comprises a heater element, wherein the passageway is in fluid communication with the heater element. The passageway comprising the heater element or in fluid communication with the heater element can comprise an aerosol generation area or chamber. In some cases, the aerosol generation area or chamber comprises the heater element. In some cases, the aerosol generation area or chamber comprises the heater element and a source of a formulation comprising an agent as provided herein. The source can be a tube, e.g., capillary tube, or a reservoir. The tube, e.g., capillary tube can be coupled to the reservoir. The reservoir can comprise the liquid formulation. The reservoir can be in fluid communication with the heater element. The reservoir can serve to deliver the liquid formulation to the heater element, wherein the liquid formulation can wick onto the heater element. The reservoir can comprise a tube, e.g., capillary tube, wherein the tube, e.g., capillary tube can deliver the liquid formulation onto the heater element.

In some cases, a device for generating a condensation aeorosol as provided herein comprises an aerosol generation chamber. The aerosol generation chamber can comprise a heater element. The aerosol generation chamber can comprise a source of a liquid formulation comprising a pharmaceutically active agent (e.g. nicotine). In some cases, the aerosol generation chamber comprises a heater element and a source of a liquid formulation comprising a pharmaceutically active agent (e.g. nicotine). The aerosol generation chamber can be within a primary flow-through passageway. In some cases, a device for producing a condensation aerosol as provided herein comprises a flow-through passageway, wherein the flow-through passageway comprises an upstream opening and a downstream opening, wherein the flow-through passageway comprises an aeorosol generation chamber between the upstream and downstream openings of the flow-through passageway. The passageway can be a primary flow-through passageway. The primary flow-through passageway can be in fluid communication with a secondary flow-through passageway as provided herein. In some cases, the aerosol generation chamber further comprises a nozzle as provided herein. In some cases, a device for generating a condensation aeorosol as provided herein comprises an aerosol generation chamber, wherein the aerosol generation chamber is within a passageway configured to limit the flow of a carrier gas through the aerosol generation chamber to a flow rate effective for producing a condensation aerosol comprising particles of a size suitable for delivery to the deep lung of a subject. The flow rate can be limited to about 1 to about 10 liters per minute (LPM) (a range from about $1.667 \times 10^{-5}$ m$^3$/s to about $1.667 \times 10^{-4}$ m$^3$/s) at, e.g., a vacuum of about 1 to about 15 inches of water (a range from about 249 Pa to about 3738 Pa).

In some cases, a device for producing a condensation aerosol as provided herein comprises a primary flow-through passageway, wherein the primary flow-through passageway comprises an upstream opening and a downstream opening, wherein the upstream opening comprises an inlet for a carrier gas (e.g., air) and the downstream opening comprises an outlet for the carrier gas (e.g., air). The passageway can be a primary flow-through passageway. The primary flow-through passageway can be in fluid communication with a secondary flow-through passageway as provided herein. The inlet can comprise a flow restrictor configured to limit the flow of the carrier gas through primary flow-through passageway to a flow rate effective for producing a condensation aerosol comprising particles of a size suitable for delivery to the deep lung of a subject. The flow restrictor can limit the flow rate to about 1 to about 10 liters per minute (LPM) (a range from about $1.667 \times 10^{-5}$ m$^3$/s to about $1.667 \times 10^{-4}$ m$^3$/s), e.g., at a vacuum of about 1 to about 15 inches of water (a range from about 249 Pa to about 3738 Pa). The flow restrictor can be a valve or an orifice comprising dimensions that limit the flow of a carrier gas (e.g., air) to a rate suitable for producing a condensation aerosol comprising particles of a size suitable for delivery to the deep lung of a subject.

An orifice for air that passes over the heater element can have a diameter of about, more than, less than, or at least 0.01, 0.012, 0.015, 0.02, 0.022, 0.025, 0.03, 0.032, 0.035, 0.04, 0.042, 0.045, 0.05, 0.052, 0.055, 0.06, 0.062, 0.065, 0.07, 0.075, 0.08, 0.085, 0.09, 0.1, 0.105, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 0.71, 0.72, 0.73, 0.74, 0.75, 0.76, 0.77, 0.78, 0.79, or 0.8 inches (a range from about 0.254 mm to about 20.32 mm). In some cases, an orifice for air that passes over a heater element has a diameter of about 0.01 to about 0.12 inches, about 0.02 to about 0.1 inches, about 0.03 to about 0.09 inches, about 0.04 to about 0.08 inches, or about 0.05 to about 0.07 inches, or about 0.15 to about 3 inches (a range from about 0.254 mm to about 76.2 mm). An orifice for bypass air (air that is routed around a heater element) can have a diameter of about, more than, less than, or at least 0.02, 0.04, 0.06, 0.08, 0.1, 0.12, 0.14, 0.16, 0.18, 0.2, 0.22, 0.24, 0.26, 0.28, 0.3, 0.32, 0.34, 0.36, 0.38, 0.4, 0.42, 0.44, 0.46, 0.48, 0.5, 0.52, 0.54, 0.56, 0.58, 0.6, 0.62, 0.64, 0.66, 0.68, 0.7, 0.8, 0.9, 1, or 1.2 inches (a range from about 0.508 mm to about 30.48 mm). In some cases, an orifice for bypass air (air that is routed around a heater element) has a diameter of about 0.05 to about 0.4 inches, about 0.1 to about 0.3 inches, or about 0.1 to about 0.4 inches (a range from about 1.27 mm to about 10.16 mm).

In some cases, a device for producing a condensation aerosol as provided herein comprises a flow-through passageway, wherein the flow-through passageway comprises an upstream opening and a downstream opening, wherein the flow-through passageway is configured to facilitate formation of a condensation aerosol comprising particles of a size effective for delivery to the deep lung of a subject. The particles can comprise an MMAD of about 1 to about 5 μm. The subject can be a human. The subject can be a human who smokes and/or uses tobacco or nicotine containing products. The condensation aerosol can comprise a pharmaceutically active agent (e.g. nicotine). The passageway can be a primary flow-through passageway. The primary flow-through passageway can be in fluid communication with a secondary flow-through passageway as provided herein. The upstream opening can be an inlet. The inlet can comprise a flow restrictor as provided herein. The downstream opening can comprise an outlet. The outlet can be a mouthpiece.

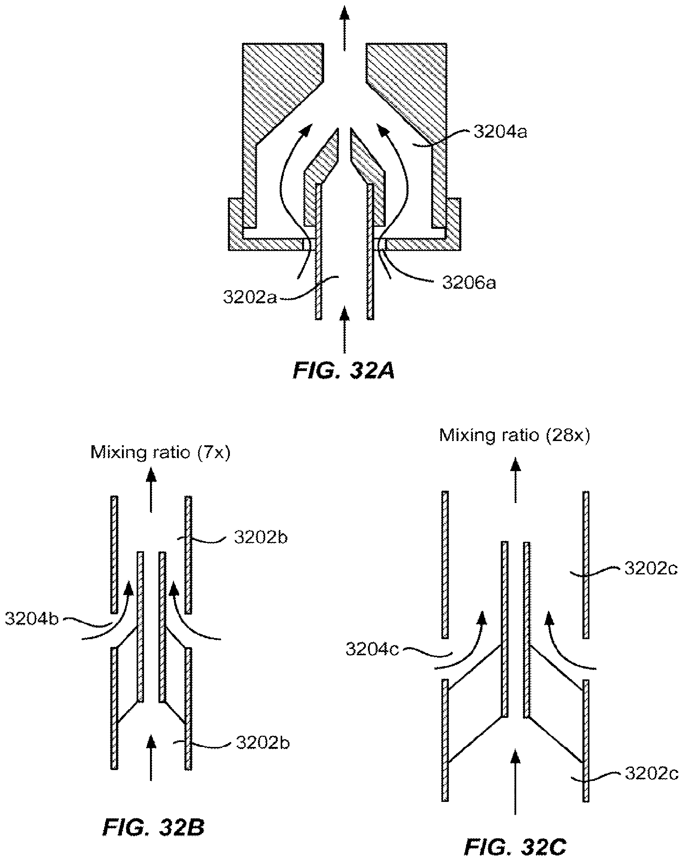

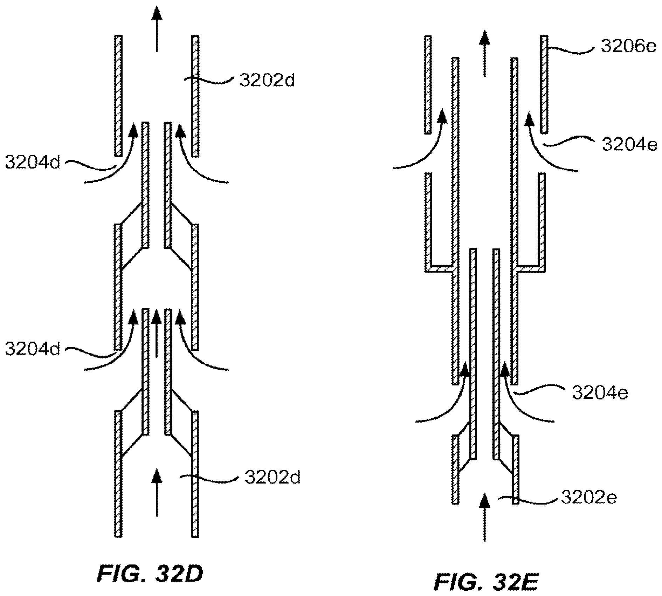

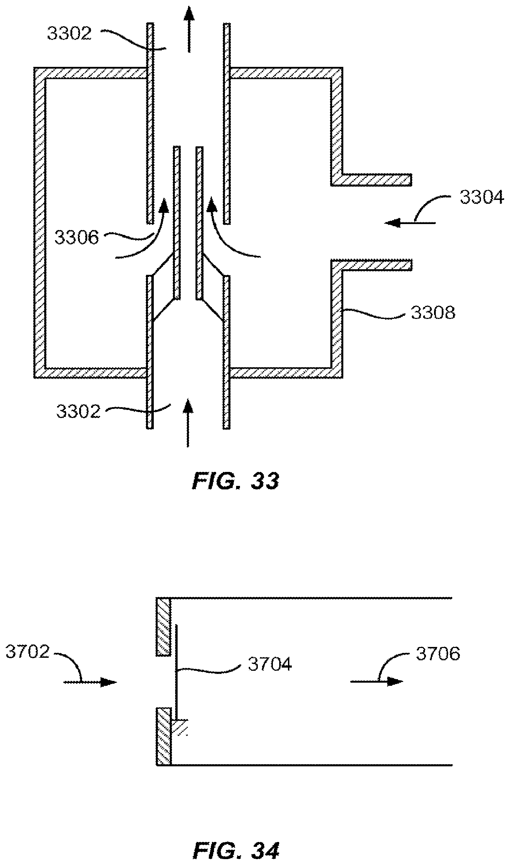

The flow-through passageway can be configured to form a narrow channel between the upstream and downstream openings. The passageway can be further configured to widen downstream of the narrow channel prior to the downstream opening of the passageway. The narrow channel can comprise an inner diameter and an outer diameter (see, e.g., **FIG. 32** and **33**). The inner diameter of the narrow channel can be exactly, about, more than, less than, at least or at most 0.01, 0.0125, 0.015, 0.0175, 0.02, 0.0225, 0.025, 0.0275, 0.03, 0.0325, 0.035, 0.0375, 0.04, 0.0425, 0.045, 0.0475, 0.05, 0.0525, 0.055, 0.0575, 0.06, 0.0625, 0.065, 0.0675, 0.07, 0.0725, 0.075, 0.0775, 0.08, 0.0825, 0.085, 0.0875, 0.09, 0.0925, 0.095, 0.0975, 0.1, 0.11, 0.115, 0.12, 0.125, 0.13, 0.135, 0.14, 0.145, or 0.15 inches (a range from about 0.254 mm to about 3.81 mm). The inner diameter of the narrow channel can be between 0.01-0.015, 0.015-0.02, 0.02-0.025, 0.025-0.03, 0.03-0.035, 0.035-0.04, 0.04-0.045, 0.045-0.05, 0.05-0.055, 0.055-0.06, 0.06-0.065, 0.065-0.07, 0.07-0.075, 0.075-0.08, 0.08-0.085, 0.085-0.09, 0.09-0.095, 0.095-0.1, 0.1-0.12, 0.12-0.13, 0.13-0.14, or 0.14-0.15 inches (a range from about 0.254 mm to about 3.81 mm). The inner diameter of the narrow channel can be about 0.01 to about 0.015, about 0.015 to about 0.02, about 0.02 to about 0.025, about 0.025 to about 03, about 0.03 to about 0.035, about 0.035 to about 0.04, about 0.04 to about 0.045, about 0.045 to about 0.05, about 0.05 to about 0.055, about 0.055 to about 0.06, about 0.06 to about 0.065, about 0.065 to about 0.07, about 0.07 to about 0.075, about 0.075 to about 0.08, about 0.08 to about 0.085, about 0.085 to about 0.09, about 0.09 to about 0.095, about 0.095 to about 0.1, or about 0.1 to about 0.15 inches (a range from about 0.254 mm to about 3.81 mm). The outer diameter of the narrow channel can be exactly, about, more than, less than, at least or at most 0.08, 0.0825, 0.085, 0.0875, 0.09, 0.0925, 0.095, 0.0975, 0.1, 0.11, 0.115, 0.12, 0.125, 0.13, 0.135, 0.14, 0.145, or 0.15 inches (a range from about 2.0 mm to about 3.81 mm). The outer diameter of the narrow channel can be between 0.08-0.085, 0.085-0.09, 0.09-0.095, 0.095-0.1, 0.1-0.12, 0.12-0.13, 0.13-0.14, or 0.14-0.15 inches (a range from about 2.0 mm to about 3.81 mm). The outer diameter of the narrow channel can be about 0.08 to about 0.085, about 0.085 to about 0.09, about 0.09 to about 0.095, about 0.095 to about 0.1, or about 0.1 to about 0.15 inches (a range from about 2.0 mm to about 3.81 mm). The inner diameter of the flow-through passageway prior to and/or downstream of the narrow channel can be exactly, about, more than, less than, at least or at most 0.2, 0.21, 0.22, 0.23, 0.24, 0.25, 0.26, 0.27, 0.28, 0.29, 0.3, 0.35, 0.4, 0.45, or 0.5 inches (a range from about 5.08 mm to about 12.7 mm). The inner diameter of the flow-through passageway prior to and/or downstream of the narrow channel can be between 0.2-0.21, 0.21-0.22, 0.22-0.23, 0.23-0.24, 0.24-0.25, 0.25-0.26, 0.26-0.27, 0.27-0.28, 0.28-0.29, 0.29-0.3, 0.3-0.35, 0.35-0.4, 0.4-0.45, or 0.45-0.5 inches (a range from about 5.08 mm to about 12.7 mm). The inner diameter of the flow-through passageway prior to and/or downstream of the narrow channel can be about 0.2 to about 0.25, about 0.25 to about 0.3, about 0.3 to about 0.35, about 0.35 to about 0.4, about 0.4 to about 0.45, or about 0.45 to about 0.5 inches (a range from about 5.08 mm to about 12.7 mm).

In some cases, a device for generating a condensation aeorosol comprising a primary flow-through passageway as provided herein further comprises a secondary flow-through passageway. The secondary flow-through passageway can be in fluid communication with the primary flow through passageway. The secondary flow-through passageway can comprise one or more channels. In some cases, the secondary flow - through channel comprises a first, a second, and a third channel. The first channel can be in fluid communication with a primary flow-through chamber upstream of an aerosol generation chamber as provided herein. The second channel can be in fluid communication with a primary flow through passageway between an aerosol generation chamber as provided herein and a downstream opening of the primary flow through passageway. The third channel can comprise a second inlet for a carrier gas (e.g. air) and can be in fluid communication with the second channel. The secondary flow-through passageway can also comprise an articuable element. The articuable element can be a diaphragm. The articuable element can be further connected to springs. The springs can control the movement of the articuable element. The articuable element can be articulated by changes in pressure within the device. The pressure that articulates the articuable element can be inhalation resistance or vacuum pressure. The inhalation resistance can be a vacuum of about 1 to about 10 inches of $H_2O$ (a range from about 249 Pa to about 2488 Pa). An increase in pressure can compress the springs. Inhalation through a device for generating a condensation aerosol as provided herein can increase the pressure in the device. The articuable element can comprise a protruding member. In some cases, one or more springs are located on a first side of an articuable element, while the protruding member is located on a second side opposite the first side. The protruding member can be configured to enter and block the third channel. A pressure differential between primary and secondary flow-through passageways within the device can cause articulation or movement of the articuable element. The pressure differential can be affected by inhalation through the downstream opening of the primary flow chamber. The pressure differential can be across the first channel of the secondary flow chamber. Under conditions of low pressure or inhalation resistance, the articuable element can block the third channel, thereby preventing entry of the carrier gas (e.g. air). Under conditions of increased pressure or inhalation resistance, the articuable element can be articulated or removed from blocking the third channel, thereby allowing the carrier gas to enter the device. In some cases, inhalation through the downstream opening of the primary flow -through passageway serves to articulate the articuable element, whereby the articulation serves to open the third channel, wherein the opening permits the carrier gas (e.g. air) to flow through the third channel of the secondary flow-through passageway and enter the primary flow through passageway through the second channel in the secondary flow-through passageway, thereby entraining the condensation aerosol in the carrier gas from the secondary flow-through passageway. Additional carrier gas entering the primary flow-through passageway through the secondary flow-through passageway as described herein can entrain the condensation aerosol in the carier gas (e.g. air) to produce a total flow rate of about 20 to about 80 LPM (a range from about $3 \times 10^{-4}$ m$^3$/s to about $1.3 \times 10^{-3}$ m$^3$/s). The device can have an interior air resistance (to inhalation) no greater than that of a cigarette. The device can have an interior air resistance (to inhalation) of about 0.05 to about 0.15 (cm $H_2O$)$^{1/2}$/LPM.

A device for generating condensation aerosols comprising a primary flow-through passageway as provided herein can further comprise one or more additional sources of carrier gas, wherein the additional sources permit the flow of carrier gas to enter the device in addition to the carrier gas flowing through the primary flow-through passageway. The one or more additional sources can be inlets or channels. The one or more additional sources can be bypass inlets or bypass channels, wherein carrier gas entering a device through the bypass inlets or channels is bypass carrier gas. The bypass carrier gas can be air. The one or more sources can be within one or more walls of the primary flow-through passageway. The one or more sources can be components of a secondary flow-through passageway as provided herein, wherein the secondary flow-through passageway can be in fluid communication with the primary flow-through passageway. The one or more sources can be within one or more walls of the secondary flow-through passageway. The one or more sources can be within one or more walls of a housing, wherein the housing surrounds or encompasses the primary flow-through passageway. The one or more sources can be flow regulators. The carrier gas entering the device through the one or more sources can be the same type or a different type of carrier gas as that flowing through a primary flow-through passageway. In some cases, the carrier gas entering through the one or more sources can be air. In some cases, the one or more sources permit flow of carrier gas to enter the device downstream of a heater element or aerosol generation chamber or area as provided herein. The flow of carrier gas entering the device through the one or more sources can mix with the carrier gas flowing through a primary flow through passageway. The mixing can be downstream of a heater element or aerosol generation chamber as provided herein but before a downstream opening or outlet of a primary passageway comprising the heater element or aersol generation chamber. The mixing of the carrier gases can produce a total flow rate exiting the device that can be similar to normal breathing of a subject. The total flow rate can be about 20 to about 80 LPM (a range from about $3 \times 10^{-4}$ m³/s to about $1.3 \times 10^{-3}$ m³/s). The subject can be a human. The subject can be a human who smokes and/or uses tobacco or nicotine containing products.

FIG. 21 illustrates an embodiment of an electronic agent (e.g., nicotine) delivery device comprising a valve system (2100) for controlling air flow for deep lung delivery and rapid PK. Upon inhalation, negative pressure in a mouthpiece (2102) increases causing a pressure drop across a gas control valve (2104). An increase in the pressure drop can cause the valve (2104) to close and prevent airflow (2106) into an aerosol generating area (2108) within a flow through chamber (2110). The aerosol generating area (2108) can comprise an agent (e.g., nicotine) reservoir comprising an agent (e.g., nicotine) formulation, any of the dosing mechanisms described herein, and a heater for vaporizing an agent (e.g., nicotine) droplets that can be released from the dosing mechanism. Closing of the valve (2104) can subsequently cause an increase in airflow (2106) from an air inlet (2112) across a backflow valve (2114) through a diversion air orifice (2116) and into a diversion air channel (2118). In this manner, the airflow over a vaporizing agent (e.g., nicotine) formulation can be regulated and controlled to an optimal level in order to achieve optimum particle sizing and dosing effectiveness. In one embodiment, the valve system produces an inhalation resistance no greater than that of a cigarette. In one embodiment, the valve system produces an inhalation resistance no greater than 0.08 (cm $H_2O)^{1/2}$/LPM.

FIG. 32 A-E illustrates multiple embodiments of a device for regulating the flow of a carrier gas (e.g. air). In each embodiment, the device comprises a primary flow-through passageway (3202A-E) and one or more sources of bypass or additional carrier gas (3204A-E). In each embodiment, the one or more sources of bypass or additional carrier gas (3204A-E) permit an additional or bypass flow of carrier gas (e.g. air) to mix with the carrier gas flowing through the primary flow-through passageway (3202A-E). In some cases, the mixing occurs downstream of an aerosol generation chamber, thereby mixing a condensation aerosol produced in the aerosol generation chamber with a larger volume of carrier gas (e.g. air). The mixing can produce a total flow rate downstream of the mixing of about 20 to about 80 liters per minute (LPM) (a range from about $3 \times 10^{-4}$ m³/s to about $1.3 \times 10^{-3}$ m³/s). FIG. 32A shows a device comprising a primary flow-through passageway (3202a) comprising an upstream and downstream section comprising an inner diameter of 0.25 inches (about 6.35 mm), and two secondary flow-through chambers (3204a), wherein bypass or additional carrier gas enters the device through two inlets (3206a) adjacent to the primary flow-through chamber (3202a). The inner diameter of the primary flow through chamber (3202a) narrows just prior to entry of the bypass carrier gas. In some cases, the narrowing of the primary flow-through passageway permits formation of condensation aerosol particles comprising particles with an MMAD of about 1 to about 5 uM. The device in FIG. 32A can permit the mixing of the bypass carrier gas with the carrier gas flow through the primary chamber at a ratio of 10:1.

FIG. 32B shows a device comprising a primary flow-through passageway (3202b) comprising an upstream and downstream section comprising an inner diameter of 0.25 inches (about 6.35 mm), and two inlets (3204b) within the wall of the primary flow-through chamber (3202b), wherein bypass or additional carrier gas enters the device. The primary flow through chamber (3202b) narrows just prior to entry of the bypass carrier gas to comprise an inner diameter of 0.084 inches (about 2.13 mm) and an outer diameter of 0.108 inches (about 2.74 mm). In some cases, the narrowing of the primary flow-through passageway (3202b) permits formation of condensation aerosol particles comprising particles with an MMAD of about 1 to about 5 um. The device in FIG. 32B can permit the mixing of the bypass carrier gas with the carrier gas flow through the primary chamber at a ratio of 7:1.

FIG. 32C shows a device comprising a primary flow-through passageway (3202c) comprising an upstream and downstream section comprising an inner diameter of 0.5 inches (about 12.7 mm), and two inlets (3204c) within the wall of the primary flow-through chamber (3202c), wherein bypass or additional carrier gas enters the device. The primary flow through chamber (3202c) narrows just prior to entry of the bypass carrier gas to comprise an inner diameter of 0.084 inches (about 2.13 mm) and an outer diameter of 0.108 inches (about 2.74 mm). In some cases, the narrowing of the primary flow-through passageway (3202c) permits formation of condensation aerosol particles comprising particles with an MMAD of about 1 to about 5 $\mu$m. The device in FIG. 32C can permit the mixing of the bypass carrier gas with the carrier gas flow through the primary chamber at a ratio of 28:1.

FIG. 32D shows a device comprising a primary flow-through passageway (3202d) comprising an upstream and downstream section comprising an inner diameter of 0.25 inches (about 6.35 mm), and two sets of two inlets (3204d) adjacent to the primary flow-through chamber (3202d), wherein bypass or additional carrier gas enters the device. The flow through chamber narrows just prior to entry of the bypass carrier gas from each set of two inlets to comprise an inner diameter of 0.096 inches (about 2.44 mm) and an outer diameter of 0.125 inches (about 3.175 mm). Following the first set of two inlets, the primary flow through passageway widens to an inner diameter of 0.250 inches (about 6.35 mm), before narrowing again. In some cases, the narrowing of the primary flow-through passageway permits formation of condensation aerosol particles comprising particles with an MMAD of about 1 to about 5 $\mu$m. The device in **FIG. 32D** can permit the mixing of the bypass carrier gas with the carrier gas flow through the primary chamber at a ratio of 35:1.

The device in **FIG. 32E** is similar to the device in **FIG. 32D,** wherein **FIG. 32E** shows a device comprising a primary flow-through passageway **(3202e)** comprising an upstream and downstream section comprising an inner diameter of 0.250 inches (about 6.35 mm), and two sets of two inlets **(3204e)** adjacent to the primary flow-through chamber **(3202e),** wherein bypass or additional carrier gas enters the device. The primary flow through chamber **(3202e)** narrows just prior to entry of the bypass carrier gas from the first set of two inlets to comprise an inner diameter of 0.096 inches (about 2.44 mm) and an outer diameter of 0.125 inches (about 3.175 mm). Following the first set of two inlets, the primary flow through passageway **(3202e)** widens to an inner diameter of 0.250 inches (about 6.35 mm) and an out diameter of 0.280 inches (about 7.112 mm). Subsequently, the primary flow-through passageway **(3202e)** opens into a secondary housing **(3206e),** which has an inner diameter of 0.466 inches (about 11.8 mm). In **FIG. 32E,** the second pair of inlets **(3204e)** are located in the wall of a secondary housing **(3206e),** which is coupled to and encompasses the primary flow-through passageway.

**FIG. 33** illustrates another embodiment of a device for regulating the flow of a carrier gas (e.g. air). **FIG. 33** shows a device comprising a primary flow-through passageway **(3302)** comprising an upstream and downstream section comprising an inner diameter of 0.25 inches (about 6.35 mm), and two inlets **(3306)** within the wall of the primary flow-through chamber **(3302),** wherein bypass or additional carrier gas enters the device. The primary flow-through chamber narrows **(3302)** just prior to entry of the bypass carrier gas to comprise an inner diameter of 0.086 inches (about 2.18 mm) and an outer diameter of 0.106 inches (about 2.69 mm). As depicted in **FIG. 33**, the section of the primary flow-through chamber **(3302)** is coupled to and encased by a secondary housing **(3308)**. The secondary housing comprises a bypass inlet **(3304),** which permits entry of bypass or additional carrier gas (e.g. air) to enter the primary flow-through passageway through the inlets **(3306)**. In some cases, the narrowing of the primary flow-through passageway permits formation of condensation aerosol particles comprising particles with an MMAD of about 1 to about 5 $\mu$m.

**FIG. 35** illustrates another embodiment a device for regulating the flow of a carrier gas (e.g. air). The device comprises a primary airway **(3504)** that comprises an aerosol generation chamber **(3528)** comprising a heater element **(3502)**, a restrictive orifice **(3514)** and a mouthpiece **(3506)**. The heater element **(3502)** comprises a coil. The heater element can be any heater element comprising a coil as provided herein. The primary airway **(3504)** is fluidically connected to a secondary airway **(3516),** through a first channel **(3518)** located (disposed) between the restrictive orifice **(3514)** and heater element **(3502),** and a second channel **(3520)** located (disposed) between the heater element **(3502)** and the mouthpiece **(3506)**. The secondary airway **(3516)** further comprises a third channel **(3530)** that is a secondary inlet **(3508)** for a carrier gas (e.g. air) and a diaphragm **(3510)**. The diaphragm **(3510)** comprises a base member that is connected to a pair of springs **(3512)** on a first side and a protruding member **(3524)** on a second side. The springs **(3512)** are additionally connected to a wall opposite the first side of the base member that is part of the housing of the secondary airway **(3516)**. The base member of the diaphragm **(3510)** is also connected to a pair of lateral springs **(3526)** on its lateral edges, which are further connected to the walls of the housing of the secondary airway **(3516)** opposite the lateral edges of the base member. The restrictive orifice **(3514)** is configured to limit the flow rate of the carrier gas (e.g. air) through the aerosol generation chamber **(3528)** in order to allow for the condensation of a liquid formulation comprising a pharmaceutically active agent as provided herein vaporized by the heater element **(3502)** to particles comprising about 1 to about 5 um MMAD. The restrictive orifice **(3514)** limits the flow rate of the carrier gas (i.e. air) about 1 to about 10 liters per minute (LPM) (a range from about $1.667 \times 10^{-5}$ m$^3$/s to about $1.667 \times 10^{-4}$ m$^3$/s) at, e.g., a vacuum of about 1 to about 15 inches of water (a range from about 249 Pa to about 3738 Pa). Inhalation through the mouthpiece **(3506)** can produce a flow of carrier gas (e.g. air) through the restrictive orifice **(3514)** that can produce an inhalation resistance. The inhalation resistance produces a pressure differential across the opening of the first channel **(3518)** connecting the primary airway **(3504)** with the secondary airway **(3516)**. The inhalation resistance causes the springs **(3512)** coupled to the first side of the diaphragm **(3510)** to compress and the lateral springs **(3526)** coupled to the lateral edges of the diaphragm **(3510)** to extend, whereby the protruding member of coupled to the second side of the diaphragm **(3510)** is removed from the third channel **(3530)** of the secondary airway **(3516)**. Removal of the protruding member **(3524)** causes an additional flow of carrier gas (e.g. air) to enter the device. The additional flow of carrier gas (e.g. air) then enters the primary airway **(3504)** downstream of the heater element **(3502)** and aerosol generation area **(3528)** through the second channel **(3520)**. The additional flow of carrier gas (e.g. air) can serve to mix or entrain the condensation aerosol comprising particles of about 1 to about 5 $\mu$m to produce a total flow rate suitable for delivery of the particles to the deep lung of a user of the device.

A device for producing a condensation aerosol as provided herein can have an interior air resistance (to inhalation) no greater than 0.08 (cm H$_2$O)$^{1/2}$/LPM. The device can have an interior air resistance (to inhalation) exactly, about, more than, less than, at least, or at most 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, 0.20, or 0.25 (cm $H_2O$)$^{1/2}$/LPM. The device can have an interior air resistance (to inhalation) between 0.01-0.02, 0.02-0.03, 0.03-0.04, 0.04-0.05, 0.05-0.06, 0.06-0.07, 0.07-0.08, 0.08-0.09, 0.09-0.10, 0.1-0.11, 0.11-0.12, 0.12-0.13, 0.13-0.14, 0.14-0.15, 0.15-0.16, 0.16-0.17, 0.17-0.18, 0.18-0.19, 0.19-0.20, or 0.20-0.25 (cm $H_2O$)$^{1/2}$/LPM. The device can have an interior air resistance (to inhalation) of about 0.01 to about 0.03, about 0.03 to about 0.05, about 0.05 to about 0.07, about 0.07 to about 0.09, about 0.09 to about 0.11, about 0.11 to about 0.13, about 0.13 to about 0.15, about 0.15 to about 0.17, about 0.17 to about 0.19, or about 0.19 to about 0.25 (cm $H_2O$)$^{1/2}$/LPM.

A device for producing a condensation aerosol as provided herein can produce a total flow rate of a carrier gas (e.g. air) of exactly, about, more than, less than, at least, or at most 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 liters per min (LPM) (a range from about $1.667 \times 10^{-5}$ m³/s to about $1.667 \times 10^{-3}$ m³/s). The total flow rate can be between 1-10, 10-20, 20-30, 30-40, 40-50, 50-60, 60-70, 70-80, 80-90, or 90-100 LPM (a range from about $1.667 \times 10^{-5}$ m³/s to about $1.667 \times 10^{-3}$ m³/s). The total flow rate can be about 1 to about 10, about 10 to about 20, about 20 to about 30, about 30 to about 40, about 40 to about 50, about 50 to about 60, about 60 to about 70, about 70 to about 80, about 80 to about 90, or about 90 to about 100 LPM (a range from about $1.667 \times 10^{-5}$ m³/s to about $1.667 \times 10^{-3}$ m³/s). The device can comprise a primary flow-through passageway for a carrier gas and one or more sources of additional or bypass carrier gas as provided herein. These flow rates can be at a vacuum of about 1 to about 15 inches of water (a range from about 249 Pa to about 3738 Pa).

The one or more sources of additional or bypass carrier gas (e.g. air) can be configured to limit the flow rate of additional or bypass carrier gas to produce a total flow rate as provided herein. The flow rate can be limited by using a restrictive orifice on the one or more sources of additional or bypass carrier gas (e.g. air). The restrictive orifice can comprise any valve or flap as known in the art. The valve or flap can be moderated at specific flow rates. The flow rates that moderate the valve or flap can be the limited to flow rates provided herein. The valve or flap can be opened at specific inhalation resistance levels. The restrictive orifice can be opened at inhalation resistances comprising a vacuum of about 1 to about 10 inches of water (a range from about 249 Pa to about 2488 Pa).

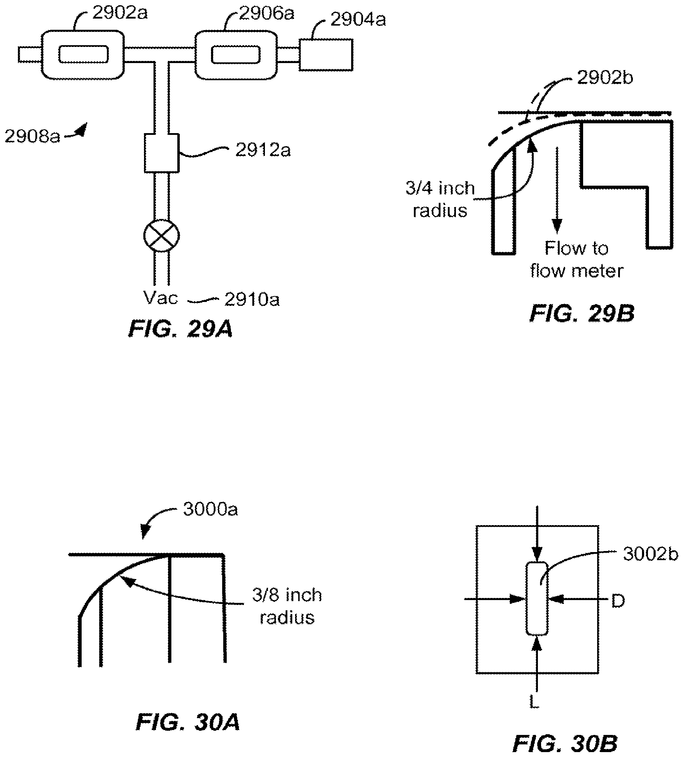

A device for producing a condensation aerosol as provided herein can be configured to limit the flow rate of a carrier gas across or through a aerosol generation area or heater element as provided herein to a flow rate of exactly, about, more than, less than, at least, or at most 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, or 16 liters per minute (LPM) (a range from about $1.667 \times 10^{-5}$ m³/s to about $2.667 \times 10^{-4}$ m³/s). A device for producing a condensation aerosol as provided herein can be configured to limit the flow rate of a carrier gas across or through a aerosol generation area or heater element to between 1-2, 2-4, 4-6, 6-8, 8-10, 10-12, 12-14, or 14-16 LPM a range from (about 1.667 x $10^{-5}$ m³/s to about 2.667 x $10^{-4}$ m³/s). A device for producing a condensation aerosol as provided herein can be configured to limit the flow rate of a carrier gas across or through a aerosol generation area or heater element to about 1 to about 2, about 2 to about 4, about 4 to about 6, about 6 to about 8, about 8 to about 10, about 10 to about 12, about 12 to about 14, or about 14 to about 16 LPM (a range from about 1.667 x $10^{-5}$ m³/s to about 2.667 x $10^{-4}$ m³/s). The flow rate can be limited by using a restrictive orifice on the inlet for a carrier gas (e.g. air). The restrictive orifice can comprise any valve or flap (see **FIG. 30A** or **FIG. 34)** and as known in the art. The valve or flap can be moderated at specific flow rates. The flow rates that moderate the valve or flap can be the limited flow rates provided herein. The valve or flap can be opened at specific inhalation resistance levels. The restrictive orifice can be opened at inhalation resistances comprising a vacuum of about 1 to about 10 inches of water (a range from about 249 Pa to about 2488 Pa). The restrictive orifice can be configured to limit the flow rates to flow rates as provided herein. The restrictive orifice can be configured into a slot as depicted in **FIG. 30B.** An aerosol generation area or heater element as provided herein can be within a flow-through passageway. The flow-through passageway can be a primary flow through passageway.

A device for producing a condensation aerosol comprising a primary flow-through passageway and one or more sources of additional or bypass carrier gas (e.g. air) as provided herein can produce a mixing ratio of bypass or additional carrier gas to carrier gas flowing through the primary flow through chamber of 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, 25:1, 26:1, 27:1, 28:1, 29:1, 30:1, 31:1, 32:1, 33:1, 34:1, 35:1, 36:1, 37:1, 38:1, 39:1, 40:1, 41:1, 42:1, 43:1, 44:1, 45:1, 46:1, 47:1, 48:1, 49:1, or 50:1. The mixing ratio can be between 1:1 and 5:1, 5:1 and 10:1, 10:1 and 15:1, 15:1 and 20:1; 20:1 and 25:1, 25:1, and 30:1, 30:1, and 35:1, 35:1 and 40:1, 40:1 and 45:1, or 45:1 and 50:1. The mixing ratio can be about 1:1 to about 5:1, about 5:1 to about 10:1, about 10:1 to about 15:1, about 15:1 to about 20:1; about 20:1 to about 25:1, about 25:1 to about 30:1, about 30:1 to about 35:1, about 35:1 to about 40:1, about 40:1 to about 45:1, or about 45:1 to about 50:1.

## Device Dimensions

In some cases, an electronic agent (e.g., nicotine) delivery device comprises the dimensions of an electronic cigarette. The electronic agent (e.g., nicotine) delivery device can have an overall cyclindrical shape. The electronic agent (e.g., nicotine) delivery device can resemble a combustible cigarette. An_electronic agent (e.g., nicotine) delivery device as provided herein can have an outer diameter of about, more than, less than, or at least 0.001, 0.0015, 0.002, 0.0025, 0.003, 0.0035, 0.004, 0.0045, 0.005, 0.0055, 0.006, 0.0065, 0.007, 0.0075, 0.008, 0.0085, 0.009, 0.0095, 0.01, 0.015, 0.02, 0.025, 0.03, 0.035, 0.04, 0.045, 0.05, 0.055, 0.06, 0.065, 0.07, 0.075, 0.08, 0.085, 0.09, 0.095, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 0.71, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, 1, 2, 3, 4, 5, 6, 7, 8, 9, 10, 11, 12, 13, 14, 15, 16, 17, 18, 19, 20, 21, 22, 23, 24, 25, 26 27, 28, 29, or 30 cm. An electronic agent (e.g., nicotine) delivery device as provided herein can have an outer diameter of about 0.5 cm to about 1 cm, about 0.25 cm to about 0.75 cm, about 0.25 cm to about 1 cm, or about 0.25 cm to about 1.5 cm.

An electronic agent (e.g., nicotine) delivery device as provided herein can have a length of about, more than, less than, or at least 20, 21, 22, 23, 24, 25, 26 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, or 150 mm. An_electronic agent (e.g., nicotine) delivery device as provided herein can have a length of about 25 mm to about 75 mm, about 75 mm to about 125 mm, about 125 mm to about 150 mm, or about 75 mm to about 150 mm

An electronic agent (e.g., nicotine) delivery device as provided herein can have a transverse dimension of about, more than, less than, or at least 1, 2, 3, 4, 5, 6, 7, 8, 9, 10, 11, 12, 13, 14, 15, 16, 17, 18, 19, 20, 21, 22, 23, 24, 25, 26 27, 28, 29, 30, 31, 32, 33, 34, 35, 36, 37, 38, 39, or 40 mm.

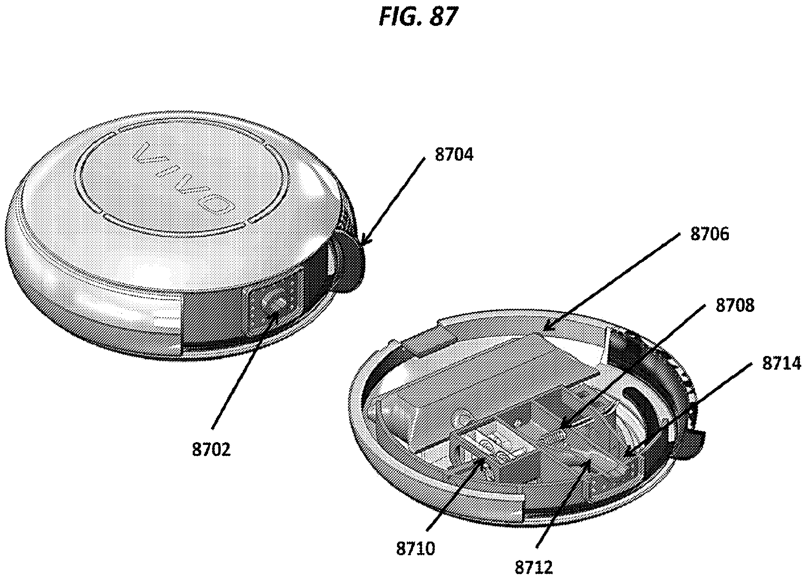

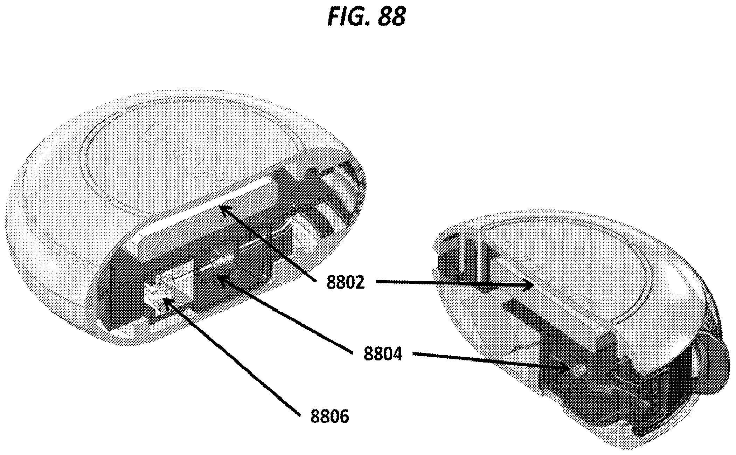

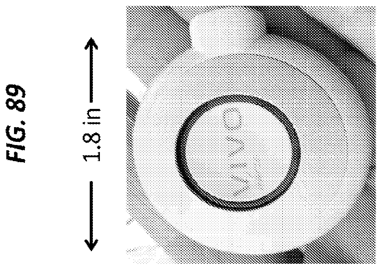

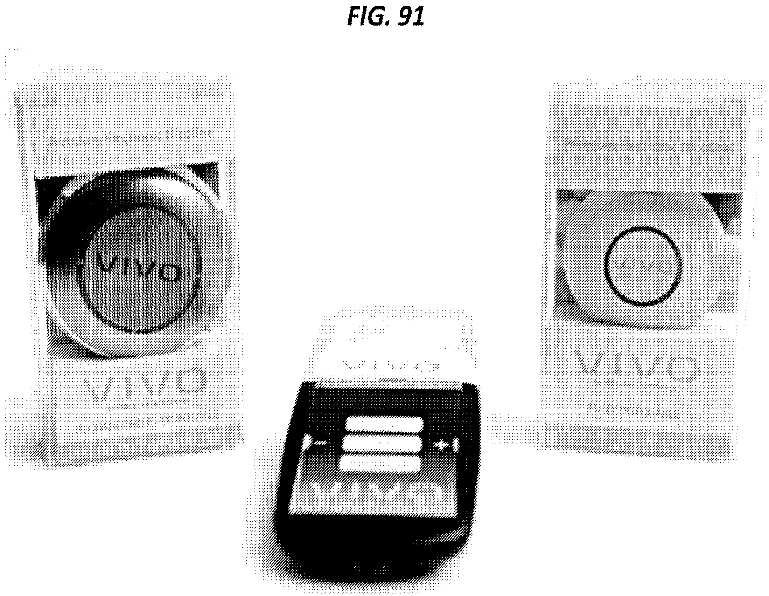

In some cases, an electronic agent (e.g., nicotine) delivery device has circular or disk-like dimensions. In some cases, the device is disk shaped. The circular or disk-shaped electronic agent (e.g., nicotine) delivery device can be a single unit. The single unit can be small enough to fit in the palm of a hand of a user of the circular electronic agent (e.g., nicotine) delivery device. The circular or disk-shaped electronic agent (e.g., nicotine) delivery device can resemble and/or have the dimensions of the structures shown in **FIGs. 86-89.** In some cases, the circular or disk-shaped electronic agent (e.g., nicotine) delivery device resembles a flattened circle comprising a same diameter in two dimensions, and a width in third dimension that is less than the diameter in the other two dimensions. The circular or disk shaped device can have an exterior diameter. The circular or disk shaped device exterior diameter can be about, more than, less than, or at least 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, or 3 inches. The exterior diameter can be from about 1 to about 3 or about 1.5 to about 2.5 inches. The exterior diameter can be between about 1 to about 1.5, about 1.5 to about 2, or about 2 to about 3 inches. The exterior diameter can be between 1 and 1.5, 1 and 2, 1 and 3, 1.5 and 2, 1.5 and 3, or 2 and 3 inches. The circular or disk shaped device can have an exterior diameter of about 1.8 or about 2.1 inches. The circular or disk shaped device can have an exterior diameter of from about 1.8 to about 2.1 inches. The circular or disk shaped device width can be about, more than, less than, or at least 0.5, 0.55, 0.6, 0.65, 0.7, 0.75, 0.8, 0.85, 0.9, 0.95, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, or 3 inches. The exterior diameter can be from about 0.5 to about 3 or about 0.5 to about 1inches. The exterior diameter can be between about 0.5 to about 1.5, about 1.5 to about 2, or about 2 to about 3 inches. The exterior diameter can be between 0.5 and 1, 1 and 2, 1 and 3, 1.5 and 2, 1.5 and 3, or 2 and 3 inches. The circular or disk-shaped device can have a width of about 0.75 inches. In some cases, the circular or disk-shaped electronic agent (e.g., nicotine) delivery device comprises a mouthpiece. The mouthpiece can comprise a door. The door can be as depicted in **FIGs. 86** and **87,** wherein the mouthpiece door **(8602; 8704)is** configured to removably cover a mouthpiece hole **(8702 in FIG. 87)** through which a user of the device inhales. The mouthpiece can comprise a protruding structure. The protruding mouthpiece can be as depicted in FIG. 89. The mouthpiece can protrude about, more than, less than, or at least 0.5, 0.55, 0.6, 0.65, 0.7, 0.75, 0.8, 0.85, 0.9, 0.95, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, or 3 inches from a side of the disk-shaped device. The circular or disk-shaped electronic agent (e.g., nicotine) delivery device can be self-contained. The circular or disk-shaped electronic agent (e.g., nicotine) delivery device can comprise a refillable or non-fillable reservoir comprising a pharmaceutically active agent (e.g., nicotine). The reservoir can be a collapsible bag. The circular or disk-shaped electronic agent (e.g., nicotine) delivery device can be self-contained can be powered by a battery as provided herein. The battery can be rechargeable. The circular or disk-shaped electronic agent (e.g., nicotine) delivery device or the reservoir can be disposable (see. FIG. 91). The reservoir can comprise an amount of an agent (e.g., nicotine) sufficient for 1, 2, 3, 4, 5, 6, 7, 8, 9, 10, 11, 12, 13, 14, 15, 16, 17, 18, 19, 20 or 21 days of use. In some cases, the reservoir comprises an amount of nicotine sufficient for 1 day of use. In some cases, the reservoir comprises an amount of nicotine sufficient for 7 days of use. In some cases, the reservoir comprises an amount of nicotine sufficient for 14 days of use. In some cases, the reservoir comprises an amount of nicotine sufficient for about 1 day to about 7 days of use. In some cases, the reservoir comprises an amount of nicotine sufficient for about 1 day to about 14 days of use. In some cases, the reservoir comprises an amount of nicotine sufficient for about 7 days to about 14 days of use. As shown in **FIG. 87,** a circular or disk-shaped device as provided herein can comprise a nicotine reservoir **(8706),** a pump **(8710),** a heater element **(8708)** and a primary **(8712)** and secondary airway **(8714).** The nicotine reservoir **(8706)** can be a collapsible bag. The pump **(8710)** can be any pump as provided herein (e.g., diaphragm or piston pump). The heater element **(8708)** can be a coil as shown in **FIG. 87** or any other heater element provided herein. The primary **(8712)** and secondary **(8714)** airways can serve to generate a condensation aerosol (primary airway; **8712)** and subsequently entrain the condensation aerosol (secondary airway; **8714)** in an airflow sufficient to deliver the condesation aerosol to the lungs of a user of the device. Additionally, **FIG. 88** shows that a device as depicted in **FIGs. 86** and **87** can further comprise a battery **(8802). FIG. 88** shows the location of the battery **(8802)** relative to the heater element **(8804)** and the pump **(8806).**

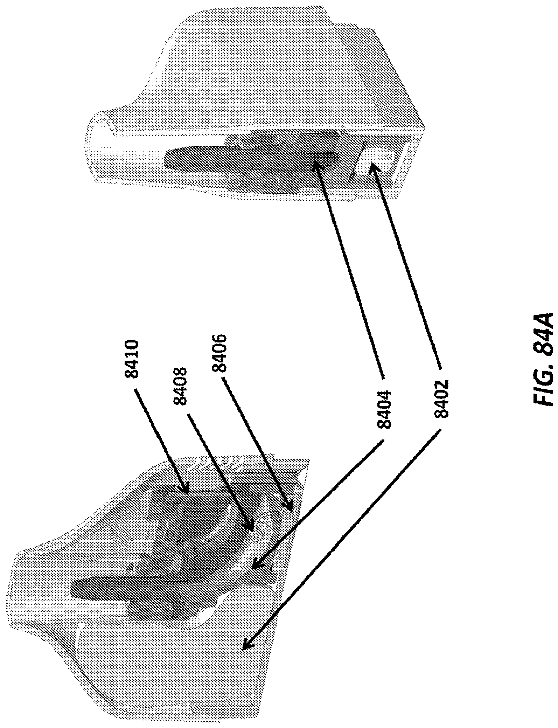

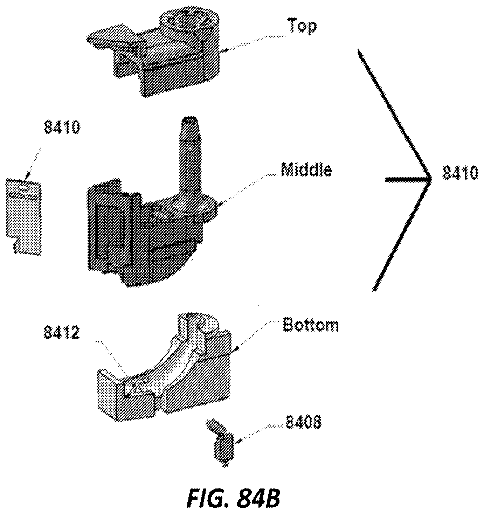

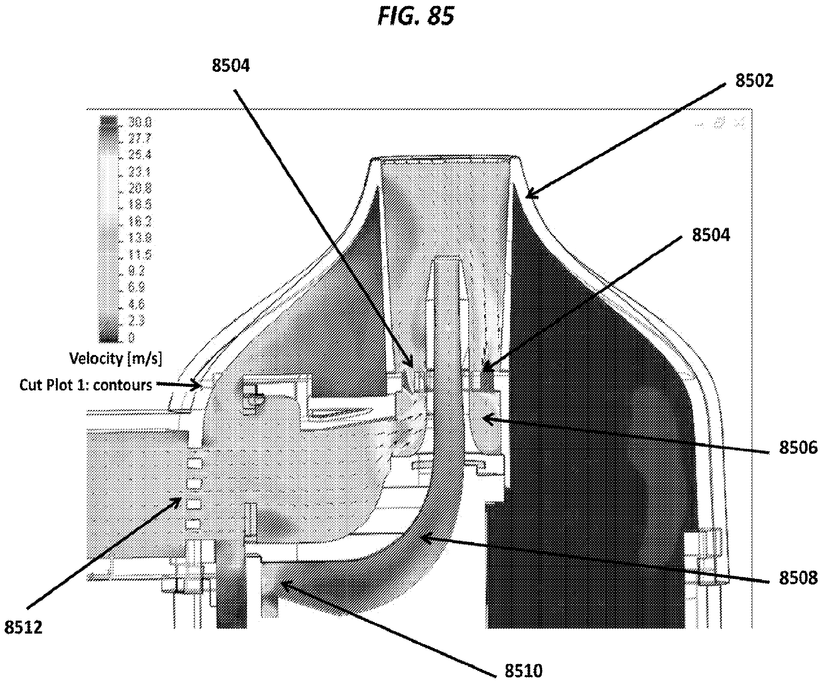

In some cases, an electronic agent (e.g., nicotine) delivery device comprises a multi-piece device as shown in **FIGs. 83A-B.** As shown in **FIGs. 83A-B,** the device can comprise three pieces. The three pieces can be detachable to and from each other. The device can comprise a controller **(8306),** a dose cartridge **(8304),** and a cap **(8302).** The cap **(8302)** can be removable and can protect from unintended use. The dose cartridge can comprise a reservoir comprising a pharmaceutically active agent as provided herein. The controller **(8306)** can have an interface. The interface can provide the calendar date and time of day. The interface can display the level of battery life or power. The interface can display connectivity (e.g., Bluetooth, infrared, cellular, etc.). The interface can display the number of doses used and/or remaining. The interface can be configured to allow scrolling to adjacent displays or screens on the interface. As shown in **FIG. 84A,** the dose cartridge can comprise a breath sensor (e.g., vane; **8410),** collapsible bag **(8402)** comprising a pharmaceutically active agent (e.g., nicotine), pump **(8406),** heater element **(8408)** as provided herein and a primary **(8404)** and/or secondary airway. As shown in **FIG. 84B,** the primary airway **(8414)** can be constructed from three parts which include bottom, middle, and top portions. The bottom portion can comprise a portion of the heater element **(8408)** as well as an outlet needle **(8412),** which can supply a volume or amount of liquid to or onto the heater element **(8408).** The middle portion can comprise the vane **(8410)** component of an inhalation sensor as provided herein. Airflow through the dose cartridge shown in **FIGs. 83A-B** and **84A-B** can be as depicted in **FIG. 85** such that outside air enters through an air inlet **(8512)** and bifurcates to flow through a primary airway **(8508)** and a secondary airway **(8506).** The primary airway can comprise the heater element in a heater element area **(8510),** and a vapor formed in the heater element area **(8510)** can ultimately condense into a condensation aerosol of a desired diameter (e.g., about 1 μm to about 5 μm). The condensation aerosol can subsequently be entrained in air flowing through the secondary airway **(8506)** that connects back up with air from the primary airway **(8508)** carrying the condensation aerosol through secondary air inlets **(8504).** Ultimately, the total volume of air carrying the condensation aerosol can flow through the mouthpiece **(8502)** into the mouth and lungs of a user of the device. (The controller can be programmable as described herein. The electronic agent (e.g., nicotine) delivery device can resemble and/or have the dimensions of the structure shown in **FIG. 83A-B, FIG. 84A-B,** and **FIG. 85.** Any of the pieces of the device can have an width of about 1.8 inches. The controller can have a length of about 2.75 inches. The cap and/or dose cartridge can have a length of about 1.25 inches.

## Agents

Any suitable agent (e.g., drug) can be used in the methods and devices described herein. Agents (e.g., pharmaceutically active agents) that can be used include, for example, drugs of one of the following classes: anesthetics, antibiotic, anticonvulsants, antidepressants, antidiabetic agents, antidotes, antiemetics, antihistamines, anti-infective agents, antineoplastics, antiparkisonian drugs, antirheumatic agents, antipsychotics, anxiolytics, appetite stimulants and suppressants, blood modifiers, cardiovascular agents, central nervous system stimulants, drugs for Alzheimer's disease management, a cold medication, COPD (chronic obstructive pulmonary disease) drug, cough medication, drugs for cystic fibrosis management, diagnostics, dietary supplements, drugs for erectile dysfunction, gastrointestinal agents, hormones, drugs for the treatment of alcoholism, drugs for the treatment of addiction, immunosuppressives, mast cell stabilizers, migraine preparations, motion sickness products, drugs for multiple sclerosis management, muscle relaxants, drugs for treating myocardial infarction, nonsteroidal anti-inflammatories, opioids, other analgesics and stimulants, opthalmic preparations, osteoporosis preparations, pain medication, panic medication, prostaglandins, respiratory agents, sedatives and hypnotics, skin and mucous membrane agents, Tourette's syndrome agents, urinary tract agents, insomnia medication, weight loss drug, and vertigo agents. In some cases, an agent is an herb, supplement, or vitamin.

An anesthetic can be ketamine, procaine, amethocaine, cocaine, prilocaine, bupivacaine, levobupivacaine, ropivacaine, mepivacaine, dibucaine, or lidocaine. An anesthetic can be desflurane, enflurane, halothane, isofurane, methoxyflurane, or sevoflurane, amobarbital, methohexital, thiamylal, thiopental, diazepam, lorazepam, midzolam, etomidate, or propofol. An anesthetic can be atracurium, ciastracurium besyalte, rapacuronium, rocuronium, succinylcholine, or suxamethonium chloride. An anesthetic can be articaine, benzocaine, benzonatate, butacaine, butanilicaine, chloroprocaine, cinchocaine, dimethocaine, eucaine, etidocaine, hexylcaine, levobupivacaine, mepivacaine, meprylcaine, metabutoxycaine, orthocaine, oxybuprocaine, phenacaine, piperocaine, pramocaine, prilocaine, procaine, proparacaine, propoxycaine, quinisocaine, ropivacaine, trimecaine, or tetracaine.

An antibiotic can be an aminoglycoside (e.g., amikacin, gentamicin, kanamycin, neomycin, netilmicin, tobramycin, paromomycin, spectinomycin); an ansamycin (e.g., geldanamycin, herbimycin, rifaximin, streptomycin); a carbacephem (e.g., loracarbef); a carbapenem (e.g., ertapenem, doripenem, imipenem/cilastatin, meropenem); a cephalosporin (first generation) (e.g., cefadroxil, cefazolin, cefalotin or cefalothin, cefalexin); a cephalosporin (second generation) (e.g., cefaclor, cefamandole, cefoxitin, cefprozil, cefuroxime); a cephalosporin (third generation) (e.g., cefixime, cefdinir, cefditoren, cefoperazone, cefotaxime, cefpodoxime, ceftazidime, ceftibuten, ceftizoxime, ceftriaxone); a cephalosporin (fourth generation) (e.g., cefepime); a cephalosporin (fifth generation) (e.g., ceftaroline fosamil, ceftobiprole); a glycopeptide (e.g., teicoplanin, vancomycin, telavancin); a lincosamide (e.g., clindamycin, lincomycin); a lipopeptide (e.g., daptomycin); a macrolide (e.g., azithromycin, clarithromycin, dirithromycin, erythromycin, roxithromycin, troleandomycin, telithromycin, spiramycin); a monobactam (e.g., aztreonam); a nitrofuran (e.g., furazolidone, nitrofurantoin); an oxazolidonone (e.g., linezolid, posizolid, radezolid, torezolid); a penicillin (e.g., amoxicillin, ampicillin, azlocillin, carbenicillin, cloxacillin, dicloxacillin, flucloxacillin, mezlocillin, methicillin, nafcillin, oxacillin, penicillin g, penicillin v, piperacillin, penicillin g, temocillin, ticarcillin); a penicillin combination (e.g., amoxicillin/clavulanate, ampicillin/sulbactam, piperacillin/tazobactam, ticarcillin/clavulanate); a polypeptide (e.g., bacitracin, colistin, polymyxin b); a quinolone (e.g., ciprofloxacin, enoxacin, gatifloxacin, levofloxacin, lomefloxacin, moxifloxacin, nalidixic acid, norfloxacin, ofloxacin, trovafloxacin, grepafloxacin, sparfloxacin, temafloxacin); a sulfonamide (e.g., mafenide, sulfacetamide, sulfadiazine, silver sulfadiazine, sulfadimethoxine, sulfamethizole, sulfamethoxazole, sulfanilimide (archaic), sulfasalazine, sulfisoxazole, trimethoprim-sulfamethoxazole (co-trimoxazole) (tmp-smx), sulfonamidochrysoidine (archaic)); a tetracycline (e.g., demeclocycline, doxycycline, minocycline, oxytetracycline, tetracycline); a drug against mycobacteria (e.g., clofazimine, dapsone, capreomycin, cycloserine, ethambutol, ethionamide, isoniazid, pyrazinamide, rifampicin (rifampin in US), rifabutin, rifapentine, streptomycin); or another antibiotic (e.g., arsphenamine, chloramphenicol, fosfomycin, fusidic acid, metronidazole, mupirocin, platensimycin, quinupristin/dalfopristin, thiamphenicol, tigecycline, tinidazole, trimethoprim).

An anticonvulsant can be an aldehyde (e.g., paraldehyde), an aromatic allylic alcohol (e.g., stiripentol), a GABA analog (e.g., gabapentin, pregabalin); a barbiturate (e.g., pentobarbital, methylpenobarital, or barbexaclone); a benzodiazepine (e.g., clonazepam, clobazam, clorazepate, diazepam, midazolam, nitrazepam, temezepam, nimetazepam, or lorazepam); a bromide (e.g., potassium bromide), a carbamate (e.g., felbamate), a caroxamide (e.g., carbamazepine, oxcarbazepine, eslicarbazepine acetate), a fatty acid (e.g., vaproate (e.g., valproic acid, sodium valproate, divalproex sodium), vigabatrin, progabide, or tiagabine), a fructose derivative (e.g., topiramate), a hydantoin (e.g., phenyloin, ethotoin, mephenytoin, or fospheytoin); an oxazolidinedione (e.g., paramethadoine, trimethadione, or ethadione), a propionate (e.g., beclamide), a pyrimidinedione (e.g., primidone), a pyrrolidine (e.g., brivaracetam, levetiracetam, seletracetam), a succinimide (e.g., ethosuximide, phensuximide, mesuximide), a sulfonamide (e.g., acetazoamide, sultiame, methazolamide, or zonisamide), a triazine (e.g., lamatrigine), a urea (e.g., pheneturide, phenacemide), a valproylamide (e.g., valpromide or valnoctamide), or a phenyltriazine (e.g., lamotrigine).

An antidepressant can be a selective serotonin reuptake inhibitor (SSRI, e.g., citalopram, escitalopram, paroxetine, fluoxetine, fluvoxamine, sertraline), a norepinephrine reuptake inhibitor (NRI, e.g., atomoxetine, reboxetine, viloxazine), a noradrenergic and specific serotonergic antidepressant (NaSSA e.g., mianserin, mirtazapine), a serotonin-norepinephrine reuptake inhibitor (SNRIs, e.g., desvenlafaxine, duloxetine, milnacipran, venlafaxine), a serotonin antagonist and reuptake inhibitor (SARIs, e.g., etoperidone, nefazodone, trazodone), a norepinephrine-dopamine reuptake inhibitor (e.g., bupropion), a selective serotonin reuptake enhancer (e.g., tianeptine, amineptine), a norepinephrine-dopamine disinhibitor (NDDIs e.g., agomelatine), a tricyclic antidepressant (e.g., tertiary amine tricyclic antidepressants (amitriptyline, clomipramine, doxepin, imipramine, trimipramine) or secondary amine tricyclic antidepressants (e.g., desipramine, nortriptyline, protriptyline)), a monoamine oxidase inhibitor (MAOIs e.g., isocarboxazid, mocolobemide, phenelzine, selegiline, tranylcypromine), nicotine, caffeine, or lithium. In some cases, the antidepressant is agomelatine, amitriptyline, amoxapine, atomoxetine, buspirone, benmoxine, butriptyline, citalopram, clomipramine, desipramine, dosulepin, doxepin, duloxetine, escitalopram, etoperidone, femoxetine, fluovoxamine, imipramine, kitanserin, lofepramine, medifoxamine, mianserin, maprotoline, mazindol, milnacipran, mirtazapine, nefzaodone, nisoxetine, nomifensine, nortriptyline, protriptyline, oxaprotiline, paroxetine, reboxetine, sertaline, trazodone, trimipramine, venlafaxine, viloxazine, zimelidine, citalopram, cotinine, duloxetine, fluoxetine, fluvoxamine, milnacipran, nisoxetine, paroxetine, reboxetine, sertraline, tianeptine, acetaphenazine, binedaline, brofaromine, cericlamine, clovoxamine, iproniazid, isocarboxazid, moclobemide, phenyhydrazine, phenelzine, selegiline, sibutramine, tranylcypromine, ademetionine, adrafinil, amesergide, amisulpride, amperozide, benactyzine, bupropion, caroxazone, gepirone, idazoxan, metralindole, milnacipran, minaprine, nefazodone, nomifensine, ritanserin, roxindole, S-adenosylmethionine, escitalopram, tofenacin, trazodone, tryptophan, or zalospirone.

An antidiabetic agent can be insulin, a sufonylurea (e.g., tolbutamide, acetohexamide, tolazmide, chlorpropamide, glyburide, glibenclamide, glimepiride, gliclazide, glycopyramide, gliquidone, or glipizide), a biguanide (e.g., metformin, phenformin, or buformin), an alpha-glucosidase inhibitor (e.g., acarbose, miglitol, or voglibose), a meglitinide (e.g., repaglinide, nateglinide), or a thiazolidinedione (e.g., pioglitazone rosiglitazone, or troglitazone). An antidiabetic agent can be an injectable glucagon-like peptide analog (e.g., exenatide, liraglutide), or a dipeptidyl peptidase-4 inhibitor (e.g., vildagliptin, sitagliptin, saxagliptin, linagliptin, allogliptin, septagliptin).

An antidote can be edrophonium chloride, flumazenil, deferoxamine, nalmefene, naloxone, or naltrexone. An antidote can be activated charcoal (e.g., with sortibal), adenosine, atropine, beta blocker, calcium chloride, calcium gluconate, a chelator (e.g, EDTA, dimercaptrol, penicillamine, EGTA, or 2,3-dimercaptosuccinic acid), a cyanide antidote (amyl nitrite, sodium nitrite, thiosulfate), cyproheptadine, deferoxamine mesylate, digoxin immune Fab antibody, diphenhydramine hydrochloride, benztorpine mesylate, ethanol, fomepizole, flumazenil, glucagon, insulin, insulin with glucagon, leucovorin, methylene blude, naloxone hydrochloride, N-acetylcysteine, octreotide, pralidoxime chloride (2-PAM), protamine sulfate, Prussian blue, physostigmine sulfate, pyridoxine, phytomenadione (vitamin K), or sodium bicarbonate.

An antiemetic can be a 5-HT3 receptor antagonist (e.g., dolasetron, granisetron, ondansetron, tropisetron, palonosetron, or mirtazapine), a dopamine antagonist (e.g., doperidone, olanzapine, droperidol, haloperidol, chlorpormaine, promethazine, prochloperazine, alizapride, prochlorperazine, metoclopramide), an NK1 receptor antagonist (e.g., aprepitant, casopitant), an antihistamine (H1 histamine receptor antagonist; e.g., cyclizine, diphenhydramine, dimenhydrinate, doxylamine, meclozine, promethazine, hydroxyzine), a cannabinoid (e.g., cannabis, dronabinol, nabilone, one of a JWH cannabinoid series), a benzodiazepine (e.g., midazolam, lorazepam), an anticholinergic (e.g., hyoscine), a steroid (e.g., dexamethasone), trimethobenzamide, ginger, emetrol, propofol, peppermint, muscimol, or ajwain. In some cases, the antiemetic is alizapride, azasetron, benzquinamide, bromopride, buclizine, chlorpromazine, cinnarizine, clebopride, cyclizine, diphenhydramine, diphenidol, dolasetron, droperidol, granisetron, hyoscine, lorazepam, dronabinol, metoclopramide, metopimazine, ondansetron, perphenazine, promethazine, prochlorperazine, scopolamine, triethylperazine, trifluoperazine, triflupromazine, trimethobenzamide, tropisetron, domperidone, or palonosetron.

An antihistamine can be an H1-receptor antagonist (e.g., acrivastine, azelastine, bromopheniramine, buclizine, bromodiphenhydramine, carbinoxamine, cetirizine, chlorpromazine, cyclizine, chlorpheniramine, chlorodiphenhydramine, celmastine, cyproheptadine, desloratadine, dexbrompheniramine, dexchlorpheniramine, dimenhydramine, doxylamine, ebastine, embramine, fexofenadine, levocetirizine, loratadine, meclozine, mirtazapine, olopatadine, orphenadrine, pheninadamine, pheniramine, phenyltooxamine, promethazine, pyrilamine, quetiapine, rupatadine, tripelennamine, triprolidine), an H2-receptor antagonist (e.g., cimetidine, famotidien, lafutidien, mizatidine, ranitidine, roxatidine), and H3-receptor antagonist (e.g., A-349,821, ABT-239, ciproxifam, clobenpropit, conessine, thioperamide), or and H4-receptor antagonist (e.g., thioperamide, JNJ 7777120, or VUF-6002). In some cases, an antihistamine can be astemizole, azatadine, brompheniramine, carbinoxamine, cetrizine, chlorpheniramine, cinnarizine, clemastine, cyproheptadine, dexmedetomidine, diphenhydramine, doxylamine, fexofenadine, hydroxyzine, loratidine, hyroxyizine, promethazine, pyrilamine or terfenidine.

A drug can be an allergy medication. In some cases, the allergy medication can be an antihistamine, montelukast, azelastine/fluticaseon propionate, beclomethasone dipropionate, budesonide, ciclesonide, cromlyn sodium, flunisollide, fluticaonse furoate, fluticasone propionate, ipratropium bromide, mometasone furoate monohydrate, olopatadine, oxymetazoline, triamcinolone acetonide, azelastine, cromolyn, emadastine, epinastine, ketorolac, ketotifen, lodoxamine, loteprednol, naphazoline, naphazoline/pheniramine, nedocromil, olopatadine, pemirolast, epinephrine, aclometasone, fluocinolone, fluocinonide, triamcinolone, desonide, fluocinolone, flurandrenolide, fluandrenolide, fluticaonse, hydrocortisone butyrate, hydrocortisone probuate, hydrocortisone valerate, mometasone, prednicarbate, triamcinolone, amcinonide, betamethazone valerate, desoximetasone, diflorasone, fluocinonide, halcononide, triamcinolone, betamethasone bipropionate, clobetasol priopionate, diflorasone, flurandrenolide, halobetasol propionate, doxepin, pimecrolimus, tacrolimus, C1 inhibitor, ecallantide, cortisone acetate, dexamethasone, hydrocortisone, methylprednisolone, prednisolone, or prednisone.

An anti-infective agent can be selected from one of the following classes: antivirals (e.g., abacavir, acyclovir, acyclovir, adefovir, amadtadine, amprenavir, ampligen, arbidol, atazanavir, atripla, balavir, boceprevirertet, cidofovir, combivir, darunavir, delavirdine, didanosine, docosanol, edoxudine, efavirenz, emtricitabine, enfuvirtide, entecavir, famciclovir, fomivirsen, fosamprenavir, foscarnet, fosfonet, ganciclovir, ibacitabine, imunovir, idoxuridine, imiquimod, indinavir, inosine, lamivudine, lipinavir, loviride, maraviroc, moroxydine, methisazone, nelfinavir, nevirapine, nexavir, oseltamivir, peginterferon alpha-2a, penciclovir, peramivir, pleconaril, podophyllotoxin, raltegravir, ribavirin, rimantadine, ritonavir, pyramidine, saquinavir, atavudine, teleprevir, tenofovir, tenofovir disoproxil, tipranavir, trifluidine, trizivir, tromantadine, truvada, valaciclovir, valganciclovir, vicriviroc, vidarabine, viramidine, zalcitabine, zanamivir, or zidovudine); AIDS adjunct agents such as dapsone; aminoglycosides (e.g., streptomycin, neomycin, framycetin, paromomycin, ribostamycin, kanamycin, amikacin, arbekacin, bekanamycin, dibekacin, tobramycin, spectinomycin, hygromycin B, paromoycin sulfate, gentamicin, netilmicin, sisomicin, isepamicin, verdamicin, or astromicin); antifungals (e.g., imidazoles, e.g., bifonazole, butoconazole, clotrimazole, econazole, fenticonazole, isoconazole, ketoconazole, miconazole, omoconazole, oxiconazole, sertaconazole, sulconazole, or tioconazole; trizoles, e.g., albaconazole, fluconazole, isavuconazole, itraconazole, posaconazole, ravuconazole, terconazole, voriconazole; thiazoles e.g., abafungin; allyamines, e.g., amorolfin, butenafine, naftifine, terbinafine; echinocandins e.g., anidulafugin, caspofungin, micafungin; benzoic acid, ciclopirox, flucytosine, griseofulvin, haloprogin, polygodial, tolnaftate, undecylenic acid, or crystal violet); antimalarial agents (e.g., quinine, chloroquine, amodiaquine, pyrimethamine, proguanil, sulfadoxine, sulfamethoxypryidazine, mefloquine, atovaquone, atovaquoneproguanil, primaquine, artemisinin, artemether, artesuante, dihyroartemisinin, arteether, halofantrine, doxycycline, clindamycin); antituberculosis agents (e.g., ethambutol, isoniazid, pyrazinamide, rifampicin); .beta.-lactams (e.g., cefmetazole, cefazolin, cephalexin, cefoperazone, cefoxitin, cephacetrile, cephaloglycin, cephaloridine; cephalosporins, such as cephalosporin C, cephalothin; cephamycins such as cephamycin A, cephamycin B, and cephamycin C, cephapirin, cephradine); leprostatics (e.g., acedapsone, clofazimine, dapsone, desoxyfructo-serotonin, diucifon, ethionamide, rifampicin, rifapentine, sulfameter, thalidomide); penicillins (e.g., ampicillin, amoxicillin, hetacillin, carfecillin, carindacillin, carbenicillin, amylpenicillin, azidocillin, benzylpenicillin, clometocillin, cloxacillin, cyclacillin, methicillin, nafcillin, 2-pentenylpenicillin, penicillin N, penicillin O, penicillin S, penicillin V, dicloxacillin; diphenicillin; heptylpenicillin; and metampicillin); quinolones (e.g., cinoxacin, nalidixic acid, oxolinic acid, piromidic acid, pipemidic acid, rosoxacin, ciprofloxacin, enoxacin, fleroxacin, lomefloxacin, madifloxacin, norfloxacin, ofloxacin, pefloxacin, rufloxacin, balofoxacin, clinafloxacin, difloxacin, grepafloxacin, levofloxacin, pazufloxacin, sparfloxacin, tosufloxacin, norfloxacin, ofloxacine, temafloxacin, clinafloxacin, gatifloxacin, gemifloxacin, moxifloxacin, sitafloxacin, trovafloxacin, prulifloxacin); tetracyclines (e.g., tetracycline, chlortetracycline, demeclocycline, doxycycline, oxytetracycline, lymecycline, meclocycline, methacycline, minocycline, rolitetracycyline, tigecycline; miscellaneous anti-infectives such as linezolide, trimethoprim and sulfamethoxazole.

An anti-neoplastic agent can be, e.g., lomustine, carmustine, steptozocin, mechlorethamine, melphalan, uracil nitrogen mustard, chlorambucil, cyclophosphamide, iphosphamide, cisplatin, carboplatin, mitomycin, thiotepa, dacarbazin, procarbazine, hexamethyl melamine, triethylene melamine, busulfan, pipobroman, mitotane, methotrexate, trimetrexate, pentostatin, cytarabine, Ara-CMP, fludarabine phosphate, hydroxyurea, fluorouracil, floxuridine, chlorodeoxyadenosine, gemcitabine, thioguanine, 6-mercaptopurine, bleomycin, toptecan, irinotecan, camptothecin sodium salt, daunorubicin, doxorubicin, idarubicin, mitoxantrone, teniposide, etoposide, dactinomycin, mithramycin, vinblastine, vincristine, nvalebine, paclitaxel, docetaxel, droloxifene, tamoxifen, or toremifene.

An antiparkisonian drug can be amantadine, baclofen, biperiden, benztropine, orphenadrine, procyclidine, trihexyphenidyl, levodopa, carbidopa, andropinirole, apomorphine, benserazide, bromocriptine, budipine, cabergoline, eliprodil, eptastigmine, ergoline, galanthamine, lazabemide, lisuride, mazindol, memantine, mofegiline, pergolide, piribedil, pramipexole, propentofylline, rasagiline, remacemide, ropinerole, selegiline, spheramine, terguride, entacapone, or tolcapone.

An antirheumatic agent can be abatacept, adalimumab, azathioprine, chloroquine, diclofenac, hydroxychloroquine, methotrexate, ciclosporin, D-penicillamine, etanercept, golimumab, infliximab, leflunomide, miocyline, rituximab, or sulfasalzine.

An antipsychotic can be acetophenazine, alizapride, amisulpride, amoxapine, amperozide, aripiprazole, asenapine, benperidol, benzquinamide, bromperidol, buramate, butaclamol, butaperazine, carphenazine, carpipramine, chlorpromazine, chlorprothixene, clocapramine, clomacran, clopenthixol, clospirazine, clothiapine, clopenthixol, clozapine, cyamemazine, droperidol, flupenthixol, fluphenazine, fluspirilene, haloperidol, loxapine, melperone, mesoridazine, levomepromazine, pimozide, metofenazate, molindrone, olanzapine, paliperidone, Iloperidone, lurasidone, penfluridol, periciazine, perphenazine, pimozide, pipamerone, piperacetazine, pipotiazine, prochlorperazine, promazine, quetiapine, remoxipride, risperidone, sertindole, spiperone, sulpiride, thioridazine, thiothixene, trifluperidol, triflupromazine, trifluoperazine, ziprasidone, zotepine, or zuclopenthixol.

An anxiolytic can be a benzodiazepine (e.g., alprazolam, chlordiazepoxide, clonazepam, diazepam, etizolam, lorazepam, oxazepam); tofisopam; a selective serotonin reuptake inhibitor (SSRI); afobazole; selank; bromantane; an azapirone (e.g., buspirone, tandospirone, gipeirone); a barbiturate; hydroxyzine; pregalalin; validol; an herbal treatment (e.g., Bacopa monnieri, Lactuca virosa, Rohodiola rosea, Hypericum perforatum, Matricaria recutita, Passiflora incarnate, Piper methysticum; Sceletium tortuosum, Scutellaria lateriflora; Valeriana officinalis; Salvia splendens; Coriandrum sativum; Myristica; Salvia elegans; Inositol; Cannabidiol); an over-the counter pharmaceutical drug (e.g., picamilon; chlorpheniramine; diphenhydramine; melatonin); BNC210; CL-218,872; L-838,417; SL-651,498; or aloradine. In some cases, an anxiolytic can be alprazolam, bromazepam, oxazepam, buspirone, hydroxyzine, mecloqualone, medetomidine, metomidate, adinazolam, chlordiazepoxide, clobenzepam, flurazepam, lorazepam, loprazolam, midazolam, alpidem, alseroxlon, amphenidone, azacyclonol, bromisovalum, captodiamine, capuride, carbcloral, carbromal, chloral betaine, enciprazine, flesinoxan, ipsapiraone, lesopitron, loxapine, methaqualone, methprylon, propanolol, tandospirone, trazadone, zopiclone, or zolpidem.

An appetite stimulant (orexigenic) can be ghrelin, orexin, neuropeptide Y; a 5-HT2c receptor antagonist (e.g., mirtazapine, alanzapine, quetiapin, amitriptyline, cyrpoheptadine); an H1 receptor antagonist (e.g., mirtazapine, olanzapine, quetiapine, amitriptyline, cyproheptadine); a dopamine antagonist (e.g., haloperidol, chlorpromazine, olanzapine, risperidone, quetiapine); an adrenergic antagonist (e.g., carvedilol, propranolol; alpha2-adrenergi agonist (e.g., clonidine); a CB1 receptor agonist (e.g., THC/dronabinol, nabilone); a corticosteroid (e.g., dexamethasone; prednisone, hydrocortisone); a pregnene steroid (e.g., oxandrolone, nandrolone, testosterone); a sufonylurea (e.g., glibenclamide, chlopropamide).

An appetite suppressant can be diethylpropion, rimonabant, oxymetazoline, fenfluramine, phentermine, sibutramine, benfluorex, butenolide, cathine, diethylpropion, FG-7142, phenmetrazine, phenylpropanolamine, pryoglutamyl-histidyl-glycine, amfepramon, amphetamine, benzphetamine, dexmethylphenidate, dextroamphetamine, glucagon, lisdexamfetamine, methamphetamine, methylphenidate, phendimetrazine, phenethylamine, or bupropion.

A blood modifier can be an anticoagulant (e.g., heparin); colony stimulating factor (e.g., fligrastim, pegfilgrastim; sargramostim); phytonadione (Vitamin K); iron; iron combination (e.g., iron + vitamin C) cilostazol, dipyridamol, abbokinase, abciximab, activase, advate, aggrastat, aggrenox, agrylin, albumin, alteplase, amicar, aminocaproic acid, anadrol, anagrelide, angiomax, anti-inhibitor coagulant complex; antihemophilic factor, antithrombin III, aprotinin, aquamephyton, aranesp, argtroban, arixtra, aspirin, aspirin + dipryidamole, benefix, bivalirudin, buminate 25%, buminate 5%, cathflo activas, clopidogrel, Coagulation Factor IX, Coagulation Factor IX Human, Coagulation Factor VIIA, Coumadin, Cyanocobalamin Nasal, cykokapron, Dalteparin, Ddavp, drotrecogin alpha, ecotrin, eltrombopag, enoxaparin, epoetin alpha, epogen, epoprostenol, eptifibatide, erythropoiesis stimulating protein, feiba VH, ferrlecit, fibrinogen human, flolan, fondaparinux subcutaneous, fragmin, gammaplex, hemofil M, human immunoglobulin G, infed, integrilin, iron dextran, jantoven, kinlytic, koate-DVI, kogenate, lepirudin, leukine, lovenox, mephyton, mononine, mozobil, nascobal, neulasta, neumega, novoseven, nplate, oprelvekin, pegfilgrastim, pentoxifylline, pentoxil, persantine, phytondione, plasbumin-25, pasbumin-5, plasma protein fraction, plasmanate, plavix, plerixafor, pletal, procrit, promacta, recombinate, refacto, refludan, reopro, riastap, romiplostim, sargramostim, sodium ferric gluconate, tenecteplase, thrombate III, thrombin, ticlid, ticlopidien, tirofiban, tnkase, tranexamic acid, trasylol, trental, urokinase, vitamin K1, warfarin, or xigris.

An asthma agent can be fluticaone, budeonside, mometasone, beclomethasone, zariflukast, zileuton, flunisolide, ciclesonide, triamcinolone, ipratropium, dyphylllin/guaifenesin, dexamethasone, prednisone, methylprednisolne, formoterol/mometeasone, triamcinolone, montelukast, isoetharine, dyphylline, salmeterol, budeonside/formoterol, mometasone/formoterol, theophylline, albuterol, levabulterol, ipratropium, omalizumab, or guaifenesin/theophylline. In some cases, the asthma medication can be an inhaled corticosteroid (e.g., beclomethasone propionate, budesonide, budesonide/formoterol, ciclesonide, blunisolide, fluticasone propionate, fluticaonse/salmeterol, mometasone, memetasone/formoterol, or triamcinolone acetonide). In some cases the asthma agent can be a long-acting beta-agonist (LABA; e.g., albuterol sulfate, formoterol fumarate, salmeterol xinafoate, or arformoterol tartrate). In some cases, the asthma agent can be cromolyn sodium or theophylline. In some cases an asthma agent can be a leukotriene modifier (e.g., montelukast, zafirlukast, zileuton). In some cases, an asthma agent can be an immunomodulator (e.g., omalizumab). In some cases, an asthma agent can be a short-acting beta-agonist (SABA; e.g., albuterol sulfate, ipratropium bromide/albuterol sulfate, ipratropium bromide HFA, levalbuterol HCI, pirbuterol, tiotropium bromide). In some cases, an asthma agent is duplilumab. In some cases, the asthma agent is bambuterol, bitolerol, doxofylline, ephedrine, epinephrine/chlorpheniramine, erythromycin, hydrocortisone, ipratropium bromide, isoetharine, isoprenaline, isoproterenol, ketotifen, metaproterenol, mometasone furoate and formoterol fumarate, nedocromil, oxtriphylline, salmeterol/fluticasone, terbutaline, tinocordin, triamcinolone, zafirlukast, or zileuton.

A cardiovascular agent can be fenoldopam, diazoxide, nitroprusside, ambrisentan, epoprostenol, treprostinil, sildenafil, bosentan, iloprost, treprostinil, epoprostenol; an aldosterone receptor antagonist (e.g., spironolactone, eplerenone); an angiotensin converting enzyme inhibitor (e.g., fosinopril, ramipril, captopril, trandolapril, moexipril, lisinopril, quinapril, enalapril, lisinopril, perinodpril, benazepril); an angiontensin II inhibitor (e.g., eprosartan, olemsartan, azilsartan medoxomil, telmisartan, losartan, valsartan, candesartan, irbesartan); an antiadrenergic agent, centrally acting (e.g., clonidine, fuanfacine, methyldopa, guanabenz); an antiadrenergic agent, peripherally acting (e.g., doxazosin, prazosin, terazosin, silodosin, alfuzosin, tamsulosin, dutasertide/tamsulosin, guanadrel, mecemylamine, guanethidine); an antianginal agent (e.g., nitroglycerin, ranolazine, isosorbide mononitrate, isosorbide dinitrate); an antiarrhythmic agent (e.g., group I (e.g., moricizine, guanidine, disopyramide, phenytoin, propafenone, flecainide, disopyramide, phenytoin, mexiletine, quinidine, tocainide, lidocaine, procainamide); group II (e.g., propranolol, esmolol, acebutolol); group III (e.g., amiodarone, sotalol, dofetilide, dronedarone, amiodarone, sotalol, ibutilid); group IV (e.g., ditiazem, verapamil); group V (e.g., adenosine, digoxin); and anticholinergic chronotropic agent (e.g., atropine); an antihypertensive combination (e.g., bendroflumethiazide/nadolol, eprosartan/hydrochlorothiazide, amlodipine/hydrochlorothiazide/valsartan, amplodipine/atorvastatin, hydrochlorothiazide/telmisartan, trandolapril/verapamil; hydrochlorthiazide/irbesartan, hydralazine/hydrochlorothiazide, hydrochlorothiazide/triamterene, diltiazem/enalapril, aliskiren/hdrochlorothiazise, amlodipine/telmisartan, amlodipine/olmesartan, atenolol/chlorthalidone, hydrochlorothiazide/moexipril, hydrochlorothiazide/olmesartan, hydrochlorothiazide/lisinopril, hydrochlorothiazide/valsartan, hydrochlorothiazide/losartan, hydrochlorthiaxide/quinapril, hyrodchlorothiazide/spironolactone, azilsartan medoxomil/chlorthalidone, amlodipine/benazepril, amiloride/hydrochlorothiazise, hydrochlrothiazide/lisinopril, amlodipine/hydrochorothiazide/olmesartan, amlodipine/valsartan, aliskirne/valsartan, hydrocholorthiazide/triamterene, bisoprolol/hydrochlorothiazide, candesartan/hydrochlorothiazide, chrlorthiazide/methyldopa, hydrochlorothiazide/triamterene, hydroclorothiazide/methyldopa, chlorothiazide/methyldopa, hydrochlrothiazide/methyldopa, amlodipine/benazepril, aliskiren/amlodipine/hydrochlorothiazide, hydrazine/hydrochlorothiazide, hydralazine/isosrbide dinitrate, captopril/hydrochlorothiazide, chlorthalidone/clonidine, bendroflumethiazide/nadolol, bendrofluemethiazide/nadolol, chlorthalidone/reserpine, hydralazine/hydrochlorothiazide/reserpine, hydrochlorothiazide/metoprolol, deserpidine/methyclothiazide, guanethidine/hydrochlorothiazide, hydrochlorothiazide/propranolol, enalapril/felodipine, polythiazide/prazosin, amiloride/hydrochlorothiazise, fosinopril/hydrochlorothiazide, hydrochlorothiazide/quinapril, chlorthalidone/reserpine, polythiazide/reerpine, aliskiren/amlodipine, atenolol/chlorthalidone, hydrochlorothiazide/timolol); a beta-adrenergic blocking agent (e.g., cardioselective beta blocker (e.g., betaxolol, bisoprolol, atenolol, metoprolol, nibivolol, esmolol, acebutolol); noncardioselective beta blocker (e.g., propranolol, nadolol, sotalol, carvedilol, labetalol, timolol, carteolol, penbutolol, pindolol)); a calcium channel blocking agent (e.g., nifedipine, diltiazem, nimodipine, verapamil, felodipine, nicardipine, isradipine, nisoldipine, clevidipine, bepridil); a peripheral vasodilator (e.g., cyclandelate, papverine, isoxsuprine); a catecholamine (e.g., epinephrine, isoproterenol, norepinephrine); a diuretic (e.g., carbonic anhydrase inhibitor (e.g., acetazolamide, dichlophenamide, methazolamide), loop diuretic (e.g., torsemide, furosemide, bumetanide, ethacrynic acid); pamabrom, mannitol; a potassium-sparing diuretic (e.g., triamterene, spironolactone, amiloride); a thiazide diuretic (e.g., indapamide, hydrochlorothiazide, metolazone, methylclothizode, hydrochlorothiazide, chlorothiazide, methyclothizide, metolazone, bendroflumethiazide, polythiazide, hydrofluemethiazide, chlorthalidone)); a inotropic agent (e.g., digoxin, dobutamine, milrinone); icatibant, cilostazol, midodrine, metyrosine, phenoxybenzamine, EDTA, phentolamine; rennin inhibitor (e.g., aliskiren); a peripheral vasodilator (e.g., cyclandelate, papaverine, isoxsuprine); a sclerosing agent (e.g., laureth-9, ethanolamine oleate, morrhuate sodium, sodium tetrdecyl sulfate); a vasodilator (e.g., nitroglycerin, alprostadil, hydralazine, minoxidil, mesiritide, nitroprusside); a vasopression antagonist (e.g., conivaptan, tolvaptan); or a vasopressor (e.g., epinephrine, isoproterenol, phenylephrine, norepinephrine, dobutamine, isoproterenol). In some cases, the cardiovascular agent can be benazepril, captopril, enalapril, quinapril, ramipril, doxazosin, prazosin, clonidine, labetolol, candesartan, irbesartan, losartan, telmisartan, valsartan, disopyramide, flecanide, mexiletine, procainamide, propafenone, quinidine, tocamide, amiodarone, dofetilide, ibutilide, adenosine, gemfibrozil, lovastatin, acebutalol, atenolol, bisoprolol, esmolol, metoprolol, nadolol, pindolol, propranolol, sotalol, diltiazem, nifedipine, verapamil, spironolactone, bumetanide, ethacrynic acid, furosemide, torsemide, amiloride, triamterene, or metolazone.

A central nervous system stimulant can be phendimetrazine, methamphetamine, diethylpropion, amphetamine/dextroamphetamine, benzphetamine, phendimetrazine, lisdexamfetamine, diethylpropion, phendimetrazine, dexmethylphenidate, armodafinil, atomexetine, doxapram, amphetamine, brucine, caffeine, dexfenfluramine, dextroamphetamine, ephedrine, fenfluramine, mazindol, methyphenidate, pemoline, phentermine, sibutramine, or modafinil.

An agent for Alzheimer's disease management can be donepezil, galanthamine, rivastigmine, tacrine, or memantine.

An agent for cystic fibrosis management can be an antibiotic (e.g., ciprofloxacin, tobramycin); a bronchodilator (e.g., albuterol or salmeterol); an anticholinergic (e.g., atrovent); a DNase (e.g., pulmozyme); a mucolytic (e.g., acetylcysteine); a saltwater solution (e.g., hypertonic saline); a nonsteroidal anti-inflammatory (NSAID; e.g., ibuprofen); a corticosteroid (e.g., fluticasone or prednisone); an enzyme replacement therapy (e.g., creon or pancreaze); CPX, IBMX, XAC and analogues; 4-phenylbutyric acid; genistein and analogous isoflavones; azithromycin, aztreonam, pancrelipase, gentamicin, ivacaftor, azithromycin, vitamin E, pancreatin, or milrinone.

A diagnostic agent can be adenosine or aminohippuric acid.

A homeopathic cold medication can be *Aconitum napellus, Allium cepa, Antimonium tartaricum, Apsi mellifica, Arsenicum album, Arum triphyllum, Belladonna, Bryonia alba, Dulcamara, Eupatorium perforliatum, Euphrasia, Ferrum phosphoricum, Gelsemium, Hepar sulphuris, Kali bichromicum, Mercurius solubilis, Natrum muriaticum, Nux vomica, Oscillococinum* (*Anas barbariase*)*,* phosphorus, *Rhus toxicodendron,* sulphur, or *Pulsatilla Sticta.*

A COPD drug can be montelukast, budesonide/formoterol, roflumilast, aclidinium, prednisone, isoetharine, dyphylline, guaifenesin/theophylline, or fluticasone/vilanterol. In some cases, a COPD drug is a bronchodilator (e.g., albuterol, levabuterol, ipratropium; or a long-acting bronchodilator (e.g., tiotropium, salmeterol, formoterol, arformoterol, indacaterol, aclidinium). In some cases, a COPD drug is a steroid (e.g., fluticasone, budesonide). In some cases, a COPD drug is a combination (e.g., salmeterol/fluticasone and formoterol/budesonide). In some cases a COPD drug is a phosphodiesterase-4 inhibitor (e.g., roflumilast). In some cases, a COPD drug is theophylline or an antibiotic.

A cough medication can be guaifenesin/hydrocodone, acetaminophen/codeine, diphenhydramine, guaifenesin/potassium guaiacolsulfonate, carbetapentane/guaifenesin, codeine/guaifenesin, dextromethorphan/guaifenesin, guaifenesin, carbinoxamine/dextromethorphan/pseudoephedrine, dextromethorphan, brompheniramine/codeine, carbetapentane/chlorpheniramine/phenylephrine, benzocaine/dextromethorphan, menthol, acetaminophen/dextromethorphan, chlophedianol/guaifenesin, acetaminophen/dextromethrophan/doxylamine, aceteaminophen/hydrocodone, glycerin, acetaminophen/dextromethorphan/phenylephrine, dexbrompheniramine/hydrocodone/phenylephrine, hydromorphone, acetaminophen/chlorpheniramine/dextromethorphan/phenylephrine,guaifenesin, carbetapentane/guaifenesin, carbinoxamine/dextromethorphan/pseudoephredrine, chlorpheniramine/dextromethorphan/methscopolamine, guaifenesin/potassium guaiacolsulfonate, homatropine/hydrocodone, dihdrocodeine/guaifenesin/pseudoephredrine, chlropheniramine/hydrocodone, codeine/guaifenesin, potassium iodide, dihydrocodeine/guaifenesin, dihydrocodeine/hydrocodone, acetaminophen/hydrocodone, chlorcyclizine/codeine/phenylephrine, codeine/pseudoephedrine/pyrilamine, hydromorphone, dihydrocodeine/guaifensesin/pseudoephedrine, chlophedianol/triprolidine, dextromethorphan/promethazine, codeine/promethazine, dextromethorphan/promethazine, carbetapentane/guaifenesin, carbetapentane/guaifenesine, dextromethorphan/guaifenesin, dextromethorphan/doxylamine, carbetapentanse, dyclonine/menthol, dextromethorphan/guaifenesin, benzonatate, acetaminophen/dextromethorphan/phenylephrine, guaifenesin/hydrocodone, carbinoxamine/hydrocodone/pseudoephedrine, codeine/guaifenesin, guaifenesin/hydrocodoen, homatropine/hydrocodone, chlorpheniramine/hydrocodone, carbetapentane/guaifenesin, acetaminophen/dextromethorphan/doxylamine/phenylephrine, acetaminophen/dextromethorphan, acetaminophen/dextromethorphan/phenylephrine, acetaminophen/hydrocodone, dihydrocodein/guaifenesin/pseudoephedrine, or benzonatate. In some cases, the cough medication can be dextromethorphan, codeine, noscapine, bromhexine, acetylcysteine, ephedrine, guaifenesin, honey, cinnaomon, honey/cinnamon, lemon, elderberry syrup, tea, Slippery Elm, peppermint, Chinese Hot Mustard, cayenne pepper (capsaicin), apple cider vinegar, wasabi, horseradish, Echinacea, vitamin c, zinc, ginger (zingiber officinale), vinegar, water, red onion, garlic, hyssop, or mullein. In some cases, a cough medication can be an antihistamine, decongestant, inhaled asthma drug, antibiotic, acid blocker, or cough suppressant. In some cases, a cough medication is licorice, horehound, mullein, peppermint, elderflower, yarrow, *Belladonna, bryonia, Gelsemium, Coccus catcti, Drosera, Dulcamara, Eupatorium, Euphrasia,* hepar suphuratum, *Kali bic, Nux vomica,* phosphorus, *Pulsatilla, Antimonium tartaricu, Rhus tox, Spongia,* or *Vincetoxicum.*

A dietary supplement can be acai, aloe vera, an anabolic steroid, astragalus, vitamin A, bilberry, beta carotene, bitter orange, Black Cohosh, Butterbur, vitamin B12, vitamin B6, calcium, carnitine, cartilage, cat's claw, chamomile, chasteberry, chondroitin, chromium, cinnamon, coenzyme Q10, colloidal silver, cranberry, vitamin C, dandelion, vitamin C, Echinacea, ephedra, essiac/flor-essence, European elder, evening primrose oil, vitamin E, fenugreek, feverfew, fish oil, flaxseed, folate, folic acid, garlic, ginger, ginkgo, ginseng, glucosamine, glucosamine with chondroitin sulfate, goldenseal, grape seed extract, green tea, hawthorn, hoodia, horse chestnut, iodine, iron, kava, vitamin K, lavender, licorice root, L-lysine, magnesium, melatonin, milk thistle, mistletoe, noni, omega-3 fatty acids, PC-SPES, peppermint oil, red clover, sage, S-adenosyl-L-methionine, saw palmetto, selenium, soy, St. John's Wort, tea, thunder god vince, turmeric, valerian, vitamin A, vitamin B1, vitamin B12, vitamin B6, vitamin C, vitamin D, vitamin E, vitamin K, yohimbe, or zinc.

An agent for erectile dysfunction can be tadalafil, sildenafil, vardenafil, alprostadil, avanafil, apomorphine, apomorphine diacetate, phentolamine, and yohimbine.

A gastrointestinal agent can be a 5-aminosalicylate (e.g., mesalamine, balsalazide, sulfasalazine, olsalazine), an antacid (e.g., aluminum hydroxide/magnesium hydroxide/simethicone; sodium barcarbonate, magaldrate/simethicone, calcium carbonate, aluminum hydroxide/magnesium hydroxide/simethicone, magnesium hydroxide, aluminum hydroxide/magnsesium hydroxide, magnesium hydroxide, alginic acid/aluminum hydroxide/magnesium trisilicate, alginic acid/aluminum hydroxide/magnesium carbonate, aluminum hydroxide/magnesium hydroxide/simethicone, calcium carbonate/magnesium hydroxide, magaldrate, magaldrate/simethicon), an antidiarrheal (e.g., bismuth subsalicylate, atropine/difenoxin, attapulgite, lactobacillus acidophilus, loperamide, atropine/diphenoxylate, saccharomyces boulardii lyo, crofelemer systemic, kaolin/pectin systemic, kaolin systemic, lactobacillus acidophilus/lactobacillus bulgaricus, loperamide/simethicone systemic), a digestive enzyme (e.g., pancrelipase, amylase/cellulose/hyoscyamine/lipase/phenyltoloxamine/protease, pancreatin, lactase), a functional bowel disorder agent (e.g., an antichoinergic/antispasmodic, e.g., hyoscyamine, atropine/hyoscyamine/Phenobarbital/scopolamine, methscopolamine, scopolamine, chlordiazepoxide/clidinium, dicyclomine, glycopyrrolate, belladonna, atropine, atropine/hyoscyamin/Phenobarbital/scopolamine, belladonna/ergotamine/phenobarbital, mepenzolate, hyoscyamine/phenyltoloxamine), a chloride channel activator (e.g., lubiprostone), a guanylate cyclase-C agonist (e.g., linaclotide), a peripheral opioid receptor antagonist (e.g., methylnaltrexone, alivmopan); a serotoninergic neuroenteric modulator (e.g., tegaserod, alosetron), a gallstone solubilizing agent (e.g., ursodiol, chenodeoxycholic acid), a gastrointestinal stimulant (e.g., metoclopramide, cisapride, choline bitartrate/dexpanthenol), H. pylori eradication agent (e.g., amoxicillin/clarithromycin/lansoprazole, bismuth subcitrate potassium/metronidazole/tetracycline, bismuth subsalicylate/metronidazole/tetracycline, amoxicillin/clarithromycin/omeprazole), an H2 antagonist (e.g., nizatidine, cimetidine, ranitidine, famotidine, cimetidine, calcium carbonate/famotdine/magnesium hydroxide), a laxative (e.g., magnesium citrate, polyethylene glycol 3350, lactulose, senna, bisacodyl, psyllium, methylcellulose, docusate, polycarbophil, sodium biphophate/sodium phosphate, docusate/senna, sodium biphosphate/sodium phosphate, polyethylene glycol 3350 with electrolytes, bisacodyl/polyethylene glycol 3350/potassium chloride/sodium bicarbonate/sodium chloride, magnesium sulfate, polycarbophil, magnesium hydroxide, mineral oil), citric acid/simethicone/sodium bicarbonate, simethicone, misoprostol, charcoal/simethicone, sucralfate, teduglutide; or a proton pump inhibitor (e.g., pantoprazole, omeprazole/sodium bicarbonate, rebeprazole, esomeprazole, lansoprazole, dexlansoprazole). In some cases, the gastrointestinal agent can be loperamide, atropine, hyoscyamine, famotidine, lansoprazole, omeprazole, or rebeprazole.

A hormone can be estrogen, progesterone, hydrocortisone, fludocortisone, throxine, progestin, testosterone, estradiol, cortisone, 1-androstendediol, 1-androstenedione, bolandiol, bolasterone, boldenone, boldione, calusterone, clostebol, danazol, dehydrochlormethyltestosterone, desoxymethyltestosterone, drostanolone, ethylestrenol, fluoxymesterone, formeboone, furazabol, gestrinone, 4-hydroxytestosterone, mestanolone, mesterolone, meenolone, methandienone, methandriol, methasterone, methyldienolone, methyl-1-testosterone, methylnortestosterone, methyltestosterone, mitribolone, mibolerone, nandrolone, 19-noradrostenedione, norboletone, norclostebol, norethandrolone, oxabolone, oxandrolone, oxymesterone, oxymetholone, prostanozol, quinbolone, stnozolol, stenbolone, 1-testosterone, tetrhydrogestrinone, trenbolone, androstenediol, androstenedionne, dihydrotestosterone, or prasterone.

An agentfor the treatment of alcoholism can be naloxone, naltrexone, acamprostate, or disulfuram.

An agent for the treatment of addiction can be disulfiram, naltrexone, acamprosate, methadone, levo-alph acetyl methadol (LAAM), or buprenorphine.

An immunosupressive can be a glucocorticoid, a cytostatic (e.g., alkyating agent, antimetabolite (e.g., methotrexate, azathiopurine, mercaptopurine, fluorouracil, a cytotoxic antibiotic (e.g., dactinomycin, an antracycline, mitomycin C, bleomycin, mithramycin), an antibody (e.g., a monoclonal antibody (e.g., IL-2 receptor directed antibody, CD3 directed antibody; a T-cell receptor directed antibody (e.g., muromonab-CD3)), a drug acting on immunophilin (e.g., ciclosporin, tacrolimus, sirolimus), other drugs (e.g., an interferon, an opioid, a TNF binding protein, a mycophenolate). In some cases, the immunosuppressive is mycophenolic acid, cyclosporin, azathioprine, tacrolimus, everolimus, or rapamycin. In some cases, the immunosuppressive agent is a calcineurin inhibitor (e.g., cyclosporine, tacrolimus), an interleukin inhibitor (e.g., rilonacept, tocilizumab, anakinra, ustekinumab, canakinumab, basiliximab, daclizumab), omalizumab, lenalidomide, azathioprine, methotrexate, pomalidomide, thalidomide, alefacept, efalizumab, mycophenolic acid, mycophenolate mofetil, fingolimod, natalizumab, belimumab, lefunomide, abatacept, lymphocyte immune globulin anti-thy (equine), teriflunomide, belatacept, muromonab-cd3, eculizumab, anti-thymocyte globulin (rabbit), or a TNF alpha inhibitor (e.g., infliximab, adalimumab, etanercept, certolizumab, golimumab).

A mast cell stabilizer can be cromolyn, pemirolast, or nedocromil.

An agent for migraine headache can be naproxen, ibuprofen, acetaminophen, almotriptan, alperopride, codeine, dihydroergotamine, ergotamine, eletriptan, frovatriptan, isometheptene, lidocaine, lisuride, metoclopramide, naratriptan, oxycodone, propoxyphene, rizatriptan, sumatriptan, tolfenamic acid, zolmitriptan, amitriptyline, atenolol, clonidine, cyproheptadine, diltiazem, doxepin, fluoxetine, lisinopril, methysergide, metoprolol, nadolol, zolmitriptan, nortriptyline, paroxetine, pizotifen, pizotyline, propanolol, protriptyline, sertraline, timolol, ergotamine/caffeine, isometheptine/dichlorphenazone/apap, or verapamil.

An agent that can be used to treat motion sickness can be diphenhydramine, dimehydrinate, cinnaizine, meclozine, promethazine, metoclopramide, prochlorperazine, ginger root, or scopolamine.

An agent for managing multiple sclerosis can be corticotropin, dalfampridine, teriflunomide, interferon beta-1a, interferon beta-1b, glatiramer, cyclophosphamide, dexamethasone, prednisone, fingolimod, azathioprine, natalizumab, bencyclane, methylprednisolone, azathioprine, mitoxantrone, or prednisolone.

A muscle relaxant can be a neuromuscular blocking agent (e.g., succinylcholine, mivacurium, cisatracurium, vecuronium, doxacurium, pancuronium, atracurium); a skeletal muscle relaxant combination (e.g., aspirin/caffeine/orphenadrine, aspirin/carisoprodol, aspirin/carisoprodol/codeine, aspirin/methocarbamol, aspirin/meprobamate); or a skeletal muscle relaxant (e.g., dantrolene, botulinum toxin type b, carisprodol, onabotulinumtoxin A, cyclobenzaprine, chlorzoxazone, chlrophenesin, tizanidine, baclofen, cyclobenzaprine, metaxalone, methocarbamol, cyclobenzaprine, orphenadrine, carisoprodol, incobotulinumtoxinA). In some cases, the muscle relaxant is decamethonium, rapacuronium, atracurium, rocuronium, alcuronium, gallamine, metocurine, pipecuronium, bubocurarine, baclofen, chlorzoxazone, cyclobenzaprine, methocarbamol, orphenadrine, quinine, carisoprodol, gabapentin, metaxalone, diazepam, dantrolene, botulinum toxin type b, onabotulinumtoxinA, chloroxazone, chlorphenesin, baclofen, methocarbamol, or ortizanidine.

A drug for treating mycocardial infarction can be urokinase, perindopril, alteplase, ramipril, aspirin, aluminum hydroxide/aspirin/calcium carbonate/magnesium hydroxide, timolol, magnesium chloride, warfarin, dalteparin, heparin, propranolol, eptifibatide, metoprolol, enoxaparin, trandolapril, nitroglycerin, clopidogrel, lisinopril, reteplase, streptokinase, atenolol, tenecteplase, or moexipril. In some cases, the drug for treating myocardial infarction can be a vasodilator. A vasodilator can be an alphaadrenoceptor antagonist (e.g., prazosin, terazosin, doxazosin, trimazosin, phentolamine, phenoxybenzamine); an angiotensin converting enzyme (ACE) inhibitor (e.g., benazepril, captopril, enalapril, fosinopril, lisinopril, moexipril, quinapril, ramipril); an angiotensin receptor blocker (ARB) (e.g., candesartan, eprosartan, irbesartan, losartan, olmesartan, telmisartan, valsartan); a beta2-adrenoceptor agonist (beta2-agonist) (e.g., epinephrine, norepinephrine, dopamine, dobutamin, isoproterenol); a calcium-channel blocker (CCB) (e.g., amlodipine, felodipine, isradipine, nicardipine, nifedipine, nimodipine, nitrendipine); a centrally acting sympatholytic (e.g., clonidine, guanabenz, guanfacine, alpha-methyldopa); a direct acting vasodilator (e.g., hydralazine); an endothelin receptor antagonist (e.g., bosetan); a ganglionic blocker (e.g., trimethaphan camsylate); a nitrodilator (e.g., isosorbide dinitrate, isosorbide mononitrate, nitroglycerin, erhthrityl tetranitrate, pentaerythritol tetranitrate, sodium nitroprusside); a phosphodiesterase inhibitor (e.g., a PDE3 inhibitor (e.g., milrinone, inamrinone, cliostazol; a PDE5 inhibitor (e.g., sildenafil, tadalafil)); a potassium-channel opener (e.g., minoxidil); or a rennin inhibitor (e.g., aliskiren). In some cases, a drug for treating myocardial infarction can be a cardiac depressant drug (e.g., a beta-adrenoceptor antagonist (beta-blocker), e.g., a non-selective beta1/beta2 drug (e.g., carteolol, carvedilol, labetalol, nadolol, penbutolol, pindolol, propranolol, sotalol, timolol) or a beta1-selective drug (e.g., acebutolol, atenolol, betaxolol, bisoprolol, esmolol, metoprolol, nebivolol); a calcium-channel blocker (e.g., amlodipine, felodipine, isradipine, nicardipine, nifedipine, nimodipine, nitrendipine); or a centrally acting sympatholytic (e.g., clonidine, guanabenz, guanfacine, alpha-methyldopa). In some cases, a drug for treating myocardial infarction can be an antiarrhythmic drug (e.g., Class I -sodium-channel blocker (e.g., Class 1A (e.g., quinidine, procainamide, disopryamide); Class 1B (e.g., lidocaine, tocainide, mexiletine); Class 1C (e.g., flecainide, propafenone, moricizine); Class II-beta blocker (e.g., a non-selective beta1/beta2 drug (e.g., carteolol, carvedilol, labetalol, nadolol, penbutolol, pindolol, propranolol, sotalol, timolol) or a beta1-selective drug (e.g., acebutolol, atenolol, betaxolol, bisoprolol, esmolol, metoprolol, nebivolol); a Class III-potassium channel blocker (e.g., amiodarone, dronedarone, bretylium, sotalol, ibutilide, dofetilide); a Class IV calcium channel blocker (e.g., amlodipine, felodipine, isradipine, nicardipine, nifedipine, nimodipine, nitrendipine); adenosine, an electrolyte supplement (e.g., magnesium, potassium); a digitalis compound (e.g., digoxin, digitoxin, ouabain); or atropine. In some cases, the drug for treating myocardial infarction is a thrombolytic drug (e.g., a tissue plasmiogen activator (e.g., alteplase, retaplase, tenecteplase); streptokinase, anistreplase, or urokinase.

A nonsteroidal anti-inflammatory can be a salicylate (e.g., aspirin (acetylsalicylic acid), diflunisal, salsalate); a propionic acid derivative (e.g., ibuprofen, dexibuprofen, naproxen, fenoprofen, ketoprofen, dexketoprofen, flurbiprofen, oxaprozin, loxoprofen); an acetic acid derivative (e.g., indomethacin, tolmetin, sulindac, etodolac, ketorolac, diclofeanc, nabumetone) a enolic acid (oxicam) derivative (e.g., piroxicam meloxicam, tenoxicam, droxicam, lornoxicam, isoxicam); a fenamic acid derivative (fenamate; e.g., mefenamic acid, meclofenamic acid, flufenamic acid, tolfenamic acid); a selective COX-2 inhibitor (coxib; e.g., celecoxib, rofecoxib, vadexocib, parecoxib, lumiracoxib, etoricoxib, firoxib); a sulphonanilide (e.g., nimesulide); licofelone, lysine clonixinate, hyperforin, figwort, calcitriol (vitamin D). In some cases, a nonsteroidal anti-inflammatory can be aceclofenac, alminoprofen, amfenac, aminopropylon, amixetrine, aspirin, benoxaprofen, bromfenac, bufexamac, carprofen, celecoxib, choline salicylate, cinchophen, cinmetacin, clopriac, clometacin, diclofenac, diclofenac potassium, diclofenac sodium, diclofenac sodium with misoprostol, diflunisal, etodolac, fenoprofen, fenoprofen calcium, flurbiprofen, ibuprofen, indomethacin, indoprofen, ketoprofen, ketorolac, magnesium salicylate, mazipredone, meclofenamate, meclofenamate sodium, mefanamic acid, meloxicam, nabumetone, naproxen, naproxen sodium, oxaprozin, parecoxib, piroxicam, pirprofen, rofecoxib, salsalate, sodium salicylate, sulindac, tolfenamate, tolmetin, tolmetin sodium,or valdecoxib.

An opioid, opioid antagonist, or inverse agonist can be an opium alkaloid (e.g., codeine, morphine, oripavine, pseudomorphine, thebaine); an alkaloid salt mixture (e.g., pantopon, papaveretum); 14-hydroxymorphine, 2,4-dinitorphenylmorphe, 6-methyldihydromorphine, 6-methylenedihydrodesoxymorphine, acetyldihydromorphine, azidomorphine, chlornaltrexamine, chloroxymorphamine, desomorphine, dihydromorphine, ehtyldihydromorphine, hydromorphinol, methyldesorphine, N-henethylnormorphine, RAM-378, 6-nicotinoyldihydromorphine, acetlypropionylmorphin, diacetyldihydromorphine, dibutyrylmorphine, dibenzoylmorphine, diformylmorphine, dipropanoylmorphine, heroin, nicomorphine, 6-Monoacetylcodeine, Benzylmorphine, Codeine methylbromide, Desocodeine, Dimethylmorphine (6-O-Methylcodeine), Ethyldihydromorphine, Methyldihydromorphine (dihydroheterocodeine), Ethylmorphine (dionine), Heterocodeine, Isocodeine, Pholcodine (morpholinylethylmorphine), Myrophine, Nalodeine (N-allyl-norcodeine), Transisocodeine, 14-Cinnamoyloxycodeinone, 14-Ethoxymetopon, 14-Methoxymetopon, 14-Phenylpropoxymetopon, 7-Spiroindanyloxymorphone, 8,14-Dihydroxydihydromorphinone, Acetylcodone, Acetylmorphone, α-hydrocodol (Dihydrocodeine), Bromoisopropropyldihydromorphinone, Codeinone, Codorphone, Codol ( Codeine Phosphate), Codoxime, IBNtxA, Thebacon (acetyldihydrocodeinone, dihydrocodeinone enol acetate), Hydrocodone, Hydromorphone, Hydroxycodeine, Metopon (Methyldihydromorphinone), Morphenol Morphinone, Morphol, N-Phenethyl-14-ethoxymetopon, Oxycodone, Oxymorphol, Oxymorphone, Pentamorphone, Semorphone, α-chlorocodide (Chlorocodide), β-chlorocodide, α-chloromorphide ( Chloromorphide), Bromocodide, Bromomorphide, Chlorodihydrocodide, Chloromorphide, Codide, 14-Hydroxydihydrocodeine, Acetyldihydrocodeine, Dihydrocodeine, Dihydrodesoxycodeine (desocodeine), Dihydroisocodeine, Nicocodeine, Nicodicodeine, 1-Nitrocodeine cas, Codeine-N-oxide, Morphine-N-oxide, Oxymorphazone, 1-Bromocodeine, 1-Chlorocodeine, 1-Iodomorphine, Codeine-N-oxide (genocodeine), Heroin-7,8-oxide, Morphine-6-glucuronide, 6-Monoacetylmorphine, Morphine-N-oxide (genomorphine), Naltrexol, Norcodeine, Normorphine, Levomethorphan, 4-chlorophenylpyridomorphinan, Cyclorphan, Dextrallorphan, Levargorphan, Levorphanol, Levophenacylmorphan, Levomethorphan, Norlevorphanol, N-Methylmorphinan, Oxilorphan, Phenomorphan, Methorphan (racemethorphan), Morphanol (racemorphanol), Ro4-1539, Stephodeline, Xorphanol, 1-Nitroaknadinine, 14-episinomenine, 5,6-Dihydronorsalutaridine, 6-Keto Nalbuphine, Aknadinine, Butorphanol, Cephakicine, Cephasamine, Cyprodime, Drotebanol, Fenfangjine G, Nalbuphine, Sinococuline, Sinomenine (cocculine), Tannagine, 5,9 alpha-diethyl-2-hydroxybenzomorphan (5,9-DEHB), 8-Carboxamidocyclazocine (8-CAC), Alazocine, Anazocine, Bremazocine, Butinazocine, Carbazocine, Cogazocine, Cyclazocine, Dezocine, Eptazocine, Etazocine, Ethylketocyclazocine, Fedotozine, Fluorophen, Gemazocine, Ibazocine, Ketazocine, Metazocine, Moxazocine, Pentazocine, Phenazocine, Quadazocine, Thiazocine, Tonazocine, Volazocine, Zenazocine, Pethidine, 4-Fluoromeperidine, Allylnorpethidine, Anileridine, Benzethidine, Carperidine, Difenoxin, Diphenoxylate, Etoxeridine (carbetidine), Furethidine, Hydroxypethidine (bemidone), Morpheridine, Meperidine-N-oxide, Oxpheneridine (carbamethidine), Pethidine (meperidine), Pethidine intermediate A, Pethidine intermediate B (norpethidine), Pethidine intermediate C (pethidinic acid), Pheneridine, Phenoperidine, Piminodine, Properidine (ipropethidine), Sameridine, Allylprodine, (α/β)-Meprodine, Desmethylprodine (MPPP), PEPAP, (α/β)-Prodine, Prosidol, Trimeperidine (promedol), Acetoxyketobemidone, Droxypropine, Ketobemidone, Methylketobemidone, Propylketobemidone, Alvimopan, Loperamide, Picenadol, Methadone, Dextromethadone, Dipipanone, Isomethadone, Levoisomethadone, Levomethadone, Methadone, Methadone intermediate, Normethadone, Norpipanone, Phenadoxone (heptazone), Pipidone (Dipipanone Hydrochloride) (6-piperidine-4,4-diphenyl-5-methyl-hexanone-3 hydrochloride), Alphaacetylmethadol, Dimepheptanol (racemethadol), Levacetylmethadol, Noracetylmethadol, Desmethylmoramide, Dextromoramide, Levomoramide, Moramide intermediate, Racemoramide, Diethylthiambutene, Dimethylthiambutene, Ethylmethylthiambutene, Piperidylthiambutene, Pyrrolidinylthiambutene, Thiambutene, Tipepidine, Dextropropoxyphene (propoxyphene), Dimenoxadol, Dioxaphetyl butyrate, Levopropoxyphene, Norpropoxyphene, Diampromide, Phenampromide, Propiram, IC-26, Isoaminile, Lefetamine, R-4066, Fentanyl, 3-Allylfentanyl, 3-Methylfentanyl, 3-Methylthiofentanyl, 4-Phenylfentanyl, Alfentanil, Alphamethylacetylfentanyl, Alphamethylfentanyl, Alphamethylthiofentanyl, Benzylfentanyl, Betahydroxyfentanyl, Betahydroxythiofentanyl, Betamethylfentanyl, Brifentanil, Carfentanil, Fentanyl, Lofentanil, Mirfentanil, Ocfentanil, Ohmefentanil, Parafluorofentanyl, Phenaridine, Remifentanil, Sufentanil, Thenylfentanyl, Thiofentanyl, Trefentanil, Thienorphine, 7-PET, Acetorphine, Alletorphine (N-allyl-noretorphine), BU-48, Buprenorphine, Cyprenorphine, Dihydroetorphine, Etorphine, Homprenorphine, 18,19-Dehydrobuprenorphine (HS-599), N-cyclopropylmethylnoretorphine, Nepenthone, Norbuprenorphine, Thevinone, Thienorphine, Ethoheptazine, Meptazinol, Metheptazine, Metethoheptazine, Proheptazine, Bezitramide, Piritramide, Clonitazene, Etonitazene, Nitazene, 18-Methoxycoronaridine, 7-Acetoxymitragynine, 7-Hydroxymitragynine, Akuammidine, Akuammine, Eseroline, Hodgkinsine, Mitragynine, Pericine, Pseudoakuammigine, BW373U86, DPI-221, DPI-287, DPI-3290, SNC-80, β-neo-endorphin, dynorphin, Big dynorphin, Dynorphin A, Dynorphin B, Endorphin, Beta-endorphin, Alpha-endorphin, Gamma-endorphin, α-neo-endorphin, β-neo-endorphin, Enkephalin, DADLE • DAMGO • Dermenkephalin, Met-enkephalin, Leuenkephalin, Adrenorphin, Amidorphin, Casomorphin, DALDA (Tyr-D-Arg-Phe-Lys-NH2), Deltorphin, Dermorphin, DPDPE, Endomorphin, Gliadorphin, Morphiceptin, Nociceptin, Octreotide, Opiorphin, Rubiscolin, TRIMU 5, 3-(3-Methoxyphenyl)-3-ethoxycarbonyltropane, AD-1211, AH-7921, Azaprocin, BDPC, Bisnortilidine, BRL-52537, Bromadoline, C-8813, Ciramadol, Doxpicomine, Enadoline, Faxeladol, GR-89696, Herkinorin, ICI-199,441, ICI-204,448, J-113,397, JTC-801, Ketamine, KNT-42, LPK-26, Methopholine, MT-45, Desmethylclozapine, NNC 63-0532, Nortilidine, O-Desmethyltramadol, Phenadone, Phencyclidine, Prodilidine, Profadol, Ro64-6198, Salvinorin A, SB-612,111, SC-17599, RWJ-394,674, TAN-67, Tapentadol, Tecodine (Oxycodone), Tifluadom, Tilidine, Tramadol, Trimebutine, U-50,488, U-69,593, Viminol, 1-(4-Nitrophenylethyl)piperidylidene-2-(4-chlorophenyl)sulfonamide (W-18), 5'-Guanidinonaltrindole, β-Funaitrexamine, 6β-Naltrexol, Alvimopan, Binaltorphimine, Chlornaltrexamine, Clocinnamox, Cyclazocine, Cyprodime, Diacetylnalorphine, Difenamizole, Diprenorphine (M5050), Fedotozine, JDTic, Levallorphan, Methocinnamox, Methylnaltrexone, Nalfurafine, Nalmefene, Nalmexone, Naloxazone, Naloxonazine, Naloxone, Naloxone benzoylhydrazone, Nalorphine, Naltrexone, Naltriben, Naltrindole, Norbinaltorphimine, Oxilorphan, S-allyl-3-hydroxy-17-thioniamorphinan (SAHTM), Alimadol, Anilopam+HCl, Asimadoline, FE 200665, Fedotozine, MCOPPB, Nalfurafine, Nalorphine, Nalorphine dinicotinate,or SoRI-9409 In some cases, the opioid can be alfentanil, allylprodine, alphaprodine, anileridine, benzylmorphine, bezitramide, buprenorphine, butorphanol, carbiphene, cipramadol, clonitazene, codeine, dextromoramide, dextropropoxyphene, diamorphine, dihydrocodeine, diphenoxylate, dipipanone, fentanyl, hydromorphone, L-alpha acetyl methadol, lofentanil, levorphanol, meperidine, methadone, meptazinol, metopon, morphine, nalbuphine, nalorphine, oxycodone, papaveretum, pethidine, pentazocine, phenazocine, oxymophone, remifentanil, sufentanil, or tramadol.

An analgesic can be merperidine, hydromorphone, fentanyl, codeine, methadone, morphine, oxycodone, oxycodone and ASA, oxycodone and acetaminophen, pentazocine, acetaminophen/caffeine/codeine, acetaminophen/codeine, acetaminophen, acetylsalicylic acid, ibuprofen, naproxen sodium, naproxen, indomethacin, diclofenac, mefenamic acid, ketorolac, celecoxib, erotamin, sumatriptan, butorphanol, zolmitriptan, naratriptan, rizatriptan, almotriptan, apazone, benzpiperylon, benzydramine, caffeine, clonixin, ethoheptazine, flupirtine, nefopam, orphenadrine, propacetamol, or propoxyphene.

An opthalmic preparation can be an anti-angiogenic ophthalmic agent (e.g., aflibercept, ranibizumab, pegaptanib); cysteamine, ocriplasmin, mitomycin, dapiprazole; a mydriatic (e.g., cyclpentolate, phenylephrine, atropine, cyclopentolate/phenylephrine, homatropine, scopolamine, phenylephrine/scopolamine, tropicamide, hydroxyamphetamine/tropicamide, tropicamide); an ophthalmic anesthetic (e.g, lidocaine, proparacaine, tetracaine); ophthalmic anti-infectives (e.g., levofloxacin, natamycin, bactiracin/neomycin/polymyxin b, bactiracin/polymyxin b, tobramycin, moxifloxacin, ciprofloxacin, gatifloxacin, azithromycin, idoxuridine, besifloxacin, norfloxacin, chloramphenicol, bacitracin/polymyxin b, sulfacetamide sodium, chloramphenicaol, boric acid, erythromycin, sulfisoxazole, gentamin, gramicidin/neomycin/polymyxin b, bacitracin, ofloxacin, polymyxin b/trimethoprim, levofloxacin, sulfacetamide sodium, oxytetracycline/polymyxin b, tobramycin, vidarabine, trifluridine, ganciclovir, gatifloxacin); an ophthalmic anti-inflammtory agent (e.g., bromfenac, nepafenac, ketorolac, cyclosporine, fluriprofen, suprofen, diclofenac, bromfenac); ophthalmic antihistamine and decongestant (e.g., ketotifen, nedocromil, azelastine, epinastine, olopatadine, naphazoline/pheniramine, olopatadine, alcaftadine, cromolyn, bepotastine, pemirolast, tetrhydrozolien, tetrahydrozoline/zinc sulfate, iodoxamide, naphazoline, phenylephrine, tetrhydrozoline, naphazoline/zinc sulfate, emedastine, naphazoline/pheniramine, levocabastine); an ophthalmic glaucoma agent (e.g., travoprost, dorzolamide/timolol, bimatoprost, latanoprost, brimonidine, brimonidine/timolol, timolol, levobunolol, brinzolamide, levobetaxolol, carbachol, dorzolamide/timolo, epinephrine/pilocarpine, epinephrine, demecarium bromide, apraclonidine, pilocarpine, acetylcholine, metipranolol, echothiophate iodide, dipivefrin, unoprostone, dorzolamide, tafluprost); an ophthalmic steroid (e.g., dexamethasone, fluocinolone, loteprednol, difluprednate, fluorometholone, loteprednol, prednisolone, medrysone, triamcinolone, rimexolone); an opthlamic steroid with an anti-infective (e.g., fluorometholone/sulfacetamide sodium, dexamethasone/meomycin, dexamethasone/tobramycin, dexamethasone/neomycin/polymyxin b), or orbetaxolol.

An osteoporosis preparation can be alendronate, ibandronate, calcium carbonate, calcium/vitamin D, estradiol, teriparatide, hydrochlorothiazide, calcitonin, conjugated estrogens, conjugated estrogens/medroxyprogesterone, denosumab, zoledronic acid, ibandronate, calcium glubionate, dihydrotachysterol, etidronate, esterified estrogens, raloxifene, alendronate/cholecalciferol, calcium phosphate tribasic, conjugated estrogens/medroxyprogesterone, calcium lactate, estropitate, risedronate or raloxifene.

A pain medication can be ibuprofen, hydroxyzine, celecoxib, meperidien, hydromorphone, amitriptyline, acetaminophen/hydrocodone, acetaminophen/codeine, tapentadol, acetaminophen/diphenhydramine, oxymorphone, oxycodone, acetaminophen, ketorolac, tramadol, diclonfenac, diphenhydramine/ibuprofen, naproxen, acetaminophen/phenyltoloxamine, aspirin/hydrocodone, acetaminophen/pheyltoloxamine, aspirin/caffeine, lidocaine, flurbiprofen, fentanyl, ketoprofen, aluminum hydroxide/aspirin/calcium carbonate/magnesium, trolamine salicylate, morphine, nortriptyline, capsaicin, aspirin/hydrocodone, magnesium salicylate, aspirin/caffeine/salicylamide, benzocain, camphor/menthol, valdecoxib, buprenorphine, aspirin/butalbital/caffeine, acetaminophen/caffeine/phenyltoloxamine/salicylamide, acetaminophen/codeine, clonidine, celecoxib, benzocaine/dextromethorphan, benzocaine, cholline salicylcate/magnesium salicylate, acetaminophen/dextromethorphan/doxylamine, sulindac, methol, ibuprofen/oxycodone, acetaminophen/gauifenesin, acetaminophen/diphenhydramine, pramoxine, aspirin/hydrocodone, acetaminophen/propoxyphene, propoxylphene, aspirin/caffeine/propoxyphene, oxycodone, meperidine, morphine liposomal, diphenhydramine, hydromorphone, diphenhydramine/magnesium salicylate, diflunisal, methadone, capsaicin, acetaminophen/phenyltoloxamine/salicylamide, acetaminophen/caffeine/magnesium, morphine/naltrexone, aspirin/codeine, acetaminophen/oxycodone, aspirin/meprobamate, acetaminophen/aspirin, acetaminophen/caffeine, bupivacaine liposome, aspirin/butalbital/caffeine, piroxicam, pentafluoropropane/tetrafluoroethane, indomethacin, acetaminophen/aspirin/caffeine/salicylamide, levorphanol, etodolac, meclofenamate, meperidine/promethazine, fenoprofen, nalbuphine, tapentadol, oxymorphone, acetaminophen/caffeine/dihydrocodeine, aspirin/caffeine/propxoyphene, menthol, mefenamic acid, propoxyphene, pramoxine, ziconotide, butorphanol, acetaminophen/pentazocine, pentazocine, naloxone/pentazocine, imipramine, tolmetin, acetaminophen/tramadol, acetaminophen/dextromethorphan, choline salicylate/magnesium salicylate, hydrocodone/ibuprofen, rofecoxib, or diclofenac. A pain medication can be a nonsteroidal anti-inflammatory drug (NSAID), a corticosteroid, an opoid, a muscle relaxant, an anti-anxiety drug, an antidepressant, or an anticonvulsant.

An anti-anxiety or panic disorder medication can be alprazolam, clomipramin, lorazepma, nortriptyline, buspirone, venlafaxine, clonazepam, lorazepam, maprotiline, paroxetine, fluoxetine, nefazodone, imipramine, sertraline; a serotonin and norepinephrine reuptake inhibitor (e.g., venlafaxine hydrochloride); a benzodiazepine (e.g., alprazolam, clonazepam, or lorazepam); or a selective serotonin reuptake inhibitor (SSRI; e.g., fluoxetine, paroxetine, or sertraline). A panic disorder medication can be a tricyclic antidepressant (TCA; imipramine hydrochloride, desipramine, clomipramine) or a monoamine oxidase inhibitor (MAOI; e.g., isocaroxazid, phenelzine, tranylcypromine). An anti-anxiety or panic medication can be lemon balm (*Melissa officinalis*), ibergoast (caraway, chamomile, licorice, milk thistle, and peppermint), hops (*Humulus lupulus*), lemon juice, ground giner, honey, catnip, chamomile (*Matricaria recutita*), fennel, L-theanine, Kava Kava, Motherwort, Passionflower, Skullcap (*Scutellaria lateriflora*), omega-3, Valerian (*Valeriana officinalis*), lavender (*Lavandula hybrida*), St. John's Wort, magnesium vitamin B12, vitamin B1, *Aconitum napellus, Argentum nitricum, Arsesnicum album, Gelsemium sempervirens, Natrum muriaticum, Calcarea carbonica, Ignatia amara, Kali arsenicosum, Kali phosphoricum, Lycopodium clavatum, Natrum muriaticum,* phosphorus, *Pulsatilla, Silicea* (Silica), Aconite (*Aconitum napellus*), *Ignatia amara,* Mercurius solubilis, phosphorus, sulphur, borax, Bryonia, Casticum, Anacardium, or Valerian Root.

A prostaglandin can be epoprostanol, dinoprostone, misoprostol, or alprostadil.

A respiratory agent can be an antiasthmatic combination (e.g., dyphylline/guaifensin, guaifenesin/theophylline), an antihistamine (e.g., fexofenadine, loratadine, phenindamine, dexchlorpheniramine, terfenadine, triprolidine, promethazine, brompheniramine, chlorpheniramine, cetirizine, diphenhydramine, carbinoxamine, diphenhydramine, chlorpheniramine, cyproheptadine, levocetirizine, desloratadine, clemastine, astemizole, tripelennamine, carboxamine, pheniramine/phenyltoloxamine/pyrilamine); an antitussive (e.g., carbetapentane, benzonatate, dextromethorphan); a bronchodilator (e.g., an adrenergic bronchodilator (e.g., epinephrine, isoproterenol, salmeterol, levalbuterol, arformoterol, metaproterenol, terbutaline, pirbuterol, albuterol, formoterol, indacaterol, racepinephrine, isoetharine, isoproterenol, bitolterol); an anticholinergic bronchodilator (e.g., ipratropium, aclidinium, tiotropium, ipratropium); a bronchodilator combination (e.g., fluticasone/salmeterol, albuterol/ipratropium, budesonide/formoterol, formoterol/mometasone, isoproterenol/phenylephrine); a methylxanthine (e.g., theophylline, oxtriphylline, dyphylline, aminophylline)); a decongestant (e.g., pseudoephedrine, phenylephrine, phenylpropanolamine, pseudophedrine); an expectorant (e.g., guaifenesin, potassium iodide, carbocysteine, or potassium guaiacolsulfonate); a leukotriene modifier (e.g., zafirlukast, monteukast, zileuton); a lung surfactant (e.g., poractant, calfactant, lucinactant, beractant); alpha 1-proteinase inhibitor, dornase alpha, sodium chloride, nitric oxide); an inhaled anti-infective (e.g., tobramycin, ribavirin, zanamivir, pentamidine); an inhaled corticosteroid (e.g., flunisolide, budesonide, fluticasone, beclomethasone, mometasone, ciclesonide); a mast cell stabilizer (e.g., cromolyn, nedocromil); a mucolytic (e.g., acetylcysteine); a selective phosphodiesterase-4 inhibitor (e.g., roflumilast); loratadine/pseudoephedrine, acetaminophen/chlorpheniramine/pseudoephedrine, chlorpheniramine/phenylephrine, acetaminophen/diphenhydramine/phenylephrine, brompheniramine/pseudoephedrine, codeine/guaifenesin, chlorpheniramine/dextromethorphan/phenylephrine, dextromethorphan/phenylephrine/pyrilamine, acetaminophen/chlorpheniramide/pheylephrine, guaifenesin/pseudoesphedrine, chlorpheniramine/phenylpropanolamine, carbetapentane/pseudoephedrine/pyrilamine, acetaminophen/chlorpheniramine/codeine, chlorpheniramine/dextromethorphan/pseudoephedrine, chlorcyclizine/phenylephrine, chlorpheniramine/pseudoephedrine, or chlorpheniramine/phenylpropanolamine. In some cases, the respiratory agent is albuterol, ephedrine, epinephrine, fomoterol, metaproterenol, terbutaline, budesonide, ciclesonide, dexamethasone, flunisolide, fluticasone propionate, triamcinolone acetonide, ipratropium bromide, pseudoephedrine, theophylline, montelukast, zafirlukast, ambrisentan, bosentan, enrasentan, sitaxsentan, tezosentan, iloprost, treprostinil, or pirfenidone.

A sedative or hypnotic can be a barbiturate (e.g., amobarbital, pentobarbital, secobarbital, phenobarbitol); a benzodiazepine (e.g., clonazepam, diazepam, estrazolam, flunitrazepam, lorazepam, midazolam, nitrazepam, oxazepma, trazolam, temazepma, chlordiazepoxide, alprazolam,); an herbal sedative (e.g., ashwagandha, Duboisia hopwoodii, Prosanthera striatiflora, catnip, kava, mandrake, valerian, marijuana); a non-benzodiazpein "z-drug" sedative (e.g., eszopiclone, zaleplon, zolpidem, zopiclone); an antihistamine (e.g., diphenhydramine, dimenhydrinate, doxylamine, pheneragn, promethazine), chloral hydrate, or alcohol. In some cases, the sedative or hypnotic is butalbital, chlordiazepoxide, diazepam, estazolam, flunitrazepam, flurazepam, lorazepam, midazolam, temazepam, triazolam, zaleplon, zolpidem, zolpidem tartrate, butisol sodim, pentobarbital or zopiclone.

A skin or mucous membrane agent can be an antibiotic (e.g., bacitracin, bacitracin zinc/polymyxin B sulfate; clindamycin phosphate, erythromycin/tretinoin, fusidate sodium, fusidic acid; gramicidin/polymyxin B sulfate; mupirocin; polymyxin B sulfate/bacitracin); an antiviral (e.g., acyclovir, idoxuridine); an antifungal (e.g., clotrimazole, ketoconazole, miconazole nitrate, nystatin, terbinafine HCI, terconazole, tolnaftate); a scabicide or pediculicide (e.g., crotamiton, isopropyl myristate, lindane, permethrin; piperonyl butoxide/pyrethrins); benzoyl peroxide, chlorheidine acetate, chorhexidine gluconate, hydrogen peroxide, metronidazole; metronidazole/avobenzone/octinoxate, metronidazole/nystatin, povidone-iodine, selenium sulfide, silver sulfadiazine, triclosan; an anti-inflammatory agent (e.g., amcinonide, beclomethasone dipropionate, betamethaseon dipropionate in propylene glycol, betamethasone dipropionate/clotrimazole, betamethasone dipropionate/salicyclic acid, betamethasone valerate, budesonide, clobetasol propionate, clobetasone butyrate, desonide, desoximetasone, diflucortolone valerate, diflucortolone valerate/salicyclic acid, fluocinolone acetonide, fluocinonide, fluticasone propionate, halobetasol propionate, hydrocortisone, hydrocortisone acetate, hydrocortisone acetate/zince sulfate, hydrocortisone acetate/zinc sulfate/prmoxine HCI, hydrocortisone valerate, hydrocortisone/dibucaine HCI/esculin/framycetin sulfate; hydrocortisone/urea, mometasone furoate, triamcinolone acetonide); an anipruritic or local anesthetic (e.g., lidocaine HCI, lidocaine/prilocaine); a cell stimulate or proliferant (e.g., tretinoin); a basic ointment or protectant (e.g., dimethicone, petrolatum, zinc oxide); a keratolytic agent (e.g., adapalene, canthadridin/podphyllin/salicyclic acid, dithranol, formaldehyde/lactic acid/salicyclic acid, latic acid/salicyclic acid, podofilox, podophyllin, salicyclic acid); a keratoplastic agent (e.g., coal tar, coal tar/juniper tar/pine tar; coal tar/juniper tar/pine tar/zinc pyrithione, coal tar/salicylic acid, coal tar/salicyclic acid/sulfur); a pigmenting agent (e.g., methoxsalen); acitretin, azelaic acid, calcipotriol, capsaicin, collagenase, fluorouracil, iostretinoin, pimecrolimus, tacrolimus, tazarotene, or vitamin E. In some cases, a skin or mucous membrane agent is isotretinoin, bergapten or methoxsalen.

A Tourette's syndrome agent can be pimozide, topiramate, olanzapine, clonidine, guanfacine, haloperidol, botulinum toxin type A, methylphenidate, dextroamphetamine, or pergolide.

A urinary tract agent can be lactobacillus acidophilus, amoxicillin, cefazolin, amoxicillin/clavulante, sulfamethoxazole/trimethoprim, cefuroxime, ciprofloxacin, ertapenem, levofloxacin, nitrofurantoin, ceftriaxone, cefixime, ampicillin/sulbactam, doxycycline, piperacillin/tazobactam, hyoscyamine/methenamine/methylene blue/phenyl salicylate, doripenem, cefadroxil, acetohydroxamic acid, nitrofurantoin, methenamine, lomefloxacin, cefepime, cefoxitin, tolteridine, darifenicin, propantheline bromide, or oxybutynin.

A vertigo agent can be promethazine, diphenidol, betahistine or meclizine.

An insomnia medication can be 5-hydroxytryptophan, diphenhydramine/ibuprofen, zolpidem, lorazepam, flurazepam, amitriptyline, triazolam, eszopiclone, estazolam, temezepam, ramelteon, doxepin, doxylamine, zaleplon, acetaminophen/diphenhydramine, diphenhydramine, 5-hydroxytryptophan, tryptophan, chloral hydrate, diphenhydramine/magnesium salicylate, quazepam, eszopiclone, secobarbital, doxepin, olanzapine, clonazepam, quazepam, lorazepam, alprazolam, oxazepam, prazepam, flunitrazepam, melatonin, valerian root, chamomile tea, lemon balm, or 5-L-5-hydroxytryptophan.

A weight loss drug can be megestrol, phentermine/topirmate, phentermine, phenylpropanolamine, lorcaserin, oxandrolone, megestrol, mazindol, orlistat, sibutramine, rimonabant, metformin, exenatide, pramlintide, conjugated linoleic acid, green tea extract, khat, lipoic acid, ECA stack, or raspberry ketone.

An herb, supplement, or vitamin can be aloe, arginine, beta-carotene, black cohosh, chocolate, chondriotin sulfate, coca, coenzyme Q10, cranberry, creatine, DHEA (dehydroepiandrosterone), dong quai, Echinacea, ephedra, evening primrose oil, flaxseed, flaxseed oil, folate, ginkgo, glucosamine, honey, *Lactobacillus acidophilus,* lycopene, marijuana, melatonin, milk thistle, niacin, omega-3 fatty acid, fish oil, alphalinolenic acid, red yeast rice, SAMe (adenosylmethionine), saw palmetto, soy, St. John's wort, tea tree oil, thiamin, vitamin A, vitamin B12, vitamin B6, vitamin C, vitamin D, vitamin E, whey protein, or zinc. An herb can be a medicinal herb. A medicinal herb can be absinthe wormwood (*Artemisia absinthium*), agrimony (*Agrimonia eupatoria*), aloe vera (*Aloe barbadensis Miller*), alpine rose (*Rhododendron ferrugineum*), angelica (*Angelica silvestris*), anise (*Pimpinella anisum*), arnica (*Arnica montana*), ash (*Fraxinus excelsior*), asparagus (*Asparagus officinalis*), barberry (*Berberis vulgaris*), barley (*Hordeum sativum*), basil (*Ocimum basilicum*), bean (*Phaseolus vulgaris*), bearberry (*Arctostaphylos uva ursi*), beet (*Beta vulgaris*), betony (*Betonica officinalis*), bilberry (*Vaccinium myrtillus*), birch (*Betula pendula*), birdweed (*Polygonum aviculare*), bistort (*Polygonum bistorta*), bitter dock (*Rumex obtusifolis*), bitter root (*Gentiana lutea*), bitterwort (*Gentiana lutea* ), blackberry (*Rubus fruticosus*), black chokeberry (*Aronia melanocarpa*), black currant (*Ribes nigrum*), black locust (*Robinia pseudoacacia*), blackthorn (*Prunus spinosa*), blue gum tree (*Eucalyptus globulus*), borage (*Borago officinalis*), broadleaf dock (*Rumex obtusifolis* ), broadleaved dock (*Rumex obtusifolis*), broccoli (*Brassica oleracea var. botrytis*), burdock (*Arctium lappa*), burnet saxifrage (*Pimpinella saxifraga*), butcher's broom (*Ruscus aculeatus*), calamus (*Acorus calamus*), calendula (*Calendula officinalis*), cannabis (*Cannabis sativa*), caraway (*Carum carvi*), carline thistle (*Carlina acaulis*), carrot (*Daucus carota*), cat's claw (*Uncaria tomentosa*), celery (*Apium graveolens*), centaury (*Centaurium umbellatum*), chamomile (*Matricaria chamomilla*), chasteberry (*Vitex agnus-castus*), chickory (*Cichorium intybus*), christ's thorn (*Paliurus spina-christi*), church steples (*Agrimonia eupatoria*), cinnamon (*Cinnamomum zeylandicum*), cinquefoil (*Potentilla reptans*), cleavers (*Galiuma aparine*), clove (*Syzygium aromaticum*), clubmoss (*Lycopodium clavatum*), coltsfoot (*Tussilago farfara*), comfrey (*Symphytum officinale*), common ivy (*Hedera helix*), common polypody (*Polypodium vulgare*), coriander (*Coriandrum sativum*), corn (corn silk ) (*Zea mays*), couch grass (*Agropyron repens*), cowslip (*Primula veris* ), cranberry (*Vaccinium oxycoccos*), cranesbill (*Geranium macrorrhizum*), creeping cinquefoil (*Potentilla reptans*), creeping thyme (*Thymus serpyllum*), cross gentian (*Gentiana cruciata*), daisy (*Bellis perennis*), dandelion (*Taraxacum officinale*), dill (*Anethum graveolens*), dog rose (*Rosa canina*), dogwood (Cornus *mas*), dwarf everlast (*Helichrysum arenarium*), echinacea (*Echinacea angustifolia*), elder (*Sambucus nigra*), elderberry (*Sambucus nigra*), elecampane (*Inula helenium*), european cornel (*Comus mas*), european wild ginger (*Asarum europaeum*), evening primrose (*Oenothera biennis*), evening star (*Oenothera biennis*), everlasting flower (*Helichrysum arenarium*), eyebright (*Euphrasia officinalis*), fennel (*Foeniculum vulgare*), fenugreek (*Trigonella foenum-graecum*), fig (*Ficus carica*), flax (*Linum usitatissimum*), garden nasturtium (*Tropaeolum majus*), garlic (*Allium sativum*), garland thorn (*Paliurus spina-christi*), ginger (*Zingiber officinalis*), ginkgo (*Ginkgo biloba*), ginseng (*Araliaceae>Panax*), glossy buckthorn (*Rhamnus frangula*), goat willow (*Salix caprea*), goosegrass (*Galiuma aparine*), goldenrod (*Solidago virgaurea*), gotu kola (*Centella asiatica*), grape vine (*Vitis vinifera*), greater celandine (*Chelidonium majus*), great sallow (*Salix caprea*), great yellow gentian (*Gentiana lutea*), green tea (*Camellia sinensis*), green-winged orchid (*Orchis morio*), ground ivy (*Glechoma hederacea*), gypsyweed (*Veronica officinalis*), haselwort (*Asarum europaeum*), hawthorn (*Crataegus laevigata*), heartsease (*Viola tricolor*), hibiscus (*Hibiscus*), hops (*Humulus lupulus*), horehound (*Marrubium vulgare*), horse chestnut (*Aesculus hippocastanum*), horse-heal (*Inula helenium*), horsetail (*Equisetum arvense*), houseleek (*Sempervivum tectorum*), hyssop (*Hyssopus officinalis*), iceland moss (*Cetraria islandica*), indian cress (*Tropaeolum majus*), ivy (*Hedera helix*), johnny jump up (*Viola tricolor*), juniper (*Juniperus communis*), kidney vetch (*Anthyllis vulneraria*), knotgrass (*Polygonum aviculare*), lady's bedstraw (*Galium verum*), lady's mantle (*Alchemilla vulgaris*), larch (*Larix europaea*), large-leaved lime (*Tilia platyphyllos*), large-leaved linden (*Tilia platyphyllos*), lavender (*Lavandula angustifolia*), lemon balm (*Melissa officinalis*), lemon, citron (*Citrus medica*), lily of the walley (*Convallaria majalis*), linseed (*Linum usitatissimum*), liquorice (*Glycyrrhiza glabra*), loosestrife (*Lythrum salicaria*), lovage (*Levisticum officinale*), lungwort (*Pulmonaria officinalis*), mallow (*Malva silvestris*), marigold (*Calendula officinalis*), marjoram (*Majorana hortensis*), marshmallow (*Althaea officinalis*), melilot, yellow (*Melilotus officinalis*), milk thistle (*Silybum marianum*), mint (*Mentha piperita*), mistletoe (*Viscum album*), monks cress (*Tropaeolum majus*), mountain germander (*Teucrium montanum*), mouse-ear hawkweed (*Pilosella officinarum*), mulberry, black (*Morus nigra*), mulberry, white (*Morus alba*), mullein (*Verbascum thapsus*), mustard, black (*Brassica nigra*), mustard, white (*Sinapis alba*), oak (*Quercus*), oat (*Avena sativa*), olive (*Olea europaea*), onion (*Allium cepa*), orchid (*Orchis morio*), oregano (*Origanum vulgare*), parsley (*Petroselinum hortense*), peach (*Prunus persica*), peppermint (*Mentha piperita*), pigweed (*Polygonum aviculare*), pink ipê (*Tabebuia impetiginosa*), plantain, greater (*Plantago major*), plantain, ribwort (*Plantago lanceolata*), plum (*Prunus domestica*), polypody (*Polypodium vulgare*), pomergranate (*Punica granatum*), pumpkin (*Cucurbita pepo L*), purple chokeberry (*Aronia prunifolia*), pussy willow (*Salix caprea*), quackgrass (*Agropyron repens*), quince (*Cydonia oblonga*), radish (*Raphanus sativus*), raspberry (*Rubus idaeus*), ramsons (*Allium ursinum*), red chokeberry (*Aronia arbutifolia*), red currant (*Ribes rubrum*), rest harrow (*Ononis spinosa*), rose de mai (*Rosa centifolia*), rosemary (*Rosmarinus officinalis*), rupturewort (*Herniaria glabra*), rustyback (*Ceterach officinarum*), sage (*Salvia officinalis*), salad burnet (*Sanguisorba minor*), saw palmetto (*Serenoa Repens*), scots pine (*Pinus silvestris*), senna (*Cassia angustifolia*), sesame (*Sesamum indicum*), shepard's purse (*Capsella bursa-pastoris*), silver thistle (*Carlina acaulis*), speedwell (*Veronica officinalis* ), starflower (*Borago officinalis*), sticklewort (*Agrimonia eupatoria*), stickyweed (*Galiuma aparine*), stickywilly (*Galiuma aparine*), stinging netle (*Urtica dioica*), st john's wort (*Hypericum perforatum*), strawberry (*Fragaria*), stone fern (*Ceterach officinarum*), sunflower (*Helianthus annuus*), sweetclover, yellow (*Melilotus officinalis*), sweet flag (*Acorus calamus*), sweet woodruff (*Asperula odorata*), taheebo tea (*Tabebuia impetiginosa*), tarragon (*Artemisia dracunculus*), thyme (*Thymus vulgaris*), tetterwort (*Chelidonium majus*), toadflax (*Linaria vulgaris*), tormentil (*Potentilla tormentilla*), valerian (*Valeriana officinalis*), vervian (*Verbena officinalis*), violet (*Viola odorata*), wall germander (*Teucrium chamaedrys*), walnut (*Juglans regia*), water dropwort (*Oenanthe aquatica*), waterlily (*Nymphaea alba*), white lotus (*Nymphaea alba*), wild apple (*Malus sylvestris*), wild cherry (*Prunus serotina*), wild ginger (*Asarum europaeum*), wild pansy (*Viola Tricolor*), wild pear (*Pyrus piraster*), wild strawberry (*Fragaria vesca*), wild thyme (*Thymus serpyllum*), willow herb (*Epilobium parviflorum*), winter savory (*Satureja montana*), woodruff (*Asperula odorata*), wormwood (*Artemisia absinthium*), woundwort (*Solidago virgaurea*), yarrow (*Achilea millefolium*), yellow sweetclover (*Melilotus officinalis*), or yucca (*Agavaceae*).

An agent can be one that is, or can be made to be, vaporizable. In some cases, the drug can be a heat stable drug. Exemplary drugs include acebutolol, acetaminophen, alprazolam, amantadine, amitriptyline, apomorphine diacetate, apomorphine hydrochloride, atropine, azatadine, betahistine, brompheniramine, bumetanide, buprenorphine, bupropion hydrochloride, butalbital, butorphanol, carbinoxamine maleate, celecoxib, chlordiazepoxide, chlorpheniramine, chlorzoxazone, ciclesonide, citalopram, clomipramine, clonazepam, clozapine, codeine, cyclobenzaprine, cyproheptadine, dapsone, diazepam, diclofenac ethyl ester, diflunisal, disopyramide, doxepin, estradiol, ephedrine, estazolam, ethacrynic acid, fenfluramine, fenoprofen, flecainide, flunitrazepam, galanthamine, granisetron, haloperidol, hydromorphone, hydroxychloroquine, ibuprofen, imipramine, indomethacin ethyl ester, indomethacin methyl ester, isocarboxazid, ketamine, ketoprofen, ketoprofen ethyl ester, ketoprofen methyl ester, ketorolac ethyl ester, ketorolac methyl ester, ketotifen, lamotrigine, lidocaine, loperamide, loratadine, loxapine, maprotiline, memantine, meperidine, metaproterenol, methoxsalen, metoprolol, mexiletine HCl, midazolam, mirtazapine, morphine, nalbuphine, naloxone, naproxen, naratriptan, nortriptyline, olanzapine, orphenadrine, oxycodone, paroxetine, pergolide, phenyloin, pindolol, piribedil, pramipexole, procainamide, prochloperazine, propafenone, propranolol, pyrilamine, quetiapine, quinidine, rizatriptan, ropinirole, sertraline, selegiline, sildenafil, spironolactone, tacrine, tadalafil, terbutaline, testosterone, thalidomide, theophylline, tocamide, toremifene, trazodone, triazolam, trifluoperazine, valproic acid, venlafaxine, vitamin E, zaleplon, zotepine, amoxapine, atenolol, benztropine, caffeine, doxylamine, estradiol 17-acetate, flurazepam, flurbiprofen, hydroxyzine, ibutilide, indomethacin norcholine ester, ketorolac norcholine ester, melatonin, metoclopramide, nabumetone, perphenazine, protriptyline HCl, quinine, triamterene, trimipramine, zonisamide, bergapten, chlorpromazine, colchicine, diltiazem, donepezil, eletriptan, estradiol-3,17-diacetate, efavirenz, esmolol, fentanyl, flunisolide, fluoxetine, hyoscyamine, indomethacin, isotretinoin, linezolid, meclizine, paracoxib, pioglitazone, rofecoxib, sumatriptan, tolterodine, tramadol, tranylcypromine, trimipramine maleate, valdecoxib, vardenafil, verapamil, zolmitriptan, zolpidem, zopiclone, bromazepam, buspirone, cinnarizine, dipyridamole, naltrexone, sotalol, telmisartan, temazepam, albuterol, apomorphine hydrochloride diacetate, carbinoxamine, clonidine, diphenhydramine, thambutol, fluticasone proprionate, fluconazole, lovastatin, lorazepam N,O-diacetyl, methadone, nefazodone, oxybutynin, promazine, promethazine, sibutramine, tamoxifen, tolfenamic acid, aripiprazole, astemizole, benazepril, clemastine, estradiol 17-heptanoate, fluphenazine, protriptyline, ethambutal, frovatriptan, pyrilamine maleate, scopolamine, and triamcinolone acetonide or pharmaceutically acceptable analogs or equivalents thereof.

In some cases, an agent is a parasympathomimetic alkaloid. In some cases, the parasympathomimetic alkaloid is nicotine, arecoline, muscarine, or pilocarpine.

In some cases, an agent is a nicotinic acetylcholine receptor agonist. In some cases, the nicotinic acetylcholine receptor agonist is nicotine, acetylcholine, choline, epibatidine, lobeline, or varenicline.

In some cases, an agent inhibits chromatin modifying enzymes (e.g., class I and II histone deaceytlases). In some cases, an agent that inhibits chromation modifying enzymes is nicotine.

In some cases, an agent is a nicotine analog or derivative. In some cases, the nicotine analog is EVP-6124. In some cases, a nicotine analog or derivative is described, e.g., in U.S. Patent Application Publication Nos. 20130157995, 20090234129, 20080108822, 20070186940, or 20080227088 or U.S. Patent Nos. 4,243,605, 5,015,741, 6,503,922, 6,995,265, or 7,132,545.

In some cases, a combination of at least or at most 1, 2, 3, 4, 5, 6, 7, 8, 9, 10, 11, 12, 13, 14, 15, 16, 17, 18, 19, or 20 agents is used. In some cases, a combination of between 1-2, 2-4, 4-6, 6-8, 8-10, 10-12, 12-14, 14-16, 16-18, or 18-20 agents is used. In some cases, a combination of between 1-5, 5-10, 10-15, or 15-20 agents is used.

**Formulations**

Any agent as provided herein for use in the methods and devices described herein can be in a formulation comprising one or more additional substances as provided herein. In some cases, the formulation comprising an agent (e.g., nicotine) and one or more additional substances is a liquid formulation. In some cases, the formulation is liquid at room temperature. In some cases, the liquid formulation is contained in a reservoir as provided herein in a device as provided herein and is liquid at an operating temperature of the device. The operating temperature of any of the devices as described herein can be at, below, or above room temperature. In some cases, the liquid formulation comprising a pharmaceutically active agent (e.g., nicotine) as provided herein is delivered as a liquid to a heater element as provided herein in a device as provided herein when a user inhales from the outlet or mouthpiece of the device. In some cases, the liquid formulation is not a viscous liquid. In some cases, the liquid formulation is not gel-like or a gel. In some cases, a liquid formulation comprising a pharmaceutically active agent (e.g., nicotine) as provided herein is not coated as a solid or film of any thickness onto a heater element as provided herein. In some cases, a liquid formulation comprising nicotine for use in the methods and devices described herein is not admixed with thickening agents and thereby has a viscosity that is reduced or is less than a liquid formulation comprising nicotine that has been admixed with a thickening agent. In some cases, a liquid formulation for use in the methods and devices as provided herein is not applied to or coated on a heater element as provided herein prior to use of the device by a user or subject as provided herein. In some cases, the liquid formulation comprising a pharmaceutically active agent is delivered as a liquid to a heater element in a device as provided herein only upon use of the device. Use of the device can be a user as provided herein inhaling or drawings on an outlet or mouthpiece on a device as provided herein. In some cases, inhalation on the outlet or mouthpiece draws carrier gas (e.g., air) ino the device through an inlet on the device as provided herein, wherein the flow of the carrier gas (e.g., air) through the inlet triggers delivery of a liquid formulation comprising a pharmaceutically active agent (e.g., nicotine) by any of the means provided herein to a heater element contained within the device.. The device can comprise one or more inlets as provided herein, wherein inhalation on an outlet draws carrier gas (e.g., air) through the one or more inlets simultaneously.

In some cases, one or more carriers or excipients is added to a liquid formulation to change a property of the formulation. One or more carrriers can be used to change the density, compressibility, specific weight, viscosity, surface tension, or vapor pressure of a liquid formulation.

In some cases, the use of any of the devices for generating a condensation aerosol comprising a pharmaceutically active agent (e.g., nicotine) as provided herein by a subject does not adversely affect functioning of the subject's bodily systems and/or organs. The bodily system can be the cardiovascular system and/or pulmonary system. The bodily organs can be the heart and/or lungs. In some cases, a subject using a device as provided herein has a substantially similar heart rate and pulse following use of the device as compared to a baseline. The baseline can be the subject's heart rate or pulse prior to using the device. In some cases, a subject using a device as provided herein has substantially similar lung function following use of the device as compared to a baseline. The baseline can be the subject's lung function prior to using the device. Lung function can be assessed by recording or measuring a subject's forced vital capacity (FVC) and/or the forced expiratory volume (FEV1), or calculating the ratio of FEV1/FVC. FEV1 is the volume of air that can forcibly be blown out in one second after full inspiration, while FVC is the maximum amount of air a person can expel from the lungs after a maximum inhalation. FVC is equal to the sum of inspiratory reserve volume, tidal volume, and expiratory reserve volume. For a healthy adult, the ratio of FEV1/FVC is approximately 75-80%. In some cases, a subject using a device as provided herein has a heart rate after use of the device that is about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% of the subject's heart rate prior to use of the device. In some cases, a subject using a device as provided herein has a pulse after use of the device that is about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% of the subject's pulse prior to use of the device. In some cases, a subject using a device as provided herein has a FEV1/FVC ratio after use of the device that is about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% of the subject's FEV1/FVC ratio prior to use of the device.

## III. eHealth tools

### Overview

Provided herein are eHealth tools which can include mobile devices, web-based devices, computer readable medium, and an eHealth-enabled electronic agent (e.g., nicotine) delivery platform. The eHealth tools can also be referred to as mobile Health tools or mHealth tools. In some cases, an eHealth-enabled electronic nicotine delivery platform can help a smoker transition to clean nicotine delivery by delivering a pre-determined nicotine dose with a pre-determined nicotine particle size at a pre-determined time for an individual user of a device. The eHealth-enabled electronic nicotine delivery platform can provide nicotine to an individual user on a particular schedule, which may involve varying the number of doses per day, timing of doses within the day, or amount of nicotine per dose over time. In one embodiment, the eHealth-enabled electronic nicotine delivery platform is used to achieve a reduction in an urge or desire of a subject to smoke a tobacco based smoking article. In another embodiment, the eHealth tools can help to ensure user safety when administering doses of nicotine from an electronic nicotine delivery device, so as to prevent overdose. In some cases, any of the devices provided herein are Bluetooth enabled. Bluetooth enabled devices as provided herein can be used to track usage of the device by a user. The mHealth tools can be used to aid or help a user transition from combustibles (e.g., tobacco cigarettes or cigars). Any of the devices as provided herein can be adapted or configured to leverage mobile technology, mHealth or eHealth tools as provided herein.

The methods can be applied to a variety of types of classifications of users of combustible tobacco products, including a new smoker, a trough maintainer smoker, an intermittent smoker, a light smoker, a weight-loss smoker, a heavy smoker, or a very heavy smoker. An intermittent smoker can be an individual who does not smoke every day. A light smoker can be an individual who smokes 1 to 9 cigarettes per day. A moderate smoker can be an individual who smokes 10 to 19 cigarettes a day. A heavy smoker can be an individual who smokes 20 to 29 cigarettes per day. A very heavy smoker can be an individual who smokes 30 or more cigarettes per day.

Provided herein is a method for managing treatment of a condition. The method can comprise providing a device for generating a condensation aerosol comprising a pharmaceutically active agent. The pharmaceutically active agent can be an agent as provided herein. In some cases, the condition is smoking or nicotine addiction. In some cases, the pharmaceutically active agent is nicotine. The device for generating the condensation aerosol can be device as provided herein. The device can comprise a heater element. The heater element can be any heater element as provided herein. The heater element can vaporize a composition comprising the pharmaceutically active agent. In some cases, the formulation is a liquid formulation. The heater element can be in fluid communication with a source of the formulation. The source of the formulation can be a reservoir. The heater element can be in fluid communication with a passageway configured for permitting the condensation of the vaporized formulation to produce particles comprising a size effective for deep lung delivery. The size of the particles can have an MMAD of about 1 to about 5 um. The device can further comprise a programmable controller, wherein the programmable controller comprises a non-transitory computer readable medium comprising one or more algorithms, and an interface for communicating with the programmable controller, wherein the interface is capable of receiving information from and/or transmitting information to a source. The source can be a user of the device, a healthcare provider and/or a counselor. The methods provided herein can include inputting, receiving and/or recording data on the device; analyzing the data; and regulating a dosage, frequency of administration and/or delivery schedule of the condensed formulation comprising the pharmaceutically active agent based on the analysis of the data by the one or more algorithms. The method as provided herein can also comprise adjusting the dosage, frequency of administration and/or delivery schedule of the condensed formulation comprising the pharmaceutically active agent based on the information received from the source. The inputting, analysis, regulating, and, optionally, adjusting can be repeated in order to manage treatment of the condition. Prior to a user engaging in a method or using a device as provided herein for a first time, the dosage, frequency of administration and/or delivery schedule of the condensed formulation comprising the pharmaceutically active agent can be pre-set by a source. The analysis of the data can be performed by the one or more algorithms. The regulation the dosage, frequency of administration and/or delivery schedule of agent as provided herein can be based on an analysis of the data by the one or more algorithms.

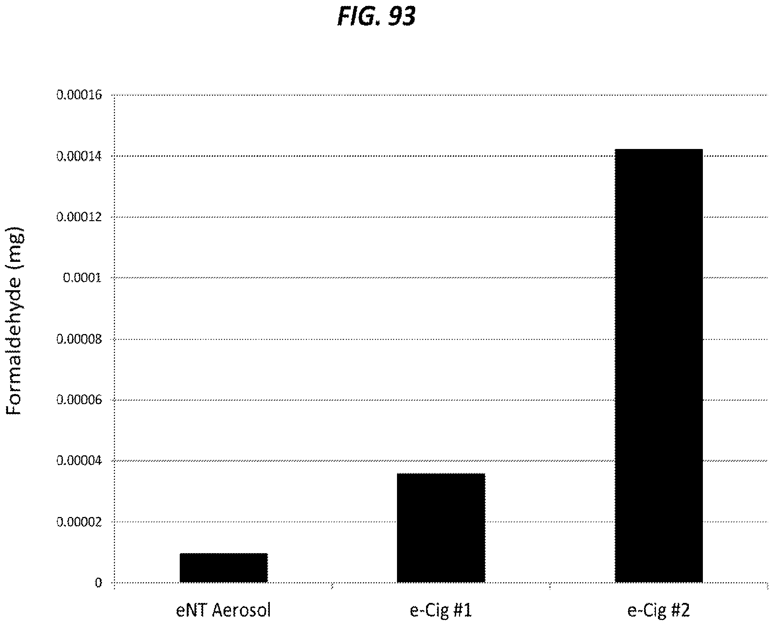

Provided herein are methods and devices for reducing an amount or level of a toxic agent in an aerosol produced by a device as provided herein. An example of the use of an aerosol generated using a device as provided herein reducing the level of a toxic agent (e.g., formaldehyde) versus commercially available e-cigarettes (e-Cig #1 and #2) is shown in **FIG. 93.** The aerosol can be a condensation aerosol. The method can comprise providing to a subject any device for generating a condensation aerosol comprising nicotine as provided herein, wherein the subject inhales the condensation aerosol comprising nicotine as generated by the device, wherein the condensation aerosol comprising nicotine from the device comprises a reduced or substantially reduced level of a toxic agent, thereby exposing the subject to the reduced or substantially reduced level of the toxic agent. The toxic agent or toxin can be any toxin or toxic agent associated with smoking or using a tobacco cigarette or commonly known e-cigarette as known in the art. In some cases, the toxic agent is formaldehyde. The device can comprise a controller. The controller can be programmable. In some cases, the condensation aerosol comprising nicotine has a diameter of from about 1 to about 5 $\mu$m. In some cases, the condensation aerosol has a diameter of from about 1 to about 3 $\mu$m. In some cases, the diameter is a mass median aerodynamic diameter (MMAD). In some cases, the diameter is a volume median diameter (VMD). The subject can be a smoker. The smoker can be a new smoker, a trough maintainer smoker, an intermittent smoker, a light smoker, a weight-loss smoker, a heavy smoker, or a very heavy smoker. An intermittent smoker can be an individual who does not smoke every day. A light smoker can be an individual who smokes 1 to 9 cigarettes per day. A moderate smoker can be an individual who smokes 10 to 19 cigarettes a day. A heavy smoker can be an individual who smokes 20to 29 cigarettes per day. Any of the devices provided herein can use up to 40-70% less nicotine than cigarettes or existing e-cigarettes. In some cases, the device delivers the condensation aerosol to the deep lung of the subject. The level or amount of a toxic agent (e.g., formaldehyde) in a condensation aerosol produced from a device for generating a condensation aerosol comprising nicotine as provided herein can be reduced by about, at least, or at most 1%, 2%, 3%,4%,5%, 6%, 7%, 8%, 9%, 10%, 11%, 12%, 13%, 14%, 15%, 16%, 17%, 18%, 19%, 20%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% as compared to a baseline. The level or amount of a toxic agent (e.g., formaldehyde) in a condensation aerosol produced from a device for generating a condensation aerosol comprising nicotine as provided herein can be1, 2, 3, 4, 5, 6, 7, 8, 9, 10, 11, 12, 14, 15, 16, 17, 18, 19, 20, 21, 22, 23, 24, 25, 26, 27, 28, 29,30, 35, 40, 45, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95 , 96, 97, 98, 99, 100, 200, 300, 400, 500 or 1000-fold less or lower than a baseline. The baseline can be the amount or level of the toxic agent (e.g., formaldehyde) in an aerosol produced froma tobacco or e-cigarette. The e-cigarette can be a commercial, conventional, or existing electronic cigarette. The level or amount of a toxic agent (e.g., formaldehyde) in a condensation aerosol produced from a device for generating a condensation aerosol comprising nicotine as provided herein can beless than 0.00002, 0.000015, 0.00001, or 0.000005 mg. The amount or level of the toxic agent (e.g., formaldehyde produced by a device provided herein can be reduced even after the device has been used mutliple times (see FIG. 93). The multiple times can be about 100 times. The multiple times can be between 50 and 100 times. The multiple times can be more than 100 times. The multiple times can be about 125 times.

**Smoking Urge**

Provided herein is a method for facilitating or treating an urge or desire of a subject to smoke. The method can comprise providing any device for generating a condensation aerosol comprising a pharmaceutically active agent as provided herein, wherein the device comprises a programmable controller. The pharmaceutically active agent can be nicotine. In some cases, the subject inhales the condensation aerosol produced by the device a plurality of times, wherein inhaling a plurality of times produces a desired nicotine blood concentration. The desired nicotine blood concentration can cause a reduction in a desire or urge in smoking. The desired nicotine blood concentration can be a plasma or serum concentration. In some cases, the desired plasma, serum or blood concentration can be an arterial plasma, serum or blood concentration. In some cases, the desired plasma, serum or blood concentration can be a venous plasma, serum or blood concentration. The desired nicotine blood, serum or plasma concentration can be about, more than, less than, or at least 1%, 2%, 3%,4%,5%, 6%, 7%, 8%, 9%, 10%, 11%, 12%, 13%, 14%, 15%, 16%, 17%, 18%, 19%, 20%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% of the nicotine plasma, serum or blood concentration achieved by smoking a cigarette. The desired nicotine plasma, serum or blood concentration can be between 1%-10%, 10%-20%, 20%-30%, 30%-40%, 40%-50%, 50%-60%, 60%-70%, 70%-80%, 80%-90%, or 90%-100% of the nicotine plasma, serum or blood concentration achieved by smoking a cigarette. The desired nicotine plasma, serum or blood concentration can be about 1% to about 10%, about 10% to about 20%, about 20% to about 30%, about 30% to about 40%, about 40% to about 50%, about 50% to about 60%, about 60% to about 70%, about 70% to about 80%, about 80% to about 90%, or about 90% to about 100% of the nicotine plasma, serum or blood concentration achieved by smoking a cigarette. Smoking a single cigarette can produce peak increments of plasma nicotine concentration of 5-30 ng/ml. The peak increments of plasma, serum or blood nicotine concentration from smoking a cigarette can be achieved within 10 minutes. The peak increments of plasma, serum or blood nicotine concentration from smoking a cigarette can be achieved within 15 minutes. The peak increments of plasma, serum or blood nicotine concentration from smoking a cigarette can be achieved within 20 minutes. The methods provided herein further comprise altering the dosage, frequency of administration, and/or delivery schedule of the condensation aerosol in order to alter the arterial nicotine plasma concentration. The alteration of the dosage, frequency of administration, and/or delivery schedule of the condensation aerosol can facilitate smoking urge reduction. In some cases, the dosage of the pharmaceutically active agent inhaled during each of the plurality of inhalations can be a percentage of a total dosage required for a specific period of time. In some cases, the pharmaceutically active agent is nicotine. In some cases, the dosage of nicotine inhaled during each of the plurality of inhalations is 25, 50, 75, or 100 μg. In some cases, the total dosage of nicotine required is 250, 500, 750, or 1000 μg. In some cases, the specific period of time is about 5 min. The period of time can be a day, wherein each inhalation of the plurality of inhalations can be a percentage of the daily dosage. Each inhalation can be about, more than, less than, or at least 1%, 5%, 10%, 15%, 20%, 25%, 30%, 33%, 50%, or 100% of a total dosage.

Provided herein is a method for treating an urge to smoke in a subject. The urge can be a desire. The urge can be a morning urge. The urge can be acute or prolonged. The method can comprise providing to a subject any device for generating a condensation aerosol comprising nicotine as provided herein, wherein the subject inhales the condensation aerosol comprising nicotine as generated by the device, wherein inhalation of the condensation aerosol comprising nicotine from the device causes a reduction in an urge to smoke in the subject using the device.. The device can comprise a controller. The controller can be programmable. In some cases, the condensation aerosol comprising nicotine has a diameter of from about 1 to about 5 μm. In some cases, the condensation aerosol has a diameter of from about 1 to about 3 μm. In some cases, the diameter is a mass median aerodynamic diameter (MMAD). In some cases, the diameter is a volume median diameter (VMD). The subject can be a smoker. The smoker can be a new smoker, a trough maintainer smoker, an intermittent smoker, a light smoker, a weight-loss smoker, a heavy smoker, or a very heavy smoker. An intermittent smoker can be an individual who does not smoke every day. A light smoker can be an individual who smokes 1 to 9 cigarettes per day. A moderate smoker can be an individual who smokes 10 to 19 cigarettes a day. A heavy smoker can be an individual who smokes 20to 29 cigarettes per day. Any of the devices provided herein can use up to 40-70% less nicotine than cigarettes or existing e-cigarettes. In some cases, the device delivers the condensation aerosol to the deep lung of the subject. The urge to smoke in the subject following use of a device as provided herein for generating a condensation aerosol comprising nicotine can be reduced by about, at least, or at most 1%, 2%, 3%,4%,5%, 6%, 7%, 8%, 9%, 10%, 11%, 12%, 13%, 14%, 15%, 16%, 17%, 18%, 19%, 20%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% as compared to a baseline. The baseline can be the urge to smoke in the subject prior to use of the device. The baseline can be the urge to smoke in the subject in comparison to a placebo or a vehicle. The placebo can be administered in an aerosol form using any of the devices provided herein. The vehicle can be a carrier. The carrier can be any carrier provided herein. In some cases, the carrier is propylene glycol, vegetible glycerin or a combination thereof. In some cases, a reduction in the urge to smoke in a subject occurs within a period of time following delivery of a condensation aerosol comprising nicotine. The reduction in the urge to smoke can occur about, less than, more than, at least or at most 1, 2, 3, 4, 5, 6, 7, 8, 9, 10, 11, 12, 14, 15, 16, 17, 18, 19, 20, 21, 22, 23, 24, 25, 26, 27, 28, 29,30, 35, 40, 45, 50, or 55 seconds, or 1, 2, 3, 4, 5, 6, 7, 8, 9, 10, 11, 12, 13, 14, 15, 16, 17, 18, 19, 20, 21, 22, 23, 24, 25, 26 27, 28, 29, 30, 31, 32, 33, 34, 35, 36, 37, 38, 39, 40, 41, 42, 43, 44, 45, 46, 47, 48, 94, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 minutes following inhalation of a condensation aerosol comprising nicotine as produced by any of the devices provided herein. The reduction in the urge to smoke as caused by the use of any of the devices provided herein for producing a condensation aerosol as provided herein can be sustained for about, less than, more than, at least, or at most 1, 2, 3, 4, 5, 6, 7, 8, 9, 10, 11, 12, 13, 14, 15, 16, 17, 18, 19, 20, 25, 30, 35, 40, 45, 50, 55, 60, 70, 80, 90, 100, 120, 140, 160, 180, 200, 250, or 300 minutes. In some cases, the reduction in the urge to smoke in a subject occurs after the subject inhales a condensation aerosol comprising nicotine from any device provided herein a plurality of times, wherein inhaling the plurality of times delivers or administers a pre-determined dose of nicotine. In some cases the pre-determined dose produces a nicotine blood or plasma concentration as provided herein. In some cases, a blood, serum or plasma nicotine concentration of from about 0.5 ng/ml to about 5 ng/ml is produced following a plurality of doses or inhalations from a device provided herein for generating a condensation aerosol comprising nicotine. In some cases, a blood, serum or plasma nicotine concentration of from about 1 ng/ml to about 2 ng/ml is produced following a plurality of doses or inhalations from a device provided herein for generating a condensation aerosol comprising nicotine. In some cases, a blood, serum or plasma nicotine concentration of from about 0.5 ng/ml to about 1 ng/ml is produced following a plurality of doses or inhalations from a device provided herein for generating a condensation aerosol comprising nicotine. In some cases, a blood, serum or plasma nicotine concentration of from about 0.5 ng/ml to about 1.5 ng/ml is produced following a plurality of doses or inhalations from a device provided herein for generating a condensation aerosol comprising nicotine. The plurality of times can be 2 to 10 doses or inhalations. The plurality of times can be 10 doses or inhalations. In some cases, the plurality of times occurs over a period of time. The period of time can be about, at least or at most 30 seconds, 1, 2, 3, 4, 5, 6, 7, 8, 9, 10, 11, 12, 13, 14, 15, 16, 17, 18, 19, or 20 minutes. In some cases, the plurality of times comprises 10 inhalations over a 5 minute period of time. The amount of nicotine per dose or inhalation can be 25, 50, 75, or 100 μg. In some cases, a blood, serum or plasma nicotine concentration of from about 0.5 ng/ml to about 5 ng/ml is produced following 10 inhalations from a device provided herein for generating a condensation aerosol comprising nicotine, wherein the condensation aerosol comprising nicotine comprises 25 μg of nicotine. In some cases, a blood, serum or plasma nicotine concentration of from about 0.5 ng/ml to about 5 ng/ml is produced following 10 inhalations from a device provided herein for generating a condensation aerosol comprising nicotine, wherein the condensation aerosol comprising nicotine comprises 50 μg of nicotine. In some cases, a blood, serum or plasma nicotine concentration of from about 0.5 ng/ml to about 2 ng/ml is produced following 10 inhalations from a device provided herein for generating a condensation aerosol comprising nicotine, wherein the condensation aerosol comprising nicotine comprises 50 μg of nicotine. In some cases, a blood, serum or plasma nicotine concentration of from about 0.5 ng/ml to about 1.5 ng/ml is produced following 10 inhalations from a device provided herein for generating a condensation aerosol comprising nicotine, wherein the condensation aerosol comprising nicotine comprises 50 $\mu$g of nicotine. In some cases, a blood, serum or plasma nicotine concentration of from about 0.5 ng/ml to about 1 ng/ml is produced following 10 inhalations from a device provided herein for generating a condensation aerosol comprising nicotine, wherein the condensation aerosol comprising nicotine comprises 50 $\mu$g of nicotine. In some cases, a blood, serum or plasma nicotine concentration of from about 0.5 ng/ml to about 5 ng/ml is produced following 10 inhalations from a device provided herein for generating a condensation aerosol comprising nicotine, wherein the condensation aerosol comprising nicotine comprises 75 $\mu$g of nicotine. In some cases, a blood, serum or plasma nicotine concentration of from about 0.5 ng/ml to about 2 ng/ml is produced following 10 inhalations from a device provided herein for generating a condensation aerosol comprising nicotine, wherein the condensation aerosol comprising nicotine comprises 75 $\mu$g of nicotine. In some cases, a blood, serum or plasma nicotine concentration of from about 0.5 ng/ml to about 1.5 ng/ml is produced following 10 inhalations from a device provided herein for generating a condensation aerosol comprising nicotine, wherein the condensation aerosol comprising nicotine comprises 75 $\mu$g of nicotine. In some cases, a blood, serum or plasma nicotine concentration of from about 0.5 ng/ml to about 1 ng/ml is produced following 10 inhalations from a device provided herein for generating a condensation aerosol comprising nicotine, wherein the condensation aerosol comprising nicotine comprises 75 $\mu$g of nicotine. In some cases, a blood, serum or plasma nicotine concentration of from about 0.5 ng/ml to about 5 ng/ml is produced following 10 inhalations from a device provided herein for generating a condensation aerosol comprising nicotine, wherein the condensation aerosol comprising nicotine comprises 100 $\mu$g of nicotine. In some cases, a blood, serum or plasma nicotine concentration of from about 0.5 ng/ml to about 2 ng/ml is produced following 10 inhalations from a device provided herein for generating a condensation aerosol comprising nicotine, wherein the condensation aerosol comprising nicotine comprises 100 $\mu$g of nicotine. In some cases, a blood, serum or plasma nicotine concentration of from about 0.5 ng/ml to about 1.5 ng/ml is produced following 10 inhalations from a device provided herein for generating a condensation aerosol comprising nicotine, wherein the condensation aerosol comprising nicotine comprises 100 $\mu$g of nicotine. In some cases, a blood, serum or plasma nicotine concentration of from about 0.5 ng/ml to about 1 ng/ml is produced following 10 inhalations from a device provided herein for generating a condensation aerosol comprising nicotine, wherein the condensation aerosol comprising nicotine comprises 100 $\mu$g of nicotine. In some cases, a blood, serum or plasma nicotine concentration of from about 0.5 ng/ml to about 5 ng/ml is produced in less than 1 minute following a plurality of doses or inhalations from a device provided herein for generating a condensation aerosol comprising nicotine. In some cases, a blood, serum or plasma nicotine concentration of from about 1 ng/ml to about 3 ng/ml is produced in less than 1 minute following a plurality of doses or inhalations from a device provided herein for generating a condensation aerosol comprising nicotine. In some cases, a blood, serum or plasma nicotine concentration of from about 0.5 ng/ml to about 5 ng/ml is produced in about 30 seconds following a plurality of doses or inhalations from a device provided herein for generating a condensation aerosol comprising nicotine. In some cases, a blood, serum or plasma nicotine concentration of from about 1 ng/ml to about 3 ng/ml is produced in less than 10 minutes following a plurality of doses or inhalations from a device provided herein for generating a condensation aerosol comprising nicotine. In some cases, a blood, serum or plasma nicotine concentration of from about 1 ng/ml to about 2 ng/ml is produced in about 30 seconds following a plurality of doses or inhalations from a device provided herein for generating a condensation aerosol comprising nicotine. The reduction in the urge to smoke in a subject following use of a device as provided herein can be substantially similar or equivalent to the reduction in the urge to smoke in a subject following use of or smoking a cigarette or use of, smoking, or vaping from an electronic cigarette. The electronic cigarette can be an electronic cigarette comprising a 4.5% nicotine solution. The reduction in the urge to smoke in a subject following use of a device as provided herein can be at least or about 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% of the reduction in the urge to smoke in a subject following use of or smoking a cigarette or use of, smoking, or vaping from an electronic cigarette. The reduction in the urge to smoke in a subject following use of a device as provided herein can be 50%-60%, 60%-70%, 70%-80%, 80%-90%, or 90%-100% of the reduction in the urge to smoke in a subject following use of or smoking a cigarette or use of, smoking, or vaping from an electronic cigarette. The reduction in the urge to smoke in a subject following use of a device as provided herein can be about 1% to about 10%, about 10% to about 20%, about 20% to about 30%, about 30% to about 40%, about 40% to about 50%, about 50% to about 60%, about 60% to about 70%, about 70% to about 80%, about 80% to about 90%, or about 90% to about 100% of the reduction in the urge to smoke in a subject following use of or smoking a cigarette or use of, smoking, or vaping from an electronic cigarette. In some cases, the reduction in the urge to smoke is assessed through the use of a psychometric response scale. The psychometric response scale can be a visual analog scale (VAS), a Likert, or a Borg scale. In some cases, the reduction in the urge to smoke is assessed using a VAS scale. The VAS scale can comprise a 100 point scale, wherein 0 = "not at all" and 100 = "extreme".

**Visible Vapor**

Provided herein is a method for reducing an amount of an exhaled vapor in a user of a cigarette or electronic cigarette. The vapor can be a visible vapor. The visible vapor can be an inhaled visible vapor and/or exhaled visible vapor. The exhaled visible vapor can be referred to as a second-hand vapor. The method comprises providing a user with any of the electronic agent (e.g., nicotine) delivery devices as provided herein, the user inhaling a condensation aerosol comprising a pharmaceutically active agent (e.g., nicotine) from the device, and the user exhaling, wherein the exhaling by the user produces a substantially reduced level of vapor. In some cases, the vapor is a visible vapor. In some cases, an electronic agent (e.g., nicotine) delivery devices as provided herein emits no visible vapor. In some cases, an electronic agent (e.g., nicotine) delivery devices as provided herein emits substantially no visible vapor. The visible vapor can be an inhaled and/or exhaled vapor. In some cases, use of (e.g., inhalation from) any device as provided herein by a user produces no or substantially no exhaled visible vapor by the user.. The reduction in an exhaled visible vapor from a subject following use of an electronic agent (e.g., nicotine) delivery device as provided herein can be at least or about 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% of the exhaled visible vapor (e.g., second hand smoke or vapor) produced by a subject following use of or smoking a cigarette or use of, smoking, or vaping from an electronic cigarette. The reduction in the exhaled visible vapor in a subject following use of a device as provided herein can be 50%-60%, 60%-70%, 70%-80%, 80%-90%, or 90%-100% of the exhaled visible vapor (e.g., second hand smoke or vapor) produced from a subject smoking a cigarette or using smoking, or vaping from an electronic cigarette. The reduction in the exhaled visible vapor in a subject following use of a device as provided herein can be about 1% to about 10%, about 10% to about 20%, about 20% to about 30%, about 30% to about 40%, about 40% to about 50%, about 50% to about 60%, about 60% to about 70%, about 70% to about 80%, about 80% to about 90%, or about 90% to about 100% of the exhaled visible vapor (e.g., second hand smoke or vapor) produced from a subject smoking a cigarette or using smoking, or vaping from an electronic cigarette. The electronic cigarette can be any commercial, conventional, or existing electronic cigarette known in the art (e.g., NJOY® King Bold, Finiti brand e-cig.). The electronic cigarette can be an electronic cigarette comprising a 4.5% nicotine solution. In some embodiments, an electronic agent (e.g., nicotine) delivery device as provided herein produces no or a substantially reduced amount of an exhaled visible vapor from a subject using said device.

An eHealth tool can be a healthcare practice supported by electronic processes and/or communication. In some cases, eHealth tools comprise healthcare practice using the Internet. The eHealth tools can be formatted for use by different types of smokers, including a new smoker, a weight loss smoker, a trough maintainer, a light smoker, a heavy smoker, or a very heavy smoker. The eHealth tools can be formatted for use by different types of patients who may be using nicotine to enhance their cognition or otherwise improve other symptoms of their illness (ulcerative colitis). In some cases the eHealth tools can communicate with a device described herein (e.g., through Bluetooth or infrared connectivity), or eHealth tools can be incorporated into a device described herein.

The eHealth tools provided herein include mechanisms for tracking use of a device. For example, the frequency of use of a device can be tracked. Also, provided herein are algorithms for analyzing the use of a device. The algorithms can be used to generate goals for a user of the device. In some cases, the algorithms can suggest a recommended dose of an agent (e.g., nicotine) for a user. The algorithms can suggest an agent (e.g., nicotine) delivery schedule for a user. Algorithms provided herein can change over time based on input from a device or feedback from the user over time. An eHealth nicotine delivery platform described herein can track use of a nicotine delivery device, assess the user in terms of their subjective nicotine craving, mood, or other psychological or behavioral parameters, and adjust nicotine delivery to accomplish desired effects. Smoking behavior can be tracked, as can other symptoms of a disease where nicotine is being used either as a treatment or to enhance deficiencies in cognition associated with a specific illness.

A smoking pattern of a user can be monitored, or use of a device described herein can be monitored. For example, tools provided herein can be used to determine if smoking or use of a device provided herein was used to satisfy a morning craving, determine if smoking occurred, or a device was used, while a subject was bored, drinking, under stress. Tools can be used to assess whether a subject smoked or used a device described herein alone or in the presence of others (e.g., friends), or whether the dose of nicotine administered was successful in enhancing cognition or improving another target medical or psychiatric symptom.

One or more algorithms can be used to devise a plan (e.g., nicotine dose, nicotine delivery schedule) for a user. In some cases, web-based tools can be used to transition a smoker to use of an electronic nicotine delivery device described herein along with customized behavioral input.

In some cases, the eHealth tools are web-based tools. The web-based tools can enable an appropriate dosing of nicotine for a user of a device described herein. In some cases, the web-based tools can track experiences of a user. In some cases, a web-based tool can track success in making a transition from smoking tobacco cigarettes. Web-based tools described herein can track health benefits derived from using devices described herein. Such tracking can enable generation of rewards (e.g., decreased health premiums). Web-based tools can enable development of constantly-improving use algorithms by obtaining use profiles from a multitude of users in the field, and can provide feedback to users. In some cases, web-based tools described herein can leverage social media to produce ideal health outcomes. The social media can be a social networking site (e.g., Facebook, Google +, MySpace, Bebo), blog or microblog (e.g., Twitter), a content community (e.g., YouTube), a virtual social world (e.g., Second Life), a virtual game world (e.g., World of Warcraft), or a collaborative project (e.g., Wikipedia). Social media can include technologies such as a blog, picture-sharing, vlog, wall-posting, email, instant messaging, music-sharing, crowdsourcing, voice over IP, Internet forums, weblog, social blog, microblog, wiki, podcast, and social bookmarking. The customized feedback can also be specific for users suffering from a medical or psychiatric disorder. For example, nicotine has been shown to have beneficial effects on cognition among patients with schizophrenia. The device could be used to deliver nicotine and also provide therapeutic input to patients to help them manage their nicotine intake in such a way as to provide maximum therapeutic advantage to their cognition or psychiatric symptom control. Other disorders where nicotine has been shown to have beneficial effects on cognition include Parkinson's disease, attention deficient disorder, mild cognitive impairment, and Alzheimer's disease.

In some cases, an eHealth tool is a mobile device. In some cases, the mobile device is an electronic nicotine delivery device. The mobile device can ensure dosing occurs at an appropriate time. The mobile device can comprise on-board tracking of dosing, can provide reminders to a subject, and can provide nicotine craving assessments. Also, a mobile device can comprise complementary advertising opportunities.

The devices provided herein can comprise electronics that control for variability in battery condition and ensure consistent heating.

## Identifying individualized user goals

eHealth tools can include Web based and mobile tools. For example, for web-based tools, self-report measures can be used to help a smoker or new user of a device provided herein identify a target goal based on their degree of nicotine dependency, health status, health goals, economic goals (i.e., decrease the amount of money spent on cigarettes), target body weight or change in body weight, or other factors.

When a mobile device is used, smoking patterns can be tracked prior to the transition to an electronic nicotine delivery platform, which can enable a real world, ecologically valid assessment of actual behavior to be used as a foundation for a subsequent prescribed pattern of use of an electronic nicotine delivery device.

## Algorithm development

By systematically tracking user characteristics at the outset, tracking their actual use of the electronic nicotine delivery device over time in terms of patterns of dosing, algorithms can be generated that can be used to suggest an optimal pattern of use, dose, pH, particle size, and other characteristics (e.g., flavoring) of the electronic nicotine delivery device to maintain use and minimize smoking urge. These algorithms can be constantly enhanced through additional user experience, adding to the empirical foundation of the algorithms and enabling more robust and finer-grained algorithms to be customized to an individual user's nicotine dependency and health goals.

For a mobile device, data can be captured from individual users in the field and can be sent to a backend web-based central database for algorithm development. The mobile device can also assess the ecological risk factors for relapse and adjust the dose or dose characteristics of nicotine accordingly to help achieve the desired outcome. An initial trial of several different types of dose characteristics may also be helpful in determining the ideal use algorithm.

In a web-based method, data from real world use of the electronic nicotine delivery device can be collected and used to predict outcomes. Users can also pick from one of several established algorithms that they think will best suit their health or other goals. The central database can issue instructions back to the electronic nicotine device, either in the form of explicit compliance reminders to use the device to achieve the optimal nicotine absorption, or implicit dosing instructions to the device to gradually taper the dose (or other characteristics of the nicotine dose, including its concentration, pH, particle size, flavorings, or flow characteristics coming from the device which can affect back of the throat impaction, which in turn can affect subjective sensations associated with the nicotine dose (i.e., tingling or burning in the back of the throat)) over the days or weeks to help achieve various health or nicotine-related goals.

## Matching users to algorithms

A user's goal when transitioning off of combustible tobacco products may change over time. By carefully matching users to an initial use and dose algorithm, and then monitoring their progress over time, adjustments can be made to ensure the maximal probability of success in their individual goals.

For a mobile device, feedback from the mobile device, both in terms of use patterns as well as real-time self-reports of cravings, and on-going tests of psychological dependency can be used help identify an initial use algorithm, as well as make changes to the use algorithm or switch to a new algorithm entirely.

For a web-based device, as new data is used to refine use algorithms, a web-based backend database can communicate subtle and/or gross changes in prescribed use algorithms to the device to help enhance the probability that a target goal will be achieved. In this way, each user can become part of a community helping to refine his/her own and others optimal algorithms to achieve a variety of goals.

## Customized dose, pH, particle size, etc.

By systematically varying different dose characteristics (e.g., dose, particle size, pH, amount of nicotine in the gas vs. particulate phase, air speed velocity coming out of a nicotine delivery device, flavorings, etc.), a differentially reinforcing subjective reward from the nicotine can be created. The probability that certain goals will be achieved can be maximized by varying dose characteristics of nicotine.

Relying on use algorithms matched to individual users regarding their stated goals, physical or psychological nicotine dependency characteristics, and/or biomarkers, the electronic nicotine delivery device can modify dose characteristics of nicotine. In some cases, the modifications can change in response to environmental triggers (e.g., by altering the mean particle size of the dose to provide an especially reinforcing dose if the subject reports on the electronic nicotine delivery device a strong craving). In some cases, the modifications can change to help the initial transition off of combustible tobacco (e.g., by altering the pH or flavor of the dose to help match previous stimulus characteristics of smoking).

## Administering nicotine challenge doses

As part of a behavioral program to achieve certain health or other nicotine-related goals, the electronic nicotine delivery device can administer one or more nicotine challenge doses. These challenge doses may contain no nicotine, less nicotine than previous doses, or doses of nicotine that vary in regards to other important characteristics (e.g., dose, particle size, pH, amount of nicotine in the gas vs. particulate phase, air speed velocity coming out of a nicotine delivery device, flavorings, etc). An electronic nicotine delivery device can then assess self-reported cravings or changes in a pattern of use that suggests increased or decreased nicotine administration. This feedback can then be used as real world data to help maintain or change the use algorithm to increase the probability that the user will achieve certain health or other nicotine-related goals.

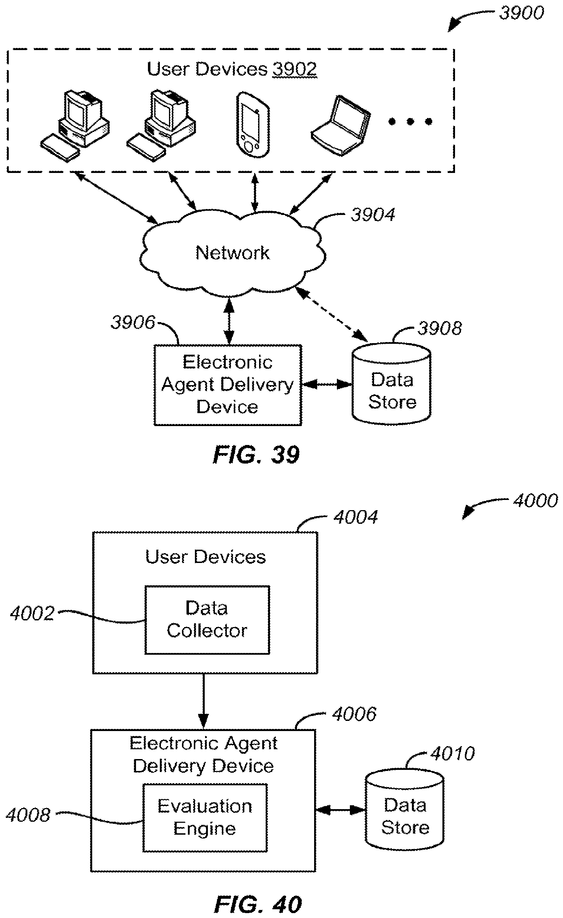

**FIG. 39** illustrates an example environment **3900** for implementing devices and methods described herein in accordance with an embodiment. As illustrated, one or more user devices **3902** connect via a network **3904** to an electronic agent (e.g., nicotine) delivery device **3906** as provided herein which can be configured to produce a condensation aerosol comprising a pharmaceutically active agent (e.g., nicotine) as provided herein. The electronic agent (e.g., nicotine) delivery device **3906** can comprise a controller, which can be programmable, as provided herein and the electronic agent (e.g., nicotine) delivery device **3906** can be connected to the network **3904** through the programmable controller. In some cases, the condensation aerosol comprising the pharmaceutically active agent (e.g., nicotine) is produced from a liquid formulation comprising the pharmaceutically active agent (e.g., nicotine) as provided herein. In various embodiments, the user devices **3902** can include any device capable of communicating with the network **3904,** such as personal computers, workstations, laptops, smartphones, mobile phones, tablet computing devices, smart TVs, game consoles, internet-connected set up boxes, and the like. In some embodiments, the user devices **3902** can include applications such as web browsers and/or applications (e.g., mobile apps) that are capable of communicating with the electronic agent (e.g., nicotine) delivery device **3906** and/or a system that uses the electronic agent (e.g., nicotine) delivery device **3906**. In some cases, the user devices **3902** communicate with the electronic agent (e.g., nicotine) delivery device **3906** via the programmable controller as provided herein. The user can be a patient, and/or a healthcare provider (e.g., physician, physician's assistant, nurse, nurse practioner, pharmacist or other medical professional). In some cases, a first user uses the device, while a second user uses the other user devices **3902**. In some cases, a first user uses the device and the other user devices **3902,** while the second user also uses the user devices **3902.**

In some embodiments, the electronic agent (e.g., nicotine) delivery device 3906 can communicate with a data store **3908** in order perform the functionalities described herein (e.g., track device usage, adjust dose, frequency of administration, delivery schedule, customize feedback, administer challenge doses, etc.). For example, the data store **3908** can be used to store historical (e.g. user use history, dosage history, delivery schedule history, frequency of administration history, etc.), evaluation rules, and the like.

In some embodiments, the data store **3908,** or any other data stores discussed herein, can include one or more data files, databases, (e.g., SQL database), data storage devices (e.g., tape, hard disk, solid-state drive), data storage servers, or the like. The data store **3908** can be connected to the electronic agent (e.g., nicotine) delivery device **3906** locally or remotely via a network. In some embodiments, data store **3908,** or any other data stores discussed herein, can comprise one or more storage services provisioned from a "cloud storage" provider, for example, Amazon Simple Storage Service ("Amazon S3"), provided by Amazon.com, Inc. of Seattle, Washington, Google Cloud Storage, provided by Google, Inc. of Mountain View, California, and the like.

In various embodiments, the network **3904** can include the Internet, a local area network ("LAN"), a wide area network ("WAN"), a cellular network, wireless network or any other public or private data and/or telecommunication network.

FIG. **40** illustrates example components of an electronic agent (e.g., nicotine) delivery system **4000,** in accordance with an embodiment. In this example, the electronic agent (e.g., nicotine) delivery system **4000** includes a data collector **4002** residing on a user or client device **4004.** The system further comprises an electronic agent (e.g., nicotine) delivery device **4006,** which can be the same as **3906** as depicted in **FIG. 39.** The electronic agent (e.g., nicotine) delivery device **4006** can comprise a programmable controller, wherein the data collector resides on the programmable controller. The data collector can be implemented as a browser script using JavaScript or any other scripting language. The data collector can be configured to communicate with a web-based backend database. For example, the data collector can be configured to collect parameter information about the electronic agent (e.g., nicotine) delivery device **4006** such as discussed herein and transmit such parameter information to the web-based backend database, for example, using an application programming interface (API) provided by the user device **4004.** In some embodiments, the collection and/or communication with the user device **4004** can be triggered by an event on the electronic agent (e.g., nicotine) delivery device **4006.** For example, the event can include a click on a portion (e.g., a button or a link) of a user display on the electronic agent (e.g., nicotine) delivery device **4006,** use of the delivery device by a user or patient, and the like. The user display can be on the programmable controller as provided herein.

In some embodiments, the electronic agent (e.g., nicotine) delivery device **4006** can be configured to receive parameter information (e.g., dosage, frequency of administration, dosing schedule, etc.) provided by the data collector of the user device and to compare and/or analyze the parameter information received from the data collector of the user device to the parameter information from use of the electronic agent (e.g., nicotine) delivery device **4006.** To that end, the electronic agent (e.g., nicotine) delivery device 4006 can utilize an evaluation engine **4008.** The evaluation engine **4008** can be configured to analyze the parameter information in order to customize or adjust output parameters of the electronic agent (e.g., nicotine) delivery device **4006.** In some embodiments, the evaluation engine **4008** can be implemented using one or more server-side library files. In some embodiments, the evaluation engine **4008** can be implemented using one or more algorithms as probided herein for analyzing the respective parameter.

In some embodiments, customized feedback or a treatment regimen (e.g., agent dosage, frequency of administration and/or delivery schedule) can be evaluated based on some or all of the parameters as provided herein. For example, a lookup table (e.g., stored in memory) can be used to determine the weight values associated with some or all of the parameters. The weight values may or may not be further weighted, combined or otherwise processed to derive a final customized feedback or treatment regimen. In some embodiments, the lookup table and the one or more algorithms for deriving the customized feedback or treatment regimen can be included on one or more rules that are pre-determined based on historical data such as past usage and/or user activities. In some embodiments, analysis of parameter information and/or generation of customized feedback or treatment regimen can be performed in real time or nearly real time with respect to the receipt of the parameter information. In other embodiments, any or all of the above operations may be performed in an asynchronous mode, for example, using batch processing.

In some embodiments, the generated feedback and/or treatment regimen can be stored in a data store **4010.** In some embodiments, the data store **4010** can include a memory of a server, one or more data storage device (e.g., SSD, hard disk, taps), or a cloud-based storage service such as discussed in connection with **FIG. 39.** The data store **4010** may or may not be owned and/or operated by the same as the provider of the electronic agent (e.g., nicotine) delivery device **4006.**

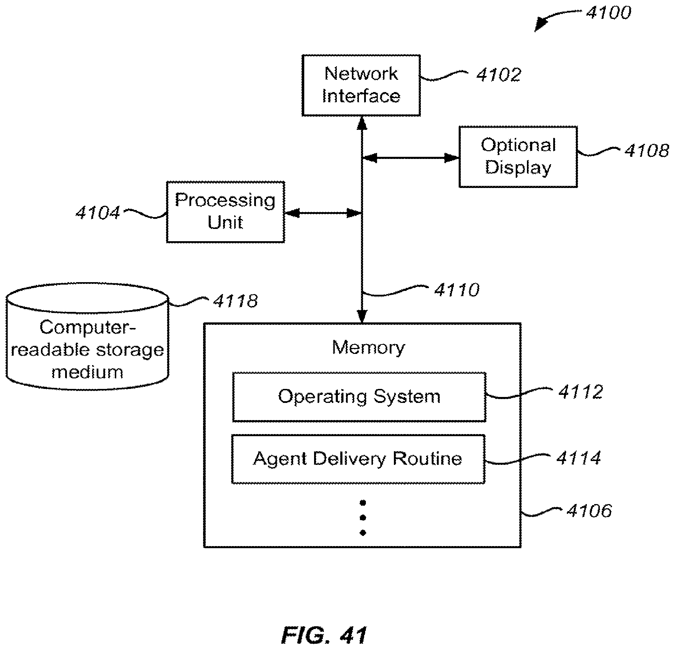

FIG. **41** illustrates example components of a computer device **4100** for implementing aspects of devices and methods described herein, in accordance with an embodiment. In another embodiment, the computer device **4100** may be configured to implement a user device such as a user device **3902** discussed in connection with **FIG. 39** and/or components or aspects of the electronic agent (e.g., nicotine) delivery device **3906** such as described in connection with **FIGs. 39** and **40.** In some embodiments, computing device **4100** can include many more components than those shown in **FIG. 4100.** However, it is not necessary that all of these components be shown in order to disclose an illustrative embodiment.

As shown in **FIG. 41,** computing device **4100** includes a network interface **4102** for connecting to a network such as discussed above. In some cases, the computing device **4100** is housed on a programmable controller on an electronic agent (e.g., nicotine) delivery device as provided herein. In various embodiments, the computing device **4100** may include one or more network interfaces **4102** for communicating with one or more types of networks such as the Internet, wireless networks, cellular networks, and any other network.

In an embodiment, computing device **4100** also includes one or more processing units **4104,** a memory **4106,** and an optional display or user interface as provided herein **4108,** all interconnected along with the network interface **4102** via a bus **4110.** The processing unit(s) **4104** can be capable of executing one or more methods or routines stored in the memory **4106.** The display **4108** can be configured to provide a graphical user interface to a user operating the computing device **4100** for receiving user input, displaying output, and/or executing applications. In some cases, such as when the computing device **4100** is a server, the display **4108** may be optional.

The memory **4106** can generally comprise a random access memory ("RAM"), a read only memory ("ROM"), and/or a permanent mass storage device, such as a disk drive. The memory **4106** may store program code for an operating system **4112,** one or more agent (e.g., nicotine) delivery routines **4114,** and other routines. In various embodiments, the program code can be stored on a computer-readable storage medium, for example, in the form of a computer program comprising a plurality of instructions executable by one or more processors. The computer-readable storage medium can be non-transitory. The one or more agent (e.g., nicotine) delivery routines **4114,** when executed, can provide various functionalities associated with the electronic agent (e.g., nicotine) delivery device as described herein.

In some embodiments, the software components discussed above can be loaded into memory **4106** using a drive mechanism associated with a non-transient computer readable storage medium **4118,** such as a floppy disc, tape, DVD/CD-ROM drive, memory card, USB flash drive, solid state drive (SSD) or the like. In other embodiments, the software components can alternatively be loaded via the network interface **4102,** rather than via a non-transient computer readable storage medium **4118.** In an embodiment, the computing device **4100** can also include an optional time keeping device (not shown) for keeping track of the timing of usage of the electronic agent (e.g., nicotine) delivery device.

In some embodiments, the computing device **4100** also communicates via bus **4110** with one or more local or remote databases or data stores such as an online data storage system via the bus **4110** or the network interface **4102.** The bus **4110** can comprise a storage area network ("SAN"), a high-speed serial bus, and/or via other suitable communication technology. In some embodiments, such databases or data stores may be integrated as part of the computing device **4100.**

## EXAMPLES

**Example 1: Effect of changes in air flow rate**, **electrical current, duration of heating, and thickness of heater element on particle size of a aerosol generated from** a **propylene glycol formulation.**

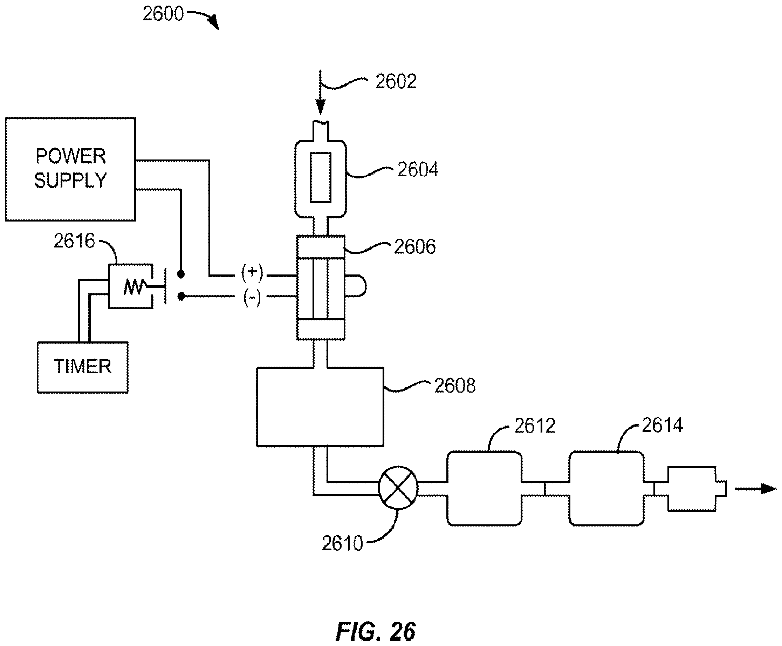

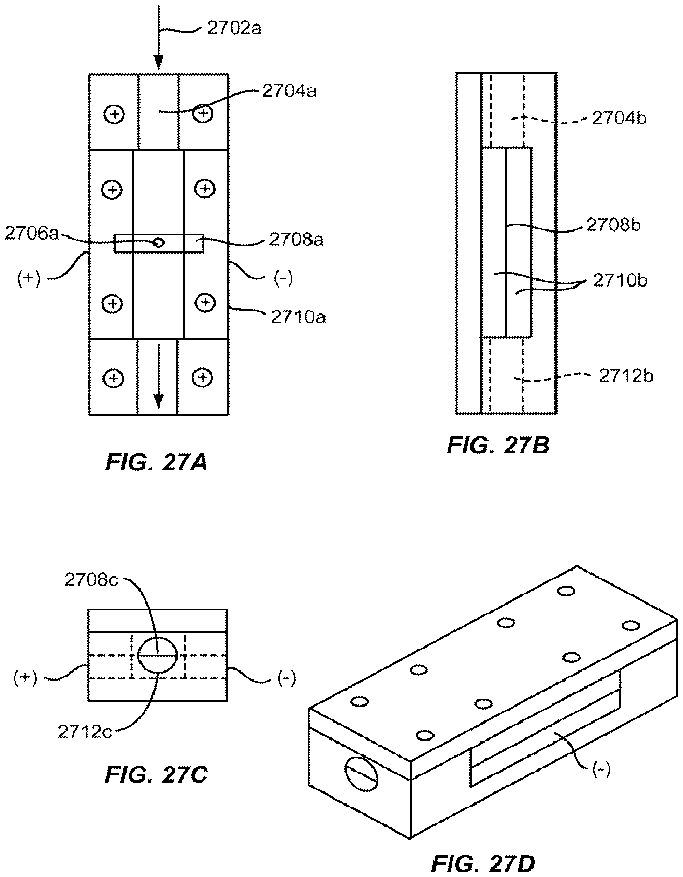

This example describes how changes in specific parameters (i.e. air flow rate, electrical current to a heater element, and thickness of a heater element) affected the size of aerosol particles generated by a test apparatus designed to comprise components and/or parameters of a nicotine delivery device as described herein. **FIG. 26** shows a schematic of the entire test apparatus while **FIGs. 27A-D** shows alternate views of the test airway used in the test apparatus. The test bed had an airway created between a block of Delrin (bottom) and a sheet of clear plexiglass (top) with brass sides used to clamp and make electrical contact with a heater element. The heater element was a stainless steel foil of variable thickness (0.0005 inches (about 0.013 mm) or 0.001 inches (about 0.025 mm)), and the formulation used to generate an aerosol was composed of propylene glycol. **FIG. 27A** shows a top view, with airflow (**2702a**) into an an inlet (**2704a**). A hole to deposite drug (**2706a**) was provided and foil was shown (**2708a**). Brass contacts (**2710a**) were provided. The length of the device was 6 inches (about 152.4 mm), and the width was 2.25 inches (about 57.15 mm). **FIG. 27B** shows a side view of the inlet (**2704b**), foil (**2708b**), brass electrical contacts (**2710b**), and outlet (**2712b**). **FIG. 27C** shows an end view of the foil (**2708c**) and (**2712c**). **FIG. 27D** shows an isometric view. **Table 2** shows the results of altering heater element thickness, air flow rate, current, and duration of heating on particle size distribution. Based on the results in **Table 2**, as the air flow rate was increased, the particle size diameter (PSD) decreased when the other parameters were held constant.

**Table 2.**

| Propylene glycol aerosol data from test airway | | | | | | | |
|---|---|---|---|---|---|---|---|
| Sequence | Material | Heater Element Thicknes s (inches) | Air Flow Rate (Liters/min ) | Dos e (mg) | Curren t (Amps ) | Duration of Heating (seconds ) | Particle Size Diamete r (microns ) |
| 1 | PG | 0.0005 | 1 | 1 | 8 | 0.5 | 2 |
| 2 | PG | 0.0005 | 1 | 1 | 6 | 1 | 2.1-3 |
| 3 | PG | 0.001 | 1 | 1 | 8 | 0.7 | 1 |
| 4 | PG | 0.001 | 3 | 1 | 7 | 1 | 1.8 |
| 5 | PG | 0.001 | 3 | 1 | 7 | 1 | 2 |

(continued)

| Propylene glycol aerosol data from test airway | | | | | | | |
|---|---|---|---|---|---|---|---|
| Sequenc e | Materia l | Heater Element Thicknes s (inches) | Air Flow Rate (Liters/min ) | Dos e (mg) | Curren t (Amps ) | Duration of Heating (seconds ) | Particle Size Diamete r (microns ) |
| 6 | PG | 0.001 | 3 | 1 | 7 | 1 | 2 |
| 7 | PG | 0.001 | 3 | 1 | 7 | 1 | 1.5-1.8 |
| 8 | PG | 0.001 | 3 | 1 | 7 | 1 | 1.4-1.8 |
| 9 | PG | 0.001 | 3 | 1 | 7 | 1 | 2 |
| 10 | PG | 0.001 | 3 | 1 | 10 | 1 | 1 |
| 11 | PG | 0.001 | 3 | 1 | 10 | 1 | 0.9 |
| 12 | PG | 0.001 | 6 | 1 | 10 | 1 | 0.6 |
| 13 | PG | 0.001 | 6 | 1 | 10 | 1 | 0.6-0.8 |
| 14 | PG | 0.001 | 12 | 1 | 10 | 1 | 0.5 |
| 15 | PG | 0.001 | 12 | 1 | 10 | 1 | 0.5 |

**Example 2: Effect of changes in air flow rate, electrical current, duration of heating, and thickness of heater element on particle size of an aerosol generated from a nicotine/propylene glycol formulation.**

This example describes how changes in specific parameters (i.e. air flow rate, and electrical current to a heater element) affected the size of aerosol particles generated from a 10% nicotine/propylene glycol formulation by a test apparatus as described in Example 1. **Table 3** shows the results of altering heater element thickness, air flow rate, current, and duration of heating on particle size distribution. As shown in **Table 3**, when air flow rate was altered while other parameters were held constant, the higher the air flow rate, the smaller the average particle size diameter (PSD).

**Table 3.**

| Nicotine/propylene glycol mixture (10%) aerosol data from test airway | | | | | | | |
|---|---|---|---|---|---|---|---|
| Sequenc e | Materia l | Heater Element Thicknes s (inches) | Air Flow Rate (Liters/ min ) | Dos e (mg) | Curren t (Amps ) | Duration of Heating (seconds ) | Average Particle Size Diamete r (microns ) |
| 1 | Nic/PG | 0.001 | 4 | 1 | 9 | 1 | 1.35 |
| 2 | Nic/PG | 0.001 | 4 | 1 | 9 | 1 | 1.45 |
| 3 | Nic/PG | 0.001 | 4 | 1 | 9 | 1 | 1.45 |
| 4 | Nic/PG | 0.001 | 2 | 1 | 9 | 1 | 1.85 |
| 5 | Nic/PG | 0.001 | 2 | 1 | 9 | 1 | 2.3 |
| 6 | Nic/PG | 0.001 | 2 | 1 | 9 | 1 | 2.3 |
| 7 | Nic/PG | 0.001 | 4 | 1 | 10 | 1 | 1.55 |
| 8 | Nic/PG | 0.001 | 4 | 1 | 10 | 1 | 1.2 |
| 9 | Nic/PG | 0.001 | 4 | 1 | 10 | 1 | 1.325 |

**Example 3: Particle size diameter ranges of aerosols generated from a test apparatus using a heater element comprising a wire coil.**

This example describes the particle size diameters of aerosols generated from either a PG formulation or 10% nicotine/PG formulation using a test apparatus as shown in **FIGs. 26** and **27A-D** and described in Example 1. In this example, the heater element was a stainless steel coil comprising 3.5 coils and a diameter of 0.10 inches (about 2.54 mm). The heater element was heated using a current of 2.5 Amps and the air flow rate was 4 Liters/min (about $6.7 \times 10^{-5}$ m$^3$/s). Table 4 shows the results.

**Table 4.**

| Sequence | Material | Air Flow Rate (Liters/min) | Dose (mg) | Current (Amps) | Duration of Heating (seconds) | Particle Size Diameter (microns) |
|---|---|---|---|---|---|---|
| 1 | PG | 4 | 1 | 2.5 | 1 | 1.5-2.2 |
| 2 | PG | 4 | 1 | 2.5 | 1 | 1.5-2.2 |
| 3 | Nic/PG | 4 | 1 | 2.5 | 1 | 1.57-2.2 |
| 4 | Nic/PG | 2 | 1 | 2.5 | 1 | 1.6-2.8 |
| 5 | Nic/PG | 2 | 1 | 2.5 | 1 | 1.52-2.2 |
| 6 | PG | 2 | 1 | 2.5 | 1 | 1.5-2.2 |
| 7 | PG | 4 | 1 | 2.5 | 1 | 1.5-2.3 |
| 8 | PG | 4 | 1 | 2.5 | 1 | 2.4-1.5 |

**Example 4: Particle size diameters of aerosols generated from commercially available e-cigarettes (eCigs).**

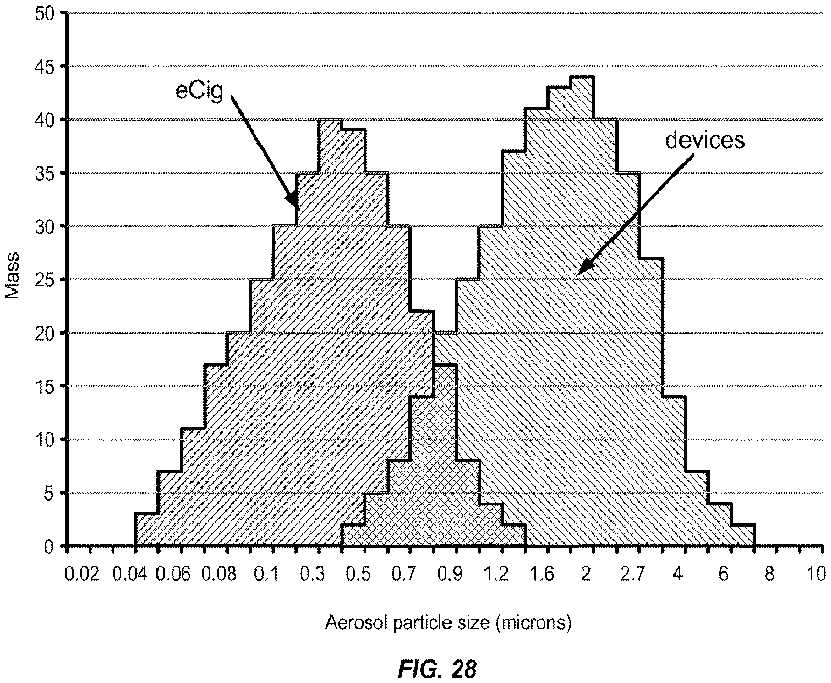

This example describes the particle size diameters of aerosols generated from either one of two brands of eCigs (Finiti and BLU). In this example, a 50 ml volume of an aerosol was pulled from either one of the two brands of eCigs over a period of 3 seconds in order to simulate a human breath. The collected aerosol was then injected into a laser particle size detector set at a flow rate of 14 Liters/min (about $2.33 \times 10^{-4}$ m$^3$/s). **Table 5** shows the particle size diameter of the aerosols generated from two brands of eCigs. FIG. **28** shows a comparison of the particle size distribution for aerosols created by eCigs vs. aerosol created by devices provided herein (devices). As shown in **FIG. 28**, the particle size distribution of aerosols generated by devices provided herein was shifted toward larger particle sizes vs. those generated by eCigs.

**Table 5.**

| Test Number | Brand | Particle Size | | |
|---|---|---|---|---|
| | | Low End | High End | Average |
| 1 | Finiti | 0.5 | 0.5 | 0.5 |
| 2 | Finiti | 0.5 | 0.6 | 0.55 |
| 3 | Finiti | 0.5 | 0.5 | 0.5 |
| 4 | Finiti | 0.5 | 0.5 | 0.5 |
| 5 | BLU | 0.5 | 0.5 | 0.5 |
| 6 | BLU | 0.5 | 0.8 | 0.65 |

**Example 5: Effect of changes in valve material, and the diameter of a bypass orifice on particle size of a aerosol generated from a propylene glycol formulation.**

This example describes how changes in specific parameters (i.e. valve material and diameter of a bypass orifice) affected the size of aerosol particles generated by a test apparatus designed to comprise components and/or parameters of a device for generating condensation aerosols as described herein. **FIG. 29A** shows a schematic of the entire test apparatus while FIG. **29B** shows an internal view of the valve **(2904a)** used in the test apparatus. The valve flap **(2902b)** had a ¾ inch diameter and the diameter of the channel downstream of the valve was 0.375 inches (about 9.53 mm) in length and 0.090 inches (about 2.29 mm) in width. The test bed had a primary airway **(2906a),** and a bypass airway **(2908a),** an aerosol generation chamber **(2912a)** and vacuum source **(2910a).** The aerosol generation chamber comprised a heater element. The inlet to the bypass airway was a slot of varying dimensions (L x W). **Table 6** shows the results using a valve of ¾ inch (about 19.05 mm) diameter and altering valve material and bypass orifice diameter.

As shown in **Table 6**, regardless of valve material type and bypass orifice diameter, above inhalation pressures of about 2 inches of H2O (about 498 Pa), the primary flow remained relatively constant, while the bypass flow increased with increasing vacuum pressure. **Table 7** shows the results using a valve of 3/8 inch diameter, a bypass orifice of varying dimensions, and altering the orifice dimensions for the inlet of the primary airway. As shown in **Table 7**, reducing the size of the orifice of the primary airway consistently reduced the flow rate through the primary airway regardless of varying vacuum pressure, dimensions of the bypass orifice, or varying the valve material.

**Table 6**: Testing of Flow Control with the device of **FIG. 29.**

| Valve Material | Flow Bypass (LPM) | Flow Primary (LPM) | $\triangle$ P Vac (inches H$_2$O) | Total Flow (LPM) | Bypass $\phi$ (inches) |
|---|---|---|---|---|---|
| .0045" Brown | 15.4 | 4.9 | 2.11 | 20.03 | .149 |
| .0045" Brown | 18.6 | 5.6 | 3 | | .149 |
| .0045" Brown | 21.5 | 6.39 | 4.2 | | .149 |
| .0045" Brown | 24.2 | 6.94 | 5.5 | | .149 |
| .0045" Brown | 28.75 | 7.62 | 8 | | .149 |
| .0045" Brown | 31.7 | 7.9 | 9.6 | | .149 |
| .0045" Brown | 34.6 | 8.2 | 11.3 | | .149 |
| .0045" Brown | 38.2 | 8.5 | 14 | | .149 |
| Green | 9.5 | 1.99 | .3 | | .199 |
| | 17.08 | 3.49 | .93 | | .199 |
| | 24.80 | 4.39 | 2.0 | | .199 |
| | 31.7 | 4.80 | 3.2 | | .199 |
| | 38.2 | 5.0 | 4.7 | | .199 |
| | 44.2 | 5.11 | 6.3 | | .199 |
| | 49.4 | 5.18 | 8.2 | | .199 |
| | 53 | 5.10 | 9.8 | | .199 |

| Valve Slot Size (inches) | Bypass $\phi$ (inches) | Bypass Flow (LPM) | Primary Flow (LPM) | $\triangle$ P Vac (inches H$_2$O) | Valve Material |
|---|---|---|---|---|---|
| .300 | .199 | 6.0 | 2.9 | .1 | Green |
| .300 | .199 | 9.2 | 4.2 | .28 | Green |
| .300 | .199 | 14.1 | 6.2 | .65 | Green |
| .300 | .199 | 17.5 | 7.4 | .99 | Green |
| .300 | .199 | 24.4 | 7.6 | 1.9 | Green |
| .300 | .199 | 28.9 | 7.5 | 2.7 | Green |
| .300 | .199 | 33.9 | 6.3 | 3.7 | Green |
| .300 | .199 | 38.0 | 5.46 | 4.8 | Green |
| .300 | .199 | 46.7 | 4.76 | 7.5 | Green |
| .300 | .199 | 50.3 | 4.6 | 8.5 | Green |
| .300 | .199 | 54 | 4.6 | 9.8 | Green |
| .300 | 1.99 | 5.9 | 2.6 | .1 | Brown |
| .300 | 1.99 | 7.9 | 3.6 | .2 | Brown |
| .300 | 1.99 | 11.8 | 5.4 | .45 | Brown |
| .300 | 1.99 | 17.7 | 7.9 | 1.0 | Brown |
| .300 | 1.99 | 23.9 | 10.48 | 1.9 | Brown |
| .300 | 1.99 | 28.59 | 11.76 | 2.7 | Brown |
| .300 | 1.99 | 33.2 | 11.9 | 3.7 | Brown |
| .300 | 1.99 | 38.5 | 10.9 | 5.0 | Brown |
| .300 | 1.99 | 42.8 | 10.3 | 6.0 | Brown |
| .300 | 1.99 | 45.5 | 10.2 | 6.8 | Brown |
| .300 | 1.99 | 48.6 | 9.6 | 7.9 | Brown |
| .300 | 1.99 | 49.5 | 9.7 | 8.3 | Brown |

**Table 7**: Re-lay out of valve with 3.8 radius and smaller slot (device of **FIG. 29**).

| Bypass $\phi$ (inches) | | Primary Slot Size (inches) | Bypass Flow (LPM) | Primary Flow (LPM) | $\Delta$P Vac (inches H$_2$O) | Flap Material (Color) |
|---|---|---|---|---|---|---|
| .265 | | .04 × .150 | 8.75 | .65 | .13 | Brown |
| .265 | | .04 × .150 | 12.5 | .95 | .23 | Brown |
| .265 | | .04 × .150 | 18.0 | 1.4 | .45 | Brown |
| .265 | | .04 × .150 | 40.3 | 3.14 | 2.02 | Brown |
| .265 | | .04 × .150 | 25.0 | 1.99 | .84 | Brown |
| .265 | | .04 × .150 | 64.0 | 4.5 | | Brown |
| .199⌀ Equivalent (EQUI) SLOT | | .04 × .150 | 18.7 | 2.82 | 1.38 | Green |
| .199⌀ SLOT | EQIU | .04 × .150 | 21.8 | 3.19 | 1.8 | Green |
| .199⌀ SLOT | EQIU | .04 × .150 | 25.5 | 3.68 | 2.54 | Green |
| .199⌀ SLOT | EQIU | .04 × .150 | 29.5 | 4.07 | 3.26 | Green |
| .199⌀ SLOT | EQIU | .04 × .150 | 34.1 | 4.45 | 4.19 | Green |
| .199⌀ SLOT | EQIU | .04 × .150 | 38.7 | 4.75 | 5.21 | Green |
| .199⌀ SLOT | EQIU | .04 × .150 | 43.3 | 4.88 | 6.2 | Green |
| .199⌀ SLOT | EQIU | .04 × .150 | 46.2 | 4.97 | 7.0 | Green |
| .199⌀ SLOT | EQIU | .04 × .150 | 54.1 | 4.79 | 9.12 | Green |
| .199⌀ SLOT | EQIU | .04 × .150 | 55.0 | 4.69 | 9.9 | Green |
| .199⌀ SLOT | EQIU | .04 × .150 | 19.8 | 1.05 | 1.5 | .001 KAPTON |
| .199⌀ SLOT | EQIU | .04 × .150 | 28.6 | 1.37 | 3.17 | .001 KAPTON |
| .199⌀ SLOT | EQIU | .04 × .150 | 35.7 | 1.10 | 4.56 | .001 KAPTON |
| .199⌀ SLOT | EQIU | .04 × .150 | 41.7 | .97 | 5.8 | .001 KAPTON |
| .199⌀ EQIU SLOT | | .04 × .150 | 46.7 | .94 | 7.1 | .001 KAPTON |
| .199⌀ EQIU SLOT | | .04 × .150 | 60.8 | .94 | 11.5 | .001 KAPTON |

| Bypass $\phi$ (inches) | | Primary Slot Size (inches) | Bypass Flow (LPM) | Primary Flow (LPM) | $\Delta$P Vac (inches H$_2$O) | Valve Material |
|---|---|---|---|---|---|---|
| .199 "SLOT" | | .040 × .275 | 16.7 | 1.79 | 1.08 | .001 KAPTON |
| .199 "SLOT" | | .040 × .275 | 18.1 | 1.87 | 1.3 | .001 KAPTON |
| .199 "SLOT" | | .040 × .275 | 25.3 | 2.12 | 3.48 | .001 KAPTON |
| .199 "SLOT" | | .040 × .275 | 35.7 | 2.7 | 4.6 | .001 KAPTON |
| .199 "SLOT" | | .040 × .275 | 43.5 | 2.8 | 6.4 | .001 KAPTON |
| .199 "SLOT" | | .040 × .275 | 50.2 | 2.8 | 8.34 | .001 KAPTON |
| .199 "SLOT" | | .040 × .275 | 54.0 | 2.72 | 9.67 | .001 KAPTON |
| .199 "SLOT" VALVE REVERSED | | .040 × .275 | 56.3 | 2.64 | 10.4 | .001 KAPTON |
| .199 "SLOT" | | .040 × .275 | 19.4 | 1.5 | 1.45 | .001 KAPTON |
| .199 "SLOT" | | .040 × .275 | 24.8 | 1.89 | 2.3 | .001 KAPTON |
| .199 "SLOT" | | .040 × .275 | 36.2 | 2.36 | 4.7 | .001 KAPTON |
| .199 "SLOT" | .040 × .275 | 41.3 | 2.5 | | 5.8 | .001 KAPTON |

(continued)

VALVE REVERSED

| | | | | | | |
|---|---|---|---|---|---|---|
| .199 "SLOT" | .040 × . 275 | 50.4 | 2.6 | | 8.3 | .001 KAPTON |
| .199 "SLOT" | .040 × . 275 | 55.9 | 2.6 | | 9.6 | .001 KAPTON |

RETEST

| | | | | | | |
|---|---|---|---|---|---|---|
| .199 "SLOT" | .040 × . 275 | 12.4 | 1.56 | | 0.6 | .001 KAPTON |
| .199 "SLOT" | .040 × . 275 | 21.1 | 1.65 | | 1.71 | .001 KAPTON |
| .199 "SLOT" | .040 × . 275 | 30.2 | 2.0 | | 3.4 | .001 KAPTON |
| .199 "SLOT" | .040 × . 275 | 41.5 | 2.08 | | 6.0 | .001 KAPTON |
| .199 "SLOT" | .040 × . 275 | 50.1 | 2.03 | | 8.4 | .001 KAPTON |
| .199 "SLOT" | .040 × . 275 | 57.5 | 1.65 | | 11.0 | .001 KAPTON |
| .199 "SLOT" | .040 × . 275 | 46.0 | 1.64 | | 7.5 | .001 KAPTON |
| .199 "SLOT" | .040 × . 275 | 33.7 | 1.55 | | 4.32 | .001 KAPTON |
| .199 "SLOT" | .040 x.275 | 19.5 | 1.36 | | 1.48 | .001 KAPTON |
| .199 "SLOT" | .040 × . 275 | 30.0 | 1.76 | | 9.39 | .001 KAPTON |

**Example 6: Particle size diameters of aerosols generated from devices comprising wire coil heater elements and bypass inlets.**

This example describes the particle size diameters (PSD) of aerosols generated from a device comprising a heater element comprising a wire coil. An example of this type of device is shown in **FIGs. 31A-D. FIG. 31A** depicts a device designated ENT-100-A, (two inches (about 50.8 mm) long) comprising a primary carrier gas inlet (**3112a**), positive and negative brass contacts (**3110a**), a heater element (**3106a**) comprising a coil located distally from the inlet to the primary airway (**3112a**) and two bypass inlets (**3104a**) located (disposed) downstream of the heater element but prior to the outlet (**3102a**). **FIG 31B** depicts a device designated ENT-100-B, which was the same as ENT-100-A except that the heater element had been moved to be proximal to the inlet of the primary airway (**3112b**). **FIG. 31C** depicts a device designated ENT-100-C, which was similar to the ENT-100-A device except that the wire coil heater element had been moved to an intermediate position relative to the location of the coil in ENT-100-A and ENT-100-B. Any of the devices depicted in **FIG. 31A-C** could have comprised the wire coil heater element designated "A Coil" (**3114e**) or "B Coil" (**3116e**) as illustrated in **FIG. 31E.** The coil in both types of heater elements comprised inner diameter of 0.26 inches (about 6.604 mm). The "A Coil" comprised a stretch of coil followed by a straight lead on either end of the coil which connected to the brass contacts. The "B Coil" comprised a stretch of coil, wherein the coil itself connected to the brass contacts.

**Tables 8-12** shows the particle size diameter of the aerosols generated from the devices depicted in **FIG. 31A-C. Table 8** shows the PSD of particles generated using an ENT-100-A device with the "B Coil". **Table 9** shows the PSD of particles generated using an ENT-100-B device with the "A Coil". **Table 10** shows the PSD of particles generated using an ENT-100-B device with the "B Coil". **Table 11** shows the PSD of particles generated using an ENT-100-C device with the "A-Coil". **Table 12** shows the PSD of particles generated using an ENT-100-C device with the "B-Coil".

**Table 8**: Testing of ENT-100-A,B prototype

| Dose = 2 mg (propylene glycol formulation), current = 3 amps, duration = 1 sec. | | | | |
|---|---|---|---|---|
| **Total Flow (LPM)** | **Primary Flow (LPM)** | **Bypass Flow (LPM)** | **PSD (microns)** | **Notes** |
| 9.7 | N/A | N/A | 1.7-1.8 | ENT-100-A Device |
| 9.7 | N/A | N/A | 1.5 - 2.1 | |
| 2.2 | 1.67 | | 0.4 - 0.5 | ENT-100-A Device w/o screen in flow valve |
| 2.2 | 1.67 | | 0.38 - 0.5 | |
| 2.2 | .7 | | 1.7-1.5 | |
| 2.2 | 2.3 | | 0.4 | w/screen |
| 32 | 1.6 | N/A | 0.4 | ENT-100-B (heater coil moved aft) |
| Ø | 0.7 | N/A | 1.7-2.0 | |
| Ø | 0.66 | N/A | 1.4-1.5 | |
| | 1.7 | Ø | 0.5 - 1.0 | Bypass taped over ENT-100-B |
| | 1.7 | Ø | 0.5 - 1.0 | Bypass taped over ENT-100-B |
| | 1.7 | Ø | 0.5 - 1.0 | Bypass taped over ENT-100-B |
| | 1.7 | Ø | 0.5 - 1.0 | Bypass taped over ENT-100-B |
| | 0.5 | Ø | 3 | Bypass taped over ENT-100-B |
| | 0.51 | Ø | 2.9 | Bypass taped over ENT-100-B |
| | .82 | Ø | 3.3/1.8 | Bypass taped over ENT-100-B |
| | .84 | Ø | 3.2 - 3.3 | Bypass taped over ENT-100-B |
| | 1.1 | Ø | 2.7 | Bypass taped over ENT-100-B |
| | 1.11 | Ø | 2.7 - 2.8 | Bypass taped over ENT-100-B |
| | 1.38 | Ø | 2.1 - 2.3 | Bypass taped over ENT-100-B |
| | 1.42 | Ø | 2.2 - 2.4 | Bypass taped over ENT-100-B |
| | 1.72 | Ø | 1.7 | Bypass taped over ENT-100-B |
| | 1.72 | Ø | 1.7-1.75 | Bypass taped over ENT-100-B |
| | 2.04 | Ø | .5 - 1.0 | Bypass taped over ENT-100-B |

(continued)

| Primary Flow (LPM) | Bypass Flow (LPM) | PSD (microns) | Notes |
|---|---|---|---|
| 1.45 | Ø | 2.3 | ENT-100-B Device Flap removed from flow valve |
| 1.45 | Ø | 2.2 - 2.4 | |
| 1.74 | Ø | 1.95-2.0 | |
| 1.75 | Ø | 1.8-1.9 | |
| 2.04 | Ø | 1.7-1.8 | |
| 2.04 | Ø | 1.6-1.7 | |
| 3.0 | Ø | 0.5 - 1.0 | |
| 3.0 | Ø | 0.5 - 1.0 | |
| 3 | Ø | 0.5 - 1.0 ST | Flow control valve removed/replaced with Black Delyrn W O.196$\phi$ hole |
| 3 | Ø | 2.0-2.3 | |
| 3 | Ø | 2.3 - 2.4 | |
| 1.04 | Ø | No trigger | |
| 2.0 | Ø | 3.8 | |
| 2.04 | Ø | 0.5 - 1.0 | With foam (open cell packing foam used to even out air flow, placed upstream from the heater element), no valve |
| 2.04 | Ø | 0.5 - 1.0 ST | |
| 1.05 | Ø | 1.8 - 2.1 | |
| 1.05 | Ø | 2.0 - 2.1 | |
| 1.5 | Ø | .79 - 1.0 | |
| 1.49 | Ø | 1.6 | |
| 1.25 | Ø | 1.6 | |
| 1.24 | Ø | 0.7 - 1.2 | |
| 1.24 | Ø | 0.7 - 1.2 | |
| 2.0 | Ø | 0.5 - 1.0 | |
| 2.0 | Ø | 0.5 - 1.0 | |

**Table 9:** Testing of ENT-100-B device with "A Coil" heater element

| Dose = 2 mg (propylene glycol formulation), 1 sec duration, current 3.1 amps | | |
|---|---|---|
| **Flow (LPM)** | **PSD (Microns)** | **Notes** |
| 1.01 | 3.4 - 3.6 | |
| 1.01 | 3.1 -3.5 | |
| 1.51 | 2.6 - 2.7 | |
| 1.51 | 2.5 - 2.7 | |
| 2.06 | 2.6 - 2.3 | |
| 2.12 | 2.15-2.2 | |
| 2.48 | 1.9-2.2 | |
| 2.49 | 1.85 - 1.9 | |
| 3.02 | 1.5-1.6 | |
| 3.02 | 1.4-1.5 | |
| 3.02 | 1.35-1.45 | |
| 3.04 | 1.45-1.6 | |
| 3.26 | 1.4-1.6 | |
| 3.27 | 1.3-1.5 | |
| 4.25 | | |

**Table 10:** Testing of ENT-100-B device with "B Coil" heater element

| Dose = 2 mg (propylene glycol formulation), Duration 1 sec, curren 2.0 amps | | | |
|---|---|---|---|
| **Dose (mg)** | **Flow (LPM)** | **PSD (microns)** | **Notes** |
| 2 | 1.5 | 2.9 - 3.1 | With foam |
| 2 | 1.53 | 2.6 - 2.8 | |
| 2 | 1.53 | 2.8 - 2.9 | |
| 2 | 2.49 | 1.8-1.9 | |
| 2 | 2.49 | 1.7-1.8 | |
| 2 | 3.01 | 1.4 | |
| 2 | 3.01 | 1.4-1.5 | |
| 2 | 3.49 | | |
| 2 | 1.55 | 2.5 | With stainless steel (SS) screen to even flow Taped up bypass |
| | 1.56 | 2.6 - 2.9 | |
| | 1.56 | 2-2.5 | |
| | 2.52 | 1.5-1.6 | |
| | 2.56 | 1.5 | |
| | 2.35 | 1.8 - 2.0 | With foam (taped up bypass) |
| | 2.51 | 1.9 - 2.0 | |
| | 2.48 | 1.9 | |
| | 1.48 | 2.9 - 3.0 | |
| | 1.50 | 2.8 - 3.0 | |
| | 1.5 | 1.8-1.9 | Bypass untaped Total flow ~ 8.5 LPM |
| | 1.52 | 1.7-1.8 | |
| | 1.48 | 1.2 - 1.1 | With 0.42 φ orifice added to primary inlet (Total flow = 24) |
| | 1.5 | 1.7-1.8 | With heater element moved aft |
| | 1.60 | 1.7-1.75 | - B configuration (Total flow 12 LPM) |

**.Table 11:** Testing of ENT-100-C with "A Coil" heater element, which has 7 coils

| Current set @ 2.0 amps, 1 sec, 2 mg dose (propylene glycol formulation) | | | | |
|---|---|---|---|---|
| **Inlet orifice (inches)** | **Primary Flow (LPM)** | **PSD (microns)** | **ΔP Vac (inches H$_2$O)** | **Notes** |
| .04 | 1.01 | 4.6 - 5 | 2.48 | No adder |
| .04 | 1.00 | 4.3 - 4.7 | 2.50 | 0.250 straight tube |
| .04 | 3.00 | 1.7 - 1.8 | 17.5 | 2.4 amps |
| .04 | 3.00 | 1.6 - 1.7 | 17.2 | 2.4 amps |
| .04 | 4.85 | ~ 1.0 | LIMIT | |
| .020 + FOAM | 0.98 | 2.2 - 2.4 | .45 | 2.4 amps - No adder |
| .020 + FOAM | 1.00 | 3.5 - 4.0 | .46 | 2.4 amps - No adder |
| .020 + FOAM | 1.00 | 4.2 - 4.7 | .46 | 2.4 amps - No adder |
| .020 + FOAM | 1.00 | 4.0 - 5.7 | .46 | 2.4 amps - No adder |
| .020 + FOAM | 1.00 | 3.0 - 4.3 | .46 | 2.4 amps - No adder |
| .020 + FOAM | 2.09 | 2.2 | 1.52 | 2.4 amps - No adder |
| .020 + FOAM | 2.07 | 2.4 - 2.5 | 1.51 | 2.4 amps - No adder |
| .020 + FOAM | 2.07 | 2.2 - 2.4 | 1.48 | 2.4 amps - No adder |
| .020 + FOAM | 2.08 | 2.4 - 2.5 | 1.53 | 2 amps |
| .020 + FOAM | 2.08 | 2.1 - 2.3 | 1.53 | 2 amps |
| .020 + FOAM | 2.09 | 2.5 - 2.6 | 1.53 | 2 amps |

**Table 12:** Testing of ENT-100-C with "B Coil" heater element, with 0.050 spacer between contacts then spread to .200 in

| Current set @ 2.0 amps, 1 sec, 2 mg dose (propylene glycol formulation) | | | | |
|---|---|---|---|---|
| **Flow (LPM)** | **PSD (microns )** | **△P Vac (inches H$_2$O)** | **Current (amps)** | **Notes** |
| .94 | 3.0-3.2 | .67 | 2.4 | |
| .94 | 2.4 - 2.5 | .67 | 2.8 | |
| .95 | 2.5 - 3.1 | .67 | 2.8 | |
| .95 | 3.3 - 3.4 | .67 | 2.8 | |
| .95 | 2.7 - 3.4 | .67 | 2.8 | |
| 2.11 | 2.3 - 2.4 | 2.58 | 2.8 | |
| 2.11 | 2.3 - 2.7 | 2.58 | 2.8 | |
| 2.11 | 2.6 - 2.7 | 2.58 | 2.8 | |
| New Heater Element .040 ID | | | | |
| 1.91 | 1.7 - 2.0 | .86 | 2.4 | |
| 1.91 | 2.4 - 2.5 | .86 | 2.6 | |
| 1.97 | 2.6-2.7 | .86 | 2.6 | |
| 1.91 | 2.4 - 2.5 | .86 | 2.6 | |
| 1.91 | 2.5 - 2.6 | .86 | 2.6 | |
| 1.91 | 2.4 - 2.5 | .86 | 2.8 | |
| 2.04 | 1.8 - 2.0 | .96 | 2.8 | |
| 2.04 | 2.4 - 2.7 | .96 | 2.8 | |
| 2.04 | 2.0 - 1.9 | .96 | 2.8 | |
| New Heater Element .032 ID 0.100 stretch | | | | |
| 2.04 | 2.0 - 2.5 | .93 | 2.6 | |
| 2.04 | 2.0 - 2.2 | .96 | 2.6 | |
| 2.04 | 2.1 - 2.3 | .96 | 2.6 | Spit (nicotine/ propylene glycol was heated under conditions (air flow, heating rate) that lead to the mixture being boiled off of the heater element and "spit" off of the heater element) |
| 2.04 | 2.1 - 2.2 | .89 | 2.6 | spit |

**Example 7: Particle size diameters of aerosols generated from heater element comprising a center exit wire lead.**

This example describes the particle size diameters (PSD) of aerosols generated from a heater element comprising a wire wherein one end of the wire wrapped around another segment of the wire, wherein a wire coil was formed with an end of the wire passing through the center of the wire coil. An example of this type of heater element is shown in **FIGs 36, 37A-B,** and **38.** In this example, the heater element was inserted into the device depicted in **FIG. 31D. FIG. 31D** depicts a device designated ENT-100-D with a primary passageway for air to flow through, brass contacts (+/-) embedded within the wall of the primary passageway, and a heater element as described in this example. The wire of the heater element had a diameter of 0.10 inches (about 2.54 mm). The wire coil of the heater element had 9 coils, and the wire coil had an inner diameter of 0.032 inches (about 0.813 mm). In this example, the liquid formulation comprised propylene glycol and it wicked onto the ends of the wire of the heater element and onto the brass contacts. **Table 13** shows the particle size diameter of the aerosols generated from a device comprising the heater element. As shown in **Table 13,** the particle size distribution of aerosols generated by devices with the heater element was unaffected by alterations in current used to heat the wire.**Table 13:** Propylene glycol (dose: 2 mg) was found to wick to ends of heater element and onto brass contacts ENT-100-D.

Heater Element: .032 10, 010 Øwire, 9 turn, center exit

| Flow (LPM) | PSD (microns ) | $\Delta$P Vac (inches $H_2O$) | Current (amps) | Notes |
|---|---|---|---|---|
| 2.01 | 2 - 2.2 | 1.14 | 2.2 | Foam |
| 2.00 | 2 - 2.2 | 1.14 | 2.2 | |
| 2.00 | 2.0-2.2 | 1.14 | 2.0 | |
| 2.0 | 2.1 - 2.2 | 1.14 | 2.0 | |
| 2.0 | 1.8 - 2.1 | 1.14 | 1.8 | |
| 2.0 | 1.9 - 2.1 | 1.14 | 1.8 | |
| 0.99 | 5.0-5.3 | .34 | 1.8 | |
| 1.00 | 5.0-5.2 | .34 | 1.8 | |
| 1.52 | 2.6-2.8 | .71 | 2.0 | |
| 1.52 | 2.6-2.7 | .71 | 2.0 | |
| 1.53 | 2.4 - 2.7 | .71 | 1.8 | |
| 1.53 | 2.5-2.7 | .71 | 1.8 | |
| 2.02 | 2.1 - 2.2 | | 2.0 | |
| 3.0 | 1.2 - 1.4 | 2.43 | 2.0 | |
| 3.0 | 0.8 - 1.4 | 2.43 | 2.0 | |
| 3.0 | 90-1.3 | 2.43 | 2.2 | |
| 3.0 | .6 - 1.3 | 2.43 | 2.2 | |

**Example 8: Particle size diameters of aerosols generated from heater element comprising a center exit wire lead when the length of the leads are increased.**

This example describes the particle size diameters (PSD) of aerosols generated from a heater element as described in **FIG. 36**. In this example, the length of the leads connecting the wire coil to the brass contacts was increased as shown in **FIGs. 37A** and **37 B**. The length of the leads in this example was 0.70 inches (about 17.78 mm). The heater element was inserted into the device depicted in **FIG. 31D. FIG. 31D** depicts a device designated ENT-100-D with a primary passageway for air to flow through, brass contacts (+/) embedded within the wall of the primary passageway, and a heater element as described in this example. In some cases, the diameter of the inlet was varied from 0.060 inches to either 0.070, 0.071, or 0.041 inches (a range from about 1.524 mm to either 1.78, 1.80, or 1.04 mm. The wire of the heater element had a diameter of 0.10 inches (about 0.254 mm). The wire coil of the heater element had a reduced number of coils, and the wire coil had an inner diameter of 0.032 inches (about 0.813 mm) . In this example, the liquid formulation comprised propylene glycol and it wicked onto the ends of the wire of the heater element and onto the brass contacts. **Table 14** shows the particle size diameter of the aerosols generated from a device comprising the heater element. As shown in **Table 14,** the particle size distribution of aerosols generated by device with the heater element was unaffected by alterations in current used to heat the wire. **Table 14** also shows the effects of altering the airway configuration in the ENT-100-D device. As shown in **Table 14**, altering the configuration of the airway of the ENT-100-D device by adding the airway depicted in **FIG. 32E** (designated the MARK V adders in **Table 14**) downstream of the heater element produced particles with a PSD of about 1 to about 2 $\mu$m.

**Table 14: Heater element leads lengthened**

| Flow (LPM) | PSD (microns ) | $\Delta$P Vac (inches $H_2O$) | Current (amps) | Notes |
|---|---|---|---|---|
| 2.0 | 3.1 - 3.2 | .96 | 2.0 | |
| 2.0 | 3.1 - 3.2 | .96 | 2.0 | |
| 2.01 | 3.1 - 3.2 | .96 | 1.8 | |
| 2.01 | 3.1 - 3.2 | .96 | 1.8 | |
| 2.02 | 3.0-3.2 | .96 | 2.2 | Orifice .060 |
| 2.02 | 2.9-3.0 | .96 | 2.2 | |
| Test of $\Delta$P affecting PS D | | | | |
| 2.06 | 3.3 - 3.4 | 1.74 | 2.0 | Orifice size = .060 |
| 2.04 | 3.2 - 3.3 | .96 | 2.0 | .071 |
| 2.04 | 3.0-3.2 | 7.00 | 2.0 | .041 |
| 2.04 | 3.1 - 3.2 | 7.08 | 2.0 | .041 |

(continued)

| Flow (LPM) | PSD (microns ) | $\Delta$P Vac (inches H$_2$O) | Current (amps) | Notes |
|---|---|---|---|---|
| Test to see affect of foam | | | | |
| 2.06 | 2.4 - 2.5 | 6.65 | 2.0 | Foam removed |
| 2.06 | 2.4 - 2.5 | 6.65 | 2.0 | |
| 2.0 | 2.7 - 2.9 | 1.63 | 2.0 | Original foam |
| 2.05 | 2.7 - 2.8 | 1.63 | 2.0 | Replaced orifice .070 |
| 2.05 | 2.7 - 2.8 | 1.70 | 2.0 | New foam |
| 2.06 | 2.7 | 1.70 | 2.0 | |
| 2.06 | 2.9-3.0 | 1.05 | 2.0 | New foam rotated 90° |
| 2.04 | 2.7 - 2.9 | .98 | 2.0 | |

| Flow (LPM) | PSD (microns ) | $\Delta$P Vac (inches H$_2$O) | Current (amps) | Notes |
|---|---|---|---|---|
| 2.0 | 2.6 | 1.47 | 2 | Foam rotated |
| 2.0 | 2.6 | 1.47 | 2 | again 90° |
| Foam replaced w/SS screen | | | | |
| 2.05 | 2.6 - 2.8 | .63 | 2 | |
| 2.04 | 2.7 - 3.0 | .63 | 2 | |
| 2.04 | 2.8 - 3.0 | .63 | 2 | |
| 2.06 | 2.8 - 3.0 | .65 | 2 | New screen |
| 2.06 | 3.0-3.1 | .65 | 2 | |
| New heater element | | | | |
| 2.03 | 3.0 - 3.2 | .62 | 2 | |
| 2.04 | 2.7 - 2.8 | .62 | 2 | |
| 2.04 | 2.7 - 2.8 | .62 | 2 | |
| 2.04 | 2.9 - 3.0 | .62 | 2 | |
| 2.50 | 2.7 - 2.9 | .9 | 2 | |
| 2.50 | 2.4 - 2.6 | .9 | 2 | |
| 2.54 | 2.6 - 2.8 | .9 | 2 | |
| 2.54 | 2.6 - 2.9 | .9 | 2 | |
| 3.52 | 1.9 | 1.60 | 2 | |
| 3.51 | 2.1 | 1.60 | 2 | |
| 4.53 | 1.8-1.9 | 2.54 | 2 | |
| 4.51 | 1.8-1.9 | 2.54 | 2 | |
| Heater element broke | | | | |
| 2.02 | 2.8 - 3.0 | .61 | 2 | Heater replaced |
| 4.52 | 1.9 | 2.53 | 2 | |
| 4.53 | 1.9 | 2.53 | 2 | |
| 6.10 | 1.3-1.5 | 4.33 | 2 | |
| 6.10 | 1.4-1.5 | 4.35 | 2 | |
| 7.03 | 1.1 - 1.2 | 5.68 | 2 | |

(continued)

| Flow (LPM) | PSD (microns ) | ∆P Vac (inches H$_2$O) | Notes | |
|---|---|---|---|---|
| 1.48 | 2.8-3 | .34 | | |
| 1.48 | 3.2 - 2.4 | .34 | | |
| 1.48 | 2.6 - 2.9 | .34 | | |
| 1.48 | 2.4 - 2.7 | .34 | | |
| 2.04 | 3-3.2 | .62 | | |
| 2.04 | 3-3.2 | .62 | | |
| .95 | 3.9 - 4.2 | 0.14 | | |
| .95 | 3.9 - 4.2 | 0.14 | | |
| | | | Bypass | Adder used (Mark V) |
| 2.08 | 1.4-1.8 | 1.06 | 14.9 | |
| 2.08 | 1.9 - 2.1 | 1.06 | 14.9 | |
| 2.08 | 2.0 - 2.1 | 1.06 | 14.9 | |
| 2.08 | 2.0 - 2.1 | 1.06 | 14.9 | |
| 3.02 | 1.7-1.8 | 2.06 | 21.0 | |
| 3.02 | 1.8 | 2.06 | 21.09 | |
| 4.48 | 1.3-1.4 | 4.22 | 30.4 | |
| 4.48 | 1.2 - 1.4 | 4.22 | 30.1 | |
| 2.0 | 1.9 - 2 | 1.08 | 0 | Flow meter taped up on bypass |
| 2.0 | 2 | 1.08 | 0 | |
| 2.0 | 2.4 - 2.5 | 1.08 | 0 | |
| 2.01 | 2.2 - 2.3 | 1.08 | 0 | |

**Example 9: Particle size diameters of aerosols generated from heater element comprising a center exit wire lead when the length of the leads are decreased.**

This example describes the particle size diameters (PSD) of aerosols generated from a heater element as described in **FIG. 36.** In this example, the length of the leads connecting the wire coil to the brass contacts was 0.30 inches (about 0.762 mm). The heater element was inserted into the device depicted in **FIG. 31D. FIG. 31D** depicts a device designated ENT-100-D with a primary passageway for air to flow through, brass contacts (+/) embedded within the wall of the primary passageway, and a heater element as described in this example. The wire of the heater element had a diameter of 0.10 inches (about 2.54 mm). The wire coil of the heater element had an increased number of coils relative to Example 8, and the wire coil had an inner diameter of 0.032 inches (about 0.813 mm). In this example, the liquid formulation comprised propylene glycol and it wicked onto the ends of the wire of the heater element and onto the brass contacts. The dose of the formulation was 2 mg. **Table 15** shows the particle size diameter of the aerosols generated from the device described in this example. As shown in **Table 15,** the particle size diameter distribution of aerosols generated by this device was unaffected by alterations in current used to heat the wire.

**Table 15:** Testing using ENT-100-D (side mount) (w/bottom leads) with leads shortened.

| | 2 mg, current 2.00 amps (U.N.O.) | | |
|---|---|---|---|
| **Dose Primary Flow (LPM)** | **PSD (microns)** | **∆P Vac (inches H$_2$O)** | **Current (amps)** |
| 2.02 | 3.0 - 3.2 | .62 | 2.0 |
| 2.02 | 2.9 - 3.2 | .62 | 2.0 |
| 1.48 | 2.3 - 2.5 | .37 | 2.0 |
| 1.48 | 2.0 - 2.4 | .37 | 2.0 |
| 1.48 | 2.0 - 2.6 | .37 | 1.8 |
| 1.48 | 2.0 - 2.5 | .37 | 1.8 |
| 1.10 | 2.8-4.1 | .20 | 1.8 |
| 1.10 | 2.3-3.4 | .20 | 1.8 |
| 2.0 | 3.1 - 3.2 | .62 | 2.0 |

(continued)

| | 2 mg, current 2.00 amps (U.N.O.) | | |
| Dose Primary Flow (LPM) | PSD (microns) | $\Delta$P Vac (inches H$_2$O) | Current (amps) |
|---|---|---|---|
| 2.12 | 2.2 | 1.16 | 2.0 |
| 2.12 | 2.2 | 1.16 | 2.0 |
| 1.01 | 2.8 | .30 | 1.8 |
| 1.01 | 2.8 - 3.0 | .30 | 1.8 |
| .49 | 4.7 - 5.4 | .08 | 1.8 |
| .49 | 4.5 - 4.8 | .09 | 1.8 |
| 4.50 | 1.4-1.6 | 4.14 | 2.0 |

**Example 10: Particle size diameters of aerosols generated from a device comprising a heater element comprising a center exit wire lead.**

This example describes the particle size diameters (PSD) of aerosols generated from a device comprising a heater element as described in **FIG. 36.** In this example, the heater element was inserted into the device depicted in **FIG. 31D. FIG. 31D** depicts a device designated ENT-100-D with a primary passageway for air to flow through, brass contacts (+/-) embedded within the wall of the primary passageway, and a heater element as described in this example. The wire of the heater element had a diameter of 0.10 inches (about 2.54 mm). The wire coil of the heater element had an inner diameter of 0.032 inches (about 0.813 mm). In this example, the liquid formulation comprised propylene glycol and it wicked onto the ends of the wire of the heater element and onto the brass contacts. The dose of the formulation in this example was 2 mg. **Table 16** shows the particle size diameter of the aerosols generated from a device comprising the heater element described in this example. As shown in **Table 16,** the particle size distribution of aerosols generated by devices with the heater element was unaffected by alterations in current used to heat the wire. Also as shown in **Table 16,** altering the configuration of the airway of the ENT-100-D device by adding the airway depicted in **FIG. 33** (designated the MARK VI adder in **Table 15)** downstream of the heater element produced particles with a PSD of about 1 to about 2 uM, which matched the PSD of the particles generated without the MARK VI adder. The MARK VI adder comprised a primary airway with an internal diameter of 0.25 inches (about 6.35 mm), which narrows to an airway comprising an internal diameter of 0.086 inches (about 2.18 mm)and an external diameter of 0.106 inches (about 2.69 mm).

**Table 16:** Testing of ENT-100-D device

| Dose = 2mg; Current 2 amps; 1 sec duration | | | | |
|---|---|---|---|---|
| **P Flow (LPM)** | **B Flow (LPM)** | **PSD (microns)** | **$\Delta$ PVac (inches H$_2$O)** | **Notes** |
| 1.97 | 0 | 3.0 - 3.1 | .58 | Straight tube |
| **P Flow (LPM)** | **B Flow (LPM)** | **PSD (microns)** | **$\Delta$ PVac (inches H$_2$O)** | **Notes** |
| 1.52 | 0 | 2.0-2.5 | .37 | |
| 1.52 | 0 | 2.4 | .36 | |
| 1.0 | 0 | 3.2 - 3.7 | .17 | |
| 3.0 | 0 | 2.0-2.3 | 1.21 | |
| 3.0 | 0 | 2.3 - 2.4 | 1.22 | |
| 4.53 | 0 | 1.6-1.8 | 2.52 | |
| 4.53 | 0 | 1.3-1.5 | 2.50 | |
| 6.08 | 0 | 1.2-1.3 | 4.23 | |
| 6.08 | 0 | 0.8 - 1.3 | 4.23 | |
| 6.11 | 0 | 0.7 - 1.2 (ST) | 7.13 | w/SS needle in |
| 6.11 | 0 | .6-1.2 | 7.13 | .250 tube |
| 4.48 | 0 | 1.5-1.6 | 4.14 | |
| 4.48 | 0 | 1.6-1.7 | 4.14 | |
| 3.01 | 0 | 1.7-1.9 | 2.05 | |
| 3.01 | 0 | 1.7-1.8 | 2.05 | |

(continued)

| P Flow (LPM) | B Flow (LPM) | PSD (microns) | △ PVac (inches $H_2O$) | Notes |
|---|---|---|---|---|
| 2.01 | 0 | 2.2 | 1.04 | |
| 2.01 | 0 | 2.2 - 2.7 | 1.04 | |
| 1.47 | 0 | 2.0 - 2.1 | .6 | |
| 1.47 | 0 | 2.1 | .6 | |
| 0.98 | 0 | 2.8-3.0 | .29 | |
| 0.98 | 0 | 2.7 - 3.0 | .29 | |
| .48 | 0 | 4.7 - 5.2 | .07 | |
| .48 | 0 | 4.4 - 5.1 | .07 | |
| 1.5 | 0 | 2.1 | .6 | Delrin "double cone" |
| 1.5 | 0 | 2.1 - 2.2 | .64 | |
| 2.05 | 0 | 2.3 | 1.04 | |
| 2.05 | 0 | 2.2 | 1.08 | |
| 2.5 | 0 | 2.1 - 2.2 | 1.48 | |
| 3.0 | 0 | 1.9 - 2.0 | 2.04 | |
| 3.0 | 0 | 1.9 - 2.0 | 2.04 | |
| 1.0 | 0 | 2.9-3.1 | .29 | |
| 1.24 | 0 | 2.6 - 2.7 | .43 | |
| 1.25 | 0 | 2.5 - 2.7 | .43 | |
| 1.75 | 0 | 2.3 - 2.4 | .76 | |
| 1.75 | 0 | 2.3 | .76 | |
| 1.49 | 0 | 2.1 -2.2 | .6 | Current changed to 2.2 |
| 1.49 | 0 | 2.1 - 2.2 | 2.41 | Back to 2.0 amps orifice changed |
| Adder installed | | | | .250 w/SS needle 6 slots .100 long x .080 |
| 3.0 | 21.16 | 1.8 | 1.98 | |
| 3.0 | 21.16 | 1.8-1.9 | 1.98 | 7x Adder |
| 2.0 | 14.13 | 2.0 - 2.1 | 1.0 | Mark VI |
| 2.0 | 14.13 | 2.0-2.1 | 1.0 | |
| .98 | 7.06 | 2.7 - 2.8 | .28 | |
| .98 | 7.00 | 2.8 - 2.9 | .29 | |
| 1.5 | 10.49 | 2.1 - 2.2 | .63 | |
| 1.53 | 10.62 | 2.0 - 2.2 | .63 | |
| P Flow (LPM) | B Flow (LPM) | PSD (microns) | △P Vac (inches $H_2O$) | Notes |
| .49 | 3.45 | 4.3 - 4.5 | .07 | |
| 4.51 | 31.4 | 1.5-1.6 | 4.09 | |
| 4.51 | 31.4 | 1.5-1.6 | 4.04 | |
| 6.1 | 4.2 | 1.2 | 7.0 | |
| 1.98 | 3.98 | 2.3 - 2.5 | .98 | |
| 1.98 | 3.98 | 2.3 - 2.4 | .98 | |
| 2.02 | 0 | 2.3 - 2.4 | 1.03 | |
| 2 | 28 | 2 | 3.52 | |
| 2 | 28 | 2.0 - 2.1 | 3.52 | |

**Example 11: Particle size diameters of aerosols generated from device comprising a bypass inlet for mixing the condensation aerosol in a larger volume of carrier gas.**

In this example, the particle size diameters (PSD) of a condensation aerosol generated by a device comprising the airway configuration depicted in **FIG. 33** was tested. The device comprised a primary airway with an internal diameter of 0.25 inches (about 6.35 mm), which narrowed to an airway comprising an internal diameter of 0.086 inches (about 2.18 mm) and an external diameter of 0.106 inches (about 2.69 mm). The airway confirguation was coupled to a heater element comprising a wire coil, wherein the heater element vaporized a liquid formulation comprising propylene glycol upstream of where the primary airway narrowed. The vaporized formulation then entered the narrowed airway and condensed into particles. The narrowed primary airway was designed to carry the vaporized formulation in a carrier gas (e.g. air) at a flow rate suitable for condensing the vapor into particles of a desired size (e.g. an MMAD of about 1 $\mu$m to about 5 $\mu$m). In this example, the narrowed primary airway opened up into a wide downstream airway comprising an internal diameter of 0.25 inches (about 6.35 mm) and the condensed particles were mixed with bypass carrier gas (e.g. air) that entered the widened primary airway from inlets located (disposed) in the walls of the primary airway. The carrier gas entering through the inlets was fed from a bypass inlet which was in a wall of a secondary housing that encompassed the primary airway. In this example, the effect of varying the flow rates of the bypass gas (B flow) on the PSD of the condensed was examined. **Table 17** shows the results. As shown in **Table 17,** different rates of B flow had no effect on the PSD. Moreover, the PSD at each B flow rate was between 1 $\mu$m and 3 $\mu$m. **Table 18** shows the effect on PSD of limiting the flow of bypass carrier gas through the bypass inlet on the secondary housing. The flow of bypass gas through the bypass inlet was limited by using either a valve or by altering the geometry of the orifice (i.e. forming a slot of different dimensions. As shown in **Table 18,** either the use of a valve or slot to control the flow of bypass gas was effective in producing particles with a PSD of about 1 $\mu$m to about 5 $\mu$m.

**Table 17:** Characterization of Primary Flow (P flow), Bypass Flow (B Flow), and particle size diameter of device comprising Mark VI Adder.

| P Flow (LPM) | B Flow (LPM) | PSD (microns) | $\Delta$P Vac (inches H$_2$O) | Notes |
|---|---|---|---|---|
| 1.01 | 7 | 2.7 - 2.8 | .29 | |
| 1.02 | 14.2 | 2.5-2.8 | 1.99 | |
| 1.0 | 14.03 | 2.5-2.7 | 2.11 | |

**Table 18**: Characterization of Primary Flow (P flow), Bypass Flow (B Flow), and particle size diameter of device comprising Mark VI Adder with addition of Flap valve to bypass inlet.

| P Flow (LPM) | B Flow (LPM) | $\Delta$P Vac (inches H$_2$O) | Orifice (inches) | Valve |
|---|---|---|---|---|
| 0 | 0 | 0 | .060 | Clear |
| 1.48 | .64 | 1 | .060 | Slot .080 |
| 2.20 | 1.58 | 2 | .060 | $\times$ 240 |
| 2.81 | 2.70 | 3.14 | .060 | |
| 3.23 | 3.72 | 4 | .060 | |
| 3.66 | 5.10 | 5 | .060 | |
| 4.42 | 7.3 | 7 | .060 | |
| 5.3 | 10.48 | 10 | .060 | |
| 1.48 | 4.86 | 1 | Tee slot | |
| 1.83 | 6.74 | 1.48 | | |
| 2.25 | 9.02 | 2.08 | | |
| 2.50 | 10.6 | 2.53 | | |
| 2.79 | 12.6 | 3.07 | | |
| 3.38 | 17.2 | 4.32 | | |
| 4.14 | 23.7 | 6.24 | | |
| 5.32 | 34.6 | 10.0 | | |
| 1.47 | 5.05 | 1.01 | Internal radius valve | |
| 1.86 | 6.34 | 1.51 | | |
| 2.23 | 7.7 | 2.06 | Blue material | |
| 2.52 | 8.7 | 2.56 | | |
| 1.5 | 5.75 | 1 | Internal radius | |
| 2.2 | 9.2 | 2 | Green | |
| 2.75 | 12.94 | 3 | | |

(continued)

| P Flow (LPM) | B Flow (LPM) | $\Delta$P Vac (inches H$_2$O) | Orifice (inches) | Valve |
|---|---|---|---|---|
| 3.27 | 17.5 | 4.06 | | |
| 4.2 | 26.2 | 6.4 | | |
| 5.4 | 38.7 | 10.5 | | |

**Example 12: Effects of gravity on particle size diameters of aerosols generated from an ENT-100-D device.**

In this example, the effects of gravity on the particle size diameters (PSD) of a condensation aerosol generated by an ENT-100-D device as depicted in **FIG. 31D** were tested. The ENT-100-D device was loaded with 2 mg of a liquid propylene glycol formulation and the device was rotated during the use of the device. The device was rotated 90 degrees in all dimensions from a stable baseline position. The particle size diameter was measured at each rotation and found not to change. As a result, the device produced particles of a consistent size regardless of the orientation in space of the device.

**Example 13: Study of the Safety, Tolerability, Pharmacokinetics, and Pharmacodynamics of the eNT-100 Nicotine Inhaler among Healthy Volunteer Cigarette Smokers-Part 1**

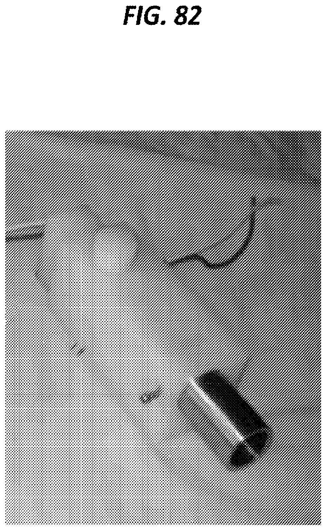

Existing electronic nicotine delivery devices tend to produce submicron particles, which have insufficient mass to settle in the deep lung, resulting in buccal delivery and slow pharmacokinetics (PK) and pharmacodynamics (PD). In contrast, 1-3 micron particles can reach the deep lung and have enough gravitational mass to settle on the alveoli, leading to rapid PK and PD effects. This example describes an ascending, placeboand vehicle-controlled, dose ranging Phase 1 study conducted to explore the tolerability, PK and PD of a novel 1-3 micron condensation aerosol of nicotine and propylene glycol (PG). In this example, Part 1 of a two-part study was conducted to examine the safety, tolerability, pharmacokinetics, and pharmacodynamics of condensation aerosol comprising nicotine produced from a liquid nicotine formulation using the ENT-100 nicotine inhaler (**FIG. 82**). The primary objectives of Part 1 were to establish the maximally tolerated dose in the range of 25-150 $\mu$g per inhalation (250-1500 $\mu$g per administration) of a condensation aerosol (i.e., 1-3 microns) comprising nicotine and propylene glycol (PG) from the eNT-100 nicotine inhaler (**FIG. 82**) when administered repreatedly (10 inhalations over 5 minutes), and to establish that use of the eNT-100 nicotine inhaler (**FIG. 82**) leads to rapid nicotine absorption with a well-tolerated dose (i.e., rapid nicotine pharmacokinetics [PK]). The secondary objectives were to: 1.) evaluate the acute tolerability and specific adverse event (AE) profile of single doses from the eNT-100 nicotine inhaler (**FIG. 82**) as compared to both placebo (air only) and a vehicle control (PG alone); 2.) evaluate the pharmacodynamics (PD) of different single doses from the eNT-100 nicotine inhaler (**FIG. 82**) in terms of their ability to reduce acute, abstinence-induced smoking urges, and also affect respiratory and other subjective sensations as compared to both placebo (air only) and a vehicle control (PG alone); 3.) evaluate the nicotine concentrations produced by single doses from the eNT-100 nicotine inhaler (**FIG. 82**) as compared to both placebo (air only) and a vehicle control; and 4.) explore the impact of inhalation on liking, statisfaction, respiratory symptoms (e.g., irritation, coughing) and craving or urge reduction The study was a single-blind, placebo and vehicle-controlled, escalating-dose design to assess the safety, tolerability, nicotine concentrations, and pharmacodynamics of a condensation aerosol comprising nicotine produced from a liquid nicotine formulation using the eNT-100 nicotine inhaler (**FIG. 82**) configured as described herein to produce a condensation aerosol of nicotine and propylene glycol (PG) of 1-3 microns. Subjects abstained from smoking for at least 12 hours prior to the experimental session. Groups of 9-12 subjects were assigned to one of seven experimental groups, based on the maximally tolerated dose (MTD) within the predetermined range of 25-150 $\mu$g of nicotine per inhalation (less than a typical cigarette inhalation per puff). Groups 1 and 2 included placebo (air only, no aerosol administration) and vehicle control (propylene glycol only) administrations, respectively. Subsequent groups were administered nicotine and PG in the predefined range of 25-150 $\mu$g nicotine per inhalation depending on the MTD.

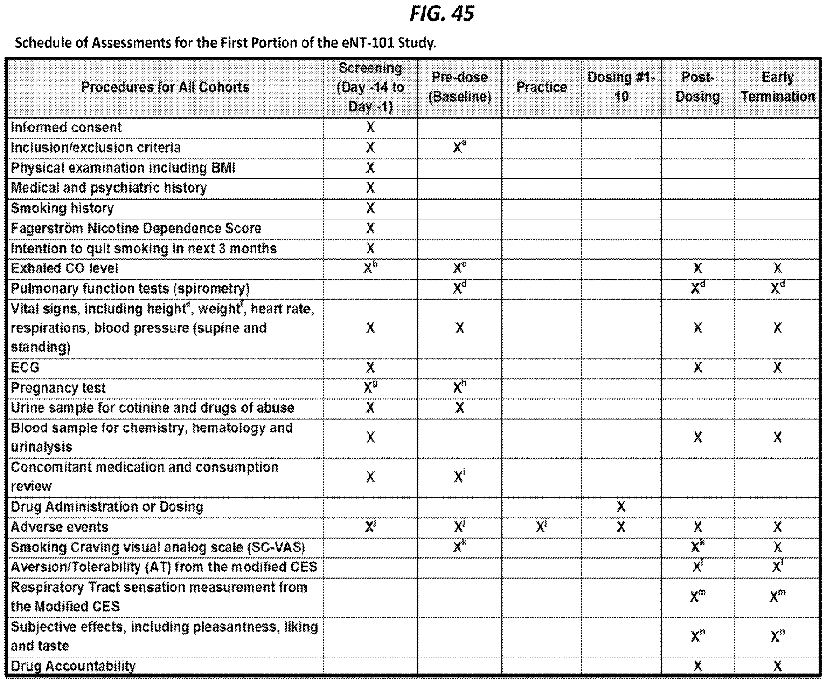

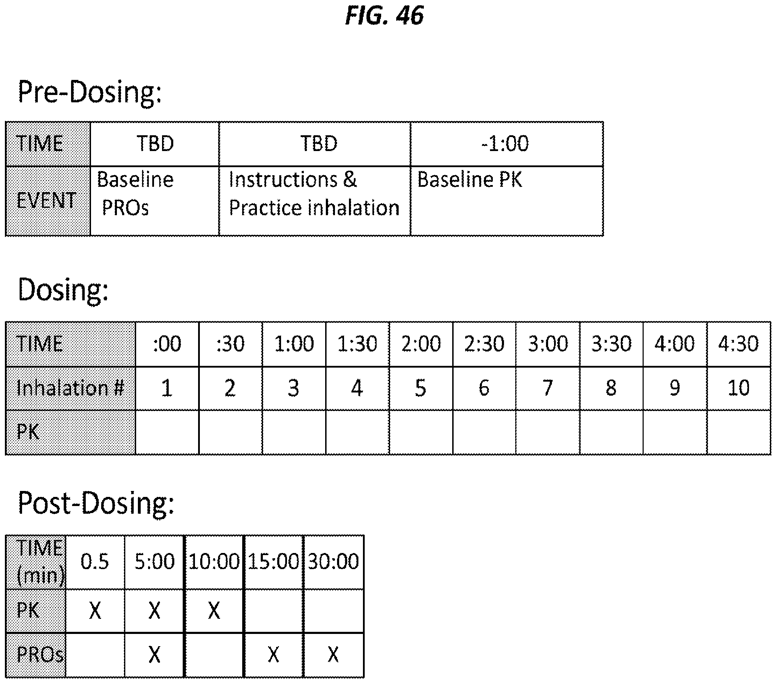

As seen in **FIG. 45**, subjects completed predose assessments of their exhaled CO, smoking urge, sampling for nicotine concentrations, spirometry, and pulse oximetry. A brief training of the use of the device (**FIG. 82**) was provided, including practice inhalations, and then, as shown in **FIG. 46**, subjects completed 10 inhalations from the eNT-100 inhaler (**FIG. 82**) at approximately 30-second intervals over a 4.5-minute period. Postdose assessments included smoking urge or craving (baseline, 1-, 15-, and 30-minutes post-dosing), spirometry, pulse oximetry, and sampling for nicotine pharmacokinetics (baseline, 30-seconds, 5-mintues post-dosing) were collected. Additionally, postdose assessments of tolerability and liking were collected using the modified Cigarette Evaluation Scale (mCES), and a product debriefing assessment questionnaire. Subjects received a follow-up phone call approximately 24 hours after dosing to assess any adverse events (AEs) that occurred since dosing. Escalation to the next dose group did not take place until adequate safety and tolerability from the previous group had been demonstrated.

**Methods:**

*Summary*

Each dose level had an enrollment target of 12 subjects (target total N = 84); however, the study recruited 77 smokers (averaging 21.2 cigarettes per day) and randomly assigned them to 7 cohorts (N=9-12) involving dosing with 10 inhalations in the following conditions: placebo (air only), vehicle (PG only), 25, 50 (both 2.5% and 5% solutions), 75 or 100 mcg of nicotine per inhalation. Outcome measures included smoking urge or craving (baseline, 1-, 15- and 30-minutes post-dosing), nicotine PK (baseline, 30-seconds, 5-minutes post-dosing), the modified Cigarette Evaluation Scale, and a product debriefing assessment. Please note that of all the 77 subjects enrolled in Part 1 of the study 75 completed the study.

*Study Inclusion and Exclusion Criteria*

Inclusion criteria included: 1. Healthy adult male and female smokers, 21 to 65 years of age, inclusive, at screening. 2. At least a 12-month smoking history prior to check-in with a cigarette smoked per day average of 10 or more manufactured cigarettes per day (no restriction on brand). Please note that 1 subject smoked filtered cigars as opposed to manufactured cigarettes. Brief periods (up to 7 consecutive days) of non-smoking (e.g., due to illness, trying to quit, participation in a study where smoking was prohibited) were permitted at the discretion of the PI. A history of occasional use of e-cigs was allowed, but the subjects confirmed that their primary source of nicotine consumption was smoking conventional cigarettes. 3. Positive urine cotinine at screening ($\geq$ 500 ng/mL). 4. Exhaled CO > 12 ppm at screening. 5. Female subjects who were heterosexually active and of childbearing potential (e.g., not surgically sterile [bilateral tubal ligation, hysterectomy, or bilateral oophorectomy at least 6 months prior to check-in] or at least 2 years naturally postmenopausal) must have been using one of the following forms of contraception and agreed to continue using it through completion of the study: hormonal method (e.g., oral, vaginal ring, transdermal patch, implant, or injection) consistently for at least 3 months prior to check-in; double barrier method (i.e., condom with spermicide or diaphragm with spermicide) consistently for at least 2 weeks prior to check-in; intrauterine device for at least 3 months prior to check-in; essure® procedure at least 6 months prior to check-in; have a partner who had been vasectomized for at least 6 months prior to check-in. 6. Female subjects of childbearing potential who were not currently engaging in heterosexual intercourse must have agreed to use one of the above methods of birth control, in the event that they had heterosexual intercourse during the course of the study. 7. Voluntary consent to participate in this study documented on the signed informed consent form (ICF). 8. Willing to comply with the requirements of the study and willing to consider using alternative inhaled forms of nicotine other than conventional cigarettes. 9. Forced Expiratory Flow (FEF) (25 - 75%) at least 60% of the normal values predicted for that individual based on age, gender, and height.

Subjects were excluded from the study if there was evidence of any of the following criteria at screening, check-in, or at any time during the study as appropriate, in the opinion of the principal investigator (PI): History or presence of clinically significant gastrointestinal, renal, hepatic, neurologic, hematologic, endocrine, oncologic, urologic, pulmonary (especially bronchospastic diseases), immunologic, psychiatric, or cardiovascular disease, or any other condition that, in the opinion of the PI, would jeopardize the safety of the subject or impact the validity of the study results; positive urine screen for alcohol or drugs of abuse at screening or any check-in; history of drug or alcohol abuse within 24 months of check-in; an acute illness (e.g., upper respiratory infection, viral infection) requiring treatment within 2 weeks prior to check-in; fever (> 100.2°F) at screening or at check-in; systolic blood pressure > 150 mmHg, diastolic blood pressure > 95 mmHg, or pulse rate > 99 bpm at screening; body mass index (BMI) < 19 kg/m2 or > 35 kg/m2 at Screening; female subjects who were pregnant, lactating, or intended to become pregnant from screening through completion of study; consumption of xanthines/caffeine, alcohol, or grapefruit juice within 24 hours of check-in and during confinement; used any OTC or prescription smoking cessation treatments, including, but not limited to, nicotine replacement therapies (gum, patches, lozenges, nasal spray, or inhalers), varenicline (Chantix®), or buproprion (Zyban®) within 3 months prior to screening and throughout the study; used prescription anti-diabetic medication and/or insulin therapy within 12 months of check-in and throughout the study; concomitantly used inhalers for any reason within 3 months prior to screening and throughout the study; plasma donation within 7 days prior to check-in, or donation of blood or blood products, had significant blood loss, or received whole blood or a blood product transfusion within 56 days prior to check-in; participation in a previous clinical study for an investigational drug, device, or biologic within 30 days prior to either check-in; used nicotine-containing products other than manufactured cigarettes and occasional e-cig use (e.g., rollyour-own cigarettes, bidis, snuff, nicotine inhaler, pipe, cigar, chewing tobacco, nicotine patch, nicotine spray, nicotine lozenge, or nicotine gum) within four weeks prior to check-in or during study; or self-reported puffers (i.e., adult smokers who draw smoke from the cigarette into the mouth and throat but do not inhale); FTND score of < 6.

*Study Restrictions:*

Concomitant Medications

Stable doses (i.e., no dosage adjustments within 30 days prior to Check-in) of prescription or over-the-counter medications required to treat a PI-approved disease or condition (e.g., hypertension) were permitted at the discretion of the PI. Hormonal contraceptives (e.g., oral, transdermal patch, implant, injection) and hormonal replacement therapy were permitted. Occasional use of over-the-counter analgesics (e.g., acetaminophen, ibuprofen), antihistamines, and nasal decongestants were permitted. Exceptions were permitted at the discretion of the PI in consultation with the Sponsor, providing the medication in question would have no impact on the study. Any exceptions were documented. All concomitant medications (and reasons for their use) taken by subjects during the study were recorded and coded using the most updated version of the WHO Drug Dictionary available at Celerion (e.g., Sep 2013 or later). During the study, up to 2 g per day of acetaminophen was administered at the discretion of the PI for intercurrent illness or adverse events. If other drug therapy was required, a joint decision was made by the PI and Sponsor to continue or discontinue the subject.

Foods and Beverages

Consumption of foods and beverages containing the following substances were prohibited as indicated: Xanthines/caffeine: 24 hours prior to Check-in and during confinement; alcohol: 24 hours prior to Check-in and during confinement; or grapefruit or grapefruit juice: 24 hours prior to Check-in and during confinement.

Activity

Subjects did not engage in strenuous activity in the 48 hours prior to and at any time during the confinement period.

Subject Numbering

Subjects were assigned a unique screening number and subject numbers for each part of the study.
Eligibility for inclusion into Part 1 was based on a screening visit(s) to assess medical history, concomitant medications, demographics, and smoking history (including the Fagerström Test for Nicotine Dependence [FTND]), an exhaled CO test, urine cotinine and drugs of abuse test, urine pregnancy test, vital signs, and BMI determination as outlined in **FIG. 45**.

**Duration of Study Conduct**

**Part 1** was completed during a screening visit, a single study visit and a follow-up phone call. The schedule of assessments used for Part 1 of the eNT-101 study is shown in **FIG. 45. FIG. 46** depicts the timing of assessments used for Part 1 of the eNT-101 study for prior to dosing (pre-dosing), during dosing (dosing), and follow completion of dosing (post-dosing).

**Study Products**

**Part 1:** Placebo (eNT-100 inhaler delivering air only); Vehicle control (eNT-100 inhaler delivering PG only); and eNT-100 Nicotine Inhaler (nicotine concentration range based on tolerability: 2.5-5% nicotine solution in PG vehicle).

**Product Administration/Experimental Sessions**

**Part 1:** Subjects participated in one of the experimental groups using the eNT-100 nicotine inhaler (**FIG. 82**) involving 10 inhalations resulting in the total nicotine delivery as listed in **Table 19** below. Subjects participating in Part 1 of the study completed in-clinic study events during a single visit that included 1 overnight confinement. As shown in **FIG. 46,** administration of the study product (i.e., eNT-100 inhaler for Part 1) included 10 inhalations at approximately 30-second intervals over a 4.5-minute period (or completion of one conventional cigarette). Administration included 10-inhalations from the eNT-100 inhaler followed by a 5-count breathe hold at 30-second intervals. Pre- and post-dose assessments were conducted as described herein. A minimum washout of 36 hours from nicotine was required prior to each study product administration.

**Table 19: Experimental Groups (Part 1)**

| Group (Total Amount of Nicotine Over 10 Inhalations) | Total Amount of Solution Aerosolized per Inhalation (mcg) | Nicotine per Inhalation ($\mu$g) | Total Technical Success: % of Doses Delivered | Nicotine Concentration (%) | Tolerability : % of Target Doses Inhaled (N) |
|---|---|---|---|---|---|
| Group #1: Placebo (air only) | 0 | 0 | 100% | 0 | 100% (120) |
| Group #2: Vehicle (PG only) | 1000 | 0 | 100% | 0 | 100% (120) |
| Group #3: 250 mcg | 1000 | 25 | 100% | 2.5 | 100% (100) |
| Group #4: 500 mcg (5.0%) | 1000 | 50 | 100% | 5.0 | 91% (90) |
| Group #4: 500 mcg (2.5%) | 2000 | 50 | 100% | 2.5 | 98% (120) |
| Group #5: 750 mcg | 2000 | 75 | 750 | 3.25 | 90% (100) |
| Group #6: 1000 mcg | 2000 | 100 | 100% | 5.0 | 90% (100) |

The demographics of the subjects in the experimental groups shown in **Table 19** are listed in **Table 20** below.

**Table 20: Demographics of Subjects in Part 1**

| Group | N | Mean Age (years) (SD) | % Male | Mean BMI (SD) | Racial Breakdown |
|---|---|---|---|---|---|
| Group #1: Placebo (air only) | 12 | 32.2 (9.6) | 83% | 25.3 (3.3) | 75% Caucasian, 25% African American |
| Group #2: Vehicle (1 mg propylene glycol only) | 12 | 34.7 (8.8) | 67% | 27.6 (3.6) | 75% Caucasian, 25% African American |
| Group #3: 250 mcg (2.5%) | 10 | 35.7 (6.8) | 80% | 26.0 (4.4) | 70% Caucasian, 20% African American, 10% Other |
| Group #4: 500 mcg (5.0%) | 9 | 35.9 (9.7) | 78% | 27.1 (5.7) | 100% Caucasian |
| Group #4: 500 mcg (2.5%) | 12 | 43.7 (13.5)* | 67% | 27.5 (3.1) | 90% Caucasian, 10% African American |
| Group #5: 750 mcg (3.25%) | 10 | 36.5 (8.6) | 40% | 26.8 (5.1) | 92% Caucasian, 8% African American |
| Group #6: 1000 mcg (5.0%) | 12 | 37.0 (3.9) | 75% | 24.6 (4.6) | 100% Caucasian |
| Overall | 77 | 36.6 (10.6) | 70% | 26.4 (4.2) | 86% Caucasian, 13% African American, 1% Other |
| *Statiscally significant difference at $p < .05$ | | | | | |

The smoking status of the subjects in the experimental groups shown in **Table 19** are listed in **Table 21** below.

**Table 21: Smoking Status of Subjects in Part 1**

| Group | Mean Years Smoking (SD) | Mean Cigs Per Day (CPD) (SD) | Mean FMean FTND* Score (SD) TND* Score |
|---|---|---|---|
| Group #1: Placebo (air only) | 14.0 (9.2) | 23.1 (10.5) | 6.3 (.65) |
| Group #2: Vehicle (PG only) | 17.9 (7.9) | 22.3 (8.6) | 7.3 (1.4) |
| Group #3: 250 mcg (2.5%) | 13.5 (8.8) | 20.5 (5.7) | 6.5 (.53) |

(continued)

| Group | Mean Years Smoking (SD) | Mean Cigs Per Day (CPD) (SD) | Mean FMean FTND* Score (SD) TND* Score |
|---|---|---|---|
| Group #4: 500 mcg (5.0%) | 17.0 (8.9) | 19.3 (3.7) | 7.1 (1.2) |
| Group #5: 500 mcg (2.5%) | 26.2 (13.8)** | 22.9 (6.6) | 6.8 (1.1) |
| Group #6: 750 mcg (3.25%) | 19.5 (9.2) | 19.3 (4.9) | 6.7(.82) |
| Group #7: 1000 mcg (5.0%) | 19.1 (12.5) | 20.2 (5.8) | 6.7 (.78) |
| Overall | 18.3(10.7) | 21.2(7.0) | 6.8 (1.0) |
| *FTND: Fagerström Test of Nicoine Dependence **Statiscally significant difference at p < .01 | | | |

In order to identify a dose that was both well-tolerated while minimizing the total amount of chronic exposure to PG that would be expected from eventual chronic use of the eNT inhaler, the total amount of solution aerosolized (starting at 1.0 mg but adjustable to a minimum of 0.5 mg or a maximum of 2.0 mg of total nicotine plus PG solution), as well as the nicotine concentration of the aerosol, was adjusted following a review of the safety and tolerability from the previous group as outlined in **FIG. 42.**

Dose-escalation decisions within Part 1 were made based on evaluation of safety data, AEs, and the pharmacodynamic (PD) assessments.

Note: % nicotine is % by volume.

**Pharmacokinetic Sample Collection, Parameters, and Analysis:**

During Part 1, serial blood samples were collected within 15 minutes prior to product administration, and at approximately 30 seconds, 5 minutes and 10 minutes after the final inhalation and were used to determine venous nicotine concentrations. Venous nicotine was measured using Celerion's proprietary GLP nicotine bioassay, which has a lower limit of quantification (LLOQ) of 0.02 ng/ml. **Table 22** outlines the blood sample collection protocol used for Part 1.

**Table 22. Part 1 Blood Sample Collection Protocol**

| Sample Type | Number of Time Points | Approximate Volume per Time Point* (mL) | Approximate Sample Volume Over Course of Study (mL) |
|---|---|---|---|
| PK | 4 | 4 | 20 |
| Total Blood Volume for Study (mL) → | | | 20 |

The following baseline-adjusted PK parameters were derived from the nicotine plasma concentration-time data: concentration at 5 minutes after the start of product administration (C5), the area under the nicotine concentration-time curve from time zero to the last measurable concentration ($AUC_{0-t}$), the maximum observed concentration ($C_{max}$), and the time of the maximum concentration ($T_{max}$).

Plasma nicotine concentrations were listed by subject and time point. Concentration data were summarized by time point using descriptive statistics (number of observations [N], arithmetic mean, standard deviation [SD], coefficient of variation [CV%], minimum, median, and maximum). PK parameters were listed by subject and summarized using descriptive statistics (N, arithmetic mean, SD, CV%, minimum, median, and maximum). In addition, geometric mean and CV% were calculated for AUC0-t, Cmax, and C5. The parameter values were imported into SAS and all descriptive statistics were calculated in SAS® Version 9.3. Figures were created to display mean and individual concentration-time curves (linear and semi-log scales). Actual sampling times were used for individual figures and nominal sampling times for mean figures.

Analysis of variance (ANOVA) were performed on the $_{Cmax}$, C5, and AUC PK parameters. The ANOVA model included sequence, study product, and period as fixed effects, and subject nested within sequence as a random effect. Sequence was tested using subject nested within sequence as the error term. Each ANOVA included calculation of least-squares means (LSMeans), differences between product LSMeans, and the standard errors associated with the LSMeans and differences. First order carry-over effect was tested. If it was not statistically significant, it was removed from the statistical model.

## Pharmacodynamic Assessment and Analysis

Smoking urge, aversion/tolerability, respiratory tract sensations, and subjective effects was evaluated via patient-reported outcome (PRO) measures following product administration. The following parameters were calculated form the smoking urge-visual analog scale (SU-VAS) response data for the product administered: area under the effect curve from time 0 to t ($AUEC_{(0-t)}$), maximum ibserved reduction ($E_{max\ reduction}$), time of the maximum reduction ($T_{max\ reduction}$).

All pharmacodynamic data obtained was listed by subject and time point. The data was summarized by time point using descriptive statistics and an appropriate statistical method (ANOVA or an appropriate non-parametric test as required by the type of data) was used to characterize the between-group comparisons. The SU-VAS measurements and response parameters ($AUEC_{(0-t)}$ and $E_{max\ reduction}$) were listed, summarized, and analyzed using an ANOVA similar to the PK concentrations and parameters.

## Safety Assessments and Analysis

Prior to inclusion into Part 1 of the study, medical history, vital signs, urine drug and alcohol screen, and pregnancy test (females only) was performed. Part 1 Check-in evaluations included vital signs, urine drug and alcohol screen, and a pregnancy test (females only) as outlined in **FIG. 45.**

In addition, vital signs were evaluated before and after study product administration.

Adverse events (AEs) spontaneously reported by the subjects or observed by the PI or other study personnel were monitored and followed up until the symptoms or values return to normal or acceptable levels or until lost to follow-up, as appropriate in the opinion of the PI or his designee.

All reported AEs were coded to a standard set of terms, using MedDRA®, Version 16.1. The number of subjects experiencing product use-emergent AEs and the number of product use-emergent AEs were summarized by preferred term for each product and overall.

Safety data were summarized by treatment and time point. Descriptive statistics (N, mean, SD, minimum, median, and maximum) were calculated for quantitative safety data and frequency counts were compiled for classification of qualitative safety data. Summary tables for actual results and change from baseline were presented for vital signs.

Other non-safety assessments as outlined in FIG. 45 were conducted and included exhaled CO, spirometry (FVC and FEV1), pulse oximetry and PRO assessments from the 13 item mCES described herein. Subjective impressions of the aerol were assessed via a 7-point product evaluation questionnaire as described herein.

Spirometry and pulse oximetry measurements were listed by subject and time point, and summarized by time point using descriptive statistics. An ANOVA was used to present the postdose to predose differences between test and reference product group comparisons. The mCES responses were listed by subject and summarized using frequency counts. Responses to the product evaluation questionnaire were listed by subject.

## Study Assessments from Part 1:

## Conclusions-Part 1:

A MTD of nicotine form the eNT-100 inhaler (FIG. 82) was not reached. Administration of up to 1000 mcg nicotine as a 5% solution with the eNT-100 nicotine inhaler appeared to be safe under the conditions used in this study.

Administration of each nicotine-containing solution produced a markedly greater plasma nicotine mean peak increase from baseline compared to placebo and vehicle control.

The smoking urge response as measured by the $E_{max}$ reduction and $AUEC_{(0-t)}$ parameters peaked with the 500 μg (2.5% nicotine solution) and the 750 μg (3.75% nicotine solution) doses from the eNT-100 inhaler, with a peak median smoking urge reduction of 62% and 64%, respectively.

Cough, throat irritation, and burning sensation were more prevalent following use of the eNT-100 inhaler with solutions containing nicotine as compared to placebo and vehicle control.

## Safety

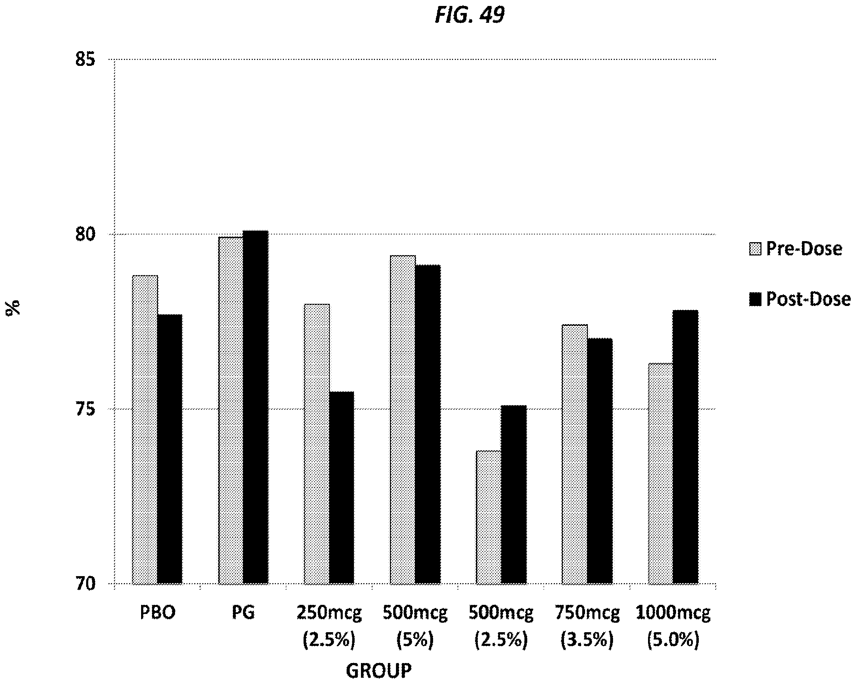

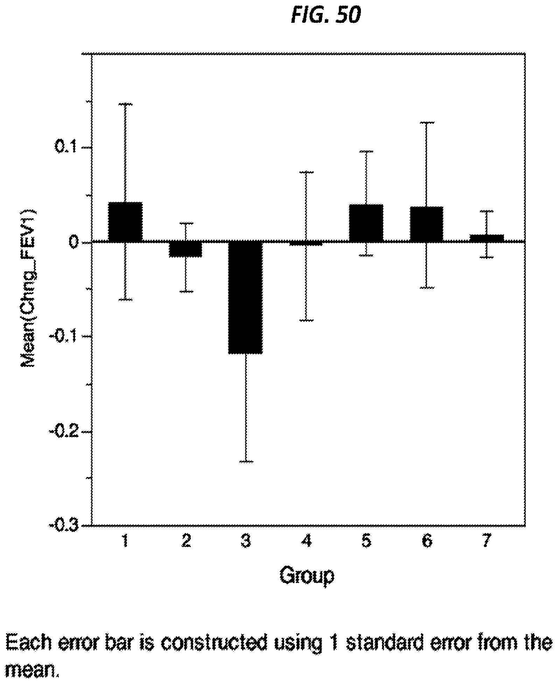

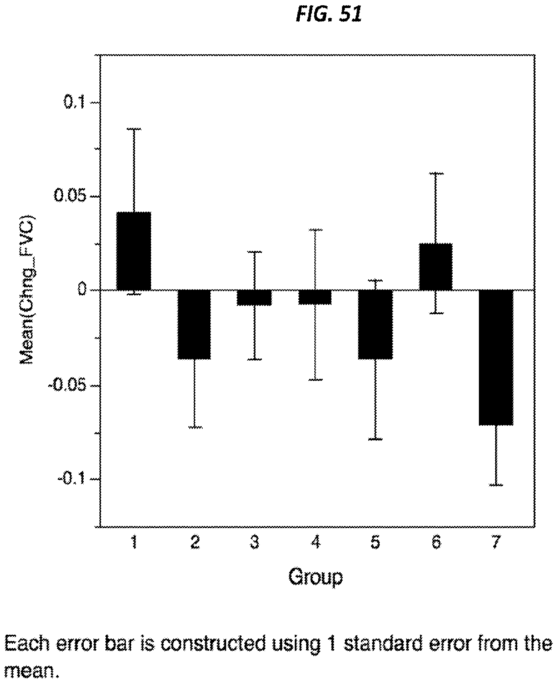

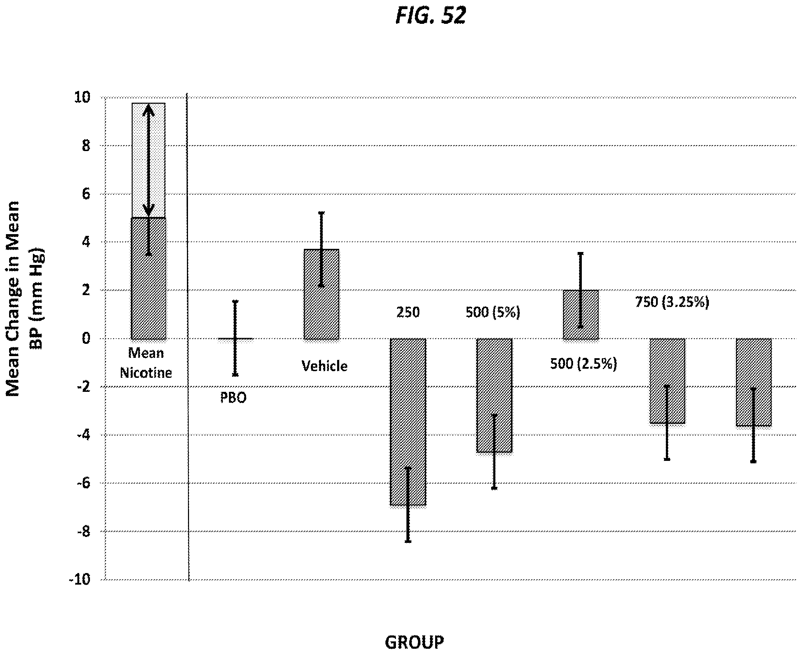

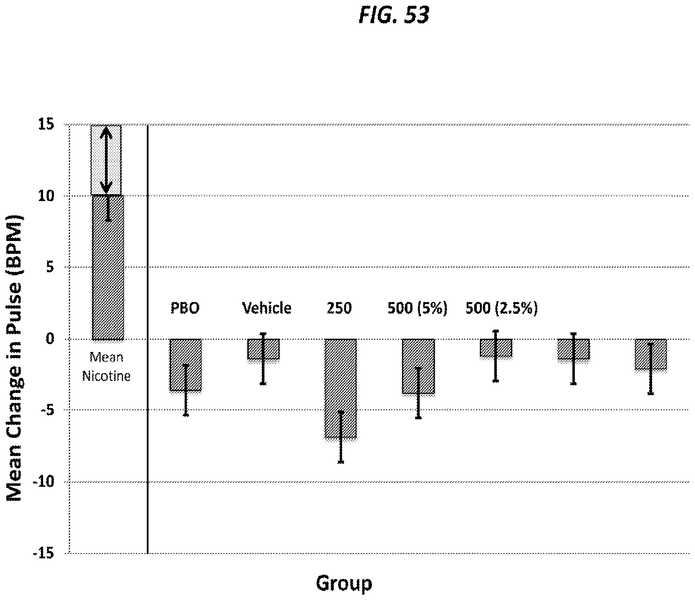

All doses appeared to be safe and well tolerated as evidenced by **FIGs. 49-53**. Lung function was assessed for each subject in each cohort by measuring the forced expiratory volume (FEV1), which is the volume of air that can forcibly be blown out in one second after full inspiration, as well as the forced vital capacity (FVC), which is the maximum amount of air a person can expel from the lungs after a maximum inhalation. FVC is equal to the sum of inspiratory reserve volume, tidal volume, and expiratory reserve volume. Both the mean change in FEV1 (**FIG. 50**) and FVC (**FIG. 51**) for each of the nicotine cohorts (groups 3-7) were not significantly different than the vehicle (group 1) or placebo (group 2) cohort values. In addition, the percentage of the ratio of FEV1/FVC (**FIG. 49**) postdose for each of the nicotine cohorts was in the range of a healthy adult (75-80%). Heart function as evidenced by the mean change in mean blood pressure (**FIG. 52**) and mean change in pulse (**FIG. 53**) was not significantly different for each of the nicotine cohorts vs. the vehicle and placebo cohorts. As a result, there were no concerns about changes in lung function, pulse, or blood pressure.

Overall, a total of 100 mild product use-emergent AEs were experienced by 74% of subjects. The incidence of AEs was higher for those groups receiving nicotine via the eNT-100 inhaler compared to subjects in the placebo and vehicle control groups. Cough, throat irritation, and burning sensation were the most frequently reported AEs in Part 1. These events accounted for the majority of AEs in Part 1 of this study, and all but 2 events of cough occurred in the subjects receiving nicotine-containing products. The study physician considered all cough, throat irritation, and burning sensation AEs to be probably related to the study product, with the exception of 1 cough AE following placebo control that was considered unrelated.These types of sensations are common in nicotine administration studies and are desired by many smokers as associated with smoking. There was a single episode of a nicotine-related AE (i.e., vomiting) in the 1000 mcg cohort. Overall, there were no unexpected AEs or significant changes in lung function or vital signs (all ps > 0.14).

**Coughing and Splatter:**

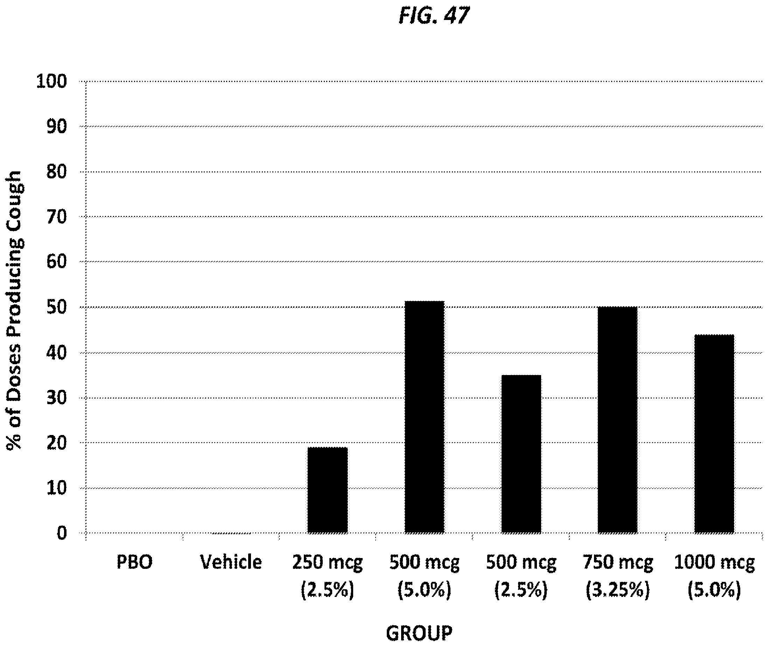

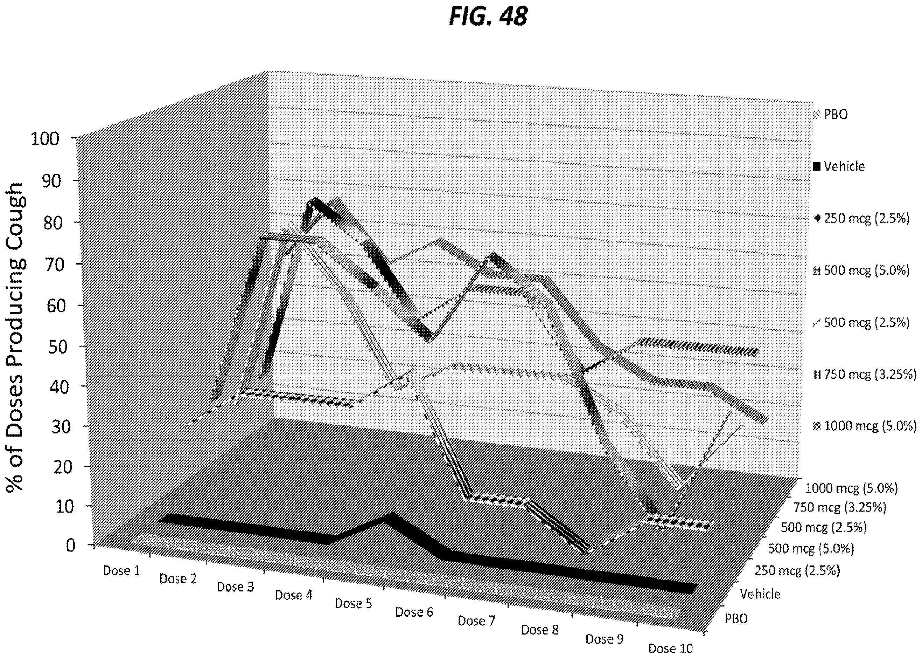

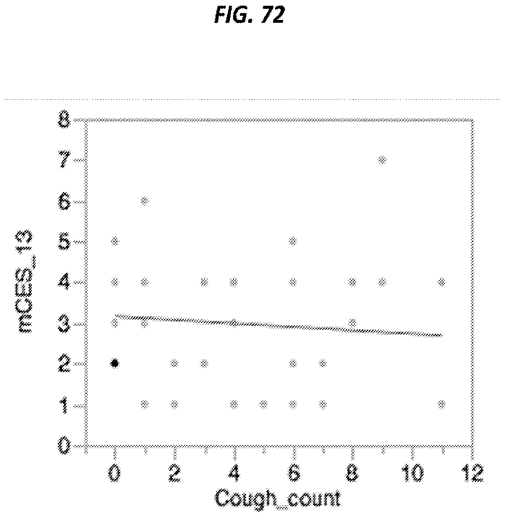

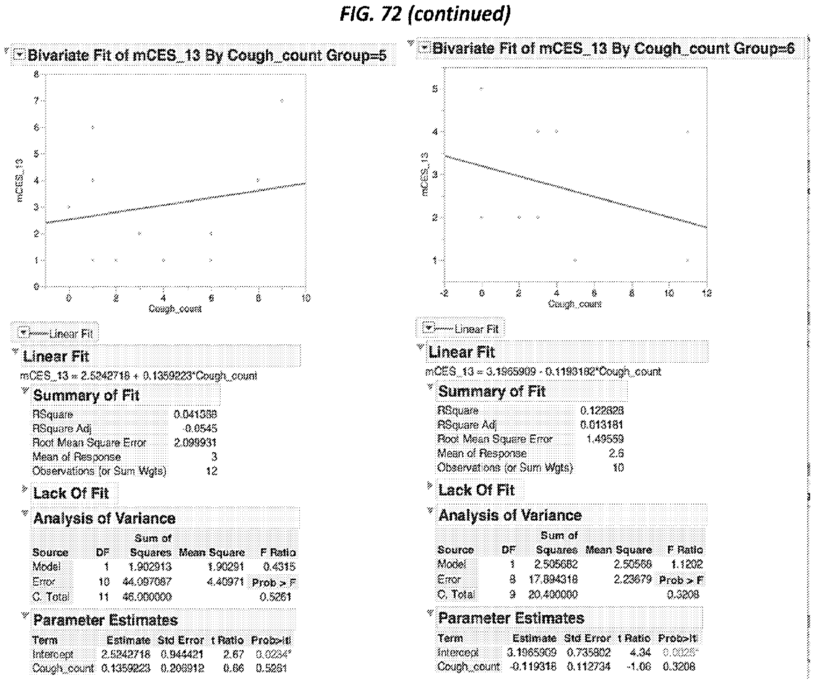

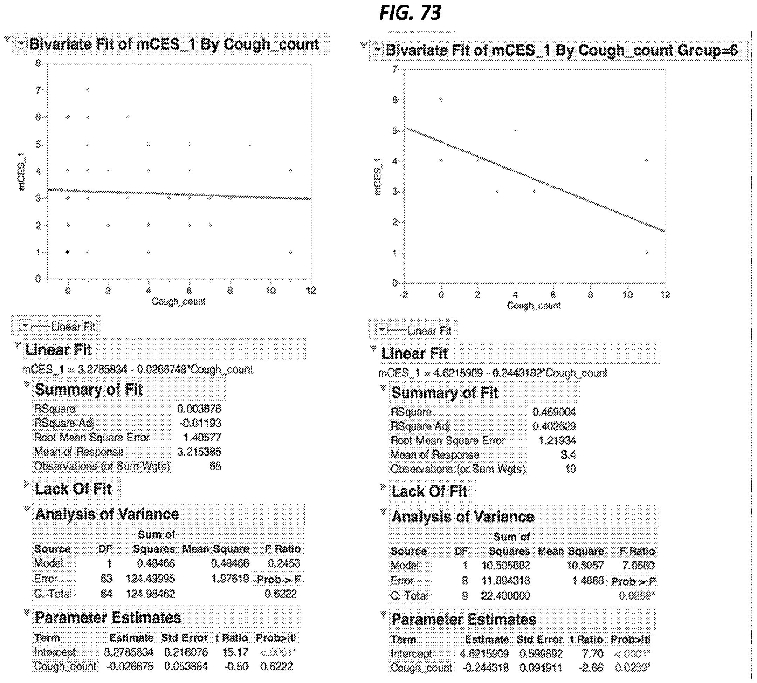

Coughing during administration of low doses was thought to be caused by large nicotine/PG particles (10-100 micron) being thrown or 'boiled' off of the heater element. As such, a large particle filter (baffle) as described herein and shown in **FIGs. 44A-C** was installed into the mouthpiece of the clinical device for the 1000 mcg cohort. Data analyzed in real-time during the course of Part 1 of the study showed that coughing was noticeably less severe with the 1000 mcg dose as compared to the 750 mcg dose, even though the dose was higher **(FIGs. 47-48).** Devices from the 500 mcg (2.5%) and 750 mcg (3.25%) solutions were examined and categorized in terms of the presence of splatter in the device: present vs. absent; severity (0 = absent, 1=mild, 2=moderate). Of the 22 devices examined to date, 77% had evidence of splatter. Subjects using devices without splatter averaged 3.2 coughs, whereas subjects using devices with splatter averaged 4.5 coughs, (ns, although a 41% increase). **Table 23** shows a breakdown of coughing data for each of the cohorts in Part 1 of the clinical study as analyzed in real-time during the course of the study. **FIG. 47** shows the percent of doses producing a cough for each cohort, while **FIG. 48** shows the percent of doses producing a cough per dose for each cohort. The 500 mcg (2.5%) cohort produced the lowest percentage of coughing for the higher dose cohorts. In addition, a bivariate analysis of coughing vs. the mCES question of "did you enjoy it?" showed that coughing was unrelated to a subjects response to "did you enjoy it?" in the 500 mcg (2.5%) group or 750 mcg (3.25%) cohorts **(FIG. 72)**. However, a bivariate analysis of coughing vs. the mCES question of "was it satisfying?" showed that less coughing did predict a increase in satisfaction overall and in the 750 (3.25%) cohort specifically **(FIG. 73)**.

Further independent analysis of the data presented in **FIGs. 47-48** by a contract research organization (CRO) (Celerion, Lincoln NE) revealed that the number of coughs observed per inhalation attempt was lower following administration of the 500 mcg 2.5% solution (33% versus 42% for the 500 mcg 5% solution) as was the number of coughs leading to a failed inhalation attempt (5% for the 2.5% solution versus 17% for the 5% solution) in agreement with the real-time data analysis performed above.

**Table 23:** Coughing Data for the cohorts in Part 1:

| Group | Total # of Coughing Episodes During Dosing | Mean Coughing Episodes per Subject (SD) | % Coughing Episodes per Dose | % of Subjects with at least 1 episode of coughing |
|---|---|---|---|---|
| Group #1: Placebo (air only) | 0 | 0(0) | 0.0% | 0% |
| Group #2: Vehicle (1 mg PG only) | 1 | 0.8 (0.29) | 0.8% | 8% |
| Group #3: 250 mcg (1 mg PG) | 19 | 1.9 (2.0) | 19% | 70% |

(continued)

| Group | Total # of Coughing Episodes During Dosing | Mean Coughing Episodes per Subject (SD) | % Coughing Episodes per Dose | % of Subjects with at least 1 episode of coughing |
|---|---|---|---|---|
| Group #4: 500 mcg (1 mg PG) | 42 | 4.7 (3.5) | 51% | 78% |
| Group #5: 500 mcg (2 mg PG) | 42 | 3.5 (3.1)** | 35% | 83% |
| Group #6: 750 mcg (2 mg PG) | 50 | 5 (4.4) | 50% | 80% |
| Group #7: 1000 mcg (2 mg PG) | 48 | 4 (2.8) | 44% | 100% |

There was no relationship between splatter or splatter severity and taste ratings or sensations in their throat and chest from the modified cigarette evaluation scale (mCES) described below.

**Pharmacodynamic Assessments:**

**Smoking Urge-Visual Analog Scale (SU-VAS):**

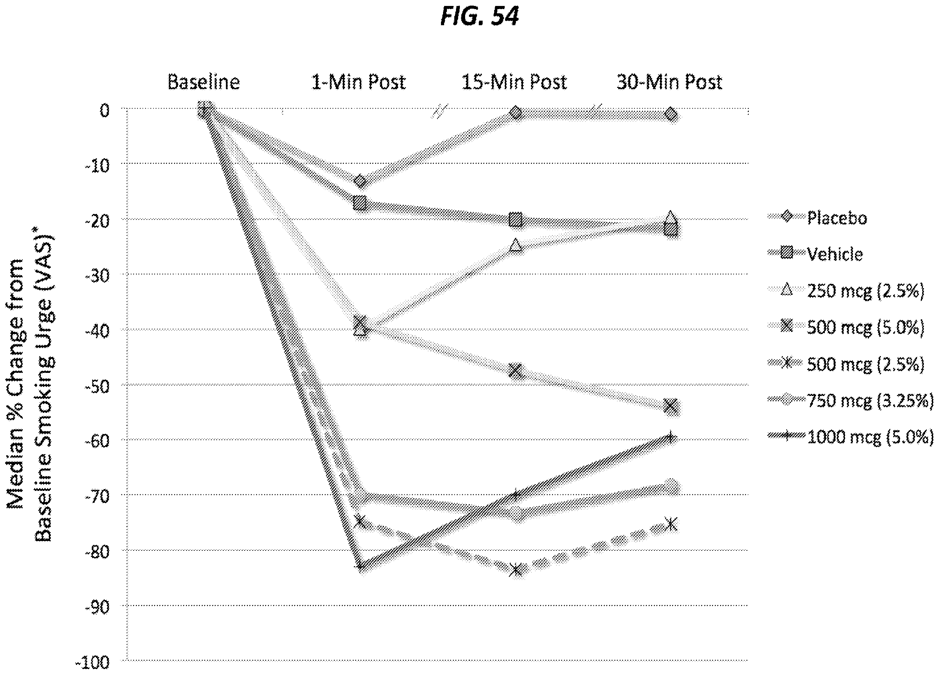

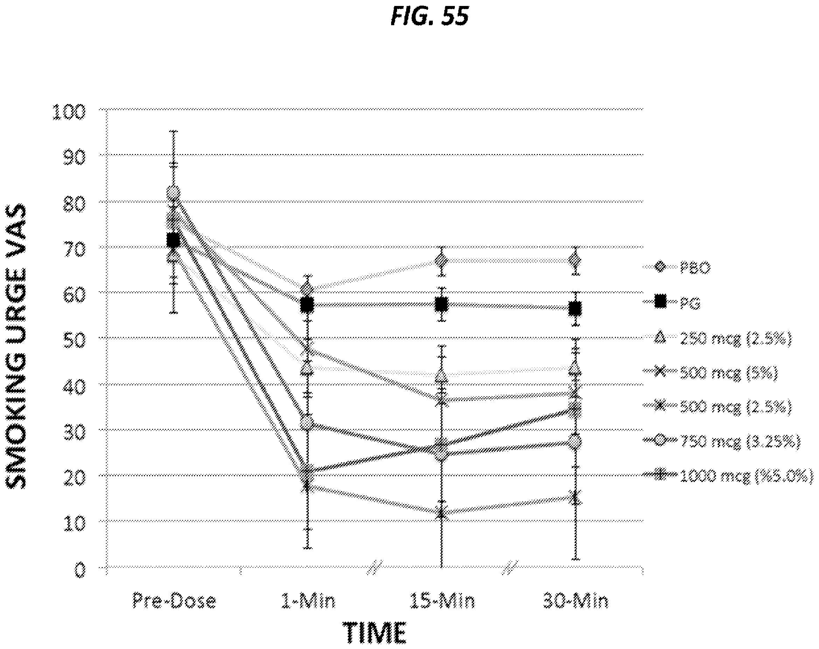

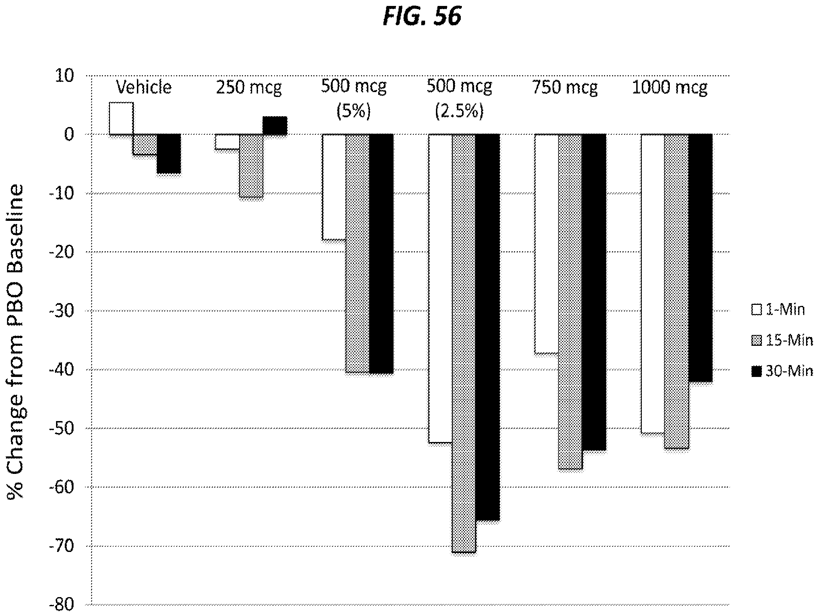

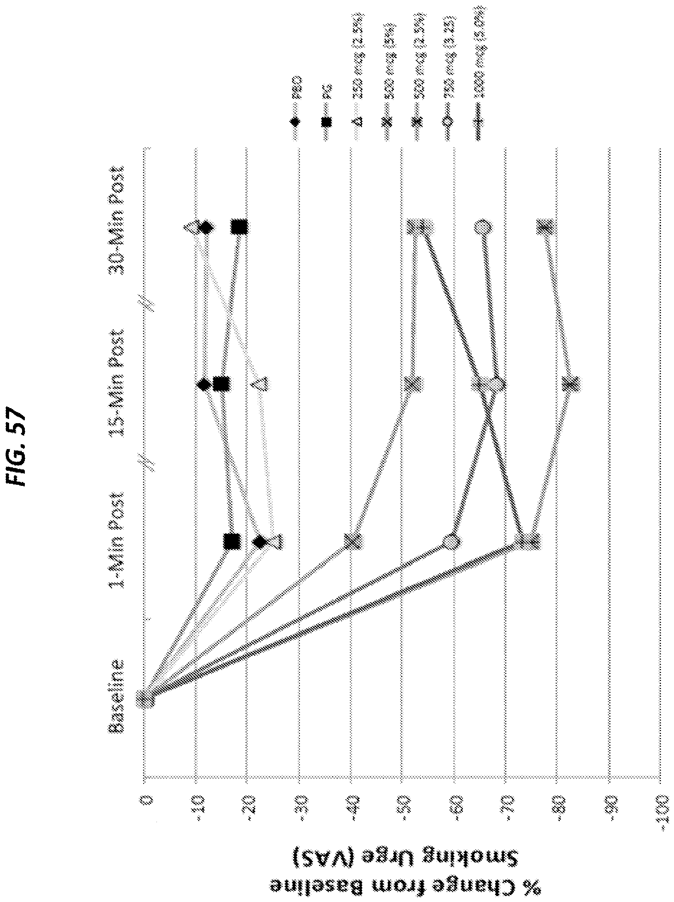

The smoking urge for each subject in each cohort was assessed by rating a subject's response to "how strong is your urge to smoke right now?" on a 0-100 mm visual analog scale (VAS), where 0 = "Not at all"; 100 = "extreme". SU-VAS assessed within 15 minutes prior to and approximately 1, 15, and 30 minutes after completion of the product administration. **FIGs. 54-64** disclose data analyzed in real-time during the course of Part 1 of the study. **FIG. 54** shows that the placebo, vehicle, 25 and 50 mcg (5.0%) dose groups reported modest median percent smoking urge reductions versus the baseline (median baseline smoking urge: 75 mm after 12 hours of overnight abstinence) of 13%, 17%, 38%, and 39%, respectively at 1-minute post-dosing, while the 50 (2.5%), 75 and 100 mcg dose groups all reported significant (p<.01) reductions in their median percent smoking urge (75%, 70%, and 83%, respectively) at 1-minute post-dosing, which were sustained over time. The raw smoking urge values from pre-dose to 1-min, 15-min, and 30-min (**FIG. 55**), % change from PBO baseline (**FIG. 56**) and % change from baseline smoking urge for each cohort showed similar trends (**FIG. 57**). In addition, multivariate analyses revealed that more dependent smokers reported a 12% greater smoking urge reduction than less dependent smokers at 15 and 30 min post-dosing, irrespective of their baseline smoking urge (p-values = 0.01).

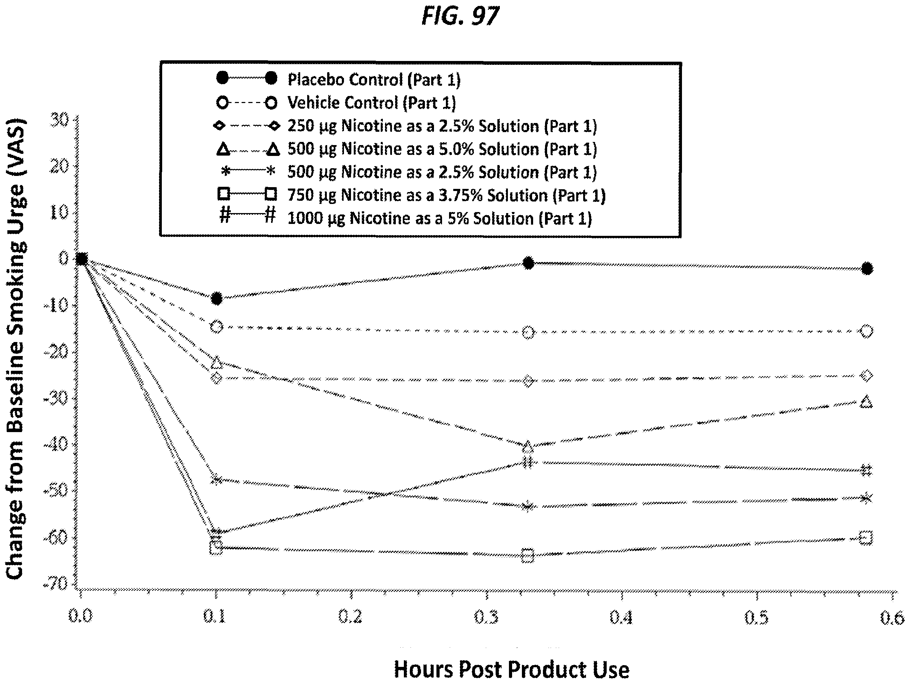

Subsequent independent data analysis of the Part 1 data by the CRO (Celerion, Lincoln NE) showed that the median baseline smoking urge for each treatment ranged from 68% to 86.5%, thus confirming the results of the real-time data analysis shown above. FIG. 97 shows that the placebo, vehicle, 25 and 50 mcg (5.0%) dose groups reported modest reductions in the % change from baseline smoking urge (vas) at 0.1 hours post-dosing, while the 50 (2.5%), 75 and 100 mcg dose groups all reported significant reductions in their % change from baseline smoking urge (vas) at 0.1 hours post-dosing, which were sustained over time. Smoking urge as assessed by the $_{AUEC(0-t)}$ and Emax reduction parameters decreased as the nicotine dose from the eNT-100 inhaler increased from 250 $\mu$g (2.5% solution) to 500 $\mu$g (5% solution) per inhalation. The response was observed to peak with the 500 $\mu$g 2.5% solution and 750 $\mu$g doses. In an effort to improve tolerability (e.g., decrease the frequency of cough), the 500 $\mu$g dose was re-administered as a 2.5% solution to determine if a reduction in nicotine concentration might reduce the cough response and subsequently enhance the smoking urge response. The number of coughs observed per inhalation attempt was lower following administration of the 2.5% solution (33% versus 42% for the 5% solution) as was the number of coughs leading to a failed inhalation attempt (5% for the 2.5% solution versus 17% for the 5% solution). Compared to the 5% solution, administration of the 2.5% solution was observed to enhance the smoking urge response, with the median $_{AUEC(0-t)}$ parameter decreasing from -20.12%*hr to -31.49%*hr and the Emax reduction parameter decreasing from -41.0% to -62.0%. Taken together, these observations may be an indication that less coughing led to a higher nicotine deposition when using the less concentrated solution. Indeed, while the data are inconclusive due to sparse PK sampling, the nicotine concentration increase from baseline was higher ~5 minutes following the start of the first inhalation of the 2.5% solution compared to the 5% solution.

**A 13-Item Modified Cigarette Evaluation Scale (mCES)**

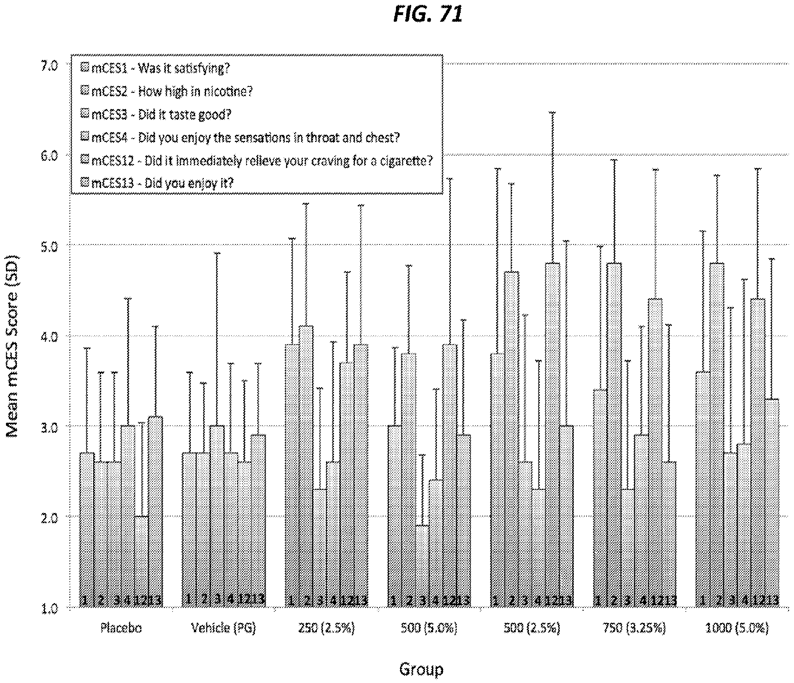

Subjects were asked to rate 13-items referred to as the modified Cigarette Evaluation Scale (mCES) using a 7-point Likert response range from 1 (not at all) to 2 (very little) to 3 (a little) to 4 (moderately) to 5 (a lot) to 6 (quite a lot) to 7 (extremely). The mCES was administered 1 minute after completion of product administration. The specific items and their respective results from Part 1 included: 'was it satisfying?' (**FIG. 58**); 'how high in nicotine?' (**FIG. 59**); did it taste good?' (**FIG. 60**); 'did you enjoy the sensations in your throat and chest?' (**FIG. 61**); 'did it calm you?' (**FIG. 62**); 'did it make you feel more awake?' (**FIG. 63**); 'did it make you fell less irritable?' (**FIG. 64**), 'did it help you concentrate?' (**FIG. 65**); 'did it reduce your hunger for food?' (**FIG. 66**); 'did it make you dizzy?' (**FIG. 67**); 'did it make you nauseous?' (**FIG. 68**); did it immediately relieve your craving for a cigarette?' (**FIG. 69**); and 'did you enjoy it?' (**FIG. 70**). **FIG. 71** shows mean mCES score for a select number of mCES questions ("was it satisfying?"; "how high in nicotine?"; "did it taste good?"; "did you enjoy the sensations in throat and chest?"; "did you enjoy it?"). In summary, subjects rated the 500 mcg (2.5%) dose as moderatelty satisfying, high in nicotine, calming, while also reduing irritability, and craving.

**Product Debriefing Assessment**

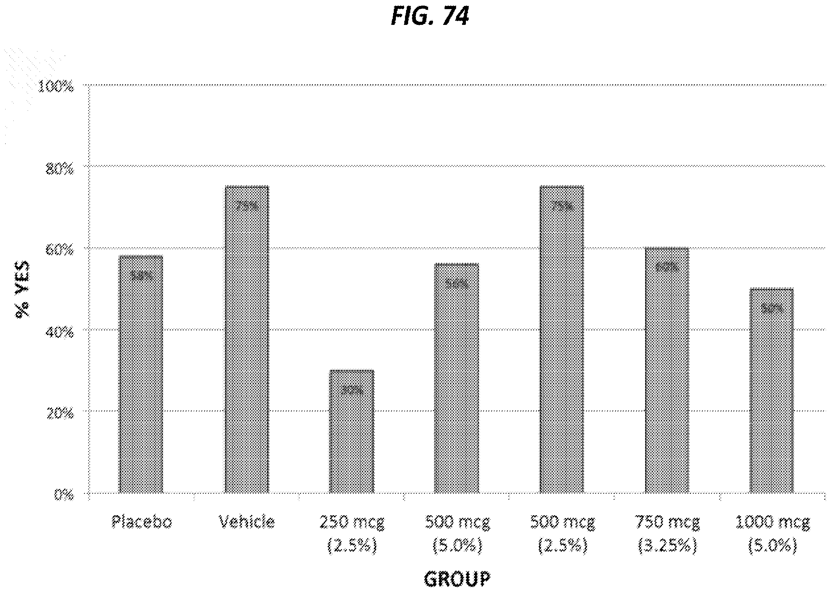

For each subject in each cohort a product debriefing assessment was conducted, which involved a series of questions. In response to the question "if a product was available that was small and easy to use and produced this aerosol, would you consider using it as a replacement for your smoking?", 75% of subjects reported that they would use the 500 mcg (2.5%) aerosol as a substitute for their smoking, as compared to 30%, 56%, 60%, and 50% for the 250, 500 (5.0%), 750, and 1000 mcg dose groups, respectively (**FIG. 74**). 58% and 75% of subjects in the placebo and vehicle groups reported that they would use the placebo and vehicle aerosol as a substitute for their smoking (**FIG. 74**). **Table 24** shows each subject's response to the question, "what did you like most about it?" for the placebo group, vehicle group, 250 mcg (5.0%); 500 mcg (5.0%); 500 mcg (2.5%); 750 mcg (3.25%); and 1000 mcg (5.0%).

**Table 24:** Subject's response to "what did you like most about it?"

| Subject No. | Placebo (air only) | Vehicle (PG only) | 250 mcg (5%) | 500 mcg (5%) | 500 mcg (2.5%) | 750 mcg (3.25%) | 1000 mcg (5%) |
|---|---|---|---|---|---|---|---|
| 1 | It makes you forget about having a cig | Smell | Tasteless . Seemed smokeles s too. | It got rid of my nicotine craving | It did seem to help with the craving | The after - taste | No smoke in the aerosol |
| 2 | It didn't taste bad | The fact that it was smooth when inhaled | How it burned the back of my throat! | Satisfying in terms of nicotine release | Nothing | It didn't burn inhaling it | It cured my craving |
| 3 | I like that it didn't have an after-taste | I really didn't. It didn't hit your lungs hard enough to feel it. | The relaxing effect after inhaling | Was a pretend smoke to trick my brain | Same with satisfying | I could feel the nicotine almost instantly | It woke me up |
| 4 | Light taste | I didn't taste it and I couldn't feel it when I inhaled. | It felt like it delivered nicotine quickly | Nicotine craving was reduced | I did not like it | The relaxed feeling after | After a bit I didn't want to smoke as bad |

(continued)

| Subject No. | Placebo (air only) | Vehicle (PG only) | 250 mcg (5%) | 500 mcg (5%) | 500 mcg (2.5%) | 750 mcg (3.25%) | 1000 mcg (5%) |
|---|---|---|---|---|---|---|---|
| 5 | Didn't get any smoke out of it, felt like air, no taste, but was lightheade d and feeling a bit high | It wasn't harsh like a cigarette | Didn't have a bad taste | Kills the craving | It did help my craving | Had nicotine | I could feel the nicotine, it relaxed me like a cigarette does |
| 6 | Was tasteless | Easy on lungs | I liked the ease to my cigarette craving | Did relieve the urge to smoke for the time being | The nicotine | That it would be a healthier choice than cigarette s | How it made me not want a cigarette |
| 7 | It seems to be quicker way of getting what I need out of it | Didn't taste bad or hurt my throat | It seemed smooth for the most part | Reduced the craving to smoke | There was no real noticeable difference between the aerosol and normal breathing | Relativel y smooth and nonirritating | That it seems to work for the time being |
| 8 | I felt that afterwards my craving for a cigarette decreased | It made me stop thinking about smoking | Calmed me down | Nothing | I like the satisfactio n of it | After taking it, didn't feel the need to smoke | The last few inhalation s |
| 9 | It did not irritate my throat | No idea | Felt ok | No smoke, good inhalation s of product | It stopped the urge to smoke | Toof edge off, calm | Had very little taste |
| 10 | That it's not hard to use | No real after - taste | It was o.k. I guess, kind of please the moment for a cigarette | | It took the craving for a cig a little bit | When it was over | It was fine |
| 11 | I liked that there was no taste to the aerosol | The taste was nice | | | It took away the craving of nicotine and I felt less frustrated | | It lasted long and tasted good |
| 12 | It wasn't a harsh feeling when I inhaled | I am indiffere nt to it | | | Stopped the urge to smoke | | Nothing |

**Table 25** shows each subject's response to the question, "what did you dislike most about it?" for the placebo group, vehicle group, 250 mcg (5.0%); 500 mcg (5.0%); 500 mcg (2.5%); 750 mcg (3.25%); and 1000 mcg (5.0%).

**Table 25:** Subject's response to "what did you dislike most about it?"

| Subject No. | Placebo (air only) | Vehicle (PG only) | 250 mcg (5%) | 500 mcg (5%) | 500 mcg (2.5%) | 750 mcg (3.25%) | 1000 mcg (5%) |
|---|---|---|---|---|---|---|---|
| 1 | That after so many tries it makes you light headed | Not feeling as if it was going into my lungs | Dried my throat out instantly | Slightly irritating my throat while inhaling | The burning it did to my throat | The first time I tried I didn't like the burning feeling in my throat | Harsh on my throat |
| 2 | It didn't seem to help much with the cravings | I was really craving a cigarette and the device didn't really stop the craving | How it burned the back of my throat! | There wasn't anything there | Like little more taste | None | The burning in back of throat |
| 3 | The clinical aspect | The above statement (i.e. Table 24 response to "what did you like most about it?") | The taste | Made me cough several time | It hurt my throat and lungs | The first inhalation was a little rough | It was very hard to breathe in |
| 4 | The tube mouthpiece | Nothing | Hard to take without coughing . Burned back of throat and dried throat out. | Too strong | It hurt my throat | The slight burning in my throat | When you have to suck in as much as possible and after a bit it starts to burn |
| 5 | No taste/ flavor | I didn't feel like it did anything | Little harsh on throat after inhale of air | I would say I coughed a little bit | It was a little harsh | Harsh on the lungs | It was pretty harsh on my throat, caused a burning sensation and coughing |
| 6 | How hard of a drag i had to take | The taste | The burn from holding it in | Making me cough | It was very dry and it caused burning sensations in the throat and chest | The taste | It was really rough on my lungs |
| 7 | The wait | Nothing came out when I exhaled | The 2nd inhalation seemed harsh on my throat | The burning in my throat when I exhaled | The coughing | Nothing | It's taste |

(continued)

| Subject No. | Placebo (air only) | Vehicle (PG only) | 250 mcg (5%) | 500 mcg (5%) | 500 mcg (2.5%) | 750 mcg (3.25%) | 1000 mcg (5%) |
|---|---|---|---|---|---|---|---|
| 8 | That there was no taste. I couldn't tell if I was inhaling anything | No taste | Burned my throat some | Burning sensation in chest and throat | At first it was harsh | While taking it, it made my throat burn and cough bad | The first few inhalation s |
| 9 | I felt like it was not serving a purpose | Nothing | nothing | The taste | The feeling | Burned or gave a spicy feeling a bit | Initially it made me cough |
| 10 | Takes a lot of breath | No real nicotine feeling | I didn't like inhaling out of a tube | | It making me a little bit lightheaded other than that it was good | The coughing | I didn't |
| 11 | I disliked that I couldn't feel like it was working | How many times I had to inhale | | | Procedure of inhaling | | It wasn't a cigarette but it was great |
| 12 | I feel like I was just inhaling | Nothing to dislike | | | | | Every thin 9 |

**Table 26** shows each subject's response to the question, "how was the taste of the aerosol?" for the placebo group , vehicle group, 250 mcg (5.0%); 500 mcg (5.0%); 500 mcg (2.5%); 750 mcg (3.25%); and 1000 mcg (5.0%). Among all nicotine cohorts (including the PBO and vehicle groups, 34% reported a negative taste, 43% a neutral taste, and 23% reported a positive taste. Among the cohorts to be included in Part 2 of the clinical study (see Example 14), 33% reported a negative taste, 50% a neutral taste, and 17% a positive taste for the 500 mcg (2.5%) cohort, while 33% reported a negative taste, 42% a neutral taste, and 25% a positive taste for the 100 mcg (5.0%) cohort. Moreover, taste tended to predict a greater change in PK (positive or negative taste reports).

**Table 26**: Subject's response to "how was the taste of the aerosol?"

| Subject No. | Placebo (air only) | Vehicle (PG only) | 250 mcg (5%) | 500 mcg (5%) | 500 mcg (2.5%) | 750 mcg (3.25%) | 1000 mcg (5%) |
|---|---|---|---|---|---|---|---|
| 1 | There was not taste | Kind of sweet, slightly | I din't taste it really | Not good | Was kind of dust taste | Ok, the after - taste was pleasant to me | No taste to the aerosol |
| 2 | Almost tasteless | The taste wa slight, it didn't really taste like muh of anything | It was not a pleasant taste | Slightly bitter | Not good at all | I didn't mind the taste | Didn't really have a taste, just a burning sensation |

(continued)

| Subject No. | Placebo (air only) | Vehicle (PG only) | 250 mcg (5%) | 500 mcg (5%) | 500 mcg (2.5%) | 750 mcg (3.25%) | 1000 mcg (5%) |
|---|---|---|---|---|---|---|---|
| 3 | It didn't seem to have a taste | No taste | It had a more chemical taste rather than mint or basic tobacco | There was none | Light | Did not notice a taste | Terrible |
| 4 | Ok | No taste | Tasteless | Not bad. Had no taste to me | Ok | Ok | It had no taste |
| 5 | Didn't tatse it | Tasted like medicine | Not bad, could be flavored | Good | Little or no taste | Bitter | It was ok. I would have preferred if it had some flavor |
| 6 | Didn't taste like anything | Plastic | Not too much of a taste at all | Not bad | Not so good but tolerable | Very harsh | I wouldn't prefer it |
| 7 | N/A there was not taste | Not bad | Sorry couldn't tell a taste of any sort | Didn't really taste anything | Mostly unnoticeable | Pleasant | Strong |
| 8 | No taste at all | There was no taste | Ok | Ok at first, but bad aftertaste | Good, you can't really taste anything | Really had no taste | Bad on first few inhalations |
| 9 | No taste at all | Ok | Good | Like tobacco | Ok | Spicy. Burning made me cough | Almost tasteless |
| 10 | Didn't taste it | Tasteless | Kind of choking but o.k. | | Bad | Not good | Good |
| 11 | There reallywas no taste to the aerosol | There really wasn't one but it was fresh like air | | | Tasted just like a strong but full flavor cigarette. Good | | Great |
| 12 | I didn't taste anything | No noticeable taste | | | Not dislikefull | | None |

**Table 27** shows each subject's response to the question, "why or why not would you use the aerosol as a substitute for smoking?" for the placebo group, vehicle group, 250 mcg (5.0%;); 500 mcg (5.0%); 500 mcg (2.5%); 750 mcg (3.25%); and 1000 mcg (5.0%).

**Table 27:** Subject's response to "why or why not would you use the aerosol as a substitute for smoking?"

| Cohort Subject No. | Placebo (air only) | Vehicle (PG only) | 250 mcg (5%) | 500 mcg (5%) | 500 mcg (2.5%) | 750 mcg (3.25%) | 1000 mcg (5%) |
|---|---|---|---|---|---|---|---|
| 1 | Because it is too hard to get it right | Less damaging to the body | Seemed too dry | Taste is terrible | Seems like good replacemen t for smoking | Because it had a pleasant taste and relieved my urge to smoke | Too harsh on my throat and non-menthol |
| 2 | I don't think it was close enough to actually smoking to help me quit | It did not satisfy my craving for nicotine the way I thought it would | For the reasons above (i.e., response s in Tables 24-26) | Prefer cigarette flavor | The way it burns my throat | It helped with nicotine cravings | Because it satisfied my craving and is simple to use |
| 3 | It seemed liek it was not anything to do with tobacco | Only if the aerosol hit harder and the taste was tasteable | The feeling or sensation received when inhaling was nice but the taste was a turn off | Was nothing | Could smoke in house | I think it would be different enough from smoking it would help break habits | It made me cough, it tastes terrible |
| 4 | Looking for a better way to enjoy nicotine | Ecause it was easy to use and tasteless | It was uncomfor table to use | East to use-no smoke-no lighter-able to use other places than cigs or cigars | It hurt | To be more healthy | Cause I probably would not smoke as much and would not smell like cigarettes |
| 5 | Not until I'm able to feel a full effect of what it does | I didn't feel like it did anything | Didn't get rid of urge to smoke just reduced it | I want to quit | Cause I'm considering quitting smoking | Easy | I have no desire to change my habits |
| 6 | If it was a lot stronger | Need flavors for me to really consider it | After smoking for so long it would be nice to quit | Expense, less smoke as far as cleanline ss | Is it safer and cleaner | The tatse was overwhelmi ng, and inhaling was more harsh than regular smoking | Did not like the feel that it had on my lungs, it was way too rough |

(continued)

| Cohort Subject No. | Placebo (air only) | Vehicle (PG only) | 250 mcg (5%) | 500 mcg (5%) | 500 mcg (2.5%) | 750 mcg (3.25%) | 1000 mcg (5%) |
|---|---|---|---|---|---|---|---|
| 7 | The quickness | I like to see somethin g come out of my mouth | Because it seems like it would be great alternativ e and to try and quit smoking | It helps with reducing the urge to smoke | It burns, there's no taste, and there is no real feeling like you smoking | Prefer smoking- might use this for times when traditional smoking is limited | Dependin g on the cost |
| 8 | Because it would be healthier | I have wanted to quit for a while. I am not sure if this would work or not. Need to use it more | Seemed 2 help get the edge off enough to help quit | Because of the burning in my throat | Cause I would like to stop smoking | I think that it would be a good way to stop smoking | Irritable inhalation |
| 9 | No smoke or 2nd hand smoke that harm anyone else | Smokeles s | I like what I smoke | No smoke is involved | It stopped the urge to smoke | Needs more flavor | Tasteless once I got used to it, did not make me cough and stopped my craving to smoke |
| 10 | Because it's pretty easy | More better than smoking | Somethin g new to me I guess | | It hurt my throat and didn't take the craving away as much as it should | It hurt my lungs and made me cough | Better for you |
| 11 | Because I feel cigarettes work better for me | Anything to help quit regular cigs | | | Because the effects do last longer you don't have to worry about finding a lighter to light cigarette | | It tasted good and lasted long |
| 12 | I didn't feel any different with it | | | | I would probably smoke less without the stink of cigarettes | | Did not like the effect |

**Nicotine Pharmacokinetic Assessments**

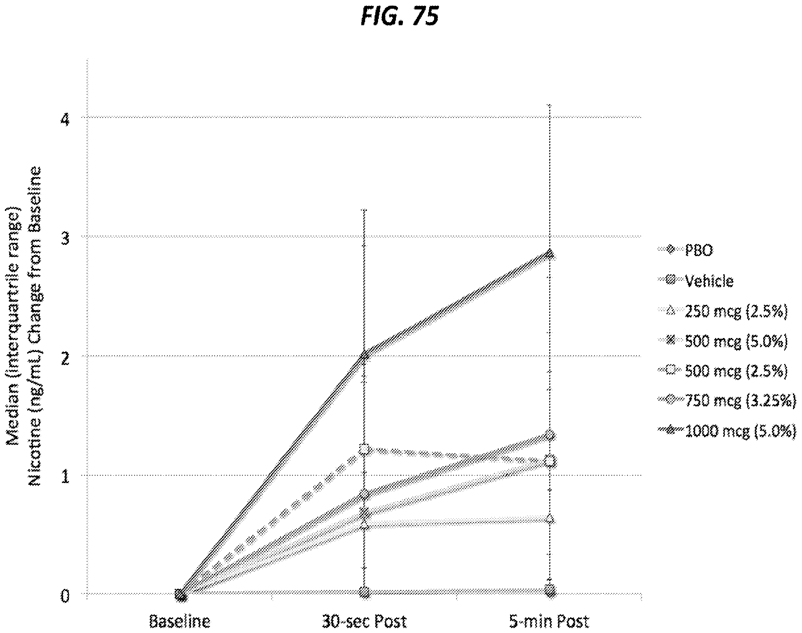

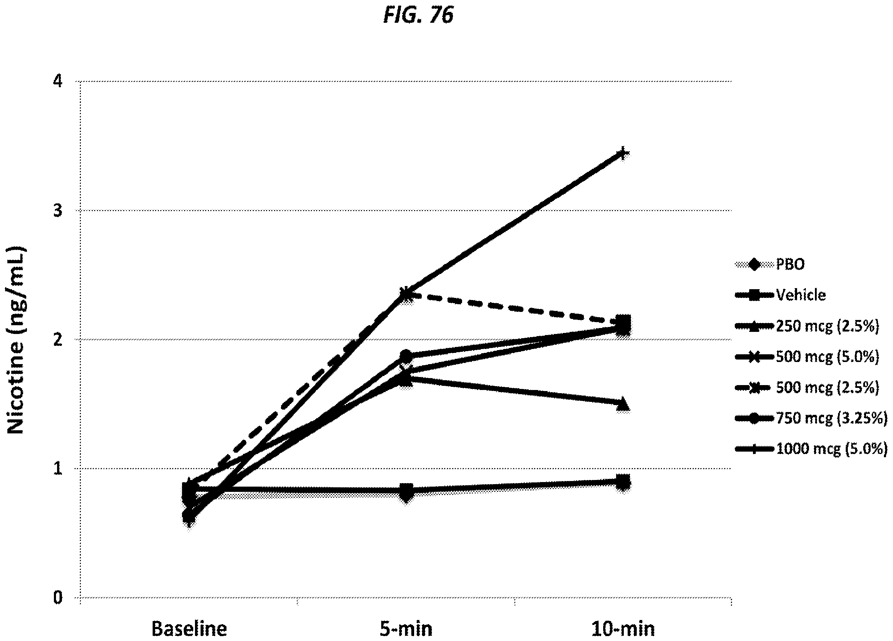

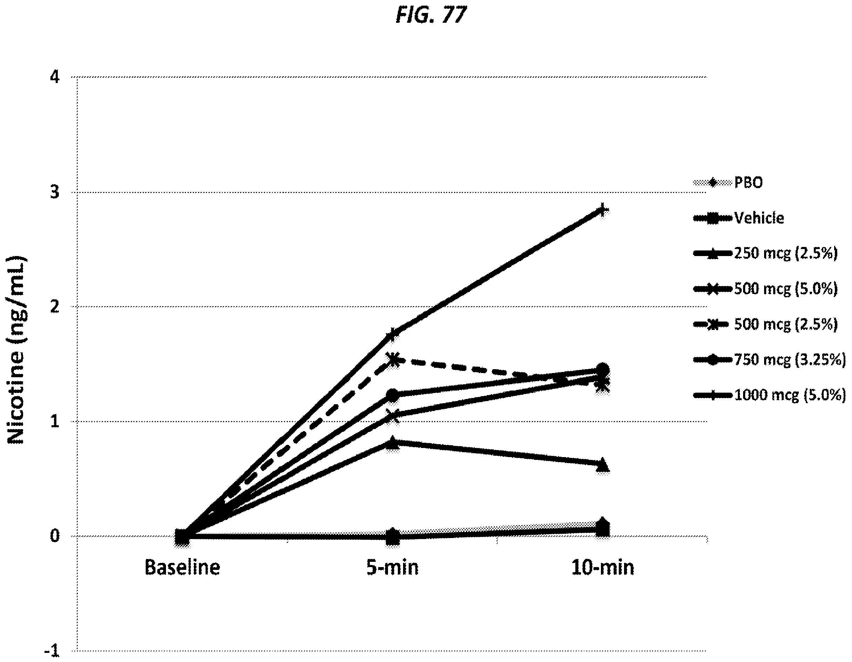

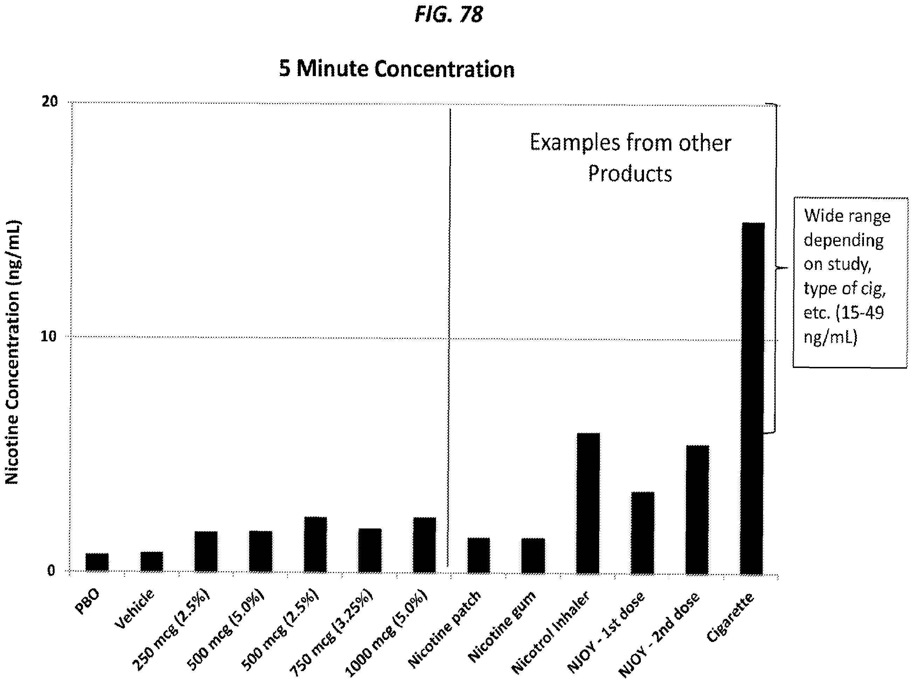

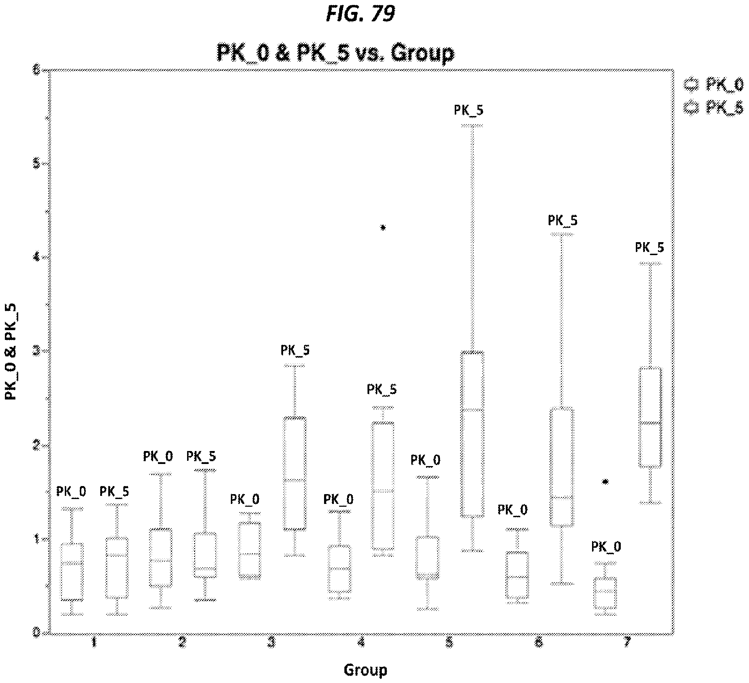

Initial, real-time data analysis of the pharmacokinetic data showed that the nicotine dosing groups as described above produced median nicotine PK changes between 0.68 and 2.0 ng/ml within 30 seconds after dosing as compared to baseline (**FIG. 75**). Of the nicotine dosing groups, the 500 mcg (2.5%) and 1000 mcg (5.0%) groups showed the highest mean nicotine concentrations for both the raw change in PK by time (**FIG. 76**), and the change in PK by time as compared to baseline (**FIG. 77**). This trend was also observed in the pre-dose and 5 minutes post-dosing for each cohort (group) in the box and whisper plots shown in **FIG. 79**. The mean nicotine concentration in each of the nicotine cohorts was less than the nicotine concentration achieved using a nicotine inhaler, a commercial electronic cigarette, or a tobacco cigarette as seen in **FIG. 78**.

Moreover, independent analysis of the PK data collected from Part 1 of the study by the CRO (Celerion, Lincoln NE) showed that administration of each nicotine-containing solution produced a markedly greater plasma nicotine mean peak increase from baseline compared to placebo and vehicle control. The rate and extent of nicotine absorption, as well as early exposure up to 5 minutes, were the highest following use of the usual brand combustible cigarette. Statistically significant differences in the exposure PK parameters were found between both the 500 $\mu$g and 1000 $\mu$g doses of the eNT inhaler and the NJOY King Bold e-cig compared to the usual brand combustible cigarette.

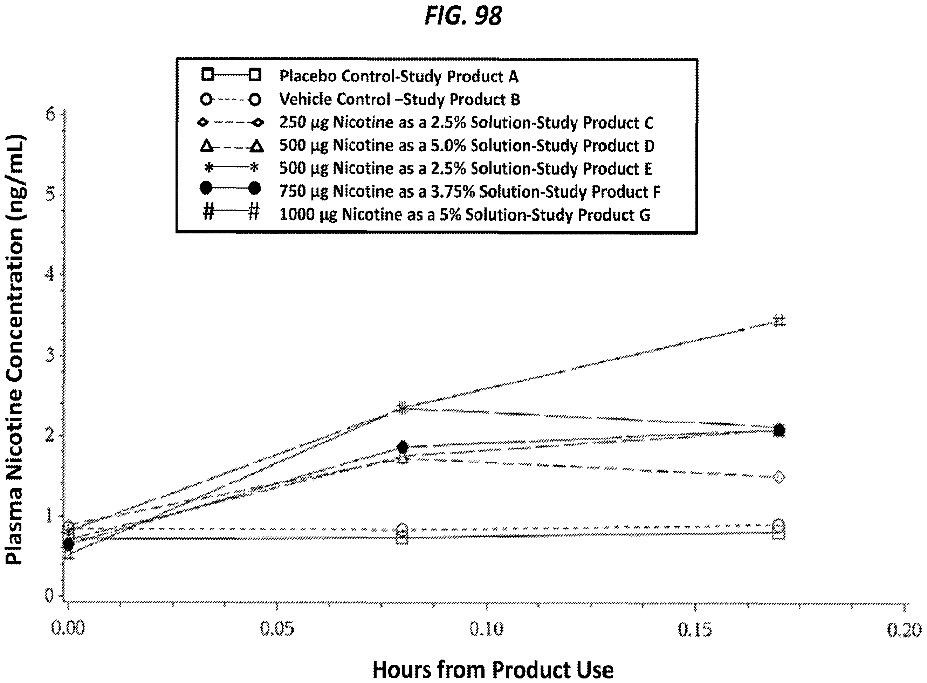

Mean peak increases from baseline at 10 mintues post-dosing for the 250 mcg dose were approximately 40-45% lower than the 500 mcg and 750 mcg doses, while the mean peak increases from baseline for the 1000 mcg dose were approximately two-fold greater than the 500 mcg and 750 mcg doses, as shown in FIG. 98.

**Example 14: Study of the Safety, Tolerability, Pharmacokinetics, and Pharmacodynamics of the eNT-100 Nicotine Inhaler among Healthy Volunteer Cigarette Smokers**

In this example, Part 2 of the two part study described in Example 13 was conducted to examine the safety, tolerability, pharmacokinetics, and pharmacodynamics of condensation aerosol comprising nicotine produced from a liquid nicotine formulation using the ENT-100 nicotine inhaler. The primary objective was be to establish the plasma level-time profiles of nicotine administered as 10 inhalations (single dose) using 500 mcg (2.5 % nicotine) and 1000 mcg (5% nicotine) doses from the eNT-100 nicotine inhaler. Secondary objective for Part 2 will be: 1.) to evaluate the pharmacokinetic (PK) profiles of nicotine administered using the eNT-100 nicotine inhaler and those of a vehicle control (PG only), a commercially available electronic-cigarette (e-cig) (NJOY® King Bold, 4.5% nicotine solution), and inhalation via cigarette smoking; 2.) to evaluate the acute tolerability and specific adverse event (AE) profiles of two different single doses from the eNT-100 nicotine inhaler, and those of a vehicle control (PG only), a commercially available e-cig (NJOY King Bold, 4.5% nicotine solution), and inhalation via cigarette smoking; 3.) to evaluate the pharmacodynamics (PD) of the eNT-100 nicotine inhaler, a vehicle control (PG only), a commercially available e-cig (NJOY King Bold, 4.5% nicotine solution), and inhalation via cigarette smoking in terms of their ability to reduce acute, abstinence-induced smoking urges, and also affect respiratory and other subjective sensations.

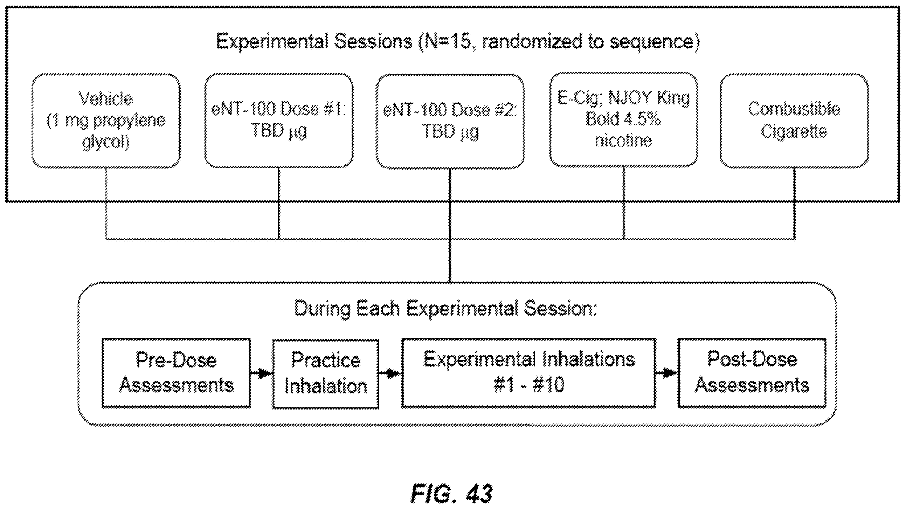

**Part 2** was a randomized, single-blind, within-subject, 5-way crossover, vehicle-, e-cig-, and combustible cigarette-controlled design to assess the safety, tolerability, pharmacokinetics and pharmacodynamic effects of the eNT-100 nicotine inhaler. The selected nicotine aerosol concentrations and doses to be administered during Part 2 were determined upon completion of Part 1 of the study described in Example 13. Control comparisons were made to the vehicle (PG only), NJOY King Bold e-cig, and the subject's usual brand combustible cigarette. All subjects participating in Part 2 had participated in Part 1. Subjects completed predose assessments of exhaled CO, their smoking urge, nicotine PK, spirometry, and pulse oximetry, and a brief training including practice inhalations as outlined in **FIG. 80**. Each administration of the study products included 10 inhalations at approximately 30-second intervals over a 4.5-minute period (or completion of one conventional cigarette). Postdose assessments included nicotine PK, safety, tolerability, liking, smoking urge, exhaled CO, spirometry, and pulse oximetry assessments. A 36-hour wash-out from nicotine was required prior to each product administration. See **FIG. 43** for the trial design of this portion of the trial (Dose #1 = 500 mcg (2.5% solution); Dose #2 = 1000 mcg (5% solution)).

*Study Population and Sample Size*

Potential subjects underwent screening procedures within 50 days prior to Check-in for Part 2 during which they were evaluated to ensure that they met the requirements for inclusion in the study.

All subjects had the study explained by the PI or his/her designee and were required to read, sign, and date an Institutional Review Board-approved informed consent form (ICF) prior to completion of Screening or other study procedures. This ICF provided the subjects in non-technical terms with the purpose of the study, the procedures to be carried out, and potential hazards. The subjects were assured that they may withdraw from the study at any time without jeopardizing medical care related to or required as a result of study participation. Subjects were given a copy of their ICF.

Like Part 1, Part 2 included healthy, adult male and female subjects. In Part 2, 15 subjects were enrolled to better ensure that 12 subjects complete the study. All 15 subjects were included in the safety, PK, PD, and other non-safety analyses with the exception of a few device failures occurring with the 1000 $\mu$g dose from the eNT-100 inhaler (details are provided below).

Subjects were healthy, adult males and females, 21 - 65 years of age inclusive, who smoke at least 10 cigarettes per day (CPD) for the last 12 months. All subjects that participated in Part 2 had participated in Part 1. Potential subjects fulfilled all of the following inclusion criteria to be eligible for participation in the study. Inclusion criteria include: 1. Healthy adult male and female smokers, 21 to 65 years of age, inclusive, at Screening. 2. At least a 12-month smoking history prior to Check-in with a cigarette smoked per day average of 10 or more manufactured cigarettes per day (no restriction on brand). Brief periods (up to 7 consecutive days) of non-smoking (e.g., due to illness, trying to quit, participation in a study where smoking was prohibited) were permitted at the discretion of the PI. A history of occasional use of e-cigs was allowed, but the subjects confirmed that their primary source of nicotine consumption was smoking conventional cigarettes. 3. Positive urine cotinine at Screening ($\geq$ 500 ng/mL). 4. Exhaled CO > 12 ppm at Screening. 5. Female subjects who were heterosexually active and of childbearing potential (e.g., not surgically sterile [bilateral tubal ligation, hysterectomy, or bilateral oophorectomy at least 6 months prior to Check-in] or at least 2 years naturally postmenopausal) must have been using one of the following forms of contraception and agree to continue using it through completion of the study: hormonal method (e.g., oral, vaginal ring, transdermal patch, implant, or injection) consistently for at least 3 months prior to Check-in; double barrier method (i.e., condom with spermicide or diaphragm with spermicide) consistently for at least 2 weeks prior to Check-in; intrauterine device for at least 3 months prior to Check-in; essure® procedure at least 6 months prior to Check-in; have a partner who has been vasectomized for at least 6 months prior to Check-in. 6. Female subjects of childbearing potential who were not currently engaging in heterosexual intercourse must have agreed to use one of the above methods of birth control, in the event that they had heterosexual intercourse during the course of the study. 7. Voluntary consent to participate in this study documented on the signed informed consent form (ICF). 8. Willing to comply with the requirements of the study and willing to consider using alternative inhaled forms of nicotine other than conventional cigarettes. 9. Forced Expiratory Flow (FEF) (25 - 75%) at least 70% of the normal values predicted for that individual based on age, gender, and height.

Subjects were excluded from the study if there was evidence of any of the following criteria at Screening, Check-in, or at any time during the study as appropriate, in the opinion of the principal investigator (PI): History or presence of clinically significant gastrointestinal, renal, hepatic, neurologic, hematologic, endocrine, oncologic, urologic, pulmonary (especially bronchospastic diseases), immunologic, psychiatric, or cardiovascular disease, or any other condition that, in the opinion of the PI, would jeopardize the safety of the subject or impact the validity of the study results; (**Part 2 only**) clinically significant abnormal findings on the physical examination, ECG, or clinical laboratory results, in the opinion of the PI; (**Part 2 only**) positive test for human immunodeficiency virus (HIV), hepatitis B surface antigen (HbsAg), or hepatitis C virus (HCV); positive urine screen for alcohol or drugs of abuse at Screening or any Check-in; history of drug or alcohol abuse within 24 months of Check-in; an acute illness (e.g., upper respiratory infection, viral infection) requiring treatment within 2 weeks prior to Check-in; fever (> 100.2°F) at Screening or at Check-in; systolic blood pressure > 150 mmHg, diastolic blood pressure > 95 mmHg, or pulse rate > 99 bpm at Screening; body mass index (BMI) < 19 kg/m2 or > 35 kg/m2 at Screening; female subjects who are pregnant, lactating, or intend to become pregnant from Screening through completion of study; consumption of xanthines/caffeine, alcohol, or grapefruit juice within 24 hours of Check-in and during confinement; use of any OTC or prescription smoking cessation treatments, including, but not limited to, nicotine replacement therapies (gum, patches, lozenges, nasal spray, or inhalers), varenicline (Chantix®), or buproprion (Zyban®) within 3 months prior to screening and throughout the study; use of prescription anti-diabetic medication and/or insulin therapy within 12 months of Check-in and throughout the study; concomitant use of inhalers for any reason within 3 months prior to screening and throughout the study; plasma donation within 7 days prior to Check-in, or donation of blood or blood products, had significant blood loss, or received whole blood or a blood product transfusion within 56 days prior to Check-in; participation in a previous clinical study for an investigational drug, device, or biologic within 30 days prior to either Check-in; use of nicotine-containing products other than manufactured cigarettes and occasional e-cig use (e.g., roll-your-own cigarettes, bidis, snuff, nicotine inhaler, pipe, cigar, chewing tobacco, nicotine patch, nicotine spray, nicotine lozenge, or nicotine gum) within four weeks prior to Check-in or during study; or self-reported puffers (i.e., adult smokers who draw smoke from the cigarette into the mouth and throat but do not inhale); FTND score of < 6.

***Study Restrictions:***

Concomitant Medications

Stable doses (i.e., no dosage adjustments within 30 days prior to Check-in) of prescription or over-the-counter medications required to treat a PI-approved disease or condition (e.g., hypertension) were permitted at the discretion of the PI. Hormonal contraceptives (e.g., oral, transdermal patch, implant, injection) and hormonal replacement therapy were permitted. Occasional use of over-the-counter analgesics (e.g., acetaminophen, ibuprofen), antihistamines, and nasal decongestants were permitted. Exceptions were permitted at the discretion of the PI in consultation with the Sponsor, providing the medication in question would have no impact on the study. Any exceptions were documented.

All concomitant medications (and reasons for their use) taken by subjects during the study were recorded and coded using the most updated version of the WHO Drug Dictionary available at Celerion (e.g., Sep 2013 or later). During the study, up to 2 g per day of acetaminophen were administered at the discretion of the PI for intercurrent illness or adverse events. If other drug therapy was required, a joint decision was made by the PI and Sponsor to continue or discontinue the subject.

Foods and Beverages

Consumption of foods and beverages containing the following substances were prohibited as indicated: Xanthines/caffeine: 24 hours prior to Check-in and during confinement; alcohol: 24 hours prior to Check-in and during confinement; or grapefruit or grapefruit juice: 24 hours prior to Check-in and during confinement.

Activity

Subjects did not engage in strenuous activity in the 48 hours prior to and at any time during the confinement period.

Subject Numbering

Like Part 1, subjects were assigned a unique screening number and subject numbers for each part of the study.
In Part 2, once enrolled for study conduct, subjects were assigned a subject number from 201 - 215.
Replacement subjects, if used, were assigned a number 1000 higher than the subject being replaced (e.g., Subject 1110 would replace Subject 110).
Eligibility for inclusion into Part 1 was based on a screening visit(s) to assess medical history, concomitant medications, demographics, and smoking history (including the Fagerström Test for Nicotine Dependence [FTND]), an exhaled CO test, urine cotinine and drugs of abuse test, urine pregnancy test, vital signs, and BMI determination.
Subjects participating in Part 2 completed additional screening events including a physical examination, ECG, clinical laboratory, and serology evaluations.

**Duration of Study Conduct**

Subjects entering into Part 2 completed an additional 11-day in-clinic confinement. Subjects were confined for the entire duration of the study from Check-in the afternoon of Day-2 through the last study procedure on Day 9.

**Study Products**

**Part** 2: The study products evaluated during Part 2 included solutions administered with the eNT-100 nicotine inhaler (500 or 1000 μg nicotine), an NJOY King Bold e-cig, and the subject's usual brand combustible cigarette.

• Treatment A: 1000 μg PG (Batch: NB1168)

• Treatment B: 500 μg of 2.5% nicotine in 2 mg of PG (Batch: DEV110-25NPG)

• Treatment C: 1000 μg of 5.0% nicotine in 2 mg of PG (Batch: DEV112-50NPG)

• Treatment D: NJOY King Bold e-cigarette (Lot No.: MS 2493)

• Treatment E: Subject supplied cigarettes (Lot No.: various)

Each administration of the eNT-100 inhaler and the NJOY e-cig included 10 inhalations followed by a 5-count breathe hold at 30-second intervals. The usual brand combustible cigarette consisted of smoking 1 cigarette to completion.
**Product Administration/Experimental SessionsPart 2:** Subjects participated in all the experimental sessions according to the randomization schedule outlined in **Table 28**. Study products as outlined above were administered on Days 1, 3, 5, 7, and 9 according to the randomization schedule in **Table 28**, while Days 2, 4, 6, and 8 were washout days during which the subjects abstained from nicotine-containing products. A minimum washout of 36 hours from nicotine was required prior to each study product administration.

**Table 28: Experimental Products (Part 2)**

| Product | Total Amount of Solution Aerosolized Inhalation (μg) | Nicotine per Inhalation (μg) | Total Nicotine over 10 Inhalations (μg) | Nicotine Concentration (%) |
|---|---|---|---|---|
| Vehicle (propylene glycol only) | 500-2000 | 0 | 0 | 0 |
| eNT-100: 500 μg dose | 2000 | 50 | 500 | 2.5 |
| eNT-100: 1000 μg dose | 2000 | 100 | 1000 | 5.0 |
| e-Cig (NJOY King Bold, 4.5% concentration) | Variable | -113 | -1130 | 4.5 |
| Combustible cigarette | N/A | 145-199 | -1450-199 | N/A |

Note: % nicotine is % by volume.

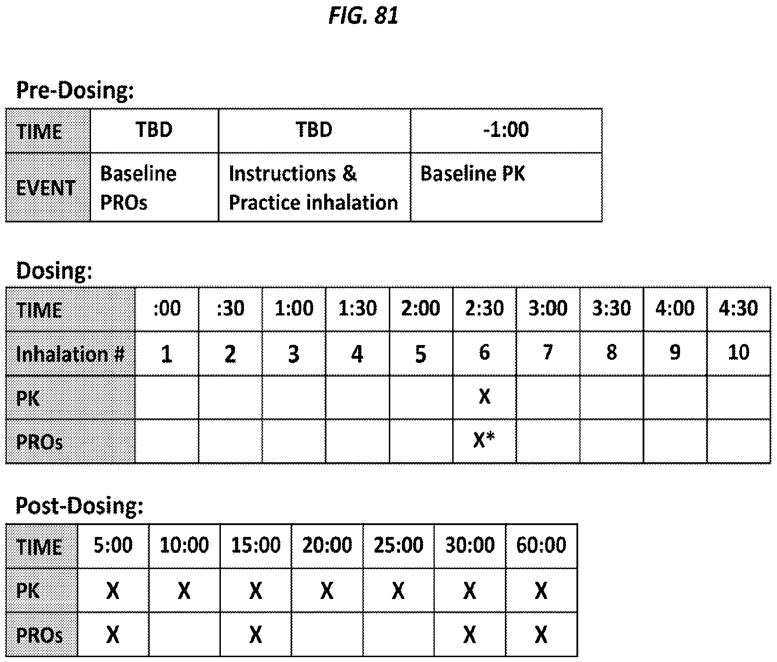

**Pharmacokinetic Sample Collection, Parameters, and Analysis:** During Part 2, as outlined in **FIG. 81,** serial blood samples were collected within 15 minutes prior to product administration and at approximately 3, 5, 10, 15, 20, 25, 30, and 60 minutes after the start of each product administration and were used to determine plasma nicotine concentrations. **Table 29** outlines the blood sample collection protocol for Part 2.

**Table 29**. Part 2 Blood Sample Collection Protocol

| Sample Type | Number of Time Points | Approximate Volume per Time Point* (mL) | Approximate Sample Volume Over Course of Study (mL) |
|---|---|---|---|
| Screening laboratory safety tests (including hematology, serum chemistry, serology). | 1 | 12.5 | 12.5 |
| On-study hematology and serum chemistry (including serum pregnancy for women) | 1 | 12.5 | 12.5 |
| PK | 45 | 4 | 180 |
| Total Blood Volume for Study (mL) → | | | 205 |

Nicotine PK parameters were computed for each nicotine-containing study product with Phoenix® WinNonlin® (Version 6.3), from the observed individual subject plasma nicotine concentration versus time profiles using noncompartmental methods and
based on actual sampling times. Plasma nicotine concentration values were adjusted for baseline concentration prior to calculating the PK parameters.

The following baseline-adjusted PK parameters were derived from the nicotine plasma concentration-time data: concentration at 5 minutes after the start of product administration (C5), the area under the nicotine concentration-time curve from time zero to the last measurable concentration $_{(AUC0-t)}$, the maximum observed concentration $_{(Cmax)}$, and the time of the maximum concentration (Tmax).

Nicotine concentrations and PK parameters were summarized by study product using descriptive statistics.

Plasma nicotine concentrations were listed by subject and time point. Concentration data were summarized by time point using descriptive statistics (number of observations [N], arithmetic mean, standard deviation [SD], coefficient of variation [CV%], minimum, median, and maximum). PK parameters were listed by subject and summarized using descriptive statistics (N, arithmetic mean, SD, CV%, minimum, median, and maximum). In addition, geometric mean and CV% were calculated for AUC0-t, Cmax, and C5. The parameter values were imported into SAS and all descriptive statistics were calculated in SAS® Version 9.3. Figures were created to display mean and individual concentration-time curves (linear and semi-log scales). Actual sampling times were used for individual figures and nominal sampling times for mean figures

Analysis of variance (ANOVA) were performed on the $_{Cmax}$, C5, and AUC PK parameters. The ANOVA model included sequence, study product, and period as fixed effects, and subject nested within sequence as a random effect. Sequence was tested using subject nested within sequence as the error term. Each ANOVA included calculation of least-squares means (LSMeans), differences between product LSMeans, and the standard errors associated with the LSMeans and differences. First order carry-over effect was tested. If it was not statistically significant, it was removed from the statistical model.

## Pharmacodynamic Assessment and Analysis

Smoking urge, aversion/tolerability, respiratory tract sensations, and subjective effects were evaluated via patient-reported outcome (PRO) measures following product administration in Part 2. The following parameters were calculated form the smoking urge-visual analog scale (SU-VAS) response data for the product administered: area under the effect curve from time 0 to t ($AUEC_{(0-t)}$), maximum ibserved reduction ($E_{max\ reduction}$), time of the maximum reduction ($T_{max\ reduction}$).

All pharmacodynamic data obtained during both parts of the study will be listed by subject and time point. The data will be summarized by time point using descriptive statistics and an appropriate statistical method (ANOVA or an appropriate non-parametric test as required by the type of data) will be used to characterize the between-group comparisons. The SU-VAS measurements and response parameters ($AUEC_{(0-t)}$ and $E_{max\ reduction}$) were listed, summarized, and analyzed using an ANOVA similar to the PK concentrations and parameters.

## Safety Assessments and Analysis

Additional safety evaluations beyond those described for Part 1 (Example 13) performed prior to inclusion in Part 2 included a physical examination, electrocardiogram (ECG), clinical laboratory (clinical chemistry, hematology, urinalysis), and serology. Part 2 Check-in evaluations included a brief physical examination (symptom-driven), vital signs, clinical laboratory (clinical chemistry, hematology, and urinalysis), urine drug and alcohol screen, and a pregnancy test (females only). End-of-Study (or Early Termination) evaluations included a brief physical examination (symptom-driven) and vital signs.

In addition, vital signs were evaluated before and after study product administration.

Adverse events (AEs) spontaneously reported by the subjects or observed by the PI or other study personnel were monitored and followed up until the symptoms or values return to normal or acceptable levels or until lost to follow-up, as appropriate in the opinion of the PI or his designee.

All reported AEs were coded to a standard set of terms, using MedDRA®, Version 16.1. The number of subjects experiencing product use-emergent AEs and the number of product use-emergent AEs were summarized by preferred term for each product and overall.

Safety data were summarized by treatment and time point. Descriptive statistics (N, mean, SD, minimum, median, and maximum) were calculated for quantitative safety data and frequency counts were compiled for classification of qualitative safety data. Summary tables for actual results and change from baseline were presented for vital signs.

## Other Non-Safety Assessments and Analysis

Other non-safety assessments were conducted as outlined in **FIG. 80** an included exhaled CO, spirometry (FVC and FEV1), pulse oximetry and PRO assessments from the 13 item mCES described herein. Subjective impressions of the aerol were assessed via a 7-point product evaluation questionnaire as described herein.

Exhaled Co (Part 2 only), spirometry, and pulse oximetry measurements were listed by subject and time point, and summarized by time point using descriptive statistics. An ANOVA was used to present the postdose to predose differences between test and reference product group comparisons. The mCES responses were listed by subject and summarized using frequency counts. Responses to the product evaluation questionnaire were listed by subject.

The dependent measures related to the spirometry device attached to the eNT-100 inhaler used to characterize subjects' inhalations was listed by subject.

## Study Assessments from Part 2:

**Conclusions:** The conclusions from Part 2 of the study were that the rate and extent of nicotine absorption, as well as early exposure up to 5 minutes, were the highest following use of the usual brand combustible cigarette. Statistically significant differences in the exposure PK parameters were found between both the 500 μg and 1000 μg doses of the eNT inhaler and the NJOY King Bold e-cig compared to the usual brand combustible cigarette. Overall, administration of nicotine in doses of 500 μg (2.5% solution) and 1000 μg (5% solution) from the eNT-100 nicotine inhaler, 10 puffs from the NJOY King Bold e-cig (4.5% nicotine solution), and a commercial combustible cigarette appeared to be safe under the conditions used in this study. Cough, throat irritation, and burning sensation were more frequently reported following nicotine dosing with the eNT-100 nicotine inhaler and the NJOY King Bold e-cig compared to the vehicle control and the combustible cigarette. All study products containing nicotine were more effective than vehicle control at reducing the urge to smoke. The Emax reduction and $_{AUEC(0-t)}$ parameter values for the vehicle control were significantly different from 0, indicating the presence of a modest placebo response. The largest response for the Emax reduction and $_{AUEC(0-t)}$ parameters was found with the combustible cigarette. None of the remaining nicotine-containing study products differed from one another, and they were all significantly greater than the vehicle control. Among the nicotine-containing products, only the difference between the 500 μg dose from the eNT-100 inhaler and the usual brand combustible cigarette was found to be statistically significant. The subjects rated the characteristics of the usual brand combustible cigarette very high on the mCES, while the characteristics of the vehicle control were rated very low. In comparison, the characteristics of the 500 μg dose from the eNT-100 inhaler, the 1000 μg dose from the eNT-100 inhaler, and the NJOY King Bold e-cig were not rated as very high or very low following a single use.

**Safety Assessments**

Overall, a total of 97 product use-emergent adverse events (AEs) were experienced by 100% of subjects. All AEs were mild in severity, with the exception of 1 moderate AE (dizziness). The incidence of AEs was lower in the vehicle control and combustible cigarette groups compared to the groups receiving nicotine via the eNT-100 inhaler and the NJOY King Bold e-cig. Cough, throat irritation, and burning sensation were the most frequently reported AEs in Part 2, experienced by 100%, 67%, and 33% of subjects, respectively. All of these events occurred following administration of the eNT-100 inhaler solutions containing nicotine and the NJOY King Bold e-cig; with only one event of cough following administration of the combustible cigarette. All cough, throat irritation, and burning sensation AEs were considered probably related to the study product by the study physician.

**Other Non-Safety Assessments:**

No statistically significant differences were noted between study products for the pulse oximetry or spirometry evaluations at any assessed time points. The exhaled CO change from pre-product administration was significantly higher (a mean difference of >5%) for the usual brand combustible cigarette compared to each of the other study products. However, there were no differences in exhaled CO change among the other products.

As indicated by the mCES responses, the usual brand combustible cigarette appeared to be the most satisfying study product overall, including characteristics such as craving reduction, enjoyment with using the study product, nicotine content, taste, and other sensory perceptions. Subjects also consistently rated the vehicle control very low on these same items. No apparent trends were evident among the 500 μg and 1000 μg doses from the eNT-100 inhaler or the NJOY King Bold e-cig for any of these items. Responses following use of these 3 study products tended to most commonly occur on the lower end of the range from "Not at all" to "Moderately". However, it is difficult to draw definitive conclusions on such subjective measures following only a single use of unfamiliar products. In addition, none of the study products tended to make the subjects nauseous, but the usual brand combustible cigarette did tend to make subjects dizzy according to Item 10 from the mCES, more so than any of the other study products.

**Pharmacodynamic Assessments:**

**Smoking Urge-Visual Analog Scale (SU-VAS):**

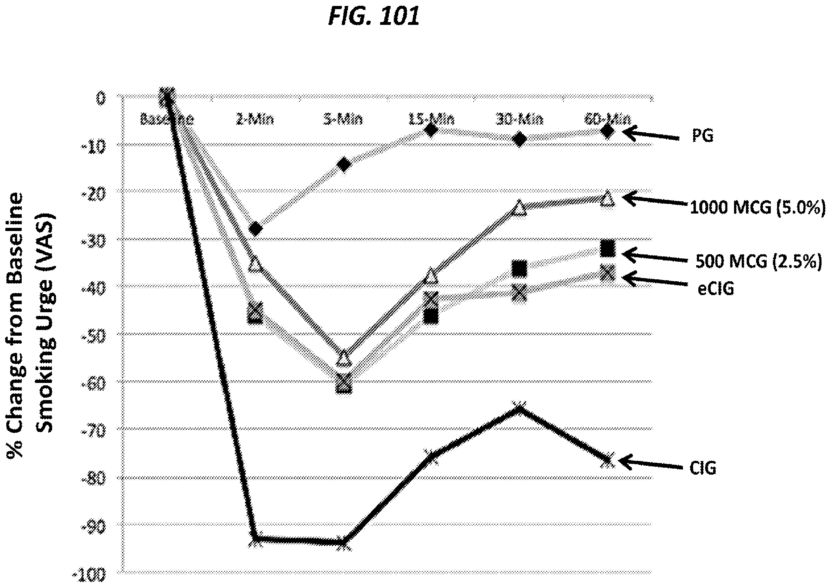

As in Part 1, the smoking urge for each subject in each cohort was assessed by rating a subject's response to "how strong is your urge to smoke right now?" on a 0-100 mm visual analog scale (VAS), where 0 = "Not at all"; 100 = "extreme". SU-VAS assessed within 15 minutes prior to and approximately 1, 15, and 30 minutes after completion of the product administration. **FIG. 101** discloses data analyzed in real-time during the course of Part 2 of the study. **FIG. 101** shows that the vehicle dose group (PG) reported a modest reduction (about 28%) in the % change from baseline smoking urge at 2 minutes post-dosing, while the 1000 mcg (5%) dose group produced an about 35% change, the 500 mcg (2.5%) mcg dose group an about 45% change, which was about the same as the NJOY King Bold e-cig, while the usual brand combustible cigarette produced a greater than 90% change at 2-minutes post-dosing. For each cohort execpt the vehicle (PG), the % change from baseline smoking urge further increased at 5-min post dosing and then gradually waned at each time point thereafter, but the 500 mcg (2.5%) cohort still showed a reduction in smoking urge at 60 min that was greater than the initial vehicle reduction (**FIG. 101**).

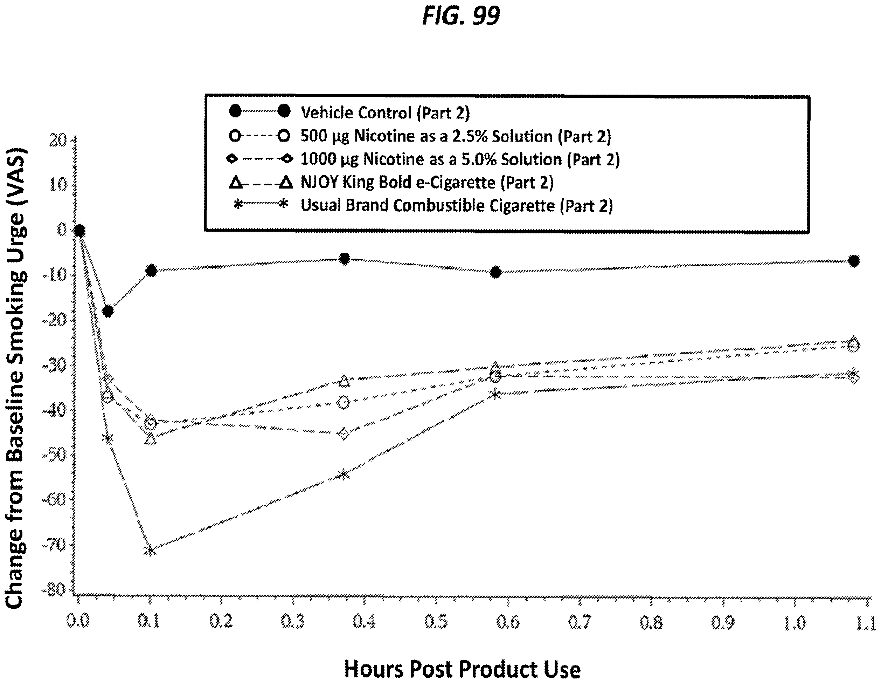

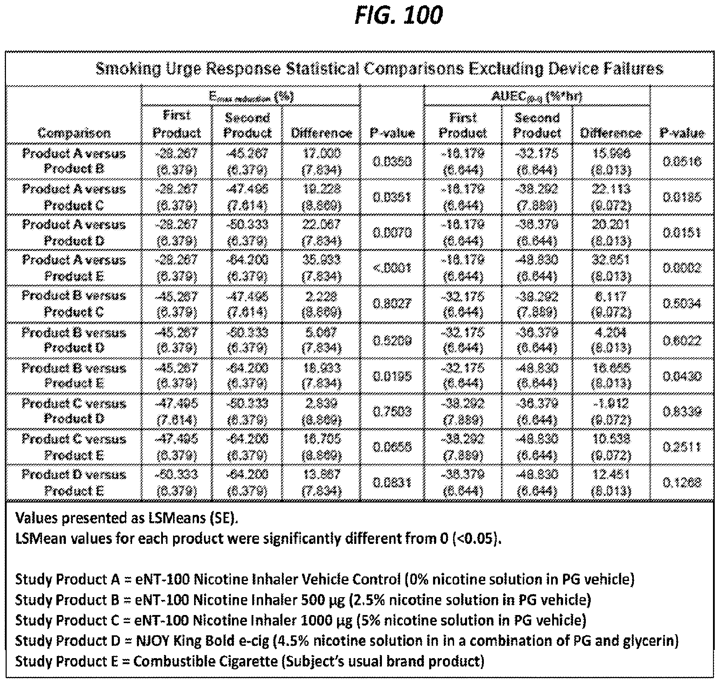

Subsequent independent analysis of the pharmacodynamic data by the CRO (Celerion, Lincoln NE) from Part 2 of the study showed that when suspected device failures were removed from consideration, the median baseline smoking urge for each treatment ranged from 77% to 86%. **FIG. 99** shows that the vehicle dose group reported a modest reduction in the % change from baseline smoking urge (vas) at 0.1 hours post-dosing, while the 500 mcg (2.5%), 1000 mcg dose groups as well as the NJOY King Bold e-cig and usual brand combustible cigarette all reported significant reductions in their % change from baseline smoking urge (vas) at 0.1 hours post-dosing, which were sustained over time. As shown in **FIG. 100,** following product use, the largest response as assessed by the mean $_{AUEC(0-t)}$ and Emax reduction parameters was found following use of the usual brand combustible cigarette. None of the other study product groups (NJOY King Bold e-cig, the 1000 μg and 500 μg doses from the eNT-100 nicotine inhaler, respectively) were significantly different from one another. However, a statistically significant difference among the nicotine-containing products was found between the 500 μg dose of the eNT inhaler and the usual brand combustible cigarette. Also of note, the $_{AUEC(0-t)}$ and Emax reduction parameters for the vehicle control group were significantly different from 0, indicating a modest placebo effect.

**A 13-Item Modified Cigarette Evaluation Scale (mCES)**

As in Part 1, subjects in Part 2 were asked to rate 13-items referred to as the modified Cigarette Evaluation Scale (mCES) using a 7-point Likert response range from 1 (not at all) to 2 (very little) to 3 (a little) to 4 (moderately) to 5 (a lot) to 6 (quite a lot) to 7 (extremely). The mCES was administered 1 minute after completion of product administration. The specific items and their respective results from Part 2 included: 'was it satisfying?' (**FIG. 102**); 'how high in nicotine?' (**FIG. 103**); did it taste good?' (**FIG. 104**); 'did you enjoy the sensations in your throat and chest?' (**FIG. 105**); 'did it calm you?' (**FIG. 106**); 'did it make you feel more awake?' (**FIG. 107**); 'did it make you fell less irritable?' (**FIG. 108**), 'did it help you concentrate?' (**FIG. 109**); 'did it reduce your hunger for food?' (**FIG. 110**); 'did it make you dizzy?' (**FIG. 111**); 'did it make you nauseous?' (**FIG. 112**); did it immediately relieve your craving for a cigarette?' (**FIG. 113**); and 'did you enjoy it?' (**FIG. 114**). In summary, subjects rated the characteristics of the usual brand combustible cigarette very high on the mCES, while the characteristics of the vehicle control were rated very low. In comparison, the characteristics of the 500 mcg dose from the eNT-100 inhaler, the 1000 mcg dose from the eNT-100 inhaler, and the NJOY King Bold e-cig were not rated very high or very low following a single use.

**Nicotine Pharmacokinetic Assessments**

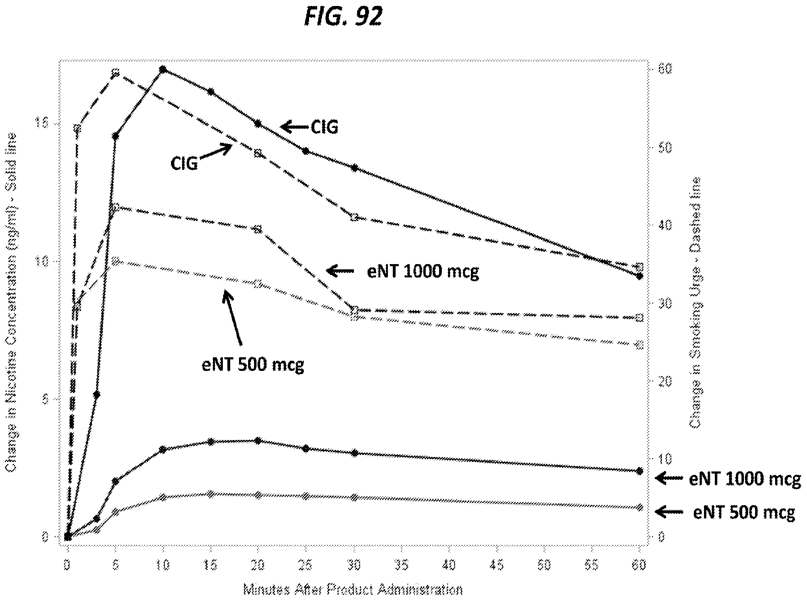

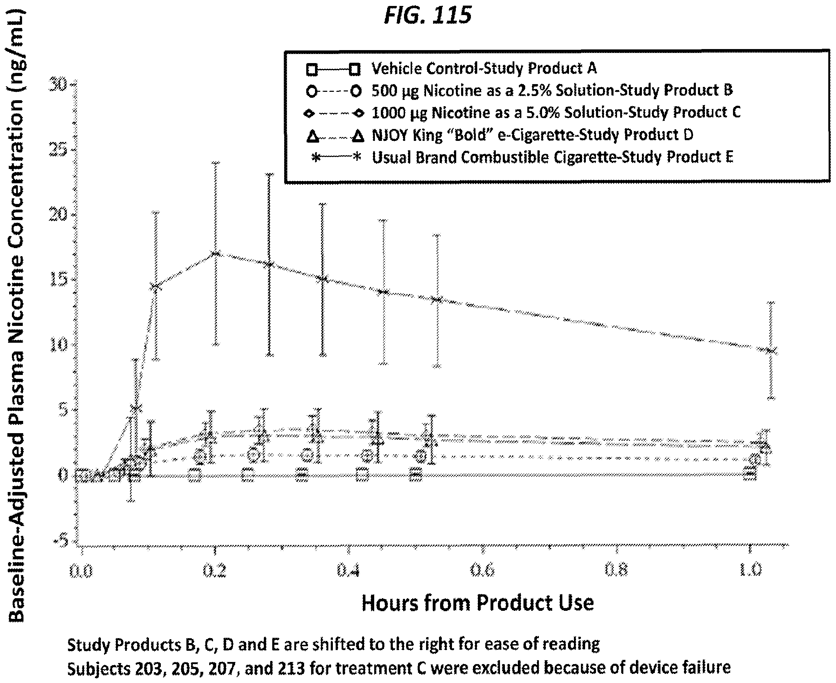

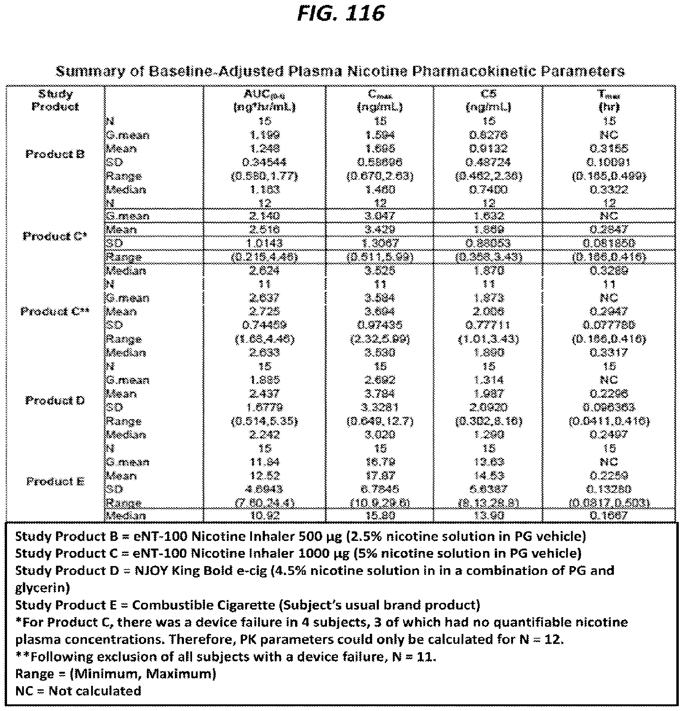

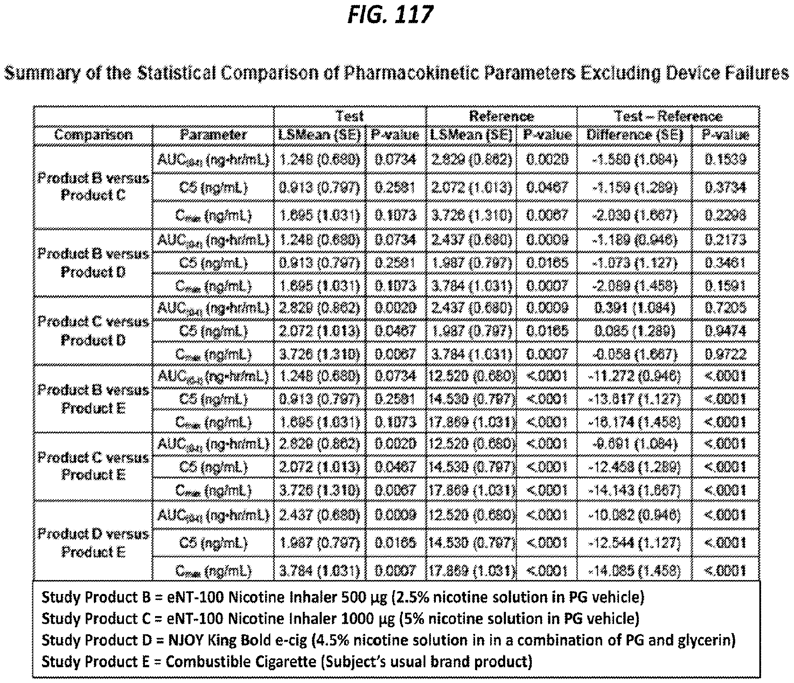

Independent anaylsis of the PK data from Part 2 of the study by the CRO (Celerion, Lincol NE) showed that administration of each nicotine-containing product produced a markedly greater mean plasma nicotine concentration from baseline **(FIG. 115).** Mean plasma nicoetine concentrations from baseline for the 500 mcg dose were lower than the 1000 mcg dose as well as the NJOY King Bol e-cig and combustible cigarette, while the combustible cigarette produced the highest adjusted plasma nicotine concentration, as shown in **FIG. 115.** Additionally, **FIG. 116** shows that the median AUC0-t, Cmax, and C5 were the highest following use of the usual brand combustible cigarette. Also, **FIG. 116** shows that the median $_{Tmax}$ varied from 10 minutes for the usual brand combustible cigarette, through 15 minutes for the NJOY King Bold e-cig, to 20 minutes for the 500 and 1000 μg doses from the eNT-100 nicotine inhaler. FIG. 117 shows that neither the $_{Cmax/C5}$ nor AUC comparisons revealed any significant differences among the 500 μg dose of the eNT inhaler (Product B), the 1000 μg dose of the eNT inhaler (Product C), and the NJOY King Bold e-cig (Product D). Statistically significant differences in AUC, $_{Cmax}$, and C5 were found between the usual brand cigarette (Product E) and both Nicotine Inhaler products (B and C) and the NJOY King Bold e-cig. Additionally, **FIG. 92** shows the efficient use of nicotine to produce a reduction in smoking urge by subjects in the 500 mcg (2.5%) and 1000 mcg (5%) cohorts from Part 2 of the study as compared to subjects in the combustible cigarette cohort. The solid lines illustrate the change in nicotine concentration (ng/ml) as a function of the minutes after product administration, while the dashed lines illustrate the change in smoking urge as a function of the minutes after product administration. The products used were CIG (tobacoo cigarette), eNT 500 mcg (eNT-101 500 mcg (2.5%) cohort), and eNT 1000 mcg (eNT-101 1000 mcg (5%) cohort).

**Description of the eNT-100 Nicotine Inhaler**

The aerosol was created inside the eNT-100 inhaler as shown in **FIG. 82**, which was itself inside a small cylindrical plastic housing that was used to blind the test subject from the test article. The test subject inhaled from a plastic tube that slides over the stainless-steel mouthpiece shown. Inside of the aerosol-generating inhaler was a small heater element that was used to vaporize the nicotine solution under flow conditions that resulted in a 1.4 to 2.5 micron aerosol particle. A typical particle size distribution as produced from the eNT-100 inhaler can be seen in **FIG. 28**, which further illustrated how the particles size distribution produced from the eNT-100 inhaler differed in size and mass from that of a commercially available e-cigarette (BLU e-cig.). The nicotine inhaler further comprised a positive displacement pump to meter out a dose of the nicotine solution onto the heater element.

The eNT-100 was designed to create the aerosol when the inhalation rate reaches 20 lpm (about $3 \times 10^{-4}$ m³/s). At that flow rate the aerosol produced had a particle size of 2.5 micron volume median diameter (VMD) with a GSD of 1.6. The upper end of the inhalation flow rate was determined by the flow rate that can be produced under what is considered an upper limit of vacuum that the human lung can produce by inhalation (13 inches of water is considered that upper limit (about 3235 Pa)). At that vacuum, the inhalation flow rate was 50 lpm (about $8.33 \times 10^{-4}$ m³/s) and the particle size was 1.4 micron VMD with a GSD of 1.2.

The bulk of the aerosol was created within 1 second of the inhaler being breath-activated. Within 1.4 seconds the entire aerosol was created. An estimate of the aerosol produced between the 1 second and the 1.4 second time point was around 5 - 10% of the total amount of the aerosol. As a result, the bulk of the aerosol was delivered to the respiratory tract in the first 1/3 to 1/2 of the volume of the total inhalation volume, thereby allowing the aerosol to be "chased" down into the deep lung by the balance of the inhalation.

The eNT-100 system can generate an emitted dose of +/-20% of the dose (or loaded dose). The dose (or loaded dose) can be the amount of nicotine solution pumped onto the heater element prior to the creation of the aerosol and can be +/-2% of the target dose (the label claimed dose or goal dose). The emitted dose can be 92% to 97% of the dose. For example, the amount actually delivered to the lung if the label claim dose is 100 $\mu$g would be between 90% and 99%.

**Example 15: Consumer Testing Study**

In this example, a comparison study was conducted to examine consumer preference between an aerosol generated using the eNT-100 device described in Examples 13 and 14 and modified aerosols generated as described below. The primary objective of the consumer testing was to aerosolize a larger amount of material in the 1 micron range to create an enhanced experience for the smoker. Two key comparisons were run: 1.) modified aerosol produced from product A vs. the results obtained in Example 14 (i.e.,Part 2 eNT-100 aerosol) and 2.) modified aerosol from product B vs. the results obtained in Example 14 (i.e., Part 2 eNT-100 aerosol). Product A comprised a device similar to eNT-100 (**FIG. 82**), but was configured to have a higher resistance than the eNT-100 device but still low enough to enable direct-to-lung inhalation (though at lower inhalation rates) and to conduct discrete doing of 2 mg of material. The flow resistance in the Product A device was about 0.4 (cm $H_2O$)$^{1/2}$/LPM. Product B comprised a device similar to eNT-100 (**FIG. 82**), but was configured to have a resistance such that a mouth-breathing maneuver (in order to mimic smoking pattern) was required and to conduct continuous dosing of 2 mg/sec of material through about 4 sec. The flow resistance in the Product B device was about 2.65 (cm $H_2O$)$^{1/2}$/LPM

**Methods:**

*Summary*

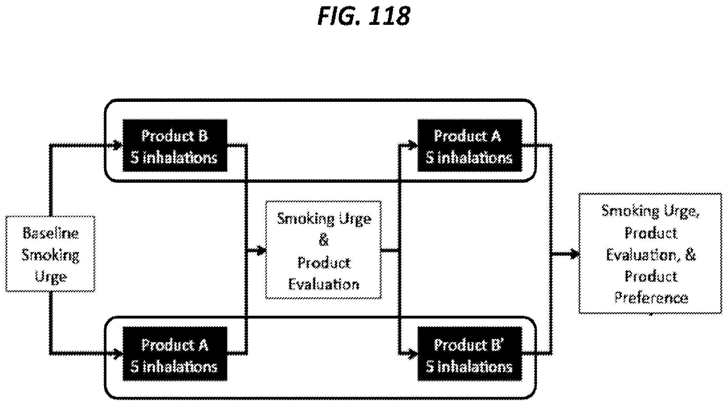

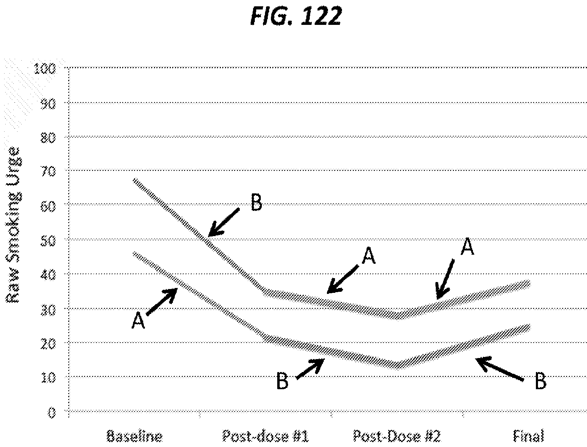

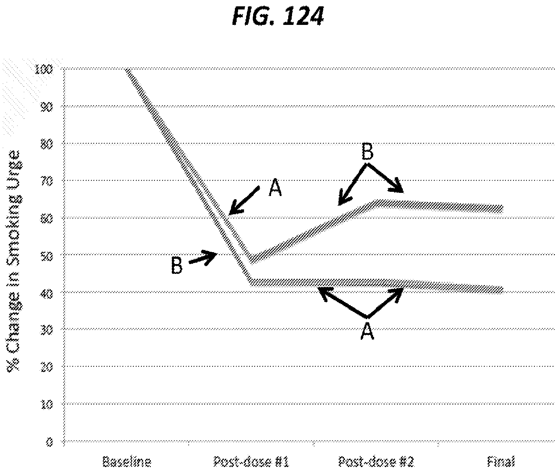

The study recruited 29 smokers (averaging 17.7 cigarettes per day) with a mean age of 43.9 years old. All smokers primarily smoked cigarettes while some also experimented with e-cigarettes. The 29 smokers had a mean years of smoking of 22.8 and a mean Fagerstrom Test of Nicotine Dependence (FTND) score of 6.7. The 29 smokers were randomly assigned to 2 groups involving dosing with 5 inhalations from each product (i.e., Product A anf B) as outlined in FIG. 118 where following baseline smoking urge assessment, the first group initially received 5 inhalations from Product B followed by a smoking urge assessment and product evaluation followed by 5 inhalations from Product A and subsequent smoking urge assessment, product evaluation and product preference assessment, while the second group initially received 5 inhalations from Product A followed by a smoking urge assessment and product evaluation followed by 5 inhalations from Product B and subsequent smoking urge assessment, product evaluation and product preference assessment. The 5 inhalations from 2 aerosols were performed in a back-to-back manner. Smoking urge assessment was performed using the smoking urge VAS as described and used in Examples 13 and 14, while product evaluation was performed using a modified mCES questionnairre described and used in Examples 13 and 14. The smoking urge VAS and mCES assessments were conducted approximately 15 seconds after the 5$^{th}$ inhalation of both Product A and Product B, while a final assessment of smoking urge VAS was conducted approximately 10 minutes after the final dosing of each subject's second product administration (see "Final" in **FIGs. 122** and **124).**

The nicotine solution used in both Products A and B was at a nicotine concentration of 2% and further contained 80% propylene glycol and 20% vegetable glycerin. The total nicotine dose administered using the products as outlined was 200 mcg (40 mcg/puff) for Product A and about 490 mcg for Product B. Product B delivered about 2.5 times more nicotine than Product A. Prior to participating, the subjects were asked to undergo nicotine deprivation from about 2 to 4 hours, which was not formally verified by study sponsor. Additionally, the endpoints in this example were subjective, single post-dose usge assessments. Comparatively, Examples 13 and 14 entailed PK and subjective data as well as extended post-dose assessments using sponsor verified 12 hour nicotine deprivation.

**Study Assessments from Consumer Testing:**

**Safety Assessments**

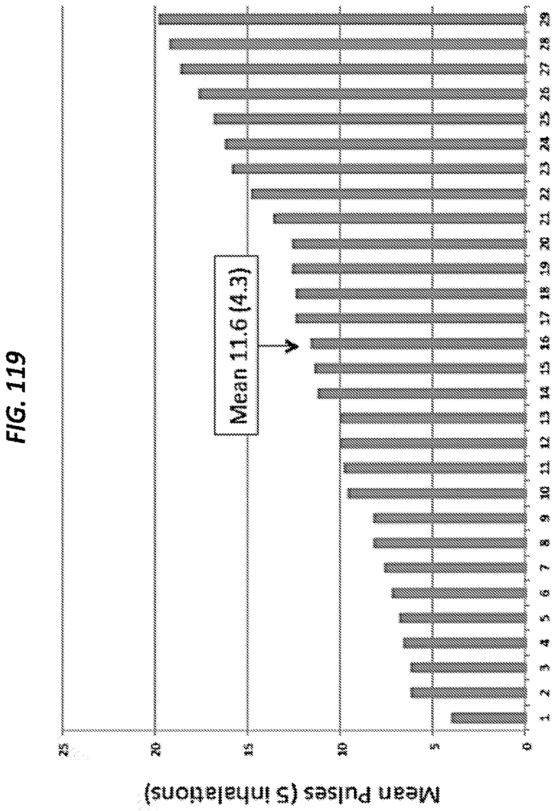

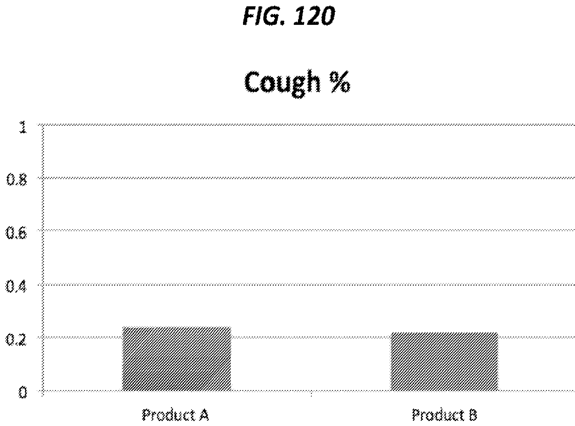

Overall, there were no unexpected nor serious adverse effects. As shown in **FIG. 120** showed that the percentage of subjects experiencing coughing was similar for both Products A and B and was only about 20%. Additionally, **FIG. 119** shows that the mean pulse of each subject over 5 inhalations was not of concern.

**Other Non-Safety Assessments:**

As indicated by the mCES responses, the aerosol produced by Product B appeared to be the most satisfying study product used in this study, including characteristics such as craving reduction, enjoyment with using the study product, nicotine content, taste, and other sensory perceptions. Additionally, when subjects were asked "did you have a preference for one aerosol vs. the other?" the subjects clearly preferred the Product B aerosol by a 55% to 45% margin. This preference was not affected by the order of aerosol administration, nor did coughing frequency predict preference. Moreover, with regards to Product B, there was little correlation between the number of pulses and satisfaction (mCES #1; -0.18) or liking (mCES #6; -0.20). Also, when offered to have more of Product A or B, 1 subject chose to use more of Product A, while 1 subject chose to use more of Product B.

**Pharmacodynamic Assessments:**

**Smoking Urge-Visual Analog Scale (VAS):**

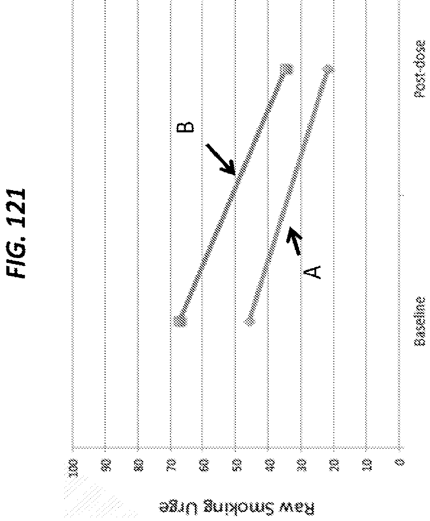

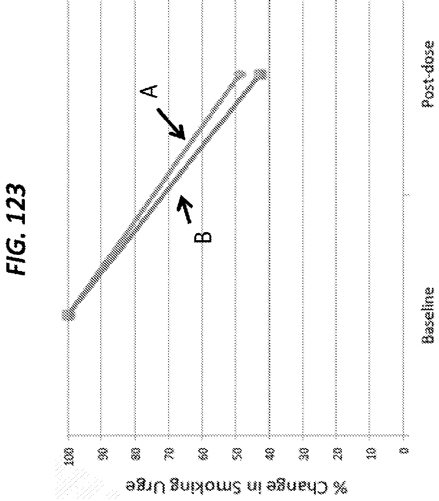

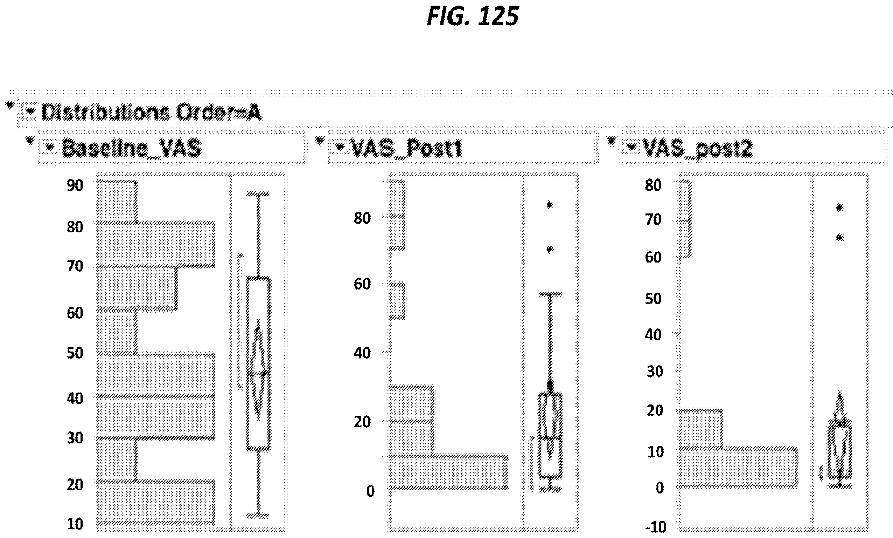

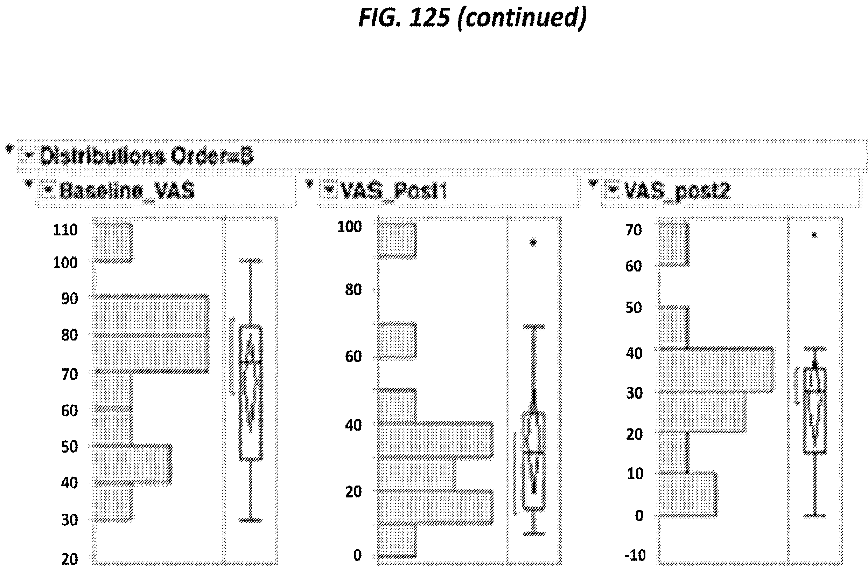

The mean number of hours of smoking deprivation prior to dosing was 3.5 hours with a standard deviation of 3. As in Examples 13 and 14, the smoking urge for each subject in each cohort was assessed by rating a subject's response to "how strong is your urge to smoke right now?" on a 0-100 mm visual analog scale (VAS), where 0 = "Not at all"; 100 = "extreme". Analysis of the raw data (**FIG. 125**) during the course of the study showed that the raw change in smoking urge following the initial dose of Product B versus Product A was slightly greater (slope of Product B line vs. slope of Product A line in **FIG. 121**). This observation was further reflected in **FIG. 123**, where the % change in smoking urge as compared to baseline for Product B was slightly less than 60% versus Product A which was slightly more than 50%. Additionally, analysis of the data following administration of the second dose showed in the raw data (**FIG. 122**) that administration of either Product A or B produced a further reduction in raw smoking urge regardless of which aerosol was received initially. However, **FIG. 124** showed that when compraed to the baseline, the % change in smoking urge baseline was maintained in the group that received Product B followed by Product A, while the satiation of smoking urge was alleviated in the group that received Product A followed by Product B.

**A 13-Item Modified Cigarette Evaluation Scale (mCES)**

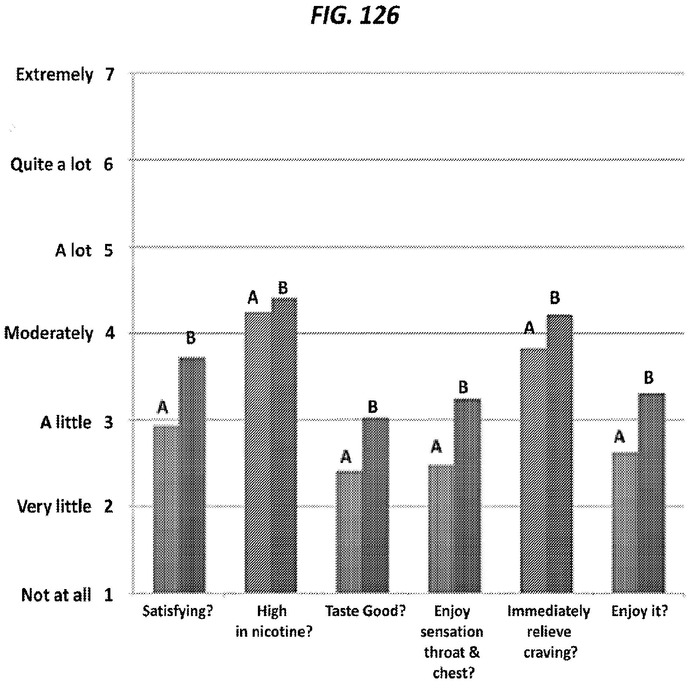

The mCES used in this example subjects were asked to rate 6-items referred to as the using a 7-point Likert response range from 1 (not at all) to 2 (very little) to 3 (a little) to 4 (moderately) to 5 (a lot) to 6 (quite a lot) to 7 (extremely). The specific items and their respective results from this Example are shown in **FIG. 126** and included: 'was it satisfying?'; 'how high in nicotine?'; did it taste good?'; 'did you enjoy the sensations in your throat and chest?'; did it immediately relieve your craving for a cigarette?'; and 'did you enjoy it?'. In summary, subjects rated the characteristics of theaerosol from Product B higher on the mCES scale than the aerosol from Product A. In comparison, the characteristics of the Product B aerosol were generally similar to the 500 mcg dose from the eNT-100 inhaler **(FIGs. 118-121** and **129-130)**,while the Product A aerosol were generally similar to the 1000 mcg dose from the eNT-100 inhaler **(FIGs. 118-121** and **129-130).**
While preferred embodiments have been shown and described herein, it will be obvious to those skilled in the art that such embodiments are provided by way of example only. Numerous variations, changes, and substitutions will now occur to those skilled in the art. It should be understood that various alternatives to the embodiments of the invention described herein may be employed. It is intended that the following claims define the scope of the invention and that methods and structures within the scope of these claims be covered thereby.

**Claims**

1.   An aerosol generating device comprising:

a liquid formulation comprising a pharmaceutically active agent,
a heater element, and a control program,
wherein the control program comprises a first phase and a second phase,
**characterized in that** the first phase controls delivery of a first amount of the liquid formulation to the heater element to generate a first aerosol comprising a first diameter and the second phase controls delivery of a second amount of the liquid formulation to the heater element to generate a second aerosol comprising a second diameter,
wherein the first amount is different from the second amount.

2. The aerosol generating device of claim 1, further comprising an airflow channel comprising an inlet and an outlet, wherein the heater element is located within the airflow channel between the inlet and the outlet.

3. The aerosol generating device of claim 2, wherein the device further comprises an additional airflow channel connected to the airflow channel.

4. The aerosol generating device of claim 3, wherein the additional airflow channel connects between the outlet and the heater element in the airflow channel.

5. The aerosol generating device of claim 3, wherein the additional airflow channel connects to the airflow channel between the inlet and the heater element.

6. The aerosol generating device of any one of claims 3 to 5, wherein the airflow channel is configured to produce the first aerosol and the second aerosol in the device.

7. The aerosol generating device of any one of claims 1 to 6, wherein the first diameter is from about 1 $\mu$m to about 5 $\mu$m .

8. The aerosol generating device of any one of claims 1 to 7, wherein the second diameter is less than about 1 $\mu$m.

9. The aerosol generating device of any one of claims 1 to 8, wherein the device further comprises a pump, wherein the first phase directs the pump to deliver the first amount to the heater element, and wherein the second phase directs the pump to deliver the second amount to the heater element.

10. The aerosol generating device of claim 9, wherein the first phase directs the pump to operate at a first rate, and wherein the second phase directs the pump to operate at a second rate, wherein the first rate and the second rate are different.

11. The aerosol generating device of any one of claims 1 to 10, wherein the heater element comprises a coil comprising electrically resistive material; and
wherein the heater element further comprises a wicking element in fluid communication with the liquid formulation comprising nicotine and wherein the coil comprising electrically resistive material is wrapped around the wicking element.

12. The aerosol generating device of claim 11, wherein the wicking element comprises electrically resistive material.

13. The aerosol generating device of any of claims 1 to 12, wherein the first phase and the second phase occur sequentially during a use of the device.

14. A method for generating aerosols from a liquid formulation comprising a pharmaceutically active agent, the method comprising:

delivering a first amount of the liquid formulation comprising a pharmaceutically active agent to a heater element in an aerosol generating device,
activating the heater element a first time, wherein the first activation of the heater element produces a first aerosol comprising a first diameter,
**characterized by**:

delivering a second amount of the liquid formulation comprising a pharmaceutically active agent to the heater element; and
activating the heater element a second time, wherein the second activation of the heater element produces a second aerosol comprising a second diameter, wherein the first amount is different than the second amount.

15. The method of claim 14, wherein the delivering the second amount occurs after the delivering of the first amount, and wherein the delivering of the first amount and the delivering of the second amount occur during a use of the device by a subject.

**Patentansprüche**

1.  Aerosolerzeugungsvorrichtung, umfassend:

    eine flüssige Formulierung, die einen pharmazeutisch aktiven Wirkstoff umfasst,
    ein Heizelement und ein Steuerprogramm,
    wobei das Steuerprogramm eine erste Phase und eine zweite Phase umfasst,
    **dadurch gekennzeichnet, dass** die erste Phase die Abgabe einer ersten Menge der flüssigen Formulierung an das Heizelement zur Erzeugung eines ersten Aerosols mit einem ersten Durchmesser steuert und die zweite Phase die Abgabe einer zweiten Menge der flüssigen Formulierung an das Heizelement zur Erzeugung eines zweiten Aerosols mit einem zweiten Durchmesser steuert,
    wobei sich die erste Menge von der zweiten Menge unterscheidet.

2.  Aerosolerzeugungsvorrichtung nach Anspruch 1, ferner umfassend einen Luftströmungskanal, der einen Einlass und einen Auslass umfasst, wobei sich das Heizelement innerhalb des Luftströmungskanals zwischen dem Einlass und dem Auslass befindet.

3.  Aerosolerzeugungsvorrichtung nach Anspruch 2, wobei die Vorrichtung ferner einen zusätzlichen Luftströmungskanal umfasst, der mit dem Luftströmungskanal verbunden ist.

4.  Aerosolerzeugungsvorrichtung nach Anspruch 3, wobei der zusätzliche Luftströmungskanal eine Verbindung zwischen dem Auslass und dem Heizelement im Luftströmungskanal bildet.

5.  Aerosolerzeugungsvorrichtung nach Anspruch 3, wobei der zusätzliche Luftströmungskanal eine Verbindung mit dem Luftströmungskanal zwischen dem Einlass und dem Heizelement bildet.

6.  Aerosolerzeugungsvorrichtung nach einem der Ansprüche 3 bis 5, wobei der Luftströmungskanal dazu konfiguriert ist, das erste Aerosol und das zweite Aerosol in der Vorrichtung zu erzeugen.

7.  Aerosolerzeugungsvorrichtung nach einem der Ansprüche 1 bis 6, wobei der erste Durchmesser zwischen etwa 1 $\mu$m und etwa 5 $\mu$m beträgt.

8.  Aerosolerzeugungsvorrichtung nach einem der Ansprüche 1 bis 7, wobei der zweite Durchmesser weniger als etwa 1 $\mu$m beträgt.

9.  Aerosolerzeugungsvorrichtung nach einem der Ansprüche 1 bis 8, wobei die Vorrichtung ferner eine Pumpe umfasst, wobei die erste Phase die Pumpe anweist, die erste Menge an das Heizelement abzugeben, und wobei die zweite Phase die Pumpe anweist, die zweite Menge an das Heizelement abzugeben.

10. Aerosolerzeugungsvorrichtung nach Anspruch 9, wobei die erste Phase die Pumpe anweist, mit einer ersten Rate zu arbeiten, und wobei die zweite Phase die Pumpe anweist, mit einer zweiten Rate zu arbeiten, wobei sich die erste Rate und die zweite Rate voneinander unterscheiden.

11. Aerosolerzeugungsvorrichtung nach einem der Ansprüche 1 bis 10, wobei das Heizelement eine Spule mit einem elektrischen Widerstandsmaterial umfasst; und wobei das Heizelement ferner ein Dochtelement umfasst, das in Fluidverbindung mit der flüssigen Formulierung, die Nikotin umfasst, steht, und wobei die Spule, die elektrisches Widerstandsmaterial umfasst, um das Dochtelement gewickelt ist.

12. Aerosolerzeugungsvorrichtung nach Anspruch 11, wobei das Dochtelement elektrisches Widerstandsmaterial umfasst.

13. Aerosolerzeugungsvorrichtung nach einem der Ansprüche 1 bis 12, wobei die erste Phase und die zweite Phase während einer Verwendung der Vorrichtung nacheinander stattfinden.

14. Verfahren zum Erzeugen von Aerosolen aus einer flüssigen Formulierung, die einen pharmazeutisch aktiven Wirkstoff umfasst, wobei das Verfahren Folgendes umfasst:

Abgeben einer ersten Menge der flüssigen Formulierung, die einen pharmazeutisch aktiven Wirkstoff umfasst, an ein Heizelement in einer Aerosolerzeugungsvorrichtung, erstmaliges Aktivieren des Heizelements, wobei die erste Aktivierung des Heizelements ein erstes Aerosol mit einem ersten Durchmesser erzeugt, **gekennzeichnet durch**:

Abgeben einer zweiten Menge der flüssigen Formulierung, die einen pharmazeutisch aktiven Wirkstoff umfasst, an das Heizelement; und zweitmaliges Aktivieren des Heizelements, wobei die zweite Aktivierung des Heizelements ein zweites Aerosol mit einem zweiten Durchmesser erzeugt, wobei sich die erste Menge von der zweiten Menge unterscheidet.

**15.** Verfahren nach Anspruch 14, wobei das Abgeben der zweiten Menge nach dem Abgeben der ersten Menge stattfindet, und wobei das Abgeben der ersten Menge und das Abgeben der zweiten Menge während einer Verwendung der Vorrichtung durch ein Subjekt stattfindet.

**Revendications**

**1.** Dispositif de génération d'aérosols comprenant:

une formulation liquide comprenant un agent pharmaceutiquement actif, un élément chauffant, et un programme de commande, où le programme de commande comprend une première phase et une seconde phase, **caractérisé en ce que** la première phase commande la distribution d'une première quantité de la formulation liquide à l'élément chauffant pour générer un premier aérosol comprenant un premier diamètre et la seconde phase commande la distribution d'une seconde quantité de la formulation liquide à l'élément chauffant pour générer un second aérosol comprenant un second diamètre, où la première quantité est différente de la seconde quantité.

**2.** Dispositif de génération d'aérosols selon la revendication 1, comprenant en outre un canal d'écoulement d'air comprenant une entrée et une sortie, où l'élément chauffant est situé dans le canal d'écoulement d'air entre l'entrée et la sortie.

**3.** Dispositif de génération d'aérosols selon la revendication 2, où le dispositif comprend en outre un canal d'écoulement d'air supplémentaire raccordé au canal d'écoulement d'air.

**4.** Dispositif de génération d'aérosols selon la revendication 3, où le canal d'écoulement d'air supplémentaire se raccorde entre la sortie et l'élément chauffant dans le canal d'écoulement d'air.

**5.** Dispositif de génération d'aérosols selon la revendication 3, où le canal d'écoulement d'air supplémentaire se raccorde au canal d'écoulement d'air entre l'entrée et l'élément chauffant.

**6.** Dispositif de génération d'aérosols selon l'une quelconque des revendications 3 à 5, où le canal d'écoulement d'air est configuré pour produire le premier aérosol et le second aérosol dans le dispositif.

**7.** Dispositif de génération d'aérosols selon l'une quelconque des revendications 1 à 6, où le premier diamètre est compris entre environ 1 et 5 $\mu$m.

**8.** Dispositif de génération d'aérosols selon l'une quelconque des revendications 1 à 7, où le second diamètre est inférieur à environ 1 $\mu$m.

**9.** Dispositif de génération d'aérosols selon l'une quelconque des revendications 1 à 8, où le dispositif comprend en outre une pompe, où la première phase indique à la pompe de distribuer la première quantité à l'élément chauffant, et où la seconde phase indique à la pompe de distribuer la seconde quantité à l'élément chauffant.

**10.** Dispositif de génération d'aérosols selon la revendication 9, où la première phase indique à la pompe de fonctionner selon un premier débit, et où la seconde phase indique à la pompe de fonctionner selon un second débit, où le premier débit et le second débit sont différents.

**11.** Dispositif de génération d'aérosols selon l'une quelconque des revendications 1 à 10, où l'élément chauffant comprend une bobine comprenant un matériau électro-résistant; et
où l'élément chauffant comprend en outre un élément à mèche en communication fluide avec la formulation liquide comprenant de la nicotine et où la bobine comprenant le matériau électro-résistant est enroulée autour de l'élément à mèche.

**12.** Dispositif de génération d'aérosols selon la revendication 11, où l'élément à mèche comprend un matériau électro-résistant.

**13.** Dispositif de génération d'aérosols selon l'une quelconque des revendications 1 à 12, où la première phase et la seconde phase se déroulent de manière séquentielle pendant une utilisation du dispositif.

**14.** Procédé de génération d'aérosols à partir d'une formulation liquide comprenant un agent pharmaceutiquement actif, le procédé comprenant:

la distribution d'une première quantité de la formulation liquide comprenant un agent pharmaceutiquement actif à un élément chauffant dans un dispositif de génération d'aérosols,
l'activation de l'élément chauffant une première fois, où la première activation de l'élément chauffant produit un premier aérosol comprenant un premier diamètre,
**caractérisé par**:

la distribution d'une seconde quantité de la formulation liquide comprenant un agent pharmaceutiquement actif à l'élément chauffant; et
l'activation de l'élément chauffant une seconde fois, où la seconde activation de l'élément chauffant produit un second aérosol comprenant un second diamètre, où la première quantité est différente de la seconde quantité.

**15.** Procédé selon la revendication 14, où la distribution de la seconde quantité se déroule après la distribution de la première quantité, et où la distribution de la première quantité et la distribution de la seconde quantité se déroulent pendant une utilisation du dispositif par un sujet.

**FIG. 1**

**FIG. 2A**

**FIG. 2B**

Area liquid is vaporized

Electrical connection

Agent
(e.g., nicotine
ejected)

Thin wall tubing
(0.0005-0.003 inches)

+ ‖‖‖ −
Battery

**FIG. 3A**

Narrower and higher
resistance section

Area liquid is vaporized

Thick walled
tubing

Agent
(e.g., nicotine
ejected)

+ ‖‖‖ −
Battery

**FIG. 3B**

**FIG. 4**

**FIG. 5**

**FIG. 6**

FIG. 7

FIG. 8

FIG. 9

**FIG. 10A**

**FIG. 10B**

**FIG. 11**

**FIG. 12**

**FIG. 13**

**FIG. 14**

**FIG. 15**

1604a

1604b

+

-

1606

1602

**FIG. 16A**

Heater surface rotated to apply mixture

**FIG. 16B**

1710a

Air Flow
1704a

1706a

1702a

1708a

**FIG. 17A**

1702b

Air Flow
1704b

1708b

1710b

**FIG. 17B**

Air routing upon valve opening

Piston

1806

Air routing after initial part of inhalation

1808

1804

1802

Air Flow

**FIG. 18**

Mean Nicotine Intake

Reduced Challenge Dose

Mean Craving Over Time

Time

**FIG. 19**

Actual Nicotine Intake

Feedback re: Nicotine Intake

Enhanced Self-Efficacy

Time

*FIG. 20*

2108

2104

2110

2114

2112

2102

2106

2116

2100

2118

*FIG. 21*

2204

(+)

(-)

2204

To Reservoir
2208

2202

2206

2200

*FIG. 22*

**FIG. 23**

**FIG. 24**

**FIG. 25A**

**FIG. 25B**

**FIG. 26**

**FIG. 27A**

**FIG. 27B**

**FIG. 27C**

**FIG. 27D**

FIG. 28

Aerosol particle size (microns)

Mass

devices

eCig

2902a

2906a

2904a

2908a

2912a

Vac — 2910a

**FIG. 29A**

2902b

3/4 inch
radius

Flow to
flow meter

**FIG. 29B**

3000a

3/8 inch
radius

**FIG. 30A**

3002b

D

L

**FIG. 30B**

**FIG. 31A**

**FIG. 31B**

3102c

3106c

(-)

(+)

3112c

**FIG. 31C**

3102d

3106d

(+)

(-)

3112d

**FIG. 31D**

3114e

3116e

**FIG. 31E**

**FIG. 32A**

**FIG. 32B**

**FIG. 32C**

3202d

3204d

3204d

3202d

**FIG. 32D**

3206e

3204e

3204e

3202e

**FIG. 32E**

3302

3304

3306

3308

3302

**FIG. 33**

3702

3704

3706

**FIG. 34**

3504    3502    3528

3514    $P_1$    3506

3518    3520

3512    $P_1$

3526

3510    $P_2$    3516

3524

3530

$P_A$

3508

**FIG. 35**

3610    3606

Air flow    3608

3604a    3604b

Brass contact (+)    Brass contact (-)
3602a    3602b

**FIG. 36**

3702a

3704a

**FIG. 37A**

3702b

3704b

**FIG. 37B**

3806

3808

3804

3810

3802

+ −

**FIG. 38**

**FIG. 39**

**FIG. 40**

*4100*

Network
Interface *4102*

Optional
Display *4108*

*4104* Processing
Unit

*4110*

*4118*

Computer-
readable storage
medium

Memory

Operating System *4112*

Agent Delivery Routine *4114*

*4106*

**FIG. 41**

**FIG. 42**

## Ascending Dose Sequence

**Group #1**: Placebo — Practice → Inhalations #1 ~ #10

**Group #2**: Vehicle (1 mg propylene glycol) — Practice → Inhalations #1 ~ #10

**Group #3**: 250 µg — Practice → 25 µg Inhalations #1 ~ #10

Safety & Tolerability Review

**Group #4**: 500 µg — Practice → 50 µg Inhalations #1 ~ #10

Safety & Tolerability Review

**Group #5**: 750 µg — Practice → 75 µg Inhalations #1 ~ #10

Safety & Tolerability Review

**Group #6**: 1000 µg — Practice → 100 µg Inhalations #1 ~ #10

Randomization

N = 12 per group

Post-Dose Assessments to Determine MTD

Safety, tolerability, liking, craving reduction

EP 3 698 832 B1

146

FIG. 43

*FIG. 44B*

A

A

*FIG. 44A*

Aerosol Inlet
4404

Flow of Aerosol
4410

4408
Carrier Gas

Baffle
4402

Large Particles
(> 5 μM)

Small Particles
(< 5 μM)

Outlet
4406

*FIG. 44C*

# FIG. 45

Schedule of Assessments for the First Portion of the eNT-101 Study.

| Procedures for All Cohorts | Screening (Day -14 to Day -1) | Pre-dose (Baseline) | Practice | Dosing #1-10 | Post-Dosing | Early Termination |
|---|---|---|---|---|---|---|
| Informed consent | X | | | | | |
| Inclusion/exclusion criteria | X | $X^a$ | | | | |
| Physical examination including BMI | X | | | | | |
| Medical and psychiatric history | X | | | | | |
| Smoking history | X | | | | | |
| Fagerström Nicotine Dependence Score | X | | | | | |
| Intention to quit smoking in next 3 months | X | | | | | |
| Exhaled CO level | $X^b$ | $X^c$ | | | X | X |
| Pulmonary function tests (spirometry) | | $X^d$ | | | $X^d$ | $X^d$ |
| Vital signs, including height$^e$, weight$^f$, heart rate, respirations, blood pressure (supine and standing) | X | X | | | X | X |
| ECG | X | | | | X | X |
| Pregnancy test | $X^g$ | $X^h$ | | | | |
| Urine sample for cotinine and drugs of abuse | X | X | | | | |
| Blood sample for chemistry, hematology and urinalysis | X | | | | X | X |
| Concomitant medication and consumption review | X | $X^i$ | | | | |
| Drug Administration or Dosing | | | | X | | |
| Adverse events | $X^j$ | $X^j$ | $X^j$ | X | X | X |
| Smoking Craving visual analog scale (SC-VAS) | | $X^k$ | | | $X^k$ | X |
| Aversion/Tolerability (AT) from the modified CES | | | | | $X^l$ | $X^l$ |
| Respiratory Tract sensation measurement from the Modified CES | | | | | $X^m$ | $X^m$ |
| Subjective effects, including pleasantness, liking and taste | | | | | $X^n$ | $X^n$ |
| Drug Accountability | | | | | X | X |

EP 3 698 832 B1

## FIG. 46

### Pre-Dosing:

| TIME | TBD | TBD | -1:00 |
|---|---|---|---|
| EVENT | Baseline PROs | Instructions & Practice inhalation | Baseline PK |

### Dosing:

| TIME | :00 | :30 | 1:00 | 1:30 | 2:00 | 2:30 | 3:00 | 3:30 | 4:00 | 4:30 |
|---|---|---|---|---|---|---|---|---|---|---|
| Inhalation # | 1 | 2 | 3 | 4 | 5 | 6 | 7 | 8 | 9 | 10 |
| PK | | | | | | | | | | |

### Post-Dosing:

| TIME (min) | 0.5 | 5:00 | 10:00 | 15:00 | 30:00 |
|---|---|---|---|---|---|
| PK | X | X | X | | |
| PROs | | X | | X | X |

FIG. 47

EP 3 698 832 B1

EP 3 698 832 B1

## FIG. 48

*FIG. 49*

FIG. 50

Each error bar is constructed using 1 standard error from the mean.

EP 3 698 832 B1

*FIG. 51*

Each error bar is constructed using 1 standard error from the mean.

FIG. 52

FIG. 53

**FIG. 54**

FIG. 55

## FIG. 56

EP 3 698 832 B1

FIG. 57

FIG. 58

FIG. 59

EP 3 698 832 B1

FIG. 60

## FIG. 61

## FIG. 62

EP 3 698 832 B1

FIG. 63

EP 3 698 832 B1

FIG. 64

EP 3 698 832 B1

EP 3 698 832 B1

**FIG. 65**

EP 3 698 832 B1

## FIG. 66

FIG. 67

FIG. 68

FIG. 69

FIG. 70

EP 3 698 832 B1

*FIG. 71*

EP 3 698 832 B1

FIG. 72

EP 3 698 832 B1

FIG. 72 (continued)

## Bivariate Fit of mCES_13 By Cough_count Group=5

Linear Fit

### Linear Fit

mCES_13 = 2.5242718 + 0.1359223*Cough_count

#### Summary of Fit

| | |
|---|---|
| RSquare | 0.041388 |
| RSquare Adj | -0.0545 |
| Root Mean Square Error | 2.099931 |
| Mean of Response | 3 |
| Observations (or Sum Wgts) | 12 |

#### Lack Of Fit

#### Analysis of Variance

| Source | DF | Sum of Squares | Mean Square | F Ratio |
|---|---|---|---|---|
| Model | 1 | 1.902913 | 1.90291 | 0.4315 |
| Error | 10 | 44.097087 | 4.40971 | Prob > F |
| C. Total | 11 | 46.000000 | | 0.5261 |

#### Parameter Estimates

| Term | Estimate | Std Error | t Ratio | Prob>|t| |
|---|---|---|---|---|
| Intercept | 2.5242718 | 0.944421 | 2.67 | 0.0234* |
| Cough_count | 0.1359223 | 0.208912 | 0.65 | 0.5261 |

## Bivariate Fit of mCES_13 By Cough_count Group=6

Linear Fit

### Linear Fit

mCES_13 = 3.1965909 - 0.1193182*Cough_count

#### Summary of Fit

| | |
|---|---|
| RSquare | 0.122823 |
| RSquare Adj | 0.013181 |
| Root Mean Square Error | 1.49559 |
| Mean of Response | 2.6 |
| Observations (or Sum Wgts) | 10 |

#### Lack Of Fit

#### Analysis of Variance

| Source | DF | Sum of Squares | Mean Square | F Ratio |
|---|---|---|---|---|
| Model | 1 | 2.505682 | 2.50568 | 1.1202 |
| Error | 8 | 17.894318 | 2.23679 | Prob > F |
| C. Total | 9 | 20.400000 | | 0.3208 |

#### Parameter Estimates

| Term | Estimate | Std Error | t Ratio | Prob>|t| |
|---|---|---|---|---|
| Intercept | 3.1965909 | 0.735802 | 4.34 | 0.0025* |
| Cough_count | -0.119318 | 0.112734 | -1.06 | 0.3208 |

EP 3 698 832 B1

## FIG. 73

Bivariate Fit of mCES_1 By Cough_count

Linear Fit

**Linear Fit**

mCES_1 = 3.2785834 - 0.0266748*Cough_count

**Summary of Fit**

| | |
|---|---|
| RSquare | 0.003878 |
| RSquare Adj | -0.01193 |
| Root Mean Square Error | 1.40577 |
| Mean of Response | 3.215385 |
| Observations (or Sum Wgts) | 65 |

**Lack Of Fit**

**Analysis of Variance**

| Source | DF | Sum of Squares | Mean Square | F Ratio |
|---|---|---|---|---|
| Model | 1 | 0.48466 | 0.48466 | 0.2453 |
| Error | 63 | 124.49995 | 1.97619 | Prob > F |
| C. Total | 64 | 124.98462 | | 0.6222 |

**Parameter Estimates**

| Term | Estimate | Std Error | t Ratio | Prob>|t| |
|---|---|---|---|---|
| Intercept | 3.2785834 | 0.216076 | 15.17 | <.0001* |
| Cough_count | -0.026675 | 0.053864 | -0.50 | 0.6222 |

Bivariate Fit of mCES_1 By Cough_count Group=6

Linear Fit

**Linear Fit**

mCES_1 = 4.6215909 - 0.2443182*Cough_count

**Summary of Fit**

| | |
|---|---|
| RSquare | 0.469004 |
| RSquare Adj | 0.402629 |
| Root Mean Square Error | 1.21934 |
| Mean of Response | 3.4 |
| Observations (or Sum Wgts) | 10 |

**Lack Of Fit**

**Analysis of Variance**

| Source | DF | Sum of Squares | Mean Square | F Ratio |
|---|---|---|---|---|
| Model | 1 | 10.505882 | 10.5057 | 7.0680 |
| Error | 8 | 11.894318 | 1.4868 | Prob > F |
| C. Total | 9 | 22.400000 | | 0.0289* |

**Parameter Estimates**

| Term | Estimate | Std Error | t Ratio | Prob>|t| |
|---|---|---|---|---|
| Intercept | 4.6215909 | 0.599892 | 7.70 | <.0001* |
| Cough_count | -0.244318 | 0.091911 | -2.66 | 0.0289* |

## FIG. 74

## FIG. 75

EP 3 698 832 B1

FIG. 76

EP 3 698 832 B1

FIG. 77

Legend: PBO, Vehicle, 250 mcg (2.5%), 500 mcg (5.0%), 500 mcg (2.5%), 750 mcg (3.25%), 1000 mcg (5.0%)

Y-axis: Nicotine (ng/mL)

X-axis: Baseline, 5-min, 10-min

FIG. 78

5 Minute Concentration

**FIG. 79**

PK_0 & PK_5 vs. Group

## FIG. 80

Schedule of Assessments for the Second Portion of the eNT-101 Study.

| Procedures for All Cohorts | Screening (Day -14-Day -1) | Pre-dose (Baseline) | Practice | Dosing #1-10 | Post-Dosing | Early Termination |
|---|---|---|---|---|---|---|
| Informed consent | X | | | | | |
| Inclusion/exclusion criteria | X | X[a] | | | | |
| Physical examination including BMI | X | | | | | |
| Medical and psychiatric history | X | | | | | |
| Smoking history | X | | | | | |
| Fagerström Nicotine Dependence Score | X | | | | | |
| Intention to quit smoking in next 3-months | X | | | | | |
| Exhaled CO level | X[b] | X[c] | | | X | X |
| Pulmonary function tests (spirometry) | | X[d] | | | X[d] | X[d] |
| Vital signs, including height[e], weight[f], heart rate, respirations, blood pressure (supine and standing) | X | X | | | X | X |
| ECG | X | | | | X | X |
| Pregnancy test | X[g] | X[h] | | | | |
| Urine sample for cotinine and drugs of abuse | X | X | | | | |
| Blood sample for chemistry, hematology and urinalysis | X | | | | X | X |
| Concomitant medication and consumption review | X | X[i] | | | | |
| Drug Administration or Dosing | | | | X | | |
| Adverse events | X[j] | X[j] | X[j] | X | X | X |
| Pharmacokinetic Venous Nicotine and Cotinine blood sample | | X[k] | | | X[k] | |
| Smoking Craving visual analog scale (SC-VAS) | | X[l] | | | X[l] | X |
| Aversion/Tolerability (AT) from the modified CES | | | | | X[m] | X[m] |
| Respiratory Tract sensation measurement from the Modified CES | | | | | X[n] | X[n] |
| Subjective effects, including pleasantness, liking and taste | | | | | X[o] | X[o] |
| Drug Accountability | | | | | X | X |

EP 3 698 832 B1

# FIG. 81

**Pre-Dosing:**

| TIME | TBD | TBD | -1:00 |
|---|---|---|---|
| EVENT | Baseline PROs | Instructions & Practice inhalation | Baseline PK |

**Dosing:**

| TIME | :00 | :30 | 1:00 | 1:30 | 2:00 | 2:30 | 3:00 | 3:30 | 4:00 | 4:30 |
|---|---|---|---|---|---|---|---|---|---|---|
| Inhalation # | 1 | 2 | 3 | 4 | 5 | 6 | 7 | 8 | 9 | 10 |
| PK | | | | | | X | | | | |
| PROs | | | | | | X* | | | | |

**Post-Dosing:**

| TIME | 5:00 | 10:00 | 15:00 | 20:00 | 25:00 | 30:00 | 60:00 |
|---|---|---|---|---|---|---|---|
| PK | X | X | X | X | X | X | X |
| PROs | X | | X | | | X | X |

EP 3 698 832 B1

*FIG. 82*

*FIG. 83A*

1.25 in

2.75 in

1.8 in

8302

8304

8306

FIG. 83B

FIG. 84A

FIG. 84B

*FIG. 85*

EP 3 698 832 B1

# FIG. 86

2.1 in

0.75 in

8602

EP 3 698 832 B1

EP 3 698 832 B1

## FIG. 87

FIG. 88

FIG. 89

EP 3 698 832 B1

## FIG. 90A

9002

*FIG. 90B*

9004

9006

9008

.280

E

E

D

D

SECTION C-C
SCALE 4 : 1

FIG. 90C

SECTION B-B
SCALE 4 : 1

9012

9014

9002

9010

.394

.280

9012

R.044

9014

9016

R.094

.157

SECTION D-D
SCALE 4 : 1

R.084

SECTION E-E
SCALE 4 : 1

FIG. 90D

FIG. 91

EP 3 698 832 B1

FIG. 92

FIG. 93

FIG. 93

9400

*FIG. 94A*

9400

*FIG. 94B*

9402

9404

9406

9408

9410

204

FIG. 94C

FIG. 95A

FIG. 95B

FIG. 95C

FIG. 96

FIG. 97

# FIG. 98

Legend:
- □———□ Placebo Control-Study Product A
- O----O Vehicle Control –Study Product B
- ◇----◇ 250 µg Nicotine as a 2.5% Solution-Study Product C
- △----△ 500 µg Nicotine as a 5.0% Solution-Study Product D
- *———* 500 µg Nicotine as a 2.5% Solution-Study Product E
- ●———● 750 µg Nicotine as a 3.75% Solution-Study Product F
- #———# 1000 µg Nicotine as a 5% Solution-Study Product G

Y-axis: Plasma Nicotine Concentration (ng/mL)

X-axis: Hours from Product Use

## FIG. 99

Legend:
- ● ——— ● Vehicle Control (Part 2)
- ○ - - - ○ 500 µg Nicotine as a 2.5% Solution (Part 2)
- ◇ - - - ◇ 1000 µg Nicotine as a 5.0% Solution (Part 2)
- △ - - - △ NJOY King Bold e-Cigarette (Part 2)
- * ——— * Usual Brand Combustible Cigarette (Part 2)

Y-axis: Change from Baseline Smoking Urge (VAS)

X-axis: Hours Post Product Use

# FIG. 100

| Smoking Urge Response Statistical Comparisons Excluding Device Failures | | | | | | | | |
|---|---|---|---|---|---|---|---|---|
| | $E_{max\ reduction}$ (%) | | | | $AUEC_{(0-4)}$ (%*hr) | | | |
| Comparison | First Product | Second Product | Difference | P-value | First Product | Second Product | Difference | P-value |
| Product A versus Product B | -28.267 (6.379) | -45.267 (6.379) | 17.000 (7.834) | 0.0350 | -16.179 (6.044) | -32.175 (6.044) | 15.996 (8.013) | 0.0516 |
| Product A versus Product C | -28.267 (6.379) | -47.495 (7.614) | 19.228 (8.889) | 0.0361 | -16.179 (6.044) | -38.292 (7.889) | 22.113 (9.072) | 0.0185 |
| Product A versus Product D | -28.267 (6.379) | -50.333 (6.379) | 22.067 (7.834) | 0.0070 | -16.179 (6.044) | -38.379 (6.044) | 20.201 (8.013) | 0.0151 |
| Product A versus Product E | -28.267 (6.379) | -64.200 (6.379) | 35.933 (7.834) | <.0001 | -16.179 (6.044) | -48.830 (6.044) | 32.651 (8.013) | 0.0002 |
| Product B versus Product C | -45.267 (6.379) | -47.495 (7.614) | 2.228 (8.889) | 0.8027 | -32.175 (6.044) | -38.292 (7.889) | 6.117 (9.072) | 0.5034 |
| Product B versus Product D | -45.267 (6.379) | -50.333 (6.379) | 5.067 (7.834) | 0.5209 | -32.175 (6.044) | -36.379 (6.044) | 4.204 (8.013) | 0.6022 |
| Product B versus Product E | -45.267 (6.379) | -64.200 (6.379) | 18.933 (7.834) | 0.0195 | -32.175 (6.044) | -48.830 (6.044) | 16.655 (8.013) | 0.0430 |
| Product C versus Product D | -47.495 (7.614) | -50.333 (6.379) | 2.839 (8.889) | 0.7503 | -38.292 (7.889) | -36.379 (6.044) | -1.912 (9.072) | 0.8339 |
| Product C versus Product E | -47.495 (6.379) | -64.200 (6.379) | 16.705 (8.889) | 0.0656 | -38.292 (7.889) | -48.830 (6.044) | 10.539 (9.072) | 0.2511 |
| Product D versus Product E | -50.333 (6.379) | -64.200 (6.379) | 13.867 (7.834) | 0.0831 | -36.379 (6.044) | -48.830 (6.044) | 12.451 (8.013) | 0.1208 |

Values presented as LSMeans (SE).
LSMean values for each product were significantly different from 0 (<0.05).

Study Product A = eNT-100 Nicotine Inhaler Vehicle Control (0% nicotine solution in PG vehicle)
Study Product B = eNT-100 Nicotine Inhaler 500 µg (2.5% nicotine solution in PG vehicle)
Study Product C = eNT-100 Nicotine Inhaler 1000 µg (5% nicotine solution in PG vehicle)
Study Product D = NJOY King Bold e-cig (4.5% nicotine solution in in a combination of PG and glycerin)
Study Product E = Combustible Cigarette (Subject's usual brand product)

## FIG. 101

EP 3 698 832 B1

FIG. 102

EP 3 698 832 B1

FIG. 103

**FIG. 104**

FIG. 105

EP 3 698 832 B1

## FIG. 106

EP 3 698 832 B1

## FIG. 107

EP 3 698 832 B1

## FIG. 108

FIG. 109

EP 3 698 832 B1

# FIG. 110

EP 3 698 832 B1

## FIG. 111

FIG. 112

EP 3 698 832 B1

**FIG. 113**

FIG. 114

EP 3 698 832 B1

## FIG. 115

**Legend:**
- □——□ Vehicle Control-Study Product A
- O----O 500 μg Nicotine as a 2.5% Solution-Study Product B
- ◇——◇ 1000 μg Nicotine as a 5.0% Solution-Study Product C
- △----△ NJOY King "Bold" e-Cigarette-Study Product D
- *——* Usual Brand Combustible Cigarette-Study Product E

**Y-axis:** Baseline-Adjusted Plasma Nicotine Concentration (ng/mL)

**X-axis:** Hours from Product Use

Study Products B, C, D and E are shifted to the right for ease of reading
Subjects 203, 205, 207, and 213 for treatment C were excluded because of device failure

EP 3 698 832 B1

## FIG. 116

### Summary of Baseline-Adjusted Plasma Nicotine Pharmacokinetic Parameters

| Study Product | | AUC$_{(0-t)}$ (ng*hr/mL) | C$_{max}$ (ng/mL) | C5 (ng/mL) | T$_{max}$ (hr) |
|---|---|---|---|---|---|
| | N | 15 | 15 | 15 | 15 |
| | G.mean | 1.199 | 1.594 | 0.6276 | NC |
| Product B | Mean | 1.248 | 1.695 | 0.9132 | 0.3165 |
| | SD | 0.34544 | 0.58696 | 0.48724 | 0.10091 |
| | Range | (0.580,1.77) | (0.670,2.63) | (0.462,2.36) | (0.165,0.499) |
| | Median | 1.183 | 1.460 | 0.7400 | 0.3322 |
| | N | 12 | 12 | 12 | 12 |
| | G.mean | 2.140 | 3.047 | 1.632 | NC |
| Product C* | Mean | 2.516 | 3.429 | 1.869 | 0.3847 |
| | SD | 1.0143 | 1.3067 | 0.86053 | 0.081850 |
| | Range | (0.215,4.46) | (0.511,5.99) | (0.358,3.43) | (0.166,0.416) |
| | Median | 2.624 | 3.525 | 1.870 | 0.3289 |
| | N | 11 | 11 | 11 | 11 |
| | G.mean | 2.637 | 3.584 | 1.873 | NC |
| Product C** | Mean | 2.725 | 3.694 | 2.006 | 0.2947 |
| | SD | 0.74459 | 0.97435 | 0.77711 | 0.077780 |
| | Range | (1.68,4.46) | (2.32,5.99) | (1.01,3.43) | (0.166,0.416) |
| | Median | 2.633 | 3.530 | 1.890 | 0.3317 |
| | N | 15 | 15 | 15 | 15 |
| | G.mean | 1.895 | 2.692 | 1.314 | NC |
| Product D | Mean | 2.437 | 3.784 | 1.987 | 0.2296 |
| | SD | 1.6779 | 3.3281 | 2.0930 | 0.096363 |
| | Range | (0.514,5.35) | (0.649,12.7) | (0.302,8.16) | (0.0411,0.416) |
| | Median | 2.242 | 3.020 | 1.290 | 0.2497 |
| | N | 15 | 15 | 15 | 15 |
| | G.mean | 11.94 | 16.79 | 13.83 | NC |
| Product E | Mean | 12.52 | 17.97 | 14.53 | 0.2259 |
| | SD | 4.6943 | 6.7845 | 5.6387 | 0.13280 |
| | Range | (7.60,24.4) | (10.9,29.6) | (8.13,28.8) | (0.0817,0.503) |
| | Median | 10.92 | 15.80 | 13.90 | 0.1667 |

Study Product B = eNT-100 Nicotine Inhaler 500 µg (2.5% nicotine solution in PG vehicle)
Study Product C = eNT-100 Nicotine Inhaler 1000 µg (5% nicotine solution in PG vehicle)
Study Product D = NJOY King Bold e-cig (4.5% nicotine solution in in a combination of PG and glycerin)
Study Product E = Combustible Cigarette (Subject's usual brand product)
*For Product C, there was a device failure in 4 subjects, 3 of which had no quantifiable nicotine plasma concentrations. Therefore, PK parameters could only be calculated for N = 12.
**Following exclusion of all subjects with a device failure, N = 11.
Range = (Minimum, Maximum)
NC = Not calculated

## FIG. 117

**Summary of the Statistical Comparison of Pharmacokinetic Parameters Excluding Device Failures**

| Comparison | Parameter | Test | | Reference | | Test – Reference | |
|---|---|---|---|---|---|---|---|
| | | LSMean (SE) | P-value | LSMean (SE) | P-value | Difference (SE) | P-value |
| Product B versus Product C | AUC$_{(0-t)}$ (ng·hr/mL) | 1.248 (0.680) | 0.0734 | 2.829 (0.882) | 0.0020 | -1.580 (1.084) | 0.1539 |
| | C5 (ng/mL) | 0.913 (0.797) | 0.2581 | 2.072 (1.013) | 0.0467 | -1.159 (1.289) | 0.3734 |
| | C$_{max}$ (ng/mL) | 1.695 (1.031) | 0.1073 | 3.726 (1.310) | 0.0067 | -2.030 (1.667) | 0.2208 |
| Product B versus Product D | AUC$_{(0-t)}$ (ng·hr/mL) | 1.248 (0.680) | 0.0734 | 2.437 (0.680) | 0.0009 | -1.189 (0.946) | 0.2173 |
| | C5 (ng/mL) | 0.913 (0.797) | 0.2581 | 1.987 (0.797) | 0.0165 | -1.073 (1.127) | 0.3461 |
| | C$_{max}$ (ng/mL) | 1.695 (1.031) | 0.1073 | 3.784 (1.031) | 0.0007 | -2.089 (1.458) | 0.1591 |
| Product C versus Product D | AUC$_{(0-t)}$ (ng·hr/mL) | 2.829 (0.862) | 0.0020 | 2.437 (0.680) | 0.0009 | 0.391 (1.084) | 0.7205 |
| | C5 (ng/mL) | 2.072 (1.013) | 0.0467 | 1.987 (0.797) | 0.0165 | 0.085 (1.289) | 0.9474 |
| | C$_{max}$ (ng/mL) | 3.726 (1.310) | 0.0067 | 3.784 (1.031) | 0.0007 | -0.058 (1.667) | 0.9722 |
| Product B versus Product E | AUC$_{(0-t)}$ (ng·hr/mL) | 1.248 (0.680) | 0.0734 | 12.520 (0.680) | <.0001 | -11.272 (0.946) | <.0001 |
| | C5 (ng/mL) | 0.913 (0.797) | 0.2581 | 14.530 (0.797) | <.0001 | -13.817 (1.127) | <.0001 |
| | C$_{max}$ (ng/mL) | 1.695 (1.031) | 0.1073 | 17.869 (1.031) | <.0001 | -16.174 (1.458) | <.0001 |
| Product C versus Product E | AUC$_{(0-t)}$ (ng·hr/mL) | 2.829 (0.862) | 0.0020 | 12.520 (0.680) | <.0001 | -9.691 (1.084) | <.0001 |
| | C5 (ng/mL) | 2.072 (1.013) | 0.0467 | 14.530 (0.797) | <.0001 | -12.458 (1.289) | <.0001 |
| | C$_{max}$ (ng/mL) | 3.726 (1.310) | 0.0067 | 17.869 (1.031) | <.0001 | -14.143 (1.667) | <.0001 |
| Product D versus Product E | AUC$_{(0-t)}$ (ng·hr/mL) | 2.437 (0.680) | 0.0009 | 12.520 (0.680) | <.0001 | -10.082 (0.946) | <.0001 |
| | C5 (ng/mL) | 1.987 (0.797) | 0.0165 | 14.530 (0.797) | <.0001 | -12.544 (1.127) | <.0001 |
| | C$_{max}$ (ng/mL) | 3.784 (1.031) | 0.0007 | 17.869 (1.031) | <.0001 | -14.085 (1.458) | <.0001 |

Study Product B = eNT-100 Nicotine Inhaler 500 µg (2.5% nicotine solution in PG vehicle)
Study Product C = eNT-100 Nicotine Inhaler 1000 µg (5% nicotine solution in PG vehicle)
Study Product D = NJOY King Bold e-cig (4.5% nicotine solution in in a combination of PG and glycerin)
Study Product E = Combustible Cigarette (Subject's usual brand product)

## FIG. 118

EP 3 698 832 B1

*FIG. 119*

EP 3 698 832 B1

## FIG. 120

## Cough %

FIG. 121

EP 3 698 832 B1

## FIG. 122

FIG. 123

EP 3 698 832 B1

## FIG. 124

## FIG. 125

EP 3 698 832 B1

FIG. 125 (continued)

## FIG. 126

EP 3 698 832 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5894841 A
- EP 0845220 B1
- WO 2013083635 A1
- US 5060671 A
- US 5093894 A
- US 5224498 A
- US 5228460 A
- US 5322075 A
- US 5353813 A
- US 5468936 A
- US 5498850 A
- US 5659656 A
- US 5498855 A
- US 5530225 A
- US 5665262 A
- US 5573692 A
- US 5591368 A
- US 20130255702 A1, Griffith
- US 20140060554 A1
- US 20050016550 A1
- US 20110094523 A1
- US 20120199146 A1
- US 20120111347 A1
- US 20120279512 A1
- US 20110209717 A1
- US 20130125906 A1
- US 7832410 B
- US 8156944 B
- US 8393331 B
- US 8375957 B
- US 20110005535 A1
- US 20120145169 A1
- US 8511318 B
- US 20060196518 A1
- US 20120090630 A1
- US 20130157995
- US 20090234129
- US 20080108822
- US 20070186940
- US 20080227088
- US 4243605 A
- US 5015741 A
- US 6503922 B
- US 6995265 B
- US 7132545 B